(19) Europäisches Patentamt
European Patent Office
Office européen des brevets

(11) EP 2 258 303 B1

(12) **EUROPEAN PATENT SPECIFICATION**

(45) Date of publication and mention of the grant of the patent:
**18.09.2013 Bulletin 2013/38**

(51) Int Cl.:
***A61C 9/00*** *(2006.01)*

(21) Application number: **10009822.7**

(22) Date of filing: **13.04.2001**

# (54) System for creating an individual three-dimensional virtual tooth model

System zur Erstellung eines individuellen virtuellen dreidimensionalen Modells eines Zahnes

Système pour créer un modèle individuel virtuel tridimensionnel d'une dent

(84) Designated Contracting States:
**AT BE CH CY DE DK ES FI FR GB GR IE IT LI LU MC NL PT SE TR**

(30) Priority:
**19.04.2000 US 552189**
**19.04.2000 US 552190**
**28.04.2000 US 560127**
**28.04.2000 US 560128**
**28.04.2000 US 560129**
**28.04.2000 US 560130**
**28.04.2000 US 560131**
**28.04.2000 US 560132**
**28.04.2000 US 560133**
**28.04.2000 US 560134**
**28.04.2000 US 560583**
**28.04.2000 US 560584**
**28.04.2000 US 560640**
**28.04.2000 US 560641**
**28.04.2000 US 560642**
**28.04.2000 US 560643**
**28.04.2000 US 560644**
**28.04.2000 US 560645**
**28.04.2000 US 560646**
**28.04.2000 US 560647**
**13.07.2000 US 616093**

(43) Date of publication of application:
**08.12.2010 Bulletin 2010/49**

(62) Document number(s) of the earlier application(s) in accordance with Art. 76 EPC:
**01928490.0 / 1 301 140**

(73) Proprietor: **OraMetrix, Inc.**
**Richardson, TX 75082-4434 (US)**

(72) Inventors:
- **Rubbert, Rüdger**
  **12101 Berlin (DE)**
- **Weise, Thomas**
  **36284 Hohenroda (DE)**
- **Sporbert, Peer**
  **10243 Berlin (DE)**
- **Imgrund, Hans**
  **10551 Berlin (DE)**
- **Leichner, Mario**
  **16540 Hohen Neuendorf (DE)**
- **Sachdeva, Rohit**
  **Plano, Texas 75093 (US)**

(74) Representative: **Stevens, Jason Paul**
**Dehns**
**St Bride's House**
**10 Salisbury Square**
**London**
**EC4Y 8JD (GB)**

(56) References cited:
**WO-A1-00/19929** **WO-A1-00/19935**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 258 303 B1

## Description

BACKGROUND OF THE INVENTION

A. Field of the Invention

This invention relates generally to the field of orthodontics. More particularly, the invention relates to a computerized, interactive method and associated system for orthodontic treatment. The system includes a hand-held optical scanner capturing 3-dimensional information of objects, and interactive computer-based treatment planning using three-dimensional tooth objects and user specified simulation of tooth movement.

B. Description of Related Art

In orthodontics, a patient suffering from a malocclusion is typically treated by bonding brackets to the surface of the patient's teeth. The brackets have slots for receiving an archwire. The bracket-archwire interaction governs forces applied to the teeth and defines the desired, direction of tooth movement. Typically, the bends in the wire are made manually by the orthodontist. During the course of treatment, the movement of the teeth is monitored. Corrections to the bracket position and/or wire shape are made manually by the orthodontist.

The key to efficiency in treatment and maximum quality in results is a realistic simulation of the treatment process. Today's orthodontists have the possibility of taking plaster models of the upper and lower jaw, cutting the model into single tooth models and sticking these tooth models into a wax bed, lining them up in the desired position, the so-called set-up. This approach allows for reaching a perfect occlusion without any guessing. The next step is to bond a bracket at every tooth model. This would tell the orthodontist the geometry of the wire to run through the bracket slots to receive exactly this result. The next step involves the transfer of the bracket position to the original malocclusion model. To make sure that the brackets will be bonded at exactly this position at the real patient's teeth, small templates for every tooth would have to be fabricated that fit over the bracket and a relevant part of the tooth and allow for reliable placement of the bracket on the patient's teeth. To increase efficiency of the bonding process, another option would be to place each single bracket onto a model of the malocclusion and then fabricate one single transfer tray per jaw that covers all brackets and relevant portions of every tooth. Using such a transfer tray guarantees a very quick and yet precise bonding using indirect bonding.

However, it is obvious that such an approach requires an extreme amount of time and labour and thus is too costly, and this is the reason why it is not practiced widely. The normal orthodontist does not fabricate set- ups; he places the brackets directly on the patient's teeth to the best of his knowledge, uses an off- the- shelf wire and hopes for the best. There is no way to confirm whether the brackets are placed correctly; and misplacement of the bracket will change the direction and/or magnitude of the forces imparted on the teeth. While at the beginning of treatment things generally run well as all teeth start to move at least into the right direction, at the end of treatment a lot of time is lost by adaptations and corrections required due to the fact that the end result has not been properly planned at any point of time. For the orthodontist this is still preferable over the lab process described above, as the efforts for the lab process would still exceed the efforts that he has to put in during treatment. And the patient has no choice and does not know that treatment time could be significantly reduced if proper planning was done.

U.S. Patent 5,431,562 to Andreiko et al. describes a computerized, appliance-driven approach to orthodontics. In this method, first certain shape information of teeth is acquired. A uniplanar target archform is calculated from the shape information. The shape of customized bracket slots, the bracket base, and the shape of an orthodontic archwire, are calculated in accordance with a mathematically-derived target archform. The goal of the. Andreiko et al. method is to give more predictability, standardization, and certainty to orthodontics by replacing the human element in orthodontic appliance design with a deterministic, mathematical computation of a target archform and appliance design. Hence the '562 patent teaches away from an interactive, computer-based system in which the orthodontist remains fully involved in patient diagnosis, appliance design, and treatment planning and monitoring.

More recently, in the late 1990's Align Technologies began offering transparent, removable aligning devices as a new treatment modality in orthodontics. In this system, a plaster model of the dentition of the patent is obtained by the orthodontist and shipped to a remote appliance manufacturing centre, where it is scanned with a laser. A computer model of the dentition in a target situation is generated at the appliance manufacturing centre and made available for viewing to the orthodontist over the Internet. The orthodontist indicates changes they wish to make to individual tooth positions. Later, another virtual model is provided over the Internet and the orthodontist reviews the revised model, and indicates any further changes. After several such iterations, the target situation is agreed upon. A series of removable aligning devices or shells are manufactured and delivered to the orthodontist. The shells, in theory, will move the patient's teeth to the desired or target position.

The art has lacked an effective, computer-based interactive orthodontic treatment planning system that provides the necessary tools to allow the orthodontist to quickly and efficiently design a treatment plan for a patient. The art has also lacked a treatment planning system in which the orthodontist-derived parameters for the treatment can be translated into a design of an orthodontic appliance in real time, while the patient is in the chair. Real-time appliance design as described herein also allows for real-time communication of the treatment plan or appliance design to occur with the patient, or transmitted over a communications link and shared with a colleague or remote appliance manufacturing facility. Alternatively, the treatment planning can be performed remotely and a digital treatment plan sent to the orthodontist for review, interactive modification, or approval.

Scanners are devices for capturing and recording information from a surface of an object. Scanners for obtaining information from a two-dimensional surface, such as reading bar codes or characters printed on a piece of paper, are widely known. Several scanners have been proposed for recording three-dimensional information as well, including the field of dentistry.

U.S. Patent 4,837,732 and U.S. Patent 4,575,805 to Brandestini and Moermann propose a scanning system for *in vivo,* non-contact scanning of teeth. The patents describe a procedure for optically mapping a prepared tooth with a non-contact scan-head. The scan-head delivers the contour data, converted to electrical format, to be stored in a memory. A computer reads the memory following a line scan pattern. A milling device is slaved to follow this pattern by means of position control signals and mills an implant for the prepared tooth cavity.

The scan-head of the ’732 and ’805 patents includes a light emitting diode, with integral lens that radiates light onto the cavity. Before reaching the object, the rays of light are reflected by a mirror and pass through a ruling consisting of a plurality of parallel slits, or an alternating pattern of parallel opaque and transparent stripes. The reflected light is focused by a lens onto a charge-coupled device (CCD) sensor. Depth information is determined in accordance with a principle known as "active triangulation", using parameters shown in Figure 9 of this document and described subsequently. Basically, the object is viewed under an angle different from the incident rays due to a parallax effect. Each light stripe will have an apparent positional shift and the amount of the shift at each point along each light stripe is proportional to the vertical height of the corresponding portion of the surface on the object.

U.S. Patent No. 5,372,502 to Massen et al. describes an optical probe for measuring teeth that works on a similar principle. As noted in the Massen et al. patent, the Brandestini et al. technique is difficult to use when there are large variations in surface topography since such large jumps displace the pattern by an amount larger than the phase constant of the pattern, making it difficult to reconstruct the pattern of lines. Furthermore, precise knowledge of the angle of incidence and angle of reflection, and the separation distance between the light source and the detector, are needed to make accurate determinations of depth. Furthermore, the scanner has to be rather carefully positioned with respect to the tooth, and would be unable to make a complete model of the dentition.

U.S. Patent No. 5,027,281 to Rekow et al. describes a scanning method using a three axis positioning head with a laser source and detector, a rotational stage and a computer controller. The computer controller positions both the rotational stage and the positioning head. An object is placed on the rotational stage and the laser beam reflects from it. The reflected laser beam is used to measure the distance between the object and the laser source. X and Y coordinates are obtained by movement of the rotational stage or the positioning head. A three-dimensional virtual model of the object is created from the laser scanning. The ’281 patent describes using this scanning method for scanning a plaster model of teeth for purposes of acquiring shape of the teeth to form a dental prosthesis. The system of the ’281 patent is not particularly flexible, since it requires the object to be placed on the rotational stage and precise control of the relative position of the object and the positioning head is required at all times. It is unsuited for *in vivo* scanning of the teeth.

U.S. Patent 5,431,562 to Andreiko et al. describes a method of acquiring certain shape information of teeth from a plaster model of the teeth. The plaster model is placed on a table and a picture is taken of the teeth using a video camera positioned a known distance away from the model, looking directly down on the model. The image is displayed on an input computer and a positioning grid is placed over the image of the teeth. The operator manually inputs X and Y coordinate information of selected points on the teeth, such as the mesial and distal contact points of the teeth. An alternative embodiment is described in which a laser directs a laser beam onto a model of the teeth and the reflected beam is detected by a sensor. The patent asserts that three-dimensional information as to teeth can be acquired from this technique but does not explain how it would be done. Neither of the techniques of Andreiko have met with widespread commercial success or acceptance in orthodontics. Neither technique achieves *in vivo* scanning of teeth. Moreover, the video technique does not produce complete three-dimensional information as to the teeth, but rather a limited amount of two-dimensional information, requiring significant manual operator input. Even using this technique, additional equipment is required even to describe the labial surface of a tooth along a single plane.

A digital dentition model and a process for manipulating the model is disclosed in WO 00/19935 to Align Technology Inc. WO 00/19929 (also to Align Technology Inc) is concerned with computer automated development of an orthodontic treatment plan, In which a computer receives data sets representing a patient’s teeth in their original and final (posttreatment) positions. The computer then uses the data sets to generate treatment paths along which the teeth will move. However, there is no explanation in this document of using virtual three-dimensional template objects corresponding to individual teeth to plan treatment.

SUMMARY OF THE INVENTION

The invention provides a system for creating an individual, three-dimensional virtual tooth model representing a tooth found in a dentition assigned to a patient, comprising: a memory storing at least one virtual three-dimensional model of a template object corresponding to a tooth, including a template tooth corresponding to said tooth; a data processing system including a memory storing a virtual three-dimensional model of at least a part of the dentition; wherein said data processing system further comprises software processing said virtual three-dimensional model of at least a part of the dentition and said virtual model of the template object and responsively deriving said individual, three-dimensional, virtual tooth model; wherein said software comprises (1) a routine superimposing said virtual template object corresponding to a tooth on said virtual model of at least a part of the dentition; (2) a routine drawing vectors from said virtual template object to a surface of said virtual model of the dentition and storing a set of points where said vectors intersect said surface; (3) a routine forming a new surface comprised of said set of points; and (4) a routine filling missing parts of said surface, if any, from said template tooth.

In a preferred implementation, an interactive, orthodontic care system is provided based on scanning of teeth. The system includes a hand-held scanner and associated processing system for capturing images of the dentition of the patient and processing the images to generate a full, virtual, three-dimensional model of the dentition. A data conditioning system processes the virtual, three-dimensional model and responsively generates a set of individual, virtual three-dimensional tooth objects representing teeth in the dentition of the patient. A workstation is provided having a user interface for display of the set of individual, virtual three-dimensional tooth objects. Interactive treatment planning software is provided on the workstation permitting a user to manipulate the virtual three-dimensional tooth objects to thereby design a target situation for the patient in three dimensions and parameters for a customized orthodontic appliance for the teeth.

The hand-held scanner and associated processing system, data conditioning system, and workstation may all be installed in an orthodontic clinic. Alternatively, the hand-held scanner and workstation are installed in an orthodontic clinic, and wherein the data conditioning system is installed in a general purpose computer at a remote location from orthodontic clinic. Further, the treatment planning software can be either installed at the clinic, and/or at a remote location, and/or at a precision appliance manufacturing centre that manufactures a custom orthodontic appliance. The type of appliance may vary considerably.

The treatment planning apparatus can be considered an interactive, computer-based computer aided design and computer aided manufacturing (CAD/CAM) system for orthodontics. The apparatus is highly interactive, in that it provides the orthodontist with the opportunity to both observe and analyze the current stage of the patient's condition and develop and specify a target or desired stage. A shortest direct path of tooth movement to the target stage can also be determined. Further, the apparatus provides for simulation of tooth movement between current and target stages.

In its broader aspects, the apparatus comprises a workstation having a processing unit and a display, and a memory storing a virtual, complete three-dimensional model representing the dentition of a patient. The virtual three-dimensional model can be obtained from one of several possible sources; in the preferred embodiment it is arrived at from a scanning of the dentition. The apparatus further includes software executable by the processing unit that accesses the model and displays the model on the display of the workstation. The software further includes navigation tools, e. g., typed commands, icons and/or graphical devices superimposed on the displayed model, that enables a user to manipulate the model on the display and simulate the movement of at least one tooth in the model relative to other teeth in the model in three-dimensional space, and quantify the amount of movement precisely. This simulation can be used, for example, to design a particular target situation for the patient.

The development of a unique target situation for the patient has utility in a variety of different orthodontic appliances, including an approach based on off-the-shelf or generic brackets and a custom orthodontic archwire. The scope of the invention is sufficient to encompass other types of appliances, such as an approach based on customized brackets, retainers, or removable aligning devices. In a bracket embodiment, the memory contains a library of virtual, three-dimensional orthodontic brackets. The software permits a user to access the virtual brackets through a suitable screen display, and place the virtual brackets on the virtual model of the dentition of the patient. This bracket bonding position can be customized on a tooth by tooth basis to suit individual patient anatomy. Because the tooth models, brackets and archwire are individual objects, and stored as such in memory, the treatment planning apparatus can simultaneously display the virtual brackets, the archwire and the virtual model of the dentition, or some lesser combination, such as just the brackets, just the dentition, or the brackets and the archwire but not the teeth. The same holds true with other appliance systems.

In a preferred embodiment, the virtual model of teeth comprises a set of virtual, individual three-dimensional tooth objects. A method of obtaining the tooth objects from a scan of teeth, and obtaining other virtual objects of associated anatomical structures, e. g., gums, roots and bone is described. When the teeth are separated from each other and from the gums, they can be individually manipulated. Thus, the individual tooth objects can be individually selected and moved relative to other teeth in the set of virtual tooth objects. This feature permits individual, customized tooth positioning on a tooth by tooth basis. These positioning can be in terms or angular rotation about three axis, or translation in transverse, sagittal or coronal planes. Additionally, various measurement features are provided for quantifying the amount of movement.

One of the primary tools in the treatment planning apparatus is the selection and customization or a desired or target archform. Again, because the teeth are individual tooth objects, they can be moved independently of each other to define an ideal arch. This development of the target archform could be calculated using interpolation or cubic spline algorithms. Alternatively, it can be customized by the user specifying a type of archform (e. g, Roth), and the tooth are moved onto that archform or some modification of that archform. The archform can be shaped to meet the anatomical constraints of the patient. After the initial archform is designed, the user can again position the teeth on the archform as they deem appropriate on a tooth by tooth basis. The treatment planning software thus enables the movement of the virtual tooth objects onto an archform which may represent, at least in part, a proposed treatment objective for the patient.

Numerous other features are possible with the treatment planning software, including movement of the teeth with respect to the other teeth in the archform, changing the position of the virtual brackets and the teeth with respect to each other, or opposing teeth with respect to the selected archform. Custom archwire bends can be simulated to provide additional corrections. Bonding corrections at the bracket-tooth interface are also possible.

In another aspect, a method of digital treatment planning for an orthodontic patient on a workstation having a processing unit, a user interface including a display and software executable by the processing unit comprises the steps of obtaining and storing a three-dimensional virtual model of teeth representing the dentition of the patient in a current or observed situation. The virtual model is displayed on the display. The method further includes the step of moving the position of teeth in the virtual model relative to each other so as to place the teeth of the virtual model into a target situation and displaying the virtual model with the teeth moved to the target situation to the user. Parameters for an orthodontic appliance to move the patient's teeth from the current situation to the target situation can be derived from the virtual model and the target situation. For example, if virtual brackets are placed on the teeth, their location in the target situation can dictate the design of an archwire to move the teeth to the target situation.

The scanner system is provided for capturing three-dimensional information of an object. The object can be virtually any object under scrutiny, however the present document will describe an application in which the object is the dentition of a patient suffering from a malocclusion.

The scanning system enables three-dimensional surface information to be obtained with a very high decree of precision. Moreover, the scanning system can be used without requiring precise movement of the scanner, or requiring the object under scrutiny to be fixed in space. Surprisingly, the scanner is able to generate precise three dimensional surface information by simply moving the scanner over the surface of the object, such as by hand, in any manner that is convenient for the user, even if the object moves in any random direction during the scanning within reasonable limits. Thus, the scanner can be used to capture the surface of a patient's dentition in a minute or two, even if the patient moves their head or jaw while the scanning is occurring. Precise knowledge of the spatial relationship between the scanner and the object is not required.

The scanner obtains a set of images, which are processed in a computer to calculate the surface configuration of the object in three dimensions of space automatically, quickly, with high precision, and with essentially no human involvement other than the act of scanning. The precision or accuracy will be dictated largely by the extent to which the object under scrutiny tends to have undercut or shadowed features which are difficult to detect, necessitating a narrow angle between the projection and imaging axes. For teeth, an accuracy of under 20 or 30 microns is possible. This accuracy can be further improved depending on the nature of the surface, such as if the surface does not have a lot of undercut or shadowed features, by increasing the angular separation of the projection axis and the imaging axis.

Each image captured by the scanner is converted to a virtual, three-dimensional point cloud or "frame". The illustrated embodiment has a relatively coarse resolution for any single frame, due to a coarse projection pattern, but a fine resolution is obtained by obtaining multiple images and performing a registration procedure on the frames, as described below. Since each point on the surface of the object is captured in a plurality of images (such as five or six in a typical example of scanning), the registration of frames results in a fine resolution. An even finer resolution can be obtained by scanning slower and capturing more images of the surface of the object from different perspectives and registering the resulting frames to each other.

This surface configuration of the object in three dimensions of space can be represented as a mathematical model, i.e., a virtual model of the object, which can be displayed on any workstation or computer using available software tools. The mathematical model can be viewed in any orientation in space, permitting detailed analysis of the surface. The model can be compared to template objects stored in a computer. Deviations in the object from the template can be quantified and analyzed. Further, the virtual model can be transported from one computer and another computer anywhere in the world essentially instantaneously over communications links such as the Internet. The model can be replicated in a computer and thus shared and used by multiple users simultaneously.

The scanner system is useful for a wide variety of industrial, medical, archaeological, forensic, archival, or other purposes. Furthermore, the scanner can be scaled down in size such that it can be hand-held and used to scan small objects, e.g., teeth or small machined parts, or scaled up in size so that it can be used to make mathematical models of larger scale objects such as works of art, sculptures, archaeological sites (e. g., the caves at Lascaux, France or the dwellings or kivas in Mesa Verde National Park), rooms or building facades.

In accordance with a preferred embodiment, the scanner system includes a projection system that projects a pattern onto the object along a first optical axis. The pattern may consist of parallel lines, parallel lines separated by shapes or colours, such as coloured dots, or other suitable pattern. The projected pattern is used to gather information as to the surface characteristics of the object in accordance with the methods and procedures described in more detail below.

The scanner further includes an electronic imaging device, preferably in the form of a charge-coupled device comprising a two-dimensional array of photo-sensitive pixels. The electronic imaging device is oriented along a second optical axis different from the first optical axis. The electronic imaging device forms an image of the pattern after reflection of the pattern off of the object under scrutiny. The surface configuration of the object will cause the projection pattern to become distorted and changed, and thereby provide information as to the surface configuration. This information as to the surface is captured by the imaging device as two-dimensional images of the reflection pattern. These images are processed in accordance with procedures described herein to derive three-dimensional information as to the surface of the object.

The scanning system, in a preferred embodiment, further includes a memory that stores data representing a three axis (X, Y, Z) calibration relationship for the scanner. The calibration relationship can be in the form of a table or in the form of a mathematical function (e. g., polynomial, spline, or other function). The calibration relationship identifies two properties of the scanner: (1) pixel coordinates for the electronic imaging device for numerous portions of the pattern, said pixel coordinates associated with distance information from the projection system in a Z direction at at least two different Z distances, and (2) distance information in X and Y directions, for the numerous portions of said pattern, at the at least two different Z distances. A method of obtaining the calibration relationship is described in detail below. While the simplest form of the relationship is a table, as described in detail below, these calibration relationships could be equivalently represented by one or more mathematical functions as will be apparent to those skilled in art.

The calibration relationship is used to derive three-dimensional coordinates for points on the surface of an object imaged by the imaging device. The generation and use of the preferred calibration table is also explained in further detail below. Other calibration tables or procedures are also possible. The use of the calibration relationship allows the scanner to operate without precise knowledge of the optical and mechanical properties of the scanner, as is required in prior art systems.

The scanning system further includes at least one data processing unit, e.g., the central processing unit of a computer or a digital signal processor, which processes the images of the projection pattern after reflection from the surface of the object. The processing unit compares data from the image (e.g., pixel locations where certain points in the projection pattern are imaged) to the calibration relationship to thereby derive spatial information, in three dimensions, of points on the object reflecting the projected pattern onto the electronic imaging device. Multiple processing units can be used to reduce the amount of time it takes to process the two-dimensional images, calculate three-dimensional coordinates for points in each image, and register frames of three-dimensional coordinates relative to each other to generate a complete virtual model of the object.

The scanning system thus derives three-dimensional information of the object from the images generated by the imaging device and from the calibration relationship stored in memory. Precise knowledge of the optical characteristics of the scanner, the angles between the first and second optical axes, the angle between the optical axis and the point in the imaging device, or the separation distance of the projection device from the imaging device are not necessary. The projection system and the imaging device can be even uncoupled relative to each other. The calibration relationship automatically and completely compensates for these issues, as well as manufacturing variations and tolerances in the scanner optics, in a highly reliable manner. Moreover, knowledge of the distance from the scanner to the object is not required. Additionally, control over the distance between the scanner and the object is not required, within reasonable limits dictated by the depth of focus of the imaging optics. Ideally, during scanning the distance from the scanner to the object is maintained within the limits of the Z distances used during calibration, and that distance is within the combined depth of focus of the projection optics and the imaging optics.

The scanning system can be constructed such that the memory, processing unit, and optical elements are in a single unit. Alternatively, the processing unit and memory can be located at a separate location, such as a scanning workstation or "scanning node", in order to reduce the size of the scanner per se. In such an embodiment, the projection system and the image-recording device can be miniaturized into a hand-held scanner device. A suitable cable connects the scanner device to the workstation to thereby supply the processing unit with scan data, and to receive commands (illumination commands, start/stop commands, etc.) from the workstation.

Numerous other features of the treatment planning software, the scanning system and related features will be more apparent from the following detailed description.

BRIEF DESCRIPTION OF THE DRAWINGS

Figure 1 is an illustration of an orthodontic care system incorporating a hand-held scanner system. The hand-held scanner is used by the orthodontist or the assistant to acquire three-dimensional information of the dentition and associated anatomical structures of a patient and provide a base of information to diagnose and plan treatment for the patient.

Figure 2 is a block-diagram of a scanning system, suitable for use in the orthodontic care system of Figure 1.

Figure 3 is a perspective view of a hand-held scanner used to acquire information of an object under scrutiny, suitable for use in the orthodontic care system of Figure 1.

Figure 4 is an illustration of a patient being scanned with the hand-held scanner of Figure 3.

Figure 5 is a block diagram of the back office server of Figure 1 showing the elements used to calculate the digital model of the patient's dentition and display the digital model on a screen display of the server.

Figure 6 is a flow diagram illustrating the sequence of steps used by the processing unit in the scanning station to calculate three-dimensional information of an object from images captured by the scanner.

Figure 7 is a cross-sectional view of the scanner of Figure 3, showing the optical elements of the projection and imaging aspects of the scanner.

Figure 8 is a perspective view of a scanner calibration apparatus of station that is used to calibrate the scanner and obtain data for entry in a calibration table stored in the memory of the scanner system.

Figure 8A is a perspective view of an alternative embodiment of a calibration device for the scanner in which the Z direction and X-Y calibration surfaces of Figure 8 are combined into a single surface.

Figure 9 is an illustration of the relevant parameters that can be used to calculate surface configuration of the object in accordance with a known fashion.

Figure 9A is an illustration of an electronic imaging device of a scanner, associated imaging lens system, and an object reflecting a projection pattern onto the imaging device at two different distances.

Figure 9B is an illustration of how the different position of the object reflecting a given ray causes the ray to impinge on a different location of the imaging device (CCD chip).

Figure 9C is an illustration of pixel coordinates in X and Y directions for portions of the pattern (crosses A, L, C, E, G, etc.) at a certain Z distances, with a best fit line or mathematical function connecting the portions together.

Figure 10 is a illustration of an arbitrary ray $R_{n,m}$ which is projected from the projection system onto a calibration surface and captured by the electronic imaging device, with the calibration surface positioned at two different distances from the scanner, Z1 and Z2 and separated by a distance $\Delta Z$. Figure 10 illustrates a fundamental principle of the technique that is used for calibration of the scanner and generation of three-dimensional information of an object, which is considered to be an improvement over the calculations required by the method of Figure 9.

Figure 11 is an illustration of a portion of a pattern that is projected from the scanner of Figure 3 onto an object, the projection pattern comprising an array of parallel lines separated from each other by coloured dots, it being understood that other types of projection patterns are possible.

Figure 12 is an illustration showing that the various rays of the projection system through the pattern of Figure 11 can be represented by an array of N x M points.

Figure 13 is a illustration of the X-Y calibration surface of Figure 8, showing the array of Q x P points in the calibration surface being organized into a coordinate system having an origin at the centre of the surface and breaking the surface up into four quadrants I-IV.

Figure 14 is an illustration of the electronic imaging device, comprising an array of pixels arranged in X columns and Y rows.

Figure 15 is an illustration showing the interpolation of pixel addresses in X and Y directions for given ray $R_{2,3}$ from a scanned object from two points of the X-Y calibration plane previously imaged by the electronic imaging device during calibration.

Figure 16 is a more detailed illustration of the location of the ray $R_{2,3}$ from the scanned object relative to the points of the X-Y calibration plane.

Figures 17, 18 and 19 illustrate the pattern recognition process for captured two dimensional images, as a first step in deriving three-dimensional information as to the object.

Figure 20 illustrates the points of the X-Y calibration plane from the calibration station of Figure 7 at two positions relative to the optical elements of the electronic imaging device.

Figure 21 illustrates the relationship between the projection of ray $R_{2,3}$ from the projection system and the X-Y calibration surface of the calibration station.

Figure 22 is an illustration showing the relationship between the unknown distance Z' of the object from the scanning device and the locations where ray $R_{n,m}$ is imaged by the electronic imaging device at distances $Z_1$ and $Z_2$.

Figure 23 is an illustration showing the coordinate system that used for the X-Y calibration surface in generating the entries for the calibration tables for the scanner.

Figure 24 is an illustration of a first calibration table for the scanner after completion of the Z calibration step;

Figure 25 is an illustration of a second calibration table for the scanner after completion of the X-Y calibration step. The entries in the second calibration table of Figure 25 are used to complete the mm entries in the calibration table of Figure 24.

Figure 26 is an illustration of the first calibration table for the scanner after the calculations from the table of Figure 25 have been performed for ray $R_{2,3}$ and the results entered in the first table. It will be understood that a similar calculation from calibration table # 2 (Figure 25) is done for each ray at both distances and the entries in mm for each ray are entered in calibration table # 1 (Figure 24).

Figure 27 is an illustration of a two-dimensional bitmap image of a tooth and associated anatomical structures captured by the electronic imaging device of the scanner of Figure 1, 2, 3 and 4, prior to any signal processing in the scanning node or workstation.

Figure 28 is an illustration of the image of Figure 27 after pattern recognition and filtering operations have been performed.

Figure 29 is an illustration of a single "frame" of data, that is, a three-dimensional point cloud of a scanned object which has been calculated from a single two dimensional image by the pattern recognition, decoding, and 3-D calculations described herein.

Figure 30 is an illustration of the points of the cloud of Figure 29 in which three adjacent points of the cloud are joined together to form triangle surfaces.

Figure 31 is a view of the three-dimensional surface formed from the triangle surfaces shown in Figure 30.

Figure 32 is a view of the surface of Figure 31, smoothed by a smoothing algorithm to give a smoother representation of the surface of the object.

Figure 33 is another example of a bitmap image obtained by the electronic imaging device of the scanner.

Figure 34 is a plan view of the three-dimensional surface obtained from the two-dimensional bitmap image of Figure 33.

Figure 35 is a perspective view of the three-dimensional surface shown in Figure 34.

Figure 36 is a flow chart shown illustrating the steps performed to generate a complete three-dimensional model of the dentition of a patient from a series of scans of the upper and lower jaws.

Figure 37A is an illustration of a mathematical model of a surface of an object after generation of several frames of data and registration of such frames relative to each other to generate a complete model of the surface of the object.

Figure 37B is a illustration of one small three-dimensional section of the model of Figure 38A, showing points from three different frames that are located in approximately the same position in three dimensional space after the registration of these frames has been completed.

Figure 37C is a screen shot on the user interface of a back office work station of Figure 1, showing triangle surfaces for points comprising one frame obtained from scanning a tooth.

Figure 37D is a screen shot showing a registration of a large number of overlapping frames on the tooth, of which the frame of Figure 37C is one frame. Figure 37D illustrates that the low resolution of the scanner's projection pattern, as indicated by the widely spaced points in Figure 37C, is compensated by registration of large overlap of frames, as illustrated in Figure 37C, and results in a high resolution surface.

Figures 38A-38C are an illustration of a two-dimensional cross-correlation procedure in the X and Y directions. The procedure, along with the procedure in Figures 37A and 37B is used to find an initial entry point into a registration algorithm between successive frames of data.

Figure 39A and 39B are an illustration of a one-dimensional correlation procedure in the Z direction for two successive frames.

Figures 40A-40D are an illustration of a frame to frame registration process for a set of frames, each frame consisting of a three-dimensional point cloud of a scanned object. Each frame is typically generated from a different spatial orientation of the scanner relative to the object due to movement of the scanner during image capture, hence the frames overlap to at least some extent. The registration process is used to find a best fit between the frames relative to each other, and thereby provide a complete three-dimensional virtual model of the surface of the object from all of the frames.

Figure 41 illustrates the normal vectors used in the process of Figure 40.

Figure 42 illustrates the summation of the normal vectors from frame 1 to reach a net normal vector $N_{net}$.

Figure 43 illustrates the vectors Vi and Ni from the process of Figure 40;

Figure 44 illustrates the cross product of vectors Vi and Ni;

Figure 45 illustrates the parameter R, which is used in the frame registration process to discard or filter out points which are greater than a distance R from the triangle surfaces.

Figure 46 is an illustration of the closeness factor or quality index, measured as the magnitude of the net normal vector $N_{net}$, as a function of iterations of the process of Figure 40, showing how the quality index improves with successive iterations of the registration process.

Figures 47A and 47B are an illustration of a cumulative registration procedure, which is an alternative to the frame to frame registration procedure of Figure 40.

Figure 48A-48C are a flow diagram of a cumulative registration process.

Figure 49 is an illustration of a set of frames illustrating a different order for frame registration than frame to frame registration, with the order based on the location on the surface of the object for a given frame relative to the location on the surface for other frames.

Figure 50 is a simplified illustration of a set of frames, showing the order in which the frames were obtained, with the neighbourliness of the frames relative to other frames being the basis for the registration order shown in Figure 49.

Figure 51 is another illustration of a set of frames, with registration of frames performed in accordance with the method of Figure 49, with the marking in frames 2, 3 6 and 7 etc. indicating that that frame has been registered. The marking is just a way of illustrating that the computer keeps track of which frames have not been registered, as a check to insure that no frames are omitted during the registration procedure of Figure 49.

Figure 52 is an illustration of cumulative registration based on the first captured frame (F1) as being the base line for all successive registrations.

Figure 53 illustrates an alternative registration procedure in which each frame in the set of frames is registered to a cumulative registration 3-dimensional model of the object, in sequential order, with one iteration of the frame registration process. This is followed by an updating of the cumulative 3-dimensional model and a repeat of the registration process with updated values for the transformation matrix [T] for each frame.

Figure 54 is a screen shot of a workstation computer (either scanning station or back office server workstation), showing the available registration parameters and variables that can be changed by the user when performing either a frame to frame registration or a cumulative registration.

Figure 55 is a screen shot from a workstation computer showing a frame to frame registration in accordance with Figure 40 for two frames in a set of frames.

Figure 56 is a screen shot showing the results after forty five iterations of the frame to frame registration process of Figure 40, and with the right hand side of the screen shot showing the two frames superimposed on each other.

Figure 57 is a screen shot showing a graphical representation of a three-dimensional model of a patient's upper front teeth after a frame to frame registration. The user is applying landmarks to the teeth as a preliminary step in treatment planning, and as a step in registering overlapping segments of a scanned upper jaw relative to each other to calculate a complete model of the upper jaw and associated dentition.

Figure 58A-58F are a series of illustrations showing the generation of an individual tooth model from a scanned tooth, shown in Figure 58A, and a template tooth, shown in figure 58B. A library of template teeth similar to Figure 58A are stored as three-dimensional computer models in computer memory. The individual tooth model is a three-dimensional tooth object having a single set of points defining the boundaries of the tooth. The individual tooth model reduces the amount of data required to represent the tooth, as compared to the data representing the tooth after a cumulative registration of a large number of frames. Individual tooth models are also invaluable in interactive orthodontic treatment planning since they can be independently moved relative to each other in simulation of treatment scenarios.

Figure 59 is an illustration of the tooth model of Figure 58D positioned in the computer model of the patient's dentition, surrounded by other anatomical structures.

Figure 60 is a screen shot from an orthodontic workstation showing the computer model of the patient's teeth positioned in a target or desired condition, as a result of the user selecting an archform for the patient and the computer placing the teeth along the arch selected by the user. Figure 60 also shows the various parameters by which the orthodontist can adjust the shape of the arch, the distance between the teeth, the distance between the molars, and other parameters, so as to provide a unique and customized target situation for the patient.

Figure 61 is another screen shot showing the computer model of the patient's teeth in a target situation, also showing the numerous parameters available to the orthodontist to customize the tooth position, orientation, angulation, torque, and other parameters on a tooth by tooth basis for the target archform.

Figure 62 is another screen shot showing a front view of the target situation and additional parameters available to the orthodontist for moving teeth relative to each other in planning treatment for the patient.

Figure 63 is a screen shot of a target situation for the patient showing the virtual tooth in a target position, a set of virtual brackets placed on the teeth, and a virtual archwire.

Figures 64A-64D are four views of a virtual model of a portion of the dentition generated by the scanning system of figure 1, illustrating alternative methods for separating teeth from associated anatomical structure, e.g., other teeth and gingival tissue, as a part of the process described in conjunction with Figure 58A-58F.

Figure 65 is an illustration of an interpolation procedure that can be used in the process described in conjunction with Figures 58A-58F to fill in holes in scan data from a template object.

Figure 66 is a screen shot of a workstation user interface display showing a patient records screen which the user access various information regarding the patient, including dental history, X-ray photographs, medical history, photographs of the face, teeth and head and the three-dimensional model of the malocclusion.

Figure 67 is an illustration of a series of icons that appear on a screen display that provide some tools for viewing the three-dimensional model of the patient's dentition.

Figure 68 is an illustration of a set of icons which are part of the screen displays which act as a navigational tool and allow the user to manipulate the three-dimensional models of teeth and brackets on the display.

Figure 69 is a screen shot from the treatment planning software showing a set of individual tooth objects representing the observed stage of a patient suffering from a malocclusion.

Figure 70 is another screen shot from the treatment planning software, showing the observed stage and the placement of virtual three-dimensional brackets on the surfaces of the teeth.

Figure 71 is another screen shot from the treatment planning software, showing several views of the observed stage and the fields by which an orthodontist can enter values to alter the relation of the upper jaw to the lower jaw as an initial step of planning treatment.

Figure 72 is another screen shot showing several views of the malocclusion displayed simultaneously, similar to Figure 71.

Figure 73 is a screen show showing a cross-section or "clipping plane" view through the upper arch in a target situation.

Figure 74 is a screen shot illustrating of a portion of a target arch, showing a vertical cross-section or clipping plane taken through the teeth. This view is helpful in adjusting the relation between the upper and lower jaw.

Figure 75 is a screen shot showing the placement of the virtual brackets on the teeth at the malocclusion, showing the user clicking on an icon to establish an initial archform for the upper arch.

Figure 76 is a screen shot showing the computer model of the patient's teeth positioned in a target or desired stage, as a result of the user selecting an archform for the patient and the computer placing the teeth along the arch selected by the user. Figure 76 also shows the various parameters by which the orthodontist can adjust the shape of the arch, the distance between the teeth, the distance between the molars, and other parameters, so as to provide a unique and customized archform for the patient.

Figure 77 is another screen shot showing the computer model of the patient's teeth in a target stage, also the brackets and the orthodontic archwire, and showing the numerous parameters available to the orthodontist to customize the tooth position, orientation, angulation, torque, and other parameters on a tooth by tooth basis for the target archform.

Figure 78 is another screen shot showing a view of the target situation, with brackets and archwire, showing fields allowing the orthodontist to moving the teeth objects relative to each other in planning treatment for the patient.

Figure 79 is a screen show showing a bracket offset correction being entered to move tooth number 16 into an improved occlusal relationship with the opposing jaw.

Figure 80 is a screen shot showing the tooth movement that occurs with tooth number 16 when the bracket offset correction is made.

Figure 81 is another screen show showing the target stage, with the brackets and archwire, showing a tooth moved in the buccal and coronal directions by an amount indicated by the orthodontist, and the correction incorporated into the archwire.

Figure 82 is another screen shot showing a space management feature by which the target situation can be adjusted by specifying spaces between teeth or by extraction of teeth.

Figure 83 is a screen shot illustrating the simulation of an extraction of a tooth number 41.

Figure 84 is a screen shot showing the user modifying the mesial gap between the teeth and showing how the user-specified mesial gap is instantaneously represented on the screen.

Figure 85 is a screen shot showing the user modifying tooth position in a target stage on a tooth by tooth basis using a bonding correction feature.

Figure 86 is a screen shot showing a wire tab, which allows the user to make changes in the shape of an archwire without changing bracket position.

Figure 87 is a screen shot showing a wire offset tab, which allows the user to change the bend size, location, etc., in the wire.

## DETAILED DESCRIPTION OF PREFERRED EMBODIMENT

### Part 1. Overview

Figure 1 is an illustration of an orthodontic care system 10 incorporating a scanner system 12. The scanner system 12 includes a hand-held scanner 14 that is used by the orthodontist or his assistant to acquire three-dimensional information of the dentition and associated anatomical structures of a patient. The images are processed in a scanning node or workstation 16 having a central processing unit, such as a general-purpose computer. The scanning node 16, either alone or in combination with a back-office server 28, generates a three-dimensional computer model 18 of the dentition and provides the orthodontist with a base of information for diagnosis, planning treatment, and monitoring care for the patient. The model 18 is displayed to the user on a monitor 20 connected to the scanning node 16.

As noted above, the scanner system 12 described in detail herein is optimized for *in vivo* scanning of teeth, or alternatively, scanning a plaster model of the teeth and/or an impression of the teeth. However, it will be apparent to persons skilled in the art that the scanning system 12 can by readily optimized for a variety of other diagnostic and/or treatment planning and/or monitoring uses in the medical arena. An example is scanning the face or head and planning plastic or orthopaedic surgery. It can be readily adapted to virtually limitless number of applications in industrial, manufacturing, forensic, archaeological, scientific, archival or other applications.

The orthodontic care system consists of a plurality of orthodontic clinics 22 which are linked via the Internet or other suitable communications medium 24 (such as the public switched telephone network, cable network, etc.) to a precision appliance service centre 26.

Each clinic 22 has a back office server work station 28 having its own user interface, including a monitor 30. The back office server 28 executes an orthodontic treatment planning software program. The software obtains the three-dimensional digital data of the patient's teeth from the scanning node 16 and displays the model 18 for the orthodontist. The treatment planning software includes features to enable the orthodontist to manipulate the model 18 to plan treatment for the patient. For example, the orthodontist can select an archform for the teeth and manipulate individual tooth positions relative to the archform to arrive at a desired or target situation for the patient. The software moves the virtual teeth in accordance with the selections of the orthodontist. The software also allows the orthodontist to selectively place virtual brackets on the tooth models and design a customized archwire for the patient given the selected bracket positions. When the orthodontist has finished designing the orthodontic appliance for the patient, digital information regarding the patient, the malocclusion, and a desired treatment plan for the patient are sent over the communications medium to the appliance service centre 26. A customized orthodontic archwire and a device for placement of the brackets on the teeth at the selected location is manufactured at the service centre and shipped to the clinic 22. The system is also applicable to other types of appliance systems ; brackets and archwires are shown in the illustrated embodiment but other types of appliance systems can benefit from the scanning system described herein, such as removable aligning devices ; retainers, Herbst appliances, etc.

As shown in Figure 1, the precision appliance service centre 26 includes a central server 32, an archwire manufacturing system 34 and a bracket placement manufacturing system 36. These details are described later.

Figure 2 is a more detailed block-diagram of the scanning system 12, suitable for use in the orthodontic care system of Figure 1. The scanning system 12 is a mechanism for capturing three-dimensional information of an object 40, which in the present example is the dentition and surrounding anatomical structures of a human patient, e.g., gums, bone and/or soft tissue. The scanning system 12 includes a scanner 14 which is used for image capture, and a processing system, which in the illustrated example consists of the main memory 42 and central processing unit 44 of the scanning node or workstation 16.

The scanner 14 includes a projection system 46 that projects a pattern onto the object 40 along a first projection axis 48. The projected pattern is formed on a slide 50 which is placed in front of a light source 53. In the illustrated embodiment, the light source 53 comprises the terminus of a fibre-optic cable 51. The cable 51 carries a high intensity flash generated by a flash lamp 52 located in a base unit 54 for the scanner. A suitable flash lamp is the model FX-1160 flash unit available from Perkin Elmer. The illuminations of the flash lamp 52 cause the pattern contained in the slide 50 to be projected onto the three-dimensional surface of the object. Further details on the types of patterns suitable for the pattern are set forth in the following co-pending patent applications of Rüdger Rubbert et al: serial no. 09/254,755 filed March 9, 1999; serial no. 09/1560,131 filed April 28, 2000, and serial no. 09/673,863 filed November 30, 2000 assigned to the assignee of the present invention. A presently preferred projection pattern is described below. The details on the optics of the projection system 46 are set forth in further detail below.

The scanner 14 further includes an electronic imaging device 56 comprising an array of photo- sensitive pixels. A preferred embodiment is an off- the- shelf, colour- sensitive, charged- coupled device (CCD) of a size of 1, 028 x 1, 028 pixels arranged in an array of rows and columns. The Sony ICX205AK CCD chip is a suitable electronic imaging device.

The electronic imaging device 56 is oriented perpendicular to a second imaging axis 58, which is off-set from the projection axis 48. The angle $\psi$ between the projection and imaging axes need not be known. However, if the 3D calculations are made in accordance with the parameters of Figure 9, then the angle and the separation distance between the centre of the imaging device 56 and the centre of the light source 53 need to be known.

The angle $\psi$ will be optimized during design and manufacture of the scanner depending on the desired resolution required by the scanner. This, in turn, is dependent on the degree to which the surface under scrutiny has undercuts and shadowing features which would result in the failure of the imaging device to detect the projection pattern. The greater the angle, the greater the accuracy of the scanner. However, as angle $\psi$ increases, the presence of undercuts and shadowing features will block the reflected pattern and prevent capture of the pattern and subsequent three-dimensional analysis of those portions of the surface. Angle $\psi$ is shown somewhat exaggerated in Figure 2, and will generally range between 10 and 30 degrees for most applications.

The electronic imaging device 56 forms an image of the projection pattern after reflection of the pattern off of the surface of the object 40. The reflected patterns imaged by the imaging device contain three-dimensional information as to the surface of the object, and this information needs to be extracted from the images. The scanning system therefore includes a processing subsystem which is used to extract this information and construct a three-dimensional virtual model of the object 40. In the preferred embodiment, this processing subsystem consists of a memory 42 storing calibration information for the scanner, and at least one processing unit, such as the central processing unit 44 of the scanning workstation 16. The location of the memory and the processing unit is not important. They can be incorporated into the scanner 14 per se. Alternatively, all processing of the images can take place in the back office server 28 or in another computer. Alternatively, two or more processing units could share the processing in order to reduce the amount of time required to generate the three-dimensional information.

The memory 42 stores a calibration relationship for the scanner 14. The calibration relationship, which can be in the form of a table or one more mathematical functions, comprises information used to compute three-dimensional coordinates of points on the object that reflected the projection pattern onto the imaging device. The information for the table is obtained during a calibration step, performed at the time of manufacture of the scanner 14. The calibration table includes an array of data storage locations that contain two pieces of information. Firstly, the calibration table stores pixel coordinates in X and Y directions for numerous portions of the projection pattern that are imaged by the electronic imaging device 56, when the pattern is projected onto a calibration surface at two different distances during a calibration procedure. Secondly, the table stores distance information, (e.g., in units of tenths of millimetres), in X and Y directions, for the portions of the projection pattern imaged at the two different distances. A preferred method for generation and use of the calibration table is explained in further detail below.

The scanning system requires at least one processing unit to perform image processing, three-dimensional calculations for each image, and registration of frames to each other. The processing unit 44 in the illustrated embodiment is the central processing unit (CPU) of the scanning work station 16. The CPU 44 processes the image of the pattern after reflection of the pattern off the surface of the object 40 and compares data from the image to the entries in the calibration table. From that comparison (or, more precisely, interpolation relative to the entries in the table, as explained below), the processing unit 44 derives spatial information, in three dimensions, of points on the object that reflect the projected pattern onto the electronic imaging device.

Basically, during operation of the scanner to scan an object of unknown surface configuration, hundreds or thousands of images are generated of the projection pattern as reflected off of the object in rapid succession as the scanner and object are moved relative to each other. For each image, pixel locations for specific portions, i.e., points, of the reflected pattern are compared to entries in the calibration table. X, Y and Z coordinates (i.e., three dimensional coordinates) are obtained for each of these specific portions of the reflected pattern. For each picture, the sum total of all of these X, Y and Z coordinates for specific points in the reflected pattern comprise a three- dimensional "frame" or virtual model of the object. When hundreds or thousands of images of the object are obtained from different perspectives, as the scanner is moved relative to the object, the system generates hundreds or thousands of these frames. These frames are then registered to each other to thereby generate a complete and highly accurate three- dimensional model of the object 40.

Stray data points are preferably cancelled out in generating the calibration table or using the calibration table to calculate three-dimensional coordinates. For example, a smoothing function such as a spline can be calculated when generating the entries for the calibration table, and the spline used to cancel or ignore data points that deviate significantly from the spline.

Figure 2 also shows a few other features of the presently preferred scanning system 12. After the CCD imaging device 56 captures a single image, the analogue voltage signals from the device 56 are amplified in an amplifier 57 and fed along a conductor 59 to an analogue to digital converter 60. The digital signal is converted into a bitmap stream of digital image data. The data is formatted by a module 61 into an IEEE 1394 "firewire" format for transmission over a second conductor 62 to the main memory 42 of the scanner work station 16. The scanning system includes an optical scanner holder 64 for the user to place the scanner after the scanning of the dentition is complete. These details are not particularly important and can vary considerably from the illustrated embodiment. As noted earlier, preferably the scanning system is constructed to provide a minimum of equipment and clutter at the chair side. Hence, the scanning work station 16 is preferably located some distance away from the chair where the patient sits. The cable leading from the scanner 14 to the base station and/or workstation 16 could be suspended from the ceiling to further eliminate chairside clutter.

The scanning work station 16 also includes the monitor 20 for displaying the scanning results as a three-dimensional model 18 of the dentition in real time as the scanning is occurring. The user interface also includes a keyboard and mouse for manipulating the virtual model of the object, and for entering or changing parameters for the scanning, identifying sections or segments of scans that have been obtained, and other features. The scanning station may also include a foot switch, not shown, for sending a signal to the CPU 44 indicating that scanning is commencing and scanning has been completed. The base station may alternatively include a voice recognition module that is trained to recognize a small set of voice commands such as START, STOP, AGAIN, REPEAT, SEGMENT, ONE, TWO, THREE, FOUR, etc., thereby eliminating the need for the foot switch. Scanner start and stop commands from the CPU 44, in the form of control signals, are sent to the light source 52, thereby controlling the illumination of the lamp 52 during scanning.

The light source 52 operates at a suitable frequency, preferably at least greater than one flash per second, such as six flashes per second, and the frame rate of the CCD imaging device 56 is synchronized with the flash rate. With a frame rate of 6 frames per second, and a scanning motion of say 1-2 centimetres per second, a large of overlap between images is obtained. The size of the mirror at the tip 68 of the scanner influences the speed at which scanning is possible. The illustrated embodiment of the mirror at the tip 68 is 18 mm square. A larger mirror reflects more surface of the object and enables faster scanning. A smaller mirror requires slower scanning. The larger the mirror, the more difficult *in vivo* scanning becomes, so some trade-off between size and utility for *in vivo* scanning exists.

This overlap between images generated by the scanner 14, and resulting three dimensional frames, allows a smooth and accurate registration of frames relative to each other. The frame rate and permissible rate of scanner motion will depend on many factors and can of course vary. Flashing a high intensity flash lamp for a brief period of time is a preferred embodiment since it is desirable to reduce the exposure time of the CCD imaging device 56 to reduce blurring since relative motion exists between the scanner and the object. A high intensity lamp is desirable to achieve sufficient signal strength from the imaging device. A preferred embodiment uses 5 μsec flash times with similar exposure periods. An alternative embodiment would use a constant illumination source of high intensity, and control exposure of the imaging device using a shutter, either a physical shutter or using electronic shutter techniques, such as draining charge accumulating in the pixels prior to generating an image. Scanning using longer exposures would be possible without image blur, using electronic image motion compensation techniques described in Lareau, et al., U.S. Patent 5,155,597.

Figure 3 is a perspective view of a hand-held scanner 14 used to acquire information of an object under scrutiny, suitable for use in the orthodontic care system of Figure 1. The projection system 46 and the electronic imaging device 56 of Figure 2 are contained in the housing 65 for the scanner. The housing 65 is sized and shaped to be held in a human hand. The scanner 14 includes an elongate distal portion 66 having a tip 68. The tip 68 is sized and shaped such that it can be inserted into and moved within an oral cavity of a human so as to enable scanning of anatomical structures inside the oral cavity. A heating mirror (not shown) is placed on the underside of the tip 68 to direct the projection pattern from the optics of the scanner onto the object and to direct the reflected pattern from the object towards the imaging optics 108 of Figure 7 associated with the electronic imaging device 56. The mirror housing has A/C conductive heating coil that heats the mirror. The mirror is heated to approximately 40 degrees to prevent fogging of the mirror while the scanner is used *in vivo.*

Figure 4 is an illustration of a patient 70 being scanned with the hand-held scanner 14 of Figure 3. The cheeks and lips are retracted from the teeth and the tip 68 of the scanner is moved over all the surfaces of the teeth in a sweeping motion at a velocity of perhaps 1-2 centimetres per second. The entire upper or lower jaw may need to be scanned in a series of scans, one for the left side, one for the right side, and one for the front. These individual scans are registered to each other as described below. Voice commands or activation of the foot switch (not shown) indicates when each scanning segment is initiated and terminated. The entire process takes just a few minutes. Depending on the colour and translucency of the object and the illumination intensity and frequency of the light source in the scanner, it may be necessary to apply a very thin coating of a bright reflective substance such as Titanium Dioxide to the object.

While Figure 4 illustrates *in vivo* scanning of a human patient, the scanner can of course be used to scan a plaster model of the dentition if that is preferred, or an impression taken from the patient. When scanning an impression or a plaster model, the scanning may be formed in a single pass, without the need for registering scan segments to each other. It is also possible that a scan of a patient may be partially taken *in vivo* and the remainder from a model or an impression.

Figure 5 is a block diagram of the back office server of Figure 1 showing the elements used to calculate the digital model of the patient's dentition. After the scanning workstation has processed all the images captured by the scanner and generated a set of three dimensional frames, the frame data is transmitted to the back office server 28. The back office server 28 performs a cumulative registration process for the frames and ultimately generates and displays the digital model on a screen display 30. The raw scanner data in the form of three-dimensional frames is stored in the main computer memory 72. The frame data for N captured images, i = 1... N from the scanner is stored in the hard disk 74. The hard disk also stores a set of (N-1) transformation matrices $[T]_i$, for i = 2 - N. The transformation matrices basically contain information as to how each frame of three-dimensional points needs to be translated and rotated in a three-axis Cartesian coordinate system in order to be registered with the other frames in a best-fit manner. One of the frames, such as the first frame in the series, is a starting point for registration and no transformation matrix is obtained for that frame. The generation of the transformation matrices, and use of the matrices with the frame data to generate the three dimensional model, is described in further detail below.

Part 2. Three-Dimensional Image Generation

With the above general introduction and overview in mind, a presently preferred process of capturing two dimensional images with the scanner and generation of a three dimensional model for each image will now be described in detail in this section. Figure 6 is a flow diagram illustrating the sequence of steps used by the processing unit 44 in the scanning station 16 (Figure 2) to calculate three-dimensional information of an object for a single image captured by the scanner 14. This process shown in Figure 6 is performed for each image.

This process can be executed in one processor, or may be performed or shared by multiple DSP processors sharing access to a memory storing the captured two-dimensional bitmap images from the scanner, e. g., memory 42 of Figure 2. The type of processor is of course not important. The goal of distributed processing is to reduce the time required for processing the scanned image. For example, one processor could perform the cross correlation process described below, another perform the pattern recognition, another decoding, and another 3-D calculation. These processes could be performed independently, each process associated with independent files shared on a disk memory. The processor assigned to one or more of the processes of Figure 6 accesses the files as needed. For example, the output file from the decoding process is written on a disk or memory available to the other processing units. The processing units are on a network in this example. The assigned to 3-D calculation access the decoding output file and writes the result, a set of coordinates for N frames and N-1 transformation matrices, onto the disk or memory. Any one of the processors assigned to perform registration could then access the N frame data and the N-1 transformation matrices and perform the registration procedures described below.

The process of Figure 6 consists of four principal steps: a pattern recognition process 80, a decoding process 82, a process 84 of derivation or calculation of three dimensional coordinates for each point in the detected and decoded pattern, and finally a step 86 of storing the three dimensional coordinates in a memory, such as the memory 42 of the scanning work station 16. Again, this process is performed for each captured image during the scanning process. In a typical scanning scenario, hundreds or even thousands of images may be captured, hence this process of Figure 6 may be performed hundreds of times. The result of the process is the storage of a large number of sets of three-dimensional coordinates, each set or "frame" associated with a single captured image. The registration of these frames relative to each other to generate a complete virtual model of the object is described in Part 3 of this document.

As the scanner is moved over the dentition, the imaging device acquires a series of bitmap images. The acquired bitmaps are analyzed using pattern recognition. Pattern recognition detects the median lines of the projected lines, endpoints of the lines and the centres of the coloured dots. Other types of patterns are of course possible, such as using triangles, squares, or other coding features. The coding is in the vertical direction (in the direction of the parallel lines), since the distortion of the projection pattern provided by the surface of the object is in this direction, as explained more fully in the Rubbert et al. patent application serial no. 09/560,131 filed April 28, 2000.

The pattern recognition process uses sub-pixel-precision. The colour of every dot is analyzed as well. Based on the knowledge of the pattern structure and using the coloured dots, the origin in the pattern for every recognized line is determined. This is necessary, as significant portions of the projected pattern may not be visible to the imaging optics due to shadowing, undercuts and un-sharp areas. A two-dimensional to three-dimensional conversion algorithm uses the knowledge of the origin of each imaged line with respect to the pattern to compute three-dimensional coordinates of the points in the object. As the lines are often captured only as fragments, the decoding algorithm does not always have sufficient information on each line to unequivocally assign that line to the pattern. The algorithm therefore examine several scenarios of possible affiliations and looks for conflicts. In this way the inconsistent scenarios are filtered out. The lines in the projection pattern do not change their order in the image. For example, if the lines in the projection pattern are sequentially numbered 1-80, and line 43 is to the left of line 44, in the captured image line 43 will be always be to the left of line 44 and never to the right of line 44. Inconsistent scenarios are indicated where the order of lines is violated. The correct order of the lines can be deduced by a suitable algorithm that examines the scenarios based on line order and eliminates all those where conflicts or inconsistent line numbering exists. A unique solution will be found.

While the preferred embodiment of the three-dimensional conversion algorithm is based on a sophisticated calibration process and does not make use of any knowledge of the optical parameters, an alternative embodiment could use general principle of analytical triangulation assuming that we do have such knowledge. Analytical triangulation will be explained with reference to Figure 9. The projecting device projects a pattern of distinguishable elements onto a surface. This projected pattern is imaged. For each captured element, is must be possible to tell its origin at the pattern. This allows us to determine the angle $\alpha$ between the optical axis of the projection device and the one ray that has projected that element. The location of the pixel at the CCD chip that has captured that element allows us to determine the angle $\beta$ between the optical axis of the imaging system and the one ray that leads from the projected element at the surface to the CCD pixel. Knowing those two angles, the angles of the two optical axes with respect to the baseline and the length of the baseline, allows us to calculate the spatial position of the element relatively to the scanning device. This calculation can be done for every detected element within one captured image and leads to a plurality of three-dimensional points. It is important to understand that as a result from this calculation we receive an undistorted, true to scale representation of the surface. While every two dimensional image shows distortions due to parallax effects, the triangulation process eliminates this effect.

The analytical triangulation method requires precise knowledge of optical parameters. The preferred embodiment using a calibration table for the scanner does not require this knowledge

A. Scanner Manufacture and Calibration

Before describing the details of the process steps shown in Figure 6, an illustrative embodiment of the scanner 14 itself and its manner of calibration will be described first.

Figure 7 is a cross-sectional view of a portion of the hand-held scanner 14 of Figure 3, showing the optical elements of the projection and imaging aspects of the scanner. The scanner includes the fibre-optic cable 51 carrying flashes from a flash lamp in the base station 54 of Figure 2 to a condenser system consisting of a group of lenses 53A. Light from the light source 53 illuminates a pattern formed in a slide 50. Wide variation is possible in the choice of the pattern. A presently preferred embodiment is described subsequently in conjunction with Figure 11. The pattern is directed by a projection lens system 102 out of the scanner to the mirror housed at the tip 68 of the scanner (Figure 3) and towards the object under investigation. The scanner further includes a several LED light sources 106 (e.g., 2 or 6), which are used to provide general illumination of the object during scanning to assist the user in scanning the dentition. A prism 104 surrounds the LED light sources. The axis of the projection system is shown as axis 48.

The projection pattern is reflected off of the object, reflected by the mirror in the tip 68 and received by an imaging lens system 108 centred about an imaging axis 58. The received pattern is reflected off a mirror 110 onto the CCD electronic imaging device 56. The CCD 56 produces a voltage signal for each pixel in the device. The level of the signal is an indication of the level of light impinging on that pixel, allowing an image to be produced from the CCD. The signals are read out of the CCD using known circuitry and amplified. The amplified analogue signal is collected and transmitted along conductors 59 to the base unit for conversion to digital form. The signal from the CCD is converted into a coloured bitmap image in the illustrated embodiment. Colour is used in the projection pattern in the illustrated embodiment, therefore a CCD chip is selected which can detect colours. A black and white system is of course also possible.

In the illustrated embodiment, the separation distance between the light source and the projection pattern is not known or needed, nor is the angle between the axes 48 and 58. However, some non-zero angle between the axes is required in order to obtain depth information. The angle selected will depend on the type of surface or object the scanner will be used for. These types of implementation details will vary considerably depending on the application. Furthermore, it is possible to make the two axes 48 and 58 completely independent of each other by locating the projection and imaging in separate, independently moveable devices. This is described in more detail in the patent application of Rüdger Rubbert et al, serial no. 09/254,843. The calibration procedures described herein are of particular advantage when the projection device and the imaging device are in two separate, independently moveable units.

Figure 8 is a perspective view of a presently preferred scanner calibration station 120 that is used to calibrate the scanner 14 at the time of manufacture. The purpose of the station 120 is to obtain data for a calibration relationship for the scanner, such as a calibration table, which is stored in the memory of the scanner system. Some variation in the design of the calibration station is possible; the principle of operation of the calibration station is that the scanner is calibrated by projecting the pattern onto a reference object of precisely known geometry at two known distances (in the Z direction) and known spatial extent (X and Y directions). A planar reference object is preferred, but it is theoretically possible to use any object of known geometry, but more computationally complex to use such objects. The goal of the calibration procedure is to compile a set of information (e.g., in the form of a table) that completely calibrates the scanner and places it in condition for use to scan objects of unknown surface geometry, without the need for precise knowledge of the mechanical or optical properties of the scanner. While the present embodiment describes a calibration table as the result of the calibration process, the information may be in other equivalent forms, such as mathematical relationships or formulae between pixel address and distance, which are operated on to derive distance information at the time of use.

Before discussing the presently preferred calibration device and calibration relationship, a discussion of the principles of the calibration will be set forth for ease of understanding.

A 3D imaging device as disclosed in this application does initially not deliver 3D information, but only 2D information as the CCD chip is a 2D imaging device. Information on the 3rd dimension therefore has to be determined in an extra processing step. The additional information that we can use to perform such a 3D calculation is the spatial arrangement of the optical components, such as shown in Figure 9A. Figure 9A shows a schematic arrangement of components. One portion of the pattern is indicated with a small square, and the ray along which this portion would be projected is also displayed. The point, where this ray would intersect with the surface of the object, is displayed as a circle. At the centre of this circle, the portion of the pattern would be projected onto the surface of the object. One of the reflected rays will run through the imaging lens system and such be projected onto the surface of the CCD chip, which will cause a corresponding signal. In this figure, only the centre rays are displayed, e.g. the rays that run through the centre of the lens systems.

Assuming that there is precise knowledge of the geometrical arrangement of the components, it would be possible to precisely calculate the spatial coordinates of the part of the surface of the object that reflects the considered portion of the pattern. This calculation is only possible under three preconditions:

(i) The geometric arrangement of all components of the scanning system must be precisely known,
(ii) The exact characteristics of all components must be known (true x/y-coordinates of all CCD pixels, including precise knowledge of the dimensions of the pattern; and
(iii) The lens systems must be 'ideal' which means that the centre ray must be an ideal straight line.

In mass production scenario for a scanner, it will be almost impossible to guarantee these preconditions. One possible approach would be to calibrate the individual devices, which means that the deviations of the characteristics from the ideal configuration are determined and noted ("compensative calibration"). The 3D calculation will then base on algorithms like described before, but will additionally take into account known deviations to compensate for individual characteristics of each device. However, this compensational calculation has to be set up very carefully, and errors in terms of plus/minus signs will not easily be detected especially when the deviations are minor.

Another challenge is presented by scanning devices like disclosed in PCT/DE97/01797 by Rubbert, where imaging device and projection device are not physically connected to each other, and therefore the geometrical relationship may be completely unknown.

The calibration procedure that is described herein does not require any pre knowledge of any dimensions of the optical and mechanical components, and thus can be termed "independent calibration". Furthermore, even any knowledge of the angle formed by the two optical axes (angle of triangulation) is not required.

The background of this procedure can be described best by again just looking at one specific portion of the pattern that is being projected along a ray onto the surface to be measured like indicated in Fig. 9B. It is important to understand that this portion of the pattern will always be projected along this specific ray R, which is defined by the optical characteristics of the lens system and the arrangement of the pattern and the lens system with respect to each other. If this portion of the pattern is being reflected from any surface sitting in the field of view, we can be absolutely sure that this point of the surface will be located somewhere along this ray R. However, we do not know at which position along this ray the point is located. To be able to determine this, the "independent calibration" method basically takes samples. The first sample will be a surface that is located at a certain distance $Z_1$ from the projection device. The reflected portion of the pattern will show up at a specific pixel at the CCD chip (or perhaps over several pixels depending on the size of the portion of the pattern). It is important to understand that every surface that is ever being hit by this ray at this Z-distance will always cause a reflection of this ray R directed to this pixel (we are dealing with diffuse reflection, so every point being reflected will send rays into many directions, regardless of the angle between ray and surface). This implies that every time when this portion of the pattern is being reflected onto the pixel, we know exactly that this point of the surface is located at distance $Z_1$.

Knowledge of the distance between $Z_1$ and the scanner is not required as long as the scanner will not be used as an absolute measuring system. If we want to use the scanning system as an absolute measuring system, which means that we want to measure the location of points relative to the scanner and not only relative to each other, we would then need to use the Z-values with respect to the origin of the coordinate system of the scanner. The illustrated embodiment is not an absolute measuring system, but nevertheless generates accurate virtual models of the object true to scale.

During the calibration process, we will acquire a plurality of such "samples" for different portions of the pattern reflected off a calibration surface at different Z-distances, where the relative Z-distances of these levels with respect to each other must be known. It will be discussed further below, how many samples will typically be required to receive a complete calibration. The result of this sampling process is the first calibration relationship that is derived for the scanner: (1) pixel coordinates for the electronic imaging device for numerous portions of the pattern, said pixel coordinates associated with distance information from the projection system in a Z direction at at least two different Z distances.

Having this first part of the calibration procedure done, we can determine the Z-component of every part of the measured surface that is reflecting the pattern onto the CCD chip. However, we do not have knowledge of the X-and Y-coordinates. To get this information, we need to perform the second part of the calibration.

Again, we will take "samples", but this time we will not make use of the pattern that is being projected onto the object during normal use of the scanner (the projection unit will be switched off) . Rather, images are obtained of a reference object in the field of view that is equipped with features of a known geometry, i.e., known X- Y spatial relationship. The simplest implementation would be a point. In Figure 9C, such a feature is a plurality of cross- hairs. The feature being projected onto the CCD chip will show up at a specific pixel (or several pixels depending on the size of the feature) . The X/Y- coordinates of the pixel at the CCD chip are then assigned to the X/Y- value of the location of the feature (and assigned to a certain Z- distance) .

It is obvious that if the feature is being moved in Z- direction, the location of the projection of the reference object at the CCD chip will change. We therefore have a dependence on the Z coordinate, which signifies that the Z-location of the feature must have a known reference to the Z- location (s) of the surfaces that have been used in the first part of the calibration. For instance, such a feature could be located at $Z_1$; other features might be located with a known reference to $Z_1$.

The first feature being captured in this manner would serve as a reference. A certain X-and Y-value (in mm or inches) would be assigned to the location of this feature. If such a feature would be placed close to the optical axis of the imaging system, it would be preferable to assign X = 0 mm and Y = 0 mm to this location. If we want to use the scanning system as an absolute measuring system, which means that we want to measure the location of points relative to the scanner and not only relative to each other, we would then need to use the X/Y/Z-values of this feature with respect to the origin of the coordinate system of the scanner.

During the calibration process, we will again acquire a plurality of such "samples" at different X-Y-and Z-locations, where the relative X-, Y-and Z-values of the locations of these features with respect to each other and with respect to the Z-values of the first part of the calibration must be known. It will be discussed further below, how many samples will typically be required to receive a complete calibration.

It is important to understand that the determined relationship between the X-and Y-coordinates of any feature being captured and specific pixel coordinates at the CCD chip exists only with respect to the Z-coordinate of the feature. A movement in Z will change the X/Y-value. Therefore, during normal operation of the scanner, when the calibration results are being used to calculate 3D coordinates, we first have to calculate the Z-coordinate of any point on a surface using the calibration values acquired in part 1, and basing on these results we can then perform the X/Y calculation, using the calibration results of part 2 of the calibration process.

There are several options with regard to the number of "samples" to take during calibration and the way how the results may be stored. The most straightforward approach would be to collect pixel coordinates for at least two Z-levels of the projected pattern. The number of pixel coordinates will depend on the resolution of the pattern. The Z-levels will preferably be defined within the depth of focus of the scanner projection lens systems and imaging lens systems, but close to the boundaries of this depth. Having collected pixel coordinates for at least two levels, would allow for interpolation of all other Z-levels. Part 2 of the calibration procedure could also comprise features (points) distributed evenly across the field of view, and those features could again be placed at two different Z-levels, which would allow for an easy interpolation of X-and Y-values. The pixel coordinates acquired in both parts of the calibration process could in the simplest embodiment be stored in a table.

However, this straightforward approach has certain disadvantages. First of all, an apparatus is required. Otherwise it would not be possible, to place the surfaces required for part 1 in a controllable manner with respect to each other, and the features being captured in part 2 also need to be precisely placed with respect to each other and to the calibration surface used in part 1. Usage of such a calibration apparatus is not a problem within an industrial production environment. But if scanners need to be calibrated for instance in an orthodontic office, it is not recommendable to always ship such a device to the location.

But there is no need to calibrate each portion of the pattern in various Z-levels. If a device is used, that comprises surfaces at different Z-levels, portions of the pattern will be projected onto levels that are closer to the scanner, and portions will be projected onto levels that are further away. It is well possible, to interpolate also the pixel coordinates that are not acquired during calibration.

Assuming that portions A and C of the pattern will be projected onto level While portions C and D will be projected onto level $Z_2$ we will receive pixel coordinates for portion A and C assigned to Level $Z_1$ ($x_{A1}$ and $y_{A1}$ for A, $x_{C1}$ and $y_{C1}$ for C) and pixel coordinates for portion B and D assigned to Level $Z_2$ ($x_{B2}$ and $y_{B2}$ for B, $x_{D2}$ and $y_{D2}$ for D). It is well possible to linearly interpolate for instance $x_{A2}$ (which has not been acquired) from $y_{B2}$ and $y_{D2}$. In the same manner $y_{B1}$ could be interpolated from $y_{A1}$ and $y_{C1}$. Another way to receive calibration values that have not been acquired directly would be to draw the acquired pixel coordinates for a certain Z-level onto a sheet of paper and then to construct a best-fit line (either straight or curved) through those points. If the mathematical function of this best-fit line is stored, the pixel coordinates can be calculated using that function instead of storing them separately. The operation of determining a best-fit line can of course also be done directly in the computer. The best fit line concept is illustrated in Figure 9C.

This procedure would work as well for part 2 of the calibration procedure where pixel coordinates are being acquired for specific features assigned to X-, Y-and Z-values. Again only a subset of features has to be captured at each Z-level, and the remaining values can be interpolated in the way described above. It would therefore also be possible to use just one calibration device that provides surfaces at least two Z-levels to perform part 1 of the calibration and comprises features at those surfaces that allow for part 2. The density of portions of the pattern, i.e., features to be captured, depends on the optical quality of the components of the scanner. We should capture at least four portions of the pattern, preferably close to the corners of the CCD imaging device 56 to provide a reliable interpolation.

The advantage of this calibration process is that it requires absolutely no pre-knowledge of the mechanical and optical characteristics of the scanner and automatically compensates for irregularities of the optical components, this including the CCD chip and the pattern. It is therefore useful to calibrate scanners that are made from cheap parts, and in can be used on scanners that have no known relationship between the imaging and the projection device.

With the foregoing in mind, a representative embodiment of a scanner calibration device and method will be described with particularity with reference to Figure 8. The presently preferred scanner calibration system includes mount or holder 122 for holding the scanner fixed in position during calibration. The holder is affixed to the top of a table 124. A calibration apparatus is positioned directly in front of the scanner 14. The calibration apparatus consists of a Z-direction carrier 126 having one portion 128 fixedly mounted to the table and a second portion 130 which can move back and forth in the Z direction between two different positions Z1 and Z2. An X-direction carrier 131 is mounted to the moveable portion 130 of the Z-direction carrier. The X-direction carrier consists a first portion 132 which is mounted to the moveable portion 130, and a second portion 134 which is moveable in the X direction relative to the first portion 132, as indicated.

The X-direction carrier 131 has mounted to its upper surface 136 two calibration devices: (1) a smooth, planar calibration surface 138 used for calibration of the scanner in the Z-direction, and (2) an X-Y calibration surface 140 used for calibration of the scanner in the X and Y direction. The X-direction carrier also contains a light 142 for providing back illumination of the X-Y calibration surface 140.

To calibrate the scanner 14, carriers 126 and 131 are moved such that the Z-direction calibration surface 138 is positioned in front of the scanner 14. An image is taken of the projection pattern reflecting off the surface with the surface 138 at some arbitrary distance Z1 from the scanner. Then the carrier 130 is moved a distance away ($\Delta$Z) to a new position Z2, and a second image is taken. Pixel addresses where the specific locations of the pattern are imaged in the electronic imaging device are determined and stored in a calibration table in a memory. The distance $\Delta$Z is also known precisely and stored in the scanner memory or in the computer that performs the scanner calibration.

Then, the carriers 126 and 131 are moved such that the X- Y calibration grid 140 is placed at the distance Z1 and an image is taken. The image is generated by activating the source 142, with light from the source 142 passing through numerous tiny apertures 143 in the calibration surface 140 and impinging on the electronic imaging device 56. (The pattern illumination source is not used in this part of the calibration) . The carrier portion 130 is moved to the position Z2, and another image is generated. Using the known separation distance between points in the X- Y calibration grid 140, X and Y distance information for points in the pattern imaged in the first part of the calibration procedure is computed. The results are stored in the calibration table. This process is described in further detail below. When the scanner calibration is finished, the scanner serial number and scanner calibration table (or other representation of the calibration relationship, such as a set of mathematical equations) are stored in memory in the scanner or in a computer associated with the scanner that processes the scanned images.

An alternative configuration of the calibration surfaces is shown in Figure 8A. The calibration device consists of a set of reflective, planar, parallel planes 144, 144', 144" and 144"', of known distance from each other and from the scanner, with apertures 143 spaced at known distances along the surfaces of the planes and at known distances from adjacent planar surfaces. A cross feature 145 at the centre forms the origin of the calibration surface, and is used as explained below. Two of the surfaces 144, 144', 144" and 144"' are used for the Z calibration surface. For X and Y calibration, the backlighting illumination source 142 is activated and light passes through the apertures 143 onto the electronic imaging device. The entire two-dimensional focal plane of the electronic imaging device is calibrated from an interpolation of known points in the surfaces 144, 144', 144" and 144"', in accordance with teachings described in detail herein. The embodiment of Figure 8A is considered more cumbersome than the embodiment of Figure 8, but is offered to illustrate that other configurations for a calibration surface are possible. In fact, curved surfaces or sloping surfaces could even be used, but the simplest surface is a planar surface oriented directly at the electronic imaging device.

Thus, in one possible alternative embodiment, a calibration device is provided for a scanner projecting a pattern onto an object and receiving a reflection of the pattern off the object. The calibration devices comprise a calibration surface 144 receiving said projected pattern comprising two or more parallel surfaces (e. g., 144 and 144") of known separation distance and spatial extent and a plurality of point sources of light 143 provided in the two or more parallel surfaces. As described herein the point sources of light are apertures which allow light to pass through the surfaces 144 from the light source 142, but other configurations are possible. For example, the point sources of light could be light emitting diodes arranged in an array in the surface 144. The apertures 143 are formed in a precise and known spatial relationship relative to each other, such as by forming the holes with a precision high powered laser on a sheet of metal. Alternatively, instead of apertures 143, black dots could be formed on paper using a highly accurate printing process, and the black dots imaged by the CCD 56.

The calibration procedure described herein represents an alternative, and more preferred way of computing three-dimensional information for images as compared to prior art methods. Figure 9 is an illustration of the relevant parameters that can be used to calculate surface configuration of the object in accordance with a known fashion. The method of Figure 9 requires knowledge of the separation distance D or baseline between the detector and the light source, the angle between the axes 48 and 58, and the angles $\alpha$ and $\beta$ shown in the Figure. The present calibration method and method of calculation of three dimensional information does not require any of this information. The calibration procedure compensates for imperfections in the optics in the optical paths, and therefore eliminates the need for high precision optics. Further, there is no need for precise knowledge of the placement of the scanner relative to the calibration planes. There is no need to know the angle between the axes 48 and 58, the separation distance between the scanner and the object being scanned, or any absolute value of the location of the object in any global coordinate system. The scanner allows for truly reference independent scanning, yet it gives very precise description of the three-dimensional surface.

The calibration will typically be performed once during manufacturing, which should be enough to last the life of the scanner. However the scanner can simply and quickly recalibrated if the need arises.

A representative example of the calibration of the scanner will be better understood from Figure 10 and the following discussion. Figure 10 is a illustration of an arbitrary, unique ray $R_{n,m}$ which is projected from the projection system of the scanner onto the smooth, planar calibration surface 138 of Figure 8 during the first part of the calibration procedure. The ray $R_{n,m}$ is captured by the electronic imaging device 56, with the calibration plane positioned at two different distances from the scanner, Z1 and Z2. The distance between the two locations is $\Delta$Z. Distance Z1 need not be known, however the separation distance $\Delta$Z is known. The separation distance $\Delta$Z will vary depending on the depth of focus of the imaging optics 108 in the scanner 14.

Figure 10 illustrates a fundamental principle of the technique that is used for calibration of the scanner and generation of three-dimensional information of an object, which is considered to be an improvement over the calculations required by the method of Figure 9. Figure 10 illustrates that when the plane 136 is at a distance Z1, the ray $R_{n,m}$ impinges on the imaging device at the location 150. When the calibration surface 136 is moved to position Z2, the ray $R_{n,m}$ impinges on the detector at point 152. The pixel coordinates for ray $R_{n,m}$ at both positions is stored in a calibration table. In actuality, the pixel coordinates for a large number of rays from the projection pattern are stored for Z1 and Z2. These pixel coordinates, along with X and Y dimension measurements from a second part of the calibration procedure, give all the information needed to create a calibration table necessary to compute three-dimensional coordinates for an object that has been scanned with the projection pattern.

Ray $R_{n,m}$ corresponds to a single point in the projection pattern. Knowledge of where in the projection pattern ray $R_{n,m}$ originated from is required. Hence, some pattern recognition and decoding of the detected pattern is needed to identify the specific portions of the pattern that are being imaged by the various portions of the CCD electronic imaging device. To understand the pattern recognition process, the reader is directed to Figures 11, 12, 17, 18 and 19 and the following discussion.

Pattern Recognition

Figure 11 is an illustration of a portion of a pattern that is projected from the scanner of Figure 3 onto an object (including the calibration surface 138). The projection pattern comprises an array of parallel lines 156 separated from each other by red, green and yellow coloured dots 158, it being understood that other types of projection patterns are possible. The sequence of the coloured dots 158 vary along the length of the lines 156, and in the direction perpendicular to the lines. This technique is used such that using pattern recognition and decoding processes, described herein, every region in the projection pattern can be decoded from the pixel data from the imaging device. Figure 11 illustrates that $Ray_{n,m}$ can be taken to have originated at the intersection on one particular line and one particular coloured dot, and this location can be determined precisely form the pattern recognition and decoding process. Similarly, the ray along the line L one row below ray $R_{n,m}$ can also be identified. In the present example, the pattern is constructed such that there are N columns or lines, and M rows of coloured dots. Figure 12 illustrates showing that the various rays of light passing through the pattern of Figure 11 can be represented by an array of N x M points.

This array of points representing the projection pattern of Figure 11 is imaged by an electronic imaging device or CCD 56 arranged in an array of row and columns, as shown in Figure 14. There are X columns of pixels in the X direction and Y rows of pixels in the Y direction. In the illustrated embodiment there are 1,028 pixels in each direction.

Figures 17, 18 and 19 illustrate the pattern recognition process for captured two dimensional images. Figure 17 shows the signal level from one row of pixels in the electronic imaging device. The signal indicates that line L of the projection pattern is imaged at pixels 20-23 of a particular row of the pixels. Averaging of the pixel values over the four pixels allows the centre point of the line relative to pixel 21 to be calculated, with sub-pixel resolution. Figure 18 shows how the line L and adjacent coloured dots are imaged on the surface of the imaging device in this example. Note that the line is not necessarily centred over any column of pixels and hence the averaging must be performed to determine the centre point in the line. A similar pattern recognition process is performed for the coloured dots, as indicated in Figure 19. The centre of each coloured dot is located, as is the centre points of the lines for every line and coloured dot imaged by the imaging device.

The pattern recognition process thus takes the output signals of the imaging device (in the form of a coloured bitmap image) and returns a set of pixel locations for centres of lines and centres of particular coloured dots. The next step in the process is correlating these pixel locations for lines and coloured dots to particular lines and coloured dots in the projection pattern. This process is referred to as decoding (process 82 in Figure 6), and is described in detail below. Decoding is not normally needed during the calibration procedure described in conjunction with Figure 8, since the Z calibration surface is planar and the arrangement of the projection pattern on the CCD 56 is preserved. Decoding is used however during use of the scanner to scan an object of unknown surface configuration.

Decoding

The decoding process is the process of converting a set of pixel addresses for lines imaged by the imaging device, and a set of pixel addresses for particular coloured dots imaged by the imaging device, to particular lines and coloured dots in the projection pattern. Decoding is not absolutely required during calibration (particularly where the Z calibration surface is a planar surface). It is used, however, during processing of images on an object having undercuts, shadow features, or other irregularities. It may be possible to decode only a portion of the received pattern, since ordering of lines in the projection pattern is preserved. For example, if lines 13 and 16 are decoded, lines 14 and 15 are also decoded since their spatial relationship relative to lines 13 and 16 are preserved.

The imaging analysis process needs to know that a particular pixel is imaging a particular line or a particular coloured dot. The projection pattern or screen 50 (Figure 2) varies continuously in both directions, due to the unique and continually varying sequence of the coloured dots. The decoding process simply examines where the red, yellow and green dots are being imaged in the imaging device, and compares these results with the known sequence of red, yellow and green dots in the projection pattern, and thereby locates or identifies each ray with reference to the projection pattern. For example, the process knows that, for example, pixel 21 in row N of the imaging device is imaging the centre of line 13, row 55, in the projection pattern.

Referring again to the calibration set-up of Figures 8 and 10, the scanner takes two images of the Z-calibration surface 138, one at distance Z1 and the other at distance Z2. The pixel addresses where each ray $R_{n,m}$ in the projection pattern is imaged by the array is stored in a calibration table referred to herein as calibration table # 1, shown in Figure 24. At this point, we know how the imaging of the projection pattern varies as the calibration surface is moved in the Z direction relative to some imaginary plane Z1 in front of the scanner. However, the X and Y relationship is not yet known. Therefore, the scanner must be calibrated in the X and Y direction using a pattern of known geometry. This is explained in conjunction with Figures 8 and 13-16.

Figure 13 is a illustration of the X-Y calibration surface 140 of Figure 8, showing the array of Q x P points (tiny apertures 143) in the calibration surface 140 being organized into a coordinate system having an origin at the centre of the surface 140, in the shape of a cross 145. The calibration surface 140 is conceptualized as consisting of four quadrants I - IV. Figure 23 shows one possible numbering convention of the points in the surface at the origin. In the illustrated embodiment, the points of the X-Y calibration surface 140 are actually tiny apertures spaced from each other a known distance (e. g., 1 mm). The apertures act as a plurality of point sources of light when the light source 142 positioned behind the surface 140 is activated. These points of light are imaged by the electronic imaging device 56 during the second part of the calibration step. By counting pixel signals (indicating the imaging of a point source in the surface 140) over from the origin in the X and Y directions, it is possible to determine which point in the surface 140 is being imaged by which pixel, again with subpixel resolution. Since we know the address of the pixels which illuminate the specific portions of the projection pattern, and we can know the distance from the origin of the surface 140 that this pixel is imaging, it is therefore possible to calibrate the pixels in the X and Y directions. A second calibration table, shown in Figure 25, is used as an interim step to generate the distance values in the X and Y directions for the principal calibration table # 1 in Figure 24.

This process will be explained by example. Figure 15 is an illustration showing the interpolation of pixel addresses in X and Y directions for a given ray $R_{2,3}$ from a scanned object from two points of the X-Y calibration plane previously imaged by the electronic imaging device during calibration. Figure 15 indicates that a given point in the projection pattern, ray $R_{2,3}$ is imaged by some pixel that is located between the pixels that imaged points in surface 140 that are in quadrant III, between 14 and 15 points to the left of the origin and between 14 and 15 points below the origin. This is suggested by Figure 21, which shows where ray $R_{2,3}$ is imaged on the CCD chip. Figure 21 also indicates where the corresponding points on the X-Y calibration surface 140 are imaged by the pixels in the electronic imaging device. As is shown in Figure 21, in the present example the pixel values are between 30 and 90 in the X and Y directions.

Figure 25 shows the X-Y calibration table # 2 that is used for generating distance entries in X and Y directions in the calibration table No. 1 shown in Figure 24. Figure 25 illustrates that for each point in the X-Y calibration surface 140, corresponding pixel addresses for pixels imaging those points are identified and stored in the table. This is done for all points in the four quadrants of the X-Y calibration surface 140. These values are obtained when the X-Y calibration surface is positioned at both distances Z1 and Z2, and an image is generated at both positions. The entries in the table are pixel addresses in X and Y directions, expressed in sub-pixel resolution. Representative entries for Quadrant I are shown, it being understood that entries are made for all the points in the X-Y calibration surface.

Now, if we know that ray $R_{2,3}$ of the projection pattern from the Z- calibration procedure (using the calibration surface 138) is being imaged at a particular location, we can use the calibration table # 2 of Figure 25 to compute an entry in mm for table # 1. Again, using the present example, assume ray $R_{2,3}$ (corresponding to line 2, row 3 in the projection pattern) is imaged by pixel having an address of 30.2 in the X direction and 36.2 in the Y direction, at Z = Z1. The distance in mm can be calculated from an interpolation of the entries in calibration table 2 of Figure 25. This is indicated for the entries in line 2, row 3. At the Z = Z1 distance, this point is imaged at pixels 30.2 in the X direction and 36.2 in the Y direction, which corresponds to a distance of- 14.6 mm in the X direction from the origin and- 14.4 mm in the Y direction. Similarly, at Z = Z2, this point is imaged by pixels 43.0 in the X direction and 31 in the Y direction, which corresponds to a distance of- 14.8 mm in the X direction and- 15.8 mm in the Y direction. This information is entered into table # 1, as shown in Figure 26.

This interpolation takes advantage of a linear relationship that exists between pixel address and distance for objects at an unknown distance Z' from the scanner. This can be best appreciated from Figure 22. Since we know that ray $R_{n,m}$ is imaged at one point 160 in the imaging device at Z = Z1, and that it is imaged at another point 162 at distance Z =Z2, the ray must fall along the dotted line 164 where Z' is between Z1 and Z2. Similarly, if Z' > Z2 it lies along the line indicated at 166. If Z' < Z1, it lies along line 168. This linear relationship between distance and pixel address is the key to obtaining Z information as to ray $R_{n,m}$ and X and Y distance in mm. Furthermore, since $\Delta Z$ is known exactly during the calibration (e. g., 7 mm in the illustrated embodiment), and a linear relationship exists between pixel address and distance, the location of exactly where ray $R_{n,m}$ lies along the line 164, 166 or 168 tells us with precision the position of the unknown object in the Z direction, relative to virtual plane Z1. This location of where ray $R_{n,m}$ is being imaged by the electronic imaging device is arrived at by the pattern recognition and decoding processes described herein.

Referring again to the example of Figure 16 and 17, we know ray $R_{n,m}$ is imaged at some region of the CCD, and this is stored in table 1 (Figure 25). From calibration table 2 (figure 26) we know the X and Y coordinates of neighbouring points in the X-Y calibration grid in the region of ray $R_{n,m}$. By interpolation of the X and Y coordinates, we can calculate the distance in mm from the origin since the points in the X-Y calibration grid are separated from each other a known distance. This is done for all N x M portions of the projection pattern.

For example, calibration table 1 of Figure 24 tells us that ray $R_{2,3}$ is imaged at pixel addresses X = 30.3 and Y = 36.2 for Z = Z1, and at pixel address X = 43 and Y = 31 at Z = Z2. We then look to the table 2 entries (Figure 25) to find the closest X and Y points in the X-Y calibration grid by looking at the pixel addresses in table 2. This is shown in Figure 16. An interpolation of the pixel addresses in table 2 to the known pixel address from table 1 results in an X, Y address in mm. In the present example, X in mm =-14.6, Y in mm = 14.4. The results are now added to table 1, see Figure 26. The same is done for the distance Z = Z2. The same is of course performed for all the N x M rays in the projection pattern, resulting in a completely filled calibration table 1. Table # 1 in Figure 26 only shows the entries for ray $R_{2,3}$, but the process is done for all rays for Z = Z1 and Z = Z2.

Figure 20 illustrates the points of the X-Y calibration plane from the calibration station of Figure 7 at two positions Z1 ands Z2, relative to the optical elements of the electronic imaging device. It will be apparent that when the Z = Z1, some points in the X-Y calibration plane will not be imaged, but will be when Z = Z2. These points, indicated by $\Delta Q$ in the Q direction, exist also in the P direction. The calibration table 2 takes these into account. Some points in all four quadrants may not be imaged at Z = Z1, but will be imaged at Z = Z2 during X-Y calibration. Points indicated at 170 are imaged at both values of Z.

From the above, in one aspect, a machine-readable memory is provided for a scanner used to calculate three dimensional information of an object scanned by the scanner. The memory may be in the scanning unit itself, in a separate work station for the scanner, or in any computing device such as a remote computer that processes acquired image data and generates a three-dimensional model of the object. The memory comprises an array of data storage locations containing a calibration relationship for the scanner, such as a table. The calibration relationship identifies pixel coordinates for numerous portions of a pattern projected onto a calibration surface located at two different distances from the scanner, and distance information in X and Y directions for the portions of the pattern for the two different distances. The calibration entries stored in memory allow the scanning system to compute three-dimensional coordinates for points on an object reflecting the projection pattern onto the electronic imaging device.

Now that the scanner has been completely calibrated, it is ready to scan objects at some unknown distance and having some unknown surface configuration. The derivation of X, Y and Z coordinates for the surface will be explained in the next section.

Derivation of 3-D Point Cloud per Image (step 84, Figure 6)

With reference to Figure 6 and 26, we now explain the derivation of spatial coordinates in three dimensions for a single captured image. With the entries in Table 1 completely filled out during calibration, the scanner is now able to derive X, Y and Z coordinates for any object at an unknown distance. The scanner has the most accuracy when the distance is between the values Z1 and Z2, such that the captured images are in focus, but distances outside of this range may still be able to be imaged and decoded.

First, the electronic imaging device 56 captures an image and the image is subject to the pattern recognition and decoding, steps 80 and 82 in Figure 6, described in detail above. This process results in a set of pixel coordinates stored in memory for all the lines and coloured dots of the pattern that are projected onto the object and imaged by the pixels of the CCD. Comparison of the pixel addresses of the captured image with the entries in Table 1 in Figure 26 (when completely filled out during calibration) yields the coordinates of every point in the captured imaged, in three dimensions.

The process is as follows :

First, compute the Z value of every portion in the projection pattern found in the captured image using table 1, given the known line and row number of the portion of the pattern, and the associated pixel number. The unknown distance, Z', for any point, measured from the virtual plane Z1 is as follows:

$$Z' \text{ (in mm)} = \Delta Z \times \frac{\text{measured pixel \# - pixel \# for line and row of pattern at Z1}}{\text{pixel \# for line and row of pattern at Z2 - pixel \# for line and row of pattern at Z1}}$$

where $\Delta Z$ is the distance from Z1 to Z2 in the calibration set up described above.

Using ray $R_{2,3}$ as an example, if this ray is imaged at pixel #35 in the X direction, from table 1 the calculation is as follows:

$$Z' \text{ (in mm)} = 7.0 \text{ mm} \times \frac{35 - 30.2}{43 - 30.2}$$

where $\Delta Z$ is 7 mm.

$$= \Delta Z \text{ X } \sigma$$

Here, $\alpha$ = 0.375, a linear scaling factor.

Therefore Z'= 0.375 x 7 mm or 2.625 mm. The point on the object reflecting ray $R_{2,3}$ is 2.625 mm from the virtual plane Z1. The Z value for all other points in the object are also measured relative to a virtual plane Z1.

Now, Table 1 (Figure 26) is referred to determine the mm values in the X and Y direction for this point in space and another interpolation is performed to the mm entries in table 1. The calculation is as follows:

Since we know we have line 2, row 3 in the pattern (from the pattern recognition and decoding process), we need to interpolate the mm entries in table 1.

X value is between -14.6 and -14.8 $\Delta X$ = 0.2 mm

Y value is between -14.4 and -15.8 $\Delta Y$ = 1.4 mm

The true value of $X = X_{at\ Z1} - (\sigma \times \Delta X)$, similarly the true value of $Y = Y_{at\ Z1} - (\sigma \times \Delta Y)$.

Therefore:

The true value of X for ray $R_{2,3}$ = -14.6 - (0.375 x 0.2) = -14.675 mm

The true value of Y for ray $R_{2,3}$ = -14.4 - (0.375 x 1.4) = -14.925 mm

Summarizing, the X, Y and Z coordinates for the point in the object reflecting ray $R_{2,3}$ is
X =- 14.675 mm
Y =- 14.925 mm
Z =- 2.625 mm

These points are stored in memory 42 of the scanning work station 16, step 86 of Figure 6. This process is performed for every ray $R_{n,m}$ in the projection pattern that is recorded in the captured image. The result is a set of three-dimensional coordinates in space, referred to herein as a "frame", for each captured image. This set of points can be calculated in a small fraction of a second with a general-purpose computer.

The pattern recognition, decoding and 3-C coordinate calculation process will now be explained with reference to two-dimensional bitmap images of teeth. The process described below is the same for any object being scanned.

Part 3 Generation of Digital Impression

A complete three-dimensional model of the patient's dentition can be generated from the scanning system. The process requires an operator moving the scanner 14 (Figure 3) over the dentition, preferably *in vivo,* and generating a series of frames of images. The frames are obtained at a rate of at least one frame per second as the scanner is moved over the teeth. The scanning of an entire jaw may require three separate scanning operations or "segments" due to manoeuvrability constraints and a break in the capturing of images. While the scanning is occurring, the four steps of Figure 6 are performed for the stream of captured images. The end result is the storage of a set of frames in the main memory of the scanning work station 16. The frames can be registered to each other using one or more of a using a variety of frame registration techniques described in detail below. Once all the frames have been registered to each other, a complete three-dimensional virtual model of the patient's dentition is displayed for the orthodontist. This computer model provides a base of information for diagnosis and planning treatment. An introduction to the treatment planning aspects of the overall orthodontic care system is set forth in Part 4.

Figure 27 is an illustration of a two-dimensional bitmap image of a tooth and associated anatomical structures captured by the electronic imaging device 56 of the scanner of Figures 1, 2, 3 and 4, prior to any signal processing in the scanning work station 16. An inspection of Figure 27 indicates that the image includes various lines and coloured dots of the projection pattern, as it is reflected off of the tooth and associated anatomical structures. The location of where these lines and coloured dots are imaged on the imaging device 56 contains information as to the three-dimensional shape of the tooth and associated anatomical structure.

Referring back to Figure 6, the first step is a pattern recognition process on the captured image. Figure 28 is an illustration of the image of Figure 27 after pattern recognition and filtering operations have been performed. The filtering operation basically returns a zero pixel value for those pixels where the pattern recognition process cannot detect the lines and the coloured dots, for example due to the surface being out of focus (i.e., out of range of the scanner optics).

After the decoding operation is done on the image of Figure 28, the result is a set of pixel locations where the decoded rays $R_{n,m}$ of the projection pattern were imaged in the imaging device 56. Step 84 is performed for all such rays, using calibration table # 1 of Figure 26 stored in memory for the scanner. The result, step 86 of Figure 6, is a set of three-dimensional coordinates for all the points in the image, a point cloud comprising a "frame". Figure 29 is an illustration of a single "frame" of data, that is, a three-dimensional point cloud of a scanned object which has been calculated from a single two dimensional image by the pattern recognition, decoding, and 3-D calculations described herein.

Figure 30 is an illustration of the points of the cloud of Figure 29, in which three adjacent points of the cloud are joined together to form triangle surfaces. The usage of the triangle surfaces in a registration process is described below. Figure 31 is another view of the three-dimensional surface formed from the triangle surfaces shown in Figure 30. Figure 32 is a view of the surface of Figure 31, smoothed by a smoothing algorithm to give a smoother representation of the surface of the object. Commercially available off-the-shelf software exists for taking a set of three dimensional coordinates and displaying them on a computer monitor, and such software is used to display the three dimensional surfaces (if desired by the user).

Figure 33 is another example of a bitmap image obtained by the electronic imaging device of the scanner. Figure 34 is a plan view of the three-dimensional surface obtained from the two-dimensional bitmap image of Figure 33, after the steps of Figure 6 have been performed.

Figure 35 is a perspective view of the three-dimensional surface shown in Figure 34. The software programs enable the surface to be rotated in any degree of freedom, allowing for complete inspection and visual analysis of the surface.

Since the scanner and scanned object move relative to each other during capture of the scanned images, the three dimensional coordinates for a large number of frames will not agree with each other. In other words, the X, Y and Z coordinates for a given point on the object will change from frame to frame since the point was imaged from a different spatial orientation for each image. Hence, the frames have to be registered to each other to generate a complete overall digital model of the object. The present system provides for various registration procedures to be performed on the frames, to find a best-fit solution for coordinates of the object in one frame vis-à-vis coordinates of the object in other frames. These registration procedures are described in the following section.

Figure 36 is a flow chart illustrating the steps performed to generate a complete three-dimensional model of the dentition of a patient from a series of scans of the upper and lower jaws. The steps include an initial step 190 of determining an entry point into a registration process, a frame to frame registration process 192 of registering one frame to another, a segment registration process 194 to register segments (i.e., portions of a scan where the scan was interrupted for some reason) to one another, and finally a cumulative registration procedure 196 in which the frames are registered to all other frames to obtain a slightly more accurate model of the object than that obtained by frame to frame registration. It will be understood that depending on the application, the step 194 may not be required, a frame to frame registration may only be needed, or the user may only desire a cumulative registration and steps 192 or 194 are not performed at all.

The result of registration is a three-dimensional model containing all the points from the frames captured by the scanner. An example of such a model is shown in Figure 37A, where surface 199 indicates the sum total of points of all the frames in three-dimensional space. Figure 37B illustrates one small section of the surface, showing for example the points in three-dimensional space from three frames.

A preferred registration technique involves registering a set of points (three-dimensional coordinates) comprising a frame to a surface formed by a previous frame (or group of frames), rather than registration of one set of points to another set of points. This is due to a relatively coarse projection pattern used in the illustrated embodiment; the points can be low in density as compared to the curvature of the object. Figure 37C shows one frame from a scan of a tooth, with the points in the frame connected by lines to form a set of triangle surfaces. The coarseness of the projection pattern (and widely spaced points in the point cloud) is compensated by the fact that a given portion of the surface is captured by overlapping frames, and the registration occurs from the points in one frame to the surface in the previous frame or a surface defined by more than one previously registered frames. The registration process described herein ultimately permits a fine resolution in the three-dimensional model. This is indicated by Figure 37D, showing all the frames for the tooth scan registered to each other to create a very fine and high resolution virtual model of the tooth.

A. Entry point into Registration (step 190, Figure 36)

Registration processes require a starting point for fitting one frame, frame i to another frame, frame i + 1. The starting point, in the illustrated embodiment, is rough calculation of the offset between overlapping points in the frames in X, Y and Z directions. Whereas prior art systems have good pre-knowledge of the spatial relationship due to the known physical arrangement of the scanner and the object, the present system does not. The starting point is the initial assumption of spatial relationship between one frame and the previous frame (and one frame and a set of previous frames).

The method of calculation of the offset in X and Y directions is illustrated in Figure 38A-C. Figures 38A-38C are an illustration of a two-dimensional cross-correlation procedure in the X and Y directions. The procedure, along with the procedure in Figures 39A and 39B, is used to find an initial entry point into a registration algorithm between successive frames of data.

It can be seen from Figures 38A and 38B that frame i + 1 is moved in the X and Y directions from frame i. To obtain the amount of movement, ΔX and ΔY, the images are projected to both X and Y axes by adding all pixel values of every row, acquiring a one dimensional Y-vector, and adding all the pixel values of every column, acquiring a one-dimensional X-vector. This is performed for both frames, resulting in $X_{frame\ i}$ and $X_{frame\ i+1}$ and $Y_{frame}$ and $Y_{frame\ i+1}$. The vectors are smoothed to suppress the influence of the projected pattern.

To compute ΔX, the absolute value of the difference between each value of the X vector of frame i (frame i $-X_{frame\ 1}$) and the X vector of frame i + 1 (frame i + 1 $- X_{frame\ i+1}$) is calculated with a varying position shift within a range of -xa < k < +xe. The sum of these values represents the resemblance of $X_{frame\ i}$ and X $frame_{i+1}$ shifted by a certain amount k. The minimum value of k is determined. This result gives the shift or movement in the ΔX direction.

The same process is also performed the Y direction. As can be seen in Figure 38C, if frame i is moved by an amount ΔX and ΔY, the overlapping points in both frames will have the same values and the sum of the difference in pixel value will be approximately zero.

Figure 39A and 39B are an illustration of a one-dimensional correlation procedure in the Z direction for two successive frames, in the present example frame 1 and frame 2. Line 200 represents a three-dimensional surface of the second frame, formed by connecting three adjacent points in the point cloud. The set of points 202 represents the points of the point cloud representing frame 1. To compute the Z offset, ΔZ, the sum is taken of all the Z values of Frame 2, and the result divided by the number of points, resulting in an average Z value of Frame 2. The same is done for Frame 1. The difference between the average Z value of Frame 2 and the average Z value of Frame 1 is the Z offset, ΔZ. Figure 37B illustrates the result after translation of Frame 1 by the amount ΔX, ΔY and ΔZ. The result is that the points of frame 1 are quite close to the triangle surfaces of Frame 2. The values of ΔX, ΔY and ΔZ are stored for Frame 1 and used as an entry point to a registration procedure.

B. Frame to Frame Registration

Frame to frame registration is a process for registering one frame with another frame, that is, finding a best-fit in terms of translation and rotation make overlapping points in the frames agree with each other. If the frames are generated in sequential order, frame to frame registration refers to registration of the second frame to the first frame, the third frame to the second frame, from the fourth frame to the third frame, etc. Frame to frame registration can be performed very quickly. It can be performed in a manner such that the operator of the scanning system sees the results of frame to frame registration on the monitor of the scanning work station while they are still scanning the patient. What they see is an accurate three-dimensional representation of the dentition on the monitor, for those portions of the dentition that have been scanned thus far. As additional frames are obtained, they are registered to the previous frame and added to the computer model. When scanning is complete, the computer model can be rotated around on the monitor and inspected to see that all relevant portions of the teeth and anatomical structures have been scanned. The user thus gets immediate feedback on the results of the scanning using frame to frame registration.

Figures 40A-40D is a flow chart of a frame to frame registration process for a set of frames, each frame consisting of a three-dimensional point cloud of a scanned object. Each frame is typically generated from a different spatial orientation of the scanner relative to the object due to movement of the scanner during image capture, hence the frames overlap to at least some extent. The registration process is used to find a best fit between the frames relative to each other, and thereby provide a complete three-dimensional virtual model of the surface of the object from all of the frames. The end result of the frame to frame registration is a substantially exact three dimensional model of the scanned object. This object is represented by a large set of point coordinates in computer memory. The result is also represented as a set of transformation matrices providing information as to how each frame of points should be translated and rotated in three dimensions in order to fit to the previous frame.

The frame to frame registration process is also an iterative process. At the end of each iteration, a comparison is made as to how" close" the two frames are to each other in three dimensions. If they are not close enough (with "closeness" determined in absolute terms by a quality index, say in microns), another iteration is done, using the results of the first iteration. The frame to frame process may continue for tens or even hundreds of iterations, depending on how fine or precise the user wants the registration to be. The process stops when a best fit between two frames has been reached or a maximum number of iterations has occurred.

Referring now to Figure 40A-40D in conjunction with Figures 39 and 41-43, an initial step 209 is performed as an entry point into the registration procedure. In this step, the processes of Figure 39A and described previously is performed to make a Z-coordinate transformation of frame i -1 and frame i. Basically, this transformation is performed by summing the Z coordinate values of both frames individually, finding a median Z value for each frame, finding the difference or ΔZ value from these median values, and shifting one frame (frame i) by this amount to bring the Z coordinates of all the points in that frame closer to the points of frame i -1. The rationale of step 209 is as follows. The Z axis measurements of a surface represent a degree of freedom of the scanner movement. Scanner movements in the Z direction do not provide significant new information on the object being scanned. However, because of the focus optics of the electronic imaging device (108 in Figure 7), the visible surface of the object becomes slightly smaller or larger depending on how much the scanner is moved in the Z direction relative to the surface, while the centre of the surface (or, loosely speaking, the "centre of gravity") of the visible area basically remains nearly the same with Z direction movement. The Z coordinate transformation step 209 eliminates this effect by normalizing the Z surface coordinates. This process also makes possible the exclusion criteria of step 2 described below, by which non-overlapping points and stray data points are excluded from the registration process.

The registration procedure itself starts with step 1, 209 in Figure 40A. At this step, minimum distance vectors 248 (N1, N2,...) are calculated from every point in Frame i to the surface of frame i - 1. The surface of a frame can be obtained easily by connecting neighbourhood points to together with a triangle or other polygon. The minimum distance vector, for each point in Frame i, is defined as the distance vector having a magnitude which is the minimum of the following three vectors : the shortest vector from the point intersecting a triangle surface in frame i - 1 normal to the triangle surface; 2) the shortest vector from the point orthogonal to the edge of a triangle surface in frame i - 1, and 3) the shortest vector from the point to the nearest point in frame i -1. Most often, but not always, this will be a normal vector, type 1) above. In the example of Figure 41, minimum distance vectors 248 are computed from the points 250 in frame i to the triangle surfaces 252 of frame i - 1, with the vectors 248 normal to the surfaces 252.

At step 2 (212 in Figure 40A), three exclusion criteria are applied to the minimum distance vectors of step 1, in order to eliminate non-overlapping data points between the two frames and to eliminate stray data points. First, all minimum distance vectors that relate to a boundary element (edge or point) in frame 1 are excluded. Second, all remaining minimum distance vectors with an amount exceeding a certain predefined value R, likely indicating stray data points, are excluded. Thirdly, only triangle surfaces are taken into consideration which form the outside surface with respect to the scanner viewing direction. Every surface has by definition two sides. We regard the "outside" surface as the surface of the object that is oriented towards the scanner.

At step 3 (214) the vector sum of all the minimum distance vectors $N_1 ... N_N$ is computed. This is shown in Figure 42, with vector 254 representing the vector sum.

At step 4 (215), the median minimal distance vector (t) is computed by multiplying the vector sum 254 by the scalar 1/N. The median minimal distance vector basically constitutes a measure of how frame i should be translated in X Y and Z directions in order to better fit to frame i - 1. Now, the registration process needs to compute a rotation factor, which is explained by steps 5-8, to indicate how frame i needs to be rotated in order to better fit frame i - 1.

At step 5 (216), the X, Y and Z components of the median minimal distance vector is subtracted from every point in frame i. This is performed by making a copy of the frame i coordinates and operating on the copy as an interim step in the procedure, the underlying data from frame i is unchanged. At the same step the "center of mass" of the points of frame i which are not excluded by step 2 is calculated. The "center of mass" is defined as the vector sum of position vectors of all mentions points scaled by the inverse of the number of points.

At step 6 (218) a calculation is made of the cross product of two vectors for every point in frame i. With reference to Figure 43, the two vectors are as follows: 1) a position vector Vi extending from the origin 253 of the global coordinate system to the points in frame i, subtracted by the vector of the center of mass of the remaining points of frame i as calculated in step 5, and 2) the identified minimum distance vector Ni for that point.

At step 7 (220), a calculation is made of the vector sum of the cross vectors calculated in step 6, that is the net cross vector $\sum_j (v_j \times n_j)$ for all i points in the frame i, where x is the cross product operator.

At step 8 (222), the vector sum of step 7 is weighted against the inverse of the sum of all squares of the position vectors (Vi) of the points in frame i, to arrive at a rotation vector U. U is interpreted as follows: The direction of U gives us the rotation axis and the magnitude of U is the angle or amount of rotation. In particular, if we consider Vi to be the position vectors from the origin of the coordinate system to the vertex of every point, and Ni being the minimal distance vectors defined above, then the weighting is as follows :

$$U = \frac{\sum_j (v_j \times n_j)}{\sum_j v_j^2}$$

The reasoning behind this weighting is as follows. If you imagine the distance vectors as the realization of linear spring elements, the vector sum of the cross products represents the aggregate moment, or rotational discrepancy, generated between both frames. In the case of small deviations between the position of frame i and its final position, it can be assumed that the rotational moment also determined the direction of the necessary adjustment. The scaling with the help of the inverse of the sum of the squares of the position vectors considers the global extension of frame i. That is, the larger the distances of the points from the center, the larger is the ratio of rotational moment and angle between the present position and the target position. In a global sense, the mentioned factor (inverse of the sum of squares of position vectors) describes this ratio.

The derivation of the proper scaling factor is by no means an exact calculation. It has, however, turned out that using this factor in all empirical cases, the iteration of defining overlapping areas and execution of transformations converges.

At step 9, the result of step 8 is scaled with an empirical "acceleration factor" f. The factor f serves to possibly accelerate this convergence. A value of f of greater than 1 is appropriate for relatively large rotational displacements, but in any event has to be determined empirically.

At step 10 (226), the result of step 9 is interpreted as an axis of rotation, the magnitude of which indicates the amount by which frame i has to be rotated in order to make the local overlapping areas of both frames lie within each other. The magnitude of the rotation vector is interpreted as the angle around which frame i has to be rotated.

A rotation transformation matrix [T] (R) is calculated for frame i. This formula shows how to convert the rotation vector resulting from step 9, where β is the original length of the net cross vector which equals the angle of rotation that is required to fit the overlapping areas of frame i to frame i-1 and u is the unit vector of U, , $u = \frac{U}{|U|}$ with components $u_x$, $u_y$, $u_z$.

$$[T](R) = \begin{pmatrix} (1-\cos\beta)u_x^2 + \cos\beta & (1-\cos\beta)u_x u_y - u_z \sin\beta & (1-\cos\beta)u_x u_z + u_y \sin\beta \\ (1-\cos\beta)u_y u_x + u_z \sin\beta & (1-\cos\beta)u_y^2 + \cos\beta & (1-\cos\beta)u_y u_z - u_x \sin\beta \\ (1-\cos\beta)u_z u_x - u_y \sin\beta & (1-\cos\beta)u_z u_y + u_x \sin\beta & (1-\cos\beta)u_z^2 + \cos\beta \end{pmatrix}$$

To obtain a unique transformation operator for calculating the translation and the rotation transformation in a closed manner a 4x4 matrix representation is used. The relation between the 4x4 representation and the three dimensional rotation represented by 3x3 Matrix [T](R) is as follows

$$[T_4](R) = \begin{pmatrix} [T](R) & 0 \\ 0 & 1 \end{pmatrix}$$

and between the 4x4 representation and the three dimensional translation, represented by vector (t)

$$[T_4](t) = \begin{pmatrix} 1 & 0 & 0 & t_x \\ 0 & 1 & 0 & t_y \\ 0 & 0 & 1 & t_z \\ 0 & 0 & 0 & 1 \end{pmatrix}$$

two apply this 4x4 matrices on the three dimensional vectors the following convention is made: A three dimensional vector is transformed into the 4 dimensional vector space by identifying the first three components of the 4 vector with the components of the three dimensional vector but the fourth component is always unique. $(x, y, z)^T \rightarrow (x, y, z, l)^T$

At step 11 (228 in Figure 40 C), a transformation matrix for frame i, [T4](i), is calculated by multiplying the rotation matrix [T4] (R) (from right) by the translation matrix [T4](t) from step 4 $[T_4](i) = [T_4](R)\ [T_4](t)$.

Alternatively, the point cloud of frame i can be separately and independently operated on by the rotation matrix and the translation vector.

At step 12 (230), a calculation is made of the square root of the sum of the squares of the minimum distance vectors calculated in step 1 (210) of Figure 40A, which indicates the closeness factor quality of the registration in this iteration, value MA below. At step 12 (232) the closeness factor MA is compared to a quality index or threshold indicative of a successful registration (e.g., 50 microns). If the value MA is greater than the quality index, the registration process is repeated another iteration. All of the points in the ith frame are updated by operation of the transformation matrix $[T4]_i$, as indicated at step 234. The process goes back to step 1 as indicated at 236, and another iteration is performed of steps 1-13.

If the closeness factor MA is less than the quality index, the registration process proceeds for the next frame. As shown in Figure 40D, at step 14 the process retrieves the next frame (frame i + 1) and frame i after application of the transformation matrix [T4] i. At step 15 (240), the iterative process of steps 1-14 is repeated for frame i and frame i + 1. This process continues until all the N frames have been registered, as indicated at step 16 (242). At the end of the process, the result is a set of points comprising the three-dimensional model, and a set of transformation matrices [T4] to $[T4]_N$ for each of the frames (other than the first frame, frame 1), which are used to generate the three dimensional model. One frame, such as frame 1, the first one generated, is chosen as the starting frame for the registration and there is no transformation matrix for that frame.

Figure 45 illustrates that a range variable R can be used to filter or limit the number of points in a frame that are used when calculating net normal vectors, triangle surface, cross vectors, etc. The purpose of the range variable R is to screen out stray data points far from the triangle surface of the previous frame. The stray data has the effect of skewing the entries of the transformation matrix and increasing the number of iterations until the quality index is met. R can be for example 1 mm, or much less such as 1/50 mm.

Figure 46 illustrates two ways in which the frame to frame registration process may terminate. As shown by line 260, the process may terminate when the closeness factor goes below the threshold, here 30 microns. Alternatively, the closeness factor may level off at some value above the quality index, despite further iterations of steps 1-13 of Figure 40. This is indicated by line 262. When the closeness factor MA does not improve by at least some amount per iteration (say 1 percent) after 10 iterations, the process is deemed complete. Here, the designation x indicates the iteration in which the amount of improvement in the quality index from the previous iteration to that iteration was first less than a threshold, e.g., 1 percent.

C. Cumulative Registration of Entire Jaw

As noted above, cumulative registration is an alternative or improvement to a frame to frame registration. The difference between the two is that frame to frame registration only registers one frame to one other frame, whereas cumulative registration is a registration of a frame to more than one other frame, such as where one frame is registered to all previously registered frames. There are numerous types of cumulative registrations that can be performed, and a few examples will be given here. An advantage of cumulative registration is more accuracy in the resulting three-dimensional model. The disadvantage is that cumulative registration can be significantly more computationally intensive, and therefore require more time to be performed using currently available low cost microprocessors or com puters.

Figures 47A and 47B are a simplified illustration of one possible cumulative registration procedure. Figure 47 A shows triangle surfaces of frame 2 and points of frame 1. The points and surfaces represented in Figure 47A actually are in three dimensions. After registration of frame 2 to frame 1, we are ready for frame 3. Unlike frame to frame registration, frame 3 is registered to both frame 1 and frame 2. In other words, triangle surfaces are formed on the points of frame 3 and minimal distance vectors are calculated for each of the points in frame 1 and frame 2 (after registration to frame 1), and the other steps of Figure 40 are performed. As each new frame is retrieved, it is registered to the total sum of points from all the previous frames, after the previous registration.

Figure 48A-48C are a flow diagram of one possible cumulative registration process. At step A (270), the points of the first frame are retrieved from memory.

At step B, the points of the second frame are retrieved from memory.

At step C, the transformation matrix $[T]_2$ is retrieved for frame 2. This process assumes that the transformation matrix for each frame has already been generated, such as after a frame to frame registration process has been performed.

At step D, the transformation matrix $[T]_2$ is applied to frame 2.

At step E, a registration is performed of the points of frame 1 to the points of frame 2, after the transformation matrix $[T]_2$ has been applied to frame 2. Basically, the steps 1-11 of Figure 40A-40B are performed.

At step F, a check is made as to whether the quality of the registration is less than a threshold. If not, the registration is performed again (with the points of frame 2 updated by a new transformation matrix). Steps 278 and 280 are performed over and over again until either the quality threshold is met or a maximum number of iterations has occurred.

If the index is met or the maximum number of iterations has been reached, the process proceeds to step G (282). The new transformation matrix for frame 2, designated $[T]_2'$ is obtained and stored.

At step H, the new transformation matrix $[T]_2'$ is applied to the points of frame 2.

At step I, the new transformed points of frame 2 are added to a "global container". The global container is merely memory locations containing the points from frame 1 and the points of frame 2 as transformed.

At step J, frame 3 and its transformation matrix $[T]_3$ is obtained from memory.

At step K, the transformation matrix $[T]_3$ is applied to the points of frame 3.

At step L, a registration is performed of frame 3, as transformed, to all the points in the global container. Steps 1-11 of Figure 40 are performed.

At step M, a check is made to see if the quality index is below the threshold. If not, another iteration of the registration process is performed. This repeats until the quality index is below the threshold or a maximum number of iterations is reached.

If the threshold is met (or the maximum number of iterations is reached), the process proceeds to step N. The new transformation matrix $[T]_3'$ is obtained and stored in memory.

At step O, this new transformation matrix is applied to the points of frame 3.

At step P, the points in frame 3 after the transformation operation is performed are added to the global container.

At step Q, the process of steps A-P of Figure 48 are performed for the rest of the N frames.

At step R, all the transformation matrices $[T]_2'$ ... $[T]_N'$ are stored on the hard disk of the back office server. These transformation matrices are used whenever the finished global container (complete three- dimensional model) needs to be generated again at a later date (or on another workstation) . The model is generated by simply applying $[T]_2'$ ... $[T]_N'$ to the raw frame data comprising frames 2... N.

At step S, the global container is displayed to the user. This can be on the monitor of the back office server 28 or on the monitor 20 of the scanning station 16 (Figure 1). Since the global container is a digital representation of the object, it can be transported across a computer network and displayed and shared by another terminal. For example, where the back office server has a connection to the Internet, the model can be transported over the Internet to the precision appliance service centre 26 and displayed there. It can also, for examples be shared among various orthodontic, periodontal or dental specialists so that they can collectively study the patient remotely and cooperatively plan care.

Figure 49 is an illustration of a set of frames, illustrating a variation from the cumulative registration set forth in Figure 48. In Figure 49, a different order of frame registration is performed from that of frame to frame registration. In particular, frame to frame registration and cumulative registration of Figure 48 are performed in the order in which the frames are obtained. This need not be the case. In fact, a more accurate registration may be obtained by registration of frames in an order based on "neighbourliness", that is, based on the concept that all frames imaging a particular portion of an object should be registered together (or in sequence such they are registered one after the other). The order of registration is indicated by the left hand column of numbers. The right hand side of Figure 49 merely illustrates that each frame consists of X, Y and Z coordinates for a set of points. The frames need not and usually will not have the same number of points.

In Figure 49, the registration order is based on the location on the surface of the object for a given frame relative to the location on the surface for other frames. Figure 50 is a simplified illustration of a set of frames, showing the order in which the frames were obtained, with the neighbourliness of the frames relative to other frames being the basis for the registration order shown in Figure 49. Frame 7 (F7) is the chosen starting frame. So, the frames surrounding frame F7 are registered next. This is shown by the order of registration being F7, F3, F4, F11, F12, F6,... as indicated.

Figure 51 is another illustration of a set of frames, with registration of frames performed in accordance with the method of Figure 49. The order of registration is indicated by the column of numbers to the right of the frames. The marking in frames 2, 3, 6 and 7 etc. indicates that those frames have been registered. The marking is just a way of illustrating that the computer keeps track of which frames have been registered, as a check to insure that no frames are omitted during the registration procedure of Figure 49 and 50. The manner of selecting the other frames, e.g., frame 4 and 5, can be based on a number of criteria, such as the order of obtaining the frame, the neighbourliness to other frames already registered, and so forth.

Figure 52 is an illustration of cumulative registration based on the first captured frame (F1) as being the base line for all successive registrations. This is essentially the technique of Figure 48. Figure 53 illustrates an alternative registration procedure in which each frame in the set of frames is registered to a cumulative registration 3-dimensional model of the object, in sequential order, with one iteration of the frame registration process. This is followed by an updating of the cumulative 3-dimensional model and a repeat of the registration process with updated values for the transformation matrix [T] for each frame. The process continues until the quality values are within acceptable limits, or after a predetermined number of iterations have been performed. Still other possibilities for cumulative registration exist. The choice of which one to use will depend on the available computing resources, the amount of time required to perform the cumulative registration for the technique, and the desired accuracy.

Figure 54 is a screen shot of a workstation computer (e.g., either a scanning station or back office server workstation), showing the available registration parameters and variables that can be changed to optimize the registration when performing either a frame to frame registration or a cumulative registration. The parameters may vary widely depending on the type of object being scanned, the amount of time needed to obtain a result from registration, the speed at which the scanner is moved relative to the object and the amount of overlap, etc. Figure 54 illustrates that the user is able to select and modify the registration procedure parameters as they see fit. Two different types of registration are indicate here, a "raw" registration, in which the quality index ("distance limit") is 250 microns, and a fine registration, wherein-the quality index is reduced to 50 microns. The distance limit is computed as the square root of the sum of the squares of the normal vectors divided by the number of points in the frame. The term "stationary count" indicates the number of iterations to continue of little or no improvement in the quality index is seen. The Radius value refers the filter R shown in Figure 45. The convergence factor 0.10 refers to the minimum amount of improvement needed between successive frames before a stationary count commences. The convergence factor is computed by taking the difference of the squares in the quality index of the ith iteration and the i - 1th iteration and dividing by the square of the quality index of the ith iteration.

The number of points to register indicates the minimum amount of overlap in points (within boundary R) needed to attempt a registration. An "accelerate" factor is shown, with a value of 1.6. This means that the points are moved in the X, Y and Z directions in the transformation matrix by an amount of the net normal vector multiplied by the accelerate factor. The use of an accelerate factor has been found to reduce the number of iterations required to meet the quality index.

The maximum iteration count value is a stop value to keep the process from running into an endless loop. The overlap size value is a limit, in terms of $mm^2$, of the size where registration is performed. This serves to screen out stray points from the registration algorithm. The minimum quota of active points is a minimum amount of overlap between two frames before registration will be attempted, expressed as a fraction of 1. The maximum triangle size is a filter to filter out triangle sizes where the size of the triangle is too large, indicating a stray data point. The maximal edge length is simply the maximum permissible length of one side of one of the triangle surfaces. The Maximal count of unsuccessful files is the number of unsuccessful sequential registrations before a failure of the registration process will be declared.

Figure 55 is a screen shot from a workstation computer showing a frame to frame registration in accordance with Figure 40 for two frames in a set of frames. The various parameters shown in Figure 54 are selected and used in the frame to frame iteration. In this instance, frame 47 is being registered to frame 46. The surface of frame 46 is shown in white, frame 47 is shown in dark tones. The left hand side of Figure 55 shows the results of each iteration, including the running time, the number of the iteration, the number of overlapping points, the overlap between frames (U), expressed as a fraction of 1, the quality index MA, and the value of the filter R. After 3 iterations, the quality index for coarse registration was met. The process continued with the fine registration. A series of fine registration iterations were performed. Note that the quality index MA improves with each registration iteration.

The data from the last twenty iterations, and the final result, of a registration of frame 2 to frame 1 in a typical scan of teeth are shown in Figure 56. After 45 iterations, the distance limit of 30 microns was met (MA = 27.686 microns). Note that the graphical representation of frame 1 (white) and frame 2 (darker tones) is such that there is essentially an equal amount of frame 1 and frame 2 in the picture. This indicates that a "best fit" between frames 1 and 2 has been achieved.

D. Segment Registration

When scanning any object, such as teeth, the situation may arise in which the operator of the scanning cannot capture all the surfaces of the object in one scanning pass. The interruption may be due to the need to physically move the scanner to a location that is impossible to reach from one location, the need for the patient to take a break from the scanning, or some other reason. When scanning teeth of a single jaw, the scanning is typically performed in two or three different segments. First, one side of the jaw is scanned, then the front of the jaw, and then the other side of the jaw. In this situation, there are three different segments of the object. All the frames of each segment are registered to each other, typically using a frame to frame registration. Then the segments are registered to each other. After this has been done, a cumulative registration is performed of the entire jaw.

To perform the segment registration, there must be some way of indicating where at least one point in one segment is common to another segment. Segment registration thus requires some overlap between segments. The scanning workstation provides a mechanism to indicate at least one point where two different segments overlap. In the case of the scanning of teeth, the operator of the scanner will typically include the canine teeth in scans of both sides of the jaw, and in the scan of the front of the teeth. The operator can also be instructed to scan these teeth in the, side and front segments. Therefore, the segment registration proceeds by the user selecting or indicating a point on the canine teeth to use for performing segment registration. A procedure referred to herein as "landmarking" is used to select the point used to register segments. It will be understood that a similar process will be performed when scanning other types of objects where more than one segment was used to completely scan the object.

E. Landmarking

Figure 57 is a screen shot showing a graphical representation of a three-dimensional model of a patient's upper front teeth (segment 1) after a frame to frame registration of this segment. The user is applying landmarks to the canine teeth as a preliminary step in treatment planning, and as a step in registering overlapping segments of a scanned upper jaw relative to each other to calculate a complete model of the upper jaw and associated dentition.

The purpose of the landmarking shown in Figure 57 is to select a point on the canine teeth which is common to the front scan and the two side scans. The landmarking is also done at a point on the labial surface of the teeth that would be a suitable location for placement of an orthodontic bracket as part of an appliance to correct a malocclusion. The landmarks are characterized by both a point location and an orientation. To place the landmarks, the user clicks on a tooth number, indicated by the row of numbers 301, and drags the cursor with a mouse to the surface on the canine teeth where they wish to place the landmark. They then release the cursor, and the landmark 302 appears on the tooth. The landmark has an arrow 304 which must point to the incisal edge of the tooth. The user can rotate the landmark to place the arrow in the proper orientation by simply clicking on the landmark and turning the mouse one way or the other. As each landmark is placed, a box below the tooth number is highlighted as indicated at 306.

The tooth numbering convention shown in Figure 57 is as follows: the first number indicates the quadrant of the patient's dentition, with 1 being upper right, 2 being upper left, 3 being lower left, 4 being lower right. The second number is the tooth number with 1 being the incisor. Thus, the landmarks are placed at teeth 13 and 23, the upper canines.

Since these canines overlap their respective side scan, and since the X, Y and Z coordinates of the point on the labial surface of the tooth where the landmark is placed is assigned in the computer, it is now possible to register the front segment shown in Figure 57 to the two side segments. This segment registration is now performed. The overlapping frames between each segment can be registered to each other, or to the entire other segment.

After segment registration is performed, a cumulative registration of the entire jaw is performed in accordance with the procedure of Figure 48. After the cumulative registration is performed, the virtual three-dimensional model of the entire jaw is presented to the orthodontist on the monitor in the back office server workstation 28.

Note that if the scanning is done in one pass, e.g., where it is performed on a plaster model, there is no need for segment registration. The landmarking step can be eliminated in that event, although it may nevertheless be performed as a step in placing virtual brackets on the teeth objects of the virtual model.

In planning treatment for the patient, the orthodontist conceptualizes teeth as individual teeth objects that can be moved independently of each other to correct the patient's malocclusion. Furthermore, orthodontists are trained to make physical models of the patient's dentition from an impression, cut the teeth from the model, and then individually move the teeth relative to each other to provide a target situation which corrects for the malocclusion. Therefore the back office server workstation 28 includes interactive treatment planning software which enables the orthodontist to do this with the virtual three-dimensional model of the patient's dentition. In order to do this treatment planning, it is highly desirable therefore to process the three dimensional model resulting from a cumulative registration by separating the teeth from the gums and other anatomical structure, and presenting just the crowns of the teeth to the orthodontist. This allows virtual individual teeth objects to be moved independently in three dimensions on the computer. This process of separation of the teeth from the cumulative registration into individual teeth objects will be described next.

The separation process described below has one further advantage, namely requiring less memory to represent an individual tooth. Cumulative registration may result in an extremely large number of points from a large number of frames to represent any given tooth. The separation process, as described below, reduces this data set to a single set of points that describe a single surface representing the surface of the tooth. Much less memory is required. Consequently, the treatment planning software can process treatment planning steps for the teeth more quickly.

F. Separation of Teeth into Individual Tooth Objects (tooth modelling)

Figure 58A-58F are a series of illustrations showing the generation of an individual tooth model from a scanned tooth. The process will now be explained in detail.

Figure 58A shows the scanned dentition and associated anatomical structure surrounding the tooth 308. This tooth is tooth number 14 in the numbering convention shown in figure 57. The back office server workstation stores a three-dimensional virtual template tooth object for each tooth in the maxilla and the mandible. The template tooth 310 for tooth number 14 is shown in Figure 58B. The template tooth object 310 is a three dimensional tooth object having a single set of points defining the boundaries of the tooth. As shown in Figure 58C, the template tooth 310 is positioned approximately in the same location in space as the tooth 308. The landmark 302 assists in providing the proper axial rotation of the template tooth to have it fit properly with respect to the tooth 308. The template tooth is placed at the point cloud of the dentition according to the labial landmark 302. The template tooth can be scaled larger or smaller or positioned arbitrarily by the user, in order to get a close a position as possible to the point cloud of the dentition.

As shown in Figure 58D, vectors are drawn from the points on the template tooth to the scanned point cloud of the tooth 308. Every ray intersects several surfaces, depending on how often the respective part of the surface has been covered during scanning. For each vector, a surface is selected. Preferably, the smallest triangle surface is selected, since this surface corresponds to an image taken by the scanner when the scanner was positioned in a more perpendicular orientation to the dentition surface, resulting in more accuracy in the determination of the coordinates of that portion of the surface. As another possibility, the outermost surface is selected, using a filter to insure that no extraneous surfaces are used. These points of the surfaces intersected by all the vectors are combined as newly generated triangle surfaces and therefore form one consistent surface shown in Figure 58E. Then, finally, missing parts of the tooth are completed from the template tooth. The result is shown in Figure 58F. In a second pass, this generated object is then used as a template tooth, and the steps indicated by Figure 58C-58F are repeated in an iterative fashion. This is done to make sure that the algorithm works even if there are significant differences between the original template tooth and the scanned point cloud, e. g, a gap in scan data, different geometry in the tooth. The goal is to provide an algorithm that does not required a closely fitting template tooth object.

The final result, an individual three-dimensional virtual tooth object 312, is then displayed to the user, as shown in Figure 59. The result may be displayed on the workstation user interface as a three-dimensional superposition of the original data (white) and the separated model of the tooth (darker tones or contrasting colour). These tones allow the user to ascertain whether there is an even distribution of white and dark tones, indicating good fit between the scanned tooth 308 and the individual tooth object 312. This step may be automated by an algorithm detecting the difference (or the sum of the differences), and repeating the process if the difference is too great.

This process is of course performed for all the teeth. The result is a set of individual tooth objects for all the teeth in the patient's dentition. The teeth can be displayed either alone, or in conjunction with the surrounding anatomical structures such as shown in Figure 59.

Some human interaction is used in the embodiment described above in context of Figure 58. While the process could be performed for all the teeth in both arches on the workstation at the orthodontic clinic, that is not necessary. In particular, since the virtual model of the dentition and the template teeth exist as digital data in memory, they can be transported to a remote location and the task of separation of the dentition into virtual teeth objects could be performed at another location. This has the advantage of not tying up the back office workstation or server 28 in the clinic unduly, and requiring less labour at the clinic. We therefore contemplate that the function could be performed as a service of the precision appliance service centre 26 of Figure 1, or perhaps even by some other entity or service provider equipped with the necessary computer hardware and software. Once the virtual tooth objects are obtained for all the teeth in the dentition, the set of virtual tooth objects could be sent over the Internet back to the clinic for treatment planning and other purposes. It would also be possible for the entity performing the separation of tooth objects to also present an initial proposed treatment to the orthodontist (such as a target situation, location of brackets, and design of orthodontic archwire), and let the orthodontist take the process from there or simply indicate her approval.

Separation of teeth from the virtual model of the dentition could also be performed automatically using algorithms to detect incisal edges of the teeth, grooves between teeth, and grooves indicating the intersection of the gums and the teeth.

Two types of errors can occur when separation of teeth objects from other structure (e. g., other teeth and gums): 1) the data is selected for a tooth that does not in actuality belong to the tooth, such as gums and adjacent teeth, and 2) data that does belong to the tooth is ignored.

We address the first problem by providing an erase mode on the workstation software that is performing the modelling process. In this mode, the user is provided with a tool that erases triangle surfaces from the 3-D data, e. g., by highlighting unwanted areas with a mouse and clicking an erase icon or other similar technique. As each tooth is modelled individually, parts of the data that represent the unwanted data, e.g., data belonging to other teeth or gingival tissue, are eliminated from the tooth. This is only a temporary process ; it is used only to model that tooth and underlying scanned data is preserved. When modelling the adjacent tooth, that data is used again. The erasing process can be performed directly on the original scan data. However, this can be inconvenient since the original scan data can consist of a huge overlay of data.

As an alternative, and more preferred approach, the user works on a tooth model that has already been created and consists of one shell of triangles. Thus, the erasing mode would be used for example after one iteration of the process of Figure 58A-F. The selection and erasing process is much faster. The modelling algorithm calculates the surfaces to be deleted from the model in a single pass. The remainder of the iterations of the process of Figure 58 can typically be performed without any further erasing.

As another alternative for elimination of unwanted data, cutter plane tools can be provided on the workstation software to assist the user in selection of correct tooth scan data. The activation of this feature is shown in Figure 64A. In this technique, two planes 1000 and 1002 are superimposed on the scanned virtual model 18 of the dentition. The user is able to translate or rotate the planes 1000 and 1002 in any orientation in space using suitable navigation tools on the workstation 28 user interface. The planes may be in different colours. The planes serve as boundaries for the selection of tooth scan data as an individual virtual tooth object, or as part of the iterative procedure of Figure 58. All 3-D data that is outside of the planes 1000 and 1002 is ignored. The intention of the planes of Figure 64A is to simulate the physical process of cutting a physical model of teeth into discrete tooth objects.

Figure 64A indicates that the two planes may not work perfectly since teeth are curved or crooked at the contact area between adjacent teeth, and the plane 1000 may in fact intersect two different teeth. In Figure 64A, the area in region 1004 indicates where some triangle surfaces from the adjacent tooth 308A are included in the region between the two planes 1000 and 1002. These parts of tooth 308A can be manually erased by the erase mode feature described above.

Another possible method for separation of the teeth, without including extraneous anatomical structures, involves allowing the user to click with a mouse multiple points on the surface of the tooth where the tooth intersects adjacent anatomical structures. In Figure 64B, the user has highlighted areas 1006 on the tooth 308 where the tooth intersects gingival tissue. As each area is highlighted and selected (e.g., by a click of the mouse), the software records the coordinates of points associated with these areas 1006. Then, a series of planes are constructed which connect these points (or surfaces) together. These planes 1008 are indicated by the hatched area of Figures 64C and 64D. The planes 1008 serve the same functions as the cutter planes 1000 and 1002 of Figure 64A ; i.e., they define a boundary separating the tooth from associated non-tooth anatomical structures, such as gums and other teeth. Depending on the anatomy of the patient, it may be necessary to highlight closely-spaced areas, as shown in Figure 64B, so that the planes 1008 match the contours of the gum and tooth.

Referring now to the second problem, the tooth separation process of Figure 58A-F can be forced to use proper data that would otherwise be ignored. Specifically, the user clicks certain areas where original scan data has been wrongfully ignored. Clicking on the area forces the modelling algorithm to pick original data points from the scan including the selected areas. For example, region 1010 in Figure 64D has scan data associated with it, but such data was ignored in a frame to frame registration process. The user highlights this area and points for those areas are filled in from the original scan data for pertinent frames covering this area.

To allow for a safe operation of this user interaction, the modelling algorithm will internally mark or classify each generated point in the virtual tooth model as being based on scan data (true points), or if it has been constructed by the algorithm due to the lack of data (artificial points, supplied by the template tooth 310 in Figure 58B). A lack of data will always occur in the spaces between teeth since the scanner cannot usually capture images of the gaps between teeth effectively. A lack of data can also occur due to improper scanning. The lack of data can be cured to a certain extent by the modelling algorithm of Figure 58, with the lack of data supplied by the template tooth, e.g., in the gaps between teeth, and adapting this template tooth to the scanned dentition as described above. Artificial points can be marked as such and displayed in a different colour or using lighter or darker tones. The manipulations of the user described above for wrongfully ignored data will have effect only on the artificial surfaces.

Missing data or gaps from the tooth scan can also be filled from the template tooth using a simple interpolation procedure, such as shown in Figure 65. The example of Figure 65 will be given in one dimension (Z); a similar process occurs in the other dimensions X and Y. As shown, the scan data for the tooth 308 includes a gap indicated at 1020. The template tooth includes a surface 1022 that corresponds to this gap. To fill in the surface, the distance in mm is determined between the point at the left hand edge of the gap 1024 and the template surface 310 in the Z direction (0.6 mm). The same is done for the right hand edge 1028 (0.9 mm). A mid point is chosen 1026 and the values are averaged to arrive at the distance for point 1026 (0.75 mm). This process is repeated for intermediate points as indicated. After a suitable number of interpolations, the points in the template tooth 310 are moved in the Z direction by the measured or calculated amounts, here 0.6 mm, 0.68 mm, 0.75 mm, 0.83 mm, 0.9 mm. These points are now connected by lines to form triangle surfaces to complete the surface of the tooth 308.

The tooth model, once created, can be modified to simulate various treatments that may be made on the tooth, such as interproximal reduction, or grinding portions of the tooth away, by using the erase mode, clipping planes similar to the planes 1000 and 1002 of Figure 64A, or some other convenient means.

The library of standardized template teeth described above is based on standard tooth models for each teeth in one possible embodiment. The library described above could be augmented to include a library of teeth based on the ethnicity, sex, age, or other factors. For example, the library could consist of one library of template teeth for Hispanics, another library of template teeth for Orientals, a third library of template teeth for Caucasians, etc. These libraries could be supplied with the treatment planning software, obtained from a third party, or even created from patient scans. For example, after each scan using the present system, the software cuts the teeth into individual tooth objects using the process described above and stores the teeth in memory, along with identifying indicia such as the race, sex and age of the patient. Over time, a large collection of virtual tooth objects for various types of patients will be obtained. These virtual teeth can be registered to each other for a given ethnic type, on a tooth by tooth basis, to result in a new library of template teeth for a given ethic group, which are stored in memory. The user can thereafter identify for the software which library of template teeth to use for a new patient based on the relevant criteria such as ethnicity.

Alternatively, in some situations it may be desirable to use a contralateral tooth as the template tooth, and not a template tooth from the library of template teeth. In this situation, a tooth object of one of the contralateral teeth is obtained from a template tooth in a library and the cumulative registration scan. Then, the tooth object of the contralateral tooth is obtained by using the contralateral tooth as the template tooth and the cumulative registration scan as explained above.

In some situations, the template tooth may need to be modified. A few example of such situations are a partially erupted molar, a tooth that has been chipped, or a tooth that has been the subject of extensive previous dental work. Since the template tooth exists as a mathematical model, it can be displayed on the user interface and modified. The modification can be made using suitable navigation software and a clipping or erasing feature to delete part of the model. One way is providing a clipping plane feature, by which a plane intersects the template tooth in a orientation and location defined by the user using suitable navigation tools. The portion of the template tooth on one side of the plane is deleted. The user positions the plane at the desired location on the template tooth to roughly match the anatomical structure of the tooth in question. This process will result in a smooth execution of the tooth separation algorithm and result in a virtual tooth model that substantially exactly matches the structure of the patient's tooth.

The virtual tooth model may be extended beyond merely the crowns of the teeth. For example, a library of standardized virtual root templates for each of the teeth may be stored in the memory of the workstation. As individual virtual models of each tooth are created, the standardized root for that tooth are matched up with the virtual tooth model to thereby created an individual virtual tooth model of the entire tooth.

This process can be extended to templates of virtual gum tissue. On one hand, after separation of the individual virtual tooth models from the gum tissue the remaining portion of the scan data depicts the gum tissue (or at least a portion of the gum tissue, depending on the tissue scanned). This gum tissue may be substantially incomplete. The incomplete portions can be supplied by a template of virtual gum tissue, e.g., gums for an entire arch. The template of virtual gum tissue can be scaled up or down or modified as may be necessary to fit the anatomical structure of the patient. A registration of the template gum to the scanned gingival tissue enables a complete three-dimensional virtual model of the gums to be created.

This process can be extended to template bones, both mandible and maxilla. The goal here is to create a three-dimensional virtual model of the patient's mandible or maxilla. A virtual three-dimensional model of the mandible and maxilla can be obtained from a variety of sources, including CAT scan data, or skeletal specimens. The models are stored in the memory of the workstation. The virtual template mandible and maxilla are then expanded, contracted, or otherwise modified to fix to the patient's anatomy. X-rays of the patient may assist in the process. When the modified virtual template mandible and maxilla are created, the virtual teeth, gums and or roots can be displayed together with the maxilla or mandible, either alone or together with the orthodontic appliance.

The concept of template virtual objects can be extended to virtual template crowns, and the scanning features and user interface on the workstation extended to simulation of virtual crowns and adaptation of virtual crowns to virtual prepared teeth. For example, a prepared tooth is scanned as described herein and represented as a virtual three-dimensional model. A template virtual three-dimensional crown for the tooth (and typically for all 32 teeth) is stored in memory on the workstation or accessed from a database. The shape of the virtual template crown is reduced or adapted to form fit the prepared tooth where crown and prepared tooth surfaces meet. The shape of the cusps of the crown can be obtained from the surface configuration of the opposing tooth and adjacent teeth, or from the surface configuration of a contralateral tooth that is also scanned and represented a virtual tooth object.

Once the crown shape has been arrived at on the workstation, it can be exported as a 3-D crown object file to a remote location such as a lab for manufacture of the crown. For example, the 3-D crown object file is fed to a stereolithography machine and a physical model of the crown is made. A mould is made from the physical model. The crown is formed in the mould from gold or porcelain made using the lost wax technique. Alternatively, the 3D crown object file can be supplied to a CNC milling machine for machining a crown. Such crowns could be made from any suitable ceramic or metallic material, including stainless steel. This process represents a substantial savings of time and labour in manufacturing crowns. The typical crown preparation process is a manual process.

The concept of virtual template tooth objects and user manipulation of tooth objects on a computer can also be used in the field of dentures. Traditionally, an impression is taken of the gums and associated bony alveolar structures and these anatomical structures are cast in plastic or wax. Pre-formed teeth are set into the wax in a desired occlusion. The dentures are cast in acrylic using the lost wax technique. This process can be automated using the scanning methods described herein and using virtual three-dimensional template teeth. First, the gums and associated anatomical structures are scanned and represented as a three-dimensional virtual model on the workstation. Then, virtual template teeth are retrieved from memory. The template teeth are sized up or down as necessary to conform to the archform represented by the virtual model of the gums. The virtual template teeth are then placed on the archform. At this point, a three-dimensional virtual model of the teeth, gums and associated anatomical structures is represented in the workstation memory as a three-dimensional virtual object. This digital object can be exported anywhere, such as to a remote location where dentures are manufactured. From this object, a denture can be manufactured from a variety of techniques, including milling and casting. For example, a stereolithographic physical model of the dentition and/or gums can be made and a denture cast in a mould obtained from the physical model using the lost wax technique.

The virtual template teeth can also be used in forensic dentistry, i.e., reconstruction of the identity of a victim from teeth. As an example, a jaw containing some teeth can be scanned as described above and represented as a three-dimensional virtual object. Missing teeth can be reconstructed by importing virtual template teeth and placing them an the virtual object. The virtual template teeth may be based on age or ethnicity if such information is known. Contralateral teeth can be constructed by using existing scanned teeth as the template tooth and placing the scanned tooth in the contralateral position. Eventually, a complete virtual representation of the dentition can be obtained and viewed on the workstation. The shape of the face of the victim can be reconstructed by adding template virtual objects comprising soft tissue, gums, lips, cheeks, skin, hair, etc., and modifying the template objects using navigational tools based on the three-dimensional object or other information known about the victim.

Another example of using template teeth is for purposes of diagnosis and detection of tooth wearing, e.g., due to bruxism. In this example, the original scan taken of the patient is converted into a three-dimensional virtual model. The individual teeth are optically separated into virtual three-dimensional tooth objects as described above. Either this original virtual model of the entire dentition or the set of virtual three-dimensional tooth objects can be considered as a template. Over the course of time, the dentition is scanned again periodically and converted into a three-dimensional virtual model as described above. The individual teeth (or the dentition as a whole) is compared to the template to identify differences due to wearing of teeth. This can be performed by overlaying the two models, each in a different colour or tones, and visually detecting where tooth surfaces were present initially but are not present in the current virtual model. Alternatively, measuring tools can be provided on the user interface to measure the height of the tooth or other distances that may be indicative of wear, and numerical values immediately presented to the user on the user interface. These measurements can be compared with measurements made of the template. Now, tooth wear can be quantified precisely.

As yet another possibility, individual tooth objects are obtained from the original scan of the patient. These tooth objects are stored in the memory. In the case of a loss of the patient's tooth due to an accident or due to an extraction, the virtual tooth objects provide a precise template for manufacture of a replacement tooth. The replacement tooth could be manufactured for example using the stereolithography and lost wax techniques referred to above.

The creation of virtual tooth models allows virtual brackets to be virtually bonded to individual virtual teeth. The virtual brackets are obtained from a 3D CAD model of the bracket obtained from the manufacturer of the bracket. Alternatively, the brackets could be scanned and virtual bracket models obtained from registration of the scan frames into a virtual three dimensional model of the bracket. In either event, the virtual brackets are stored in memory and later accessed from the user interface of the orthodontic workstation. For example, the virtual brackets are placed on the teeth at the location of the landmarks and then moved by the user accessing suitable navigational tools provided by the user interface.

The virtual bonding of the brackets is merely a superposition of the virtual bracket onto the virtual surface of the tooth. Since both the bracket and the tooth are separate and independent virtual objects, they can be moved freely relative to each other, for example to optimize the position of the bracket. Preferably, the treatment planning software allows the user to interactively position the brackets along any combination of X, Y and Z directions, as wells as rotation about three orthogonal rotational axes. In one possible embodiment, the bracket placement correction is made by the user performing the following steps :

1) navigating through the treatment planning software until the virtual model of the dentition and the virtual brackets are displayed (this can be either the target situation or the malocclusion);
2) selecting a bracket for movement by either clicking on the bracket or selecting a bracket number from a drop-down menu;
3) accessing navigational controls for the bracket, such as by clicking on an icon that displays navigational controls for moving virtual objects such as brackets;
4) allowing the user to select either move the teeth with the bracket or move the bracket freely in three dimensions; and
5) using the navigational controls to move the brackets in three dimensions as desired.

If the bracket is moved independent of the tooth model, when the user is finished with the movement of the bracket the virtual tooth is moved to the location of the bracket. Bonding corrections for bonding the bracket to the tooth are updated. The bracket is then virtually bonded to the tooth. This process can be performed for each tooth. The result is that the orthodontist customized the placement of virtual brackets to the teeth. The archwire, which passes through the bracket slots, will have the required bends to move the teeth to the desired target situation regardless of the positioning of the brackets on the teeth.

The combination of the displayed set of virtual orthodontic brackets, together with the virtual orthodontic archwire, thus presents to the user a customized virtual orthodontic appliance. The virtue of the customized virtual orthodontic appliance is that it can be studied, modified, shared between two computers, and transported electronically over a communications medium for fabrication of the orthodontic appliance. The treatment planning software is essentially a specialized CAD/CAM system that allows the design of virtually any configuration of tooth objects, bracket objects, wire objects and other appliances and objects. Because these objects exist as independent mathematical objects, they can be selectively displayed together or alone. For example, the treatment planning software displays an icon or button on the user interface that allows the user to select or deselect the teeth, wires, brackets or virtual objects or appliances, as desired. For example, the teeth and archwire can be displayed together with the brackets deleted from the user interface. The orthodontist can then select an individual tooth object, move it in three dimensions, and the movement of the tooth carried over to a repositioning of the bracket in three dimensions and a changing of the shape of the archwire.

Furthermore, while the above process of creation of tooth models has been described in conjunction with the scan data from the hand- held scanner, this is not required. The separation of tooth objects can be performed with any three- dimensional model of the teeth, regardless of how the three- dimensional model is obtained. The three- dimensional model could be acquired from a CT scan, a laser scan from a plaster impression, or otherwise.

Part 4. Treatment Planning

Introduction

Treatment planning software is provided on the workstation of the orthodontic clinic, and possibly at other remote locations such as the precision appliance centre of Figure 1. The treatment planning software can be considered an interactive, computer-based computer aided design and computer aided manufacturing (CAD/CAM) system for orthodontics. The apparatus is highly interactive, in that it provides the orthodontist with the opportunity to both observe and analyze the current stage of the patient's condition and develop and specify a target or desired stage. A shortest direct path of tooth movement to the target stage can also be determined. Further, the apparatus provides for simulation of tooth movement between current and target.stages.

In its broader aspects, the apparatus comprises a workstation having a processing unit and a display, and a memory storing a virtual, complete three-dimensional model representing the dentition of a patient. The virtual three-dimensional model can be obtained from one of several possible sources; in the preferred embodiment it is arrived at from a scanning of the dentition. The apparatus further includes software executable by the processing unit that accesses the model and displays the model on the display of the workstation. The software further includes navigation tools, e.g., typed commands, icons and/or graphical devices superimposed on the displayed model, that enables a user to manipulate the model on the display and simulate the movement of at least one tooth in the model relative to other teeth in the model in three-dimensional space, and quantify the amount of movement precisely. This simulation can be used, for example, to design a particular target situation for the patient.

The development of a unique target situation for the patient has utility in a variety of different orthodontic appliances, including an approach based on off-the-shelf or generic brackets and a custom orthodontic archwire. The scope of the invention is sufficient to encompass other types of appliances, such as an approach based on customized brackets, retainers, or the removable aligning devices mentioned earlier. In a bracket embodiment, the memory contains a library of virtual, three-dimensional orthodontic brackets. The software permits a user to access the virtual brackets through a suitable screen display, and place the virtual brackets on the virtual model of the dentition of the patient. This bracket bonding position can be customized on a tooth by tooth basis to suit individual patient anatomy. Because the tooth models, brackets and archwire are individual objects, and stored as such in memory, the treatment planning apparatus can simultaneously display the virtual brackets, the archwire and the virtual model of the dentition, or some lesser combination, such as just the brackets, just the dentition, or the brackets and the archwire but not the teeth. The same holds true with other appliance systems.

In a preferred embodiment, the virtual model of teeth comprises a set of virtual, individual three-dimensional tooth objects. A method of obtaining the tooth objects from a scan of teeth, and obtaining other virtual objects of associated anatomical structures, e.g., gums, roots and bone is described. When the teeth are separated from each other and from the gums, they can be individually manipulated. Thus, the individual tooth objects can be individually selected and moved relative to other teeth in the set of virtual tooth objects. This feature permits individual, customized tooth positioning on a tooth by tooth basis. These positioning can be in terms or angular rotation about three axis, or translation in transverse, sagittal or coronal planes. Additionally, various measurement features are provided for quantifying the amount of movement.

One of the primary tools in the treatment planning apparatus is the selection and customization or a desired or target archform. Again, because the teeth are individual tooth objects, they can be moved independently of each other to define an ideal arch. This development of the target archform could be calculated using interpolation or cubic spline algorithms. Alternatively, it can be customized by the user specifying a type of archform (e.g, Roth), and the tooth are moved onto that archform or some modification of that archform. The archform can be shaped to meet the anatomical constraints of the patient. After the initial archform is designed, the user can again position the teeth on the archform as they deem appropriate on a tooth by tooth basis. The treatment planning software thus enables the movement of the virtual tooth objects onto an archform which may represent, at least in part, a proposed treatment objective for the patient.

Numerous other features are possible with the treatment planning software, including movement of the teeth with respect to the other teeth in the archform, changing the position of the virtual brackets and the teeth with respect to each other, or opposing teeth with respect to the selected archform. Custom archwire bends can be simulated to provide additional corrections. Bonding corrections at the bracket-tooth interface are also possible.

In another aspect, a method is provided for digital treatment planning for an orthodontic patient on a workstation having a processing unit, a user interface including a display and software executable by the processing unit. The method comprises the steps of obtaining and storing a three-dimensional virtual model of teeth representing the dentition of the patient in a current or observed situation. The virtual model is displayed on the display. The method further includes the step of moving the position of teeth in the virtual model relative to each other so as to place the teeth of the virtual model into a target situation and displaying the virtual model with the teeth moved to the target situation to the user. Parameters for an orthodontic appliance to move the patient's teeth from the current situation to the target situation can be derived from the virtual model and the target situation. For example, if virtual brackets are placed on the teeth, their location in the target situation can dictate the design of an archwire to move the teeth to the target situation.

In a preferred embodiment, the method includes the step of providing screen displays on the display enabling a user of the workstation to operate the user interface so as to place virtual three-dimensional objects representing orthodontic appliances, e.g., brackets, onto the surface of teeth in the virtual model. A library of the virtual brackets can be stored in memory and a landmarking procedure used to place the brackets on the teeth at the desired location. Anatomical considerations may dictate movement of the brackets from their originally selected. position to a new position. Accordingly, the software provides navigational tools enabling a user to change the position of the brackets relative to the teeth.

The treatment planning system is based on individual tooth objects which can be moved to any position in three dimensional space. They can be moved in several ways by direct user specified movement, and by adding an object comprising an orthodontic appliance and changing the configuration of the appliance to cause the teeth to move. For example brackets can be virtually bonded to the teeth and the position of the brackets changed in three dimensions to move the teeth. Alternatively, an archwire shape can be defined which fits into the slots in the brackets. Movement of the archwire can be simulated, resulting in a simulation of tooth movement.

The treatment planning software includes features enabling more accurate diagnosis. For one thing, the virtual model of the dentition can be manipulated in three dimensions at will, resulting in complete visual assessment of the model. Measurement tools are also provided by which the orthodontist can determine the distance between any two points on the model. This allows the user to quantify the patient's morphology both at initial and at target stages. Thus, treatment progress, proposed changes in appliance design, or tooth movement can be quantified precisely. By measuring the differences and changes in morphology during the care cycle, the orthodontist can quickly and accurately assess patient treatment. Changes in treatment can be made early on. The result is shorter treatment times (and the ability for the orthodontist to service more patients per year).

The treatment planning system incorporates virtual objects comprising orthodontic appliances that may be used to treat the patient. The system provides for design of complete appliance systems and simulation of various treatment scenarios prior to initiation of treatment.

A bite registration scan is obtained from the patient to spatially correlate the scans of the upper and lower jaws when the dentition is clenched. This scan is used to provide a registration of the upper and lower jaw to determine the correct relative position. This bite registration scan is usually needed at the beginning of treatment to set the relation between the upper and lower jaws.

Landmarks such as shown in Figure 57 are then placed on the labial surfaces of all the teeth. The illustrated embodiment places landmarks manually, but this process could be automated. The landmarks are placed initially on the molars and the front teeth, and an estimated position for the landmarks on the other teeth can be made, such as in the same plane, based on the relative position of the landmark with respect to the gingival tissue and incisal edge of the tooth, or other factors.

The landmarks are placed at the location where the orthodontist expects to place an orthodontic bracket to correct the malocclusion. The bracket shape is shown on the monitor 30 (Figure 1). Three-dimensional templates for a variety of commercially available brackets are stored in memory and the software asks the orthodontist to select a particular manufacturer and style of bracket to use with the patient. Thus, as the landmarks are placed, virtual brackets appear in the computer model on the labial surfaces of the teeth where the orthodontist desires to place the brackets. The orthodontist can move the bracket position depending on the type of forces the orthodontist wishes to create on teeth to correct the malocclusion.

Figure 60 is a screen shot from an orthodontic workstation showing the computer model of the patient's teeth objects 312 positioned in a target or desired condition. The illustration is the result of the user selecting an archform for the patient from a known type of archform (e. g., Roth), and the computer placing the teeth along the arch selected by the user. This is executed by placing the virtual brackets the orthodontist placed on the teeth along the curve selected by the orthodontist. The brackets are omitted from Figure 60, but are shown in Figure 61. The software allows the orthodontist to change many variables in the target situation, simply by entering new values in the slide line area 320 of the screen display, by mouse operation of up and down arrows to scroll through available values, or by mouse operation of a bar to change the values, or other similar technique. Figure 60 shows some of the parameters by which the orthodontist can adjust the shape of the arch, the distance between the teeth, the distance between the molars, and other parameters, so as to provide a unique and customized target situation for the patient.

Figure 61 is another screen shot showing the computer model of the patient's teeth in a target situation, also showing the numerous parameters available to the orthodontist to customize the tooth position, orientation, angulation, torque, and other parameters on a tooth by tooth basis for the target archform. Virtual brackets 322 are positioned on the tooth objects 312 at the location where the user placed the landmarks. A virtual archwire 324 passes through the slots in each virtual bracket.

Figure 62 is another screen shot showing a front view of the target situation and additional parameters available to the orthodontist for simulating the movement and positioning of teeth relative to each other in planning treatment for the patient. For example, in Figure 62, the cursor is moved onto the virtual tooth 41 (in the tooth numbering convention) and the mouse is clicked. Tooth 41 is then highlighted. If the orthodontist wants to extract that tooth, they then click on the box 22 to extract the tooth in the simulation. Alternatively, tooth 41 could be rotated about any of three axis of rotation, moved in the X, Y or Z direction, or a larger or smaller gap could be created between teeth.

Figure 63 shows the target situation for the upper arch, with the virtual brackets 322 in place. The orthodontist can adjust the bracket 322 position, archwire shape 324, or tooth 312 position, on a tooth by tooth basis to thereby optimize treatment planning for the patient.

The result of the treatment planning is the generation of a set of bracket placement positions and the display on the monitor of the shape of a customized orthodontic archwire to treat the malocclusion. Information as to the location of the brackets, the three-dimensional model of the malocclusion, the three dimensional model of the target situation, and the type of archwire to use are sent to the precision appliance centre 26 of Figure 1. A customized orthodontic archwire is manufactured in accordance with the bracket location and type and the target situation for the patient. Additionally, a transfer tray is manufactured to assist the orthodontist to place the brackets at the proper location. The transfer tray, brackets and archwire are shipped to the orthodontist's clinic 22. The orthodontic appliance is then applied to the patient and treatment commences.

Because the hand-held scanner allows for scans of the dentition in a matter of minutes, the scanner becomes an important tool in monitoring treatment. As the treatment progresses, the movement and position of the teeth during treatment can be quantified with a high degree of precision. The orthodontist can discern during treatment that corrections in the wire need to be made, for example due to biological influences affecting tooth movement. The treatment planning software on the workstation displays the current situation, and also the target situation. A new customized archwire is designed on the computer. The relevant information for making the new archwire is sent to the precision appliance service centre and a new archwire is manufactured and shipped to the clinic.

Monitoring scans are taken during treatment to measure and quantify progress and detect deviations from the expected treatment. Since each of the tooth objects is already stored, the monitoring scan need not be of the entire dentition, but rather needs to only be of one surface, such as the occlusal surface, or the lingual surface, or some combination of the two. A bite scan with the teeth in a clenched condition is also taken to get the current upper and lower relation. The position of the rest of the teeth is obtained from the virtual tooth objects 312 of the patient's teeth (Figure 58F). After the monitoring scans are performed, a registration is performed of the scanned data relative to the tooth models to complete the teeth and derive a current virtual model of the patient at that point in treatment. Study of this virtual model and comparison to the target virtual model of the teeth and the virtual model at the beginning of treatment can indicate whether progress is as anticipated or if additional correction to the orthodontic appliance need to be made. These corrections will typically be carried out as wire changes in an archwire orthodontic treatment regime, in which new bends are placed in the orthodontic archwire.

Initial virtual bracket placement

With the individual teeth now cut from the three-dimensional model of the dentition and represented as tooth objects, they can be moved relative to each other in three dimensions. Since orthodontics assumes that a bracket is fixedly bonded to a tooth, by moving the bracket one moves the tooth. The next step in the process is thus selecting an initial location to bond the brackets to the tooth. As noted below, this initial location can be adjusted by the treatment planning software. The spatial location of the surfaces of the bracket and the surfaces of the corresponding tooth are known. Collision avoidance algorithms are used to keep the bracket positioned on the surface of the tooth and prevent the virtual bracket from entering the tooth itself, a clinically undesirable result. The user is able to move the bracket independently of the tooth by activating an icon (such as one shaped like a magnet to signify the mating of the bracket to the tooth). When the bracket is moved to the new location, the tooth matches up to the surface of the bracket.

The brackets are represented in the software as virtual three-dimensional objects, and the surface of all the brackets and the teeth are known in three-dimensional spatial coordinates. Accordingly, collision detection algorithms are employed to detect when simulated tooth or bracket movement would result in a collision between brackets and teeth. Similar collision algorithms are provided to prevent the adhesion surface of the bracket from migrating into the body of the virtual tooth object and to keep the brackets located on the surface of the teeth. If the user wishes to move the location of the brackets, the movement of the teeth follows the movement of the bracket. Also, again since the bracket is a three-dimensional virtual object with known spatial coordinates, the user is provided with a tool (such as an icon) which when activated allows the user to move the bracket about one plane or axis, and freeze the movement in the other directions.

Initial virtual bracket placement is done as follows. Landmarks 302 such as shown in Figure 57 are placed on the labial surfaces of all the teeth. The landmarks are placed at the location where the orthodontist expects to place an orthodontic bracket to correct the malocclusion. The bracket shape is shown on the monitor. Three-dimensional templates for a variety of commercially available brackets are stored in memory and the software asks the orthodontist to select a particular manufacturer and style of bracket to use with the patient. Thus, as the landmarks 302 are placed, virtual brackets appear in the computer model on the labial surfaces of the teeth where the orthodontist desires to place the brackets. The orthodontist can move the bracket position depending on the type of forces the orthodontist wishes to create on teeth to correct the malocclusion. Because the brackets are individual objects and stored in memory, when they are placed on the surface of virtual teeth complete position information is known in three dimensions. As such, the brackets can be displayed either alone, or in conjunction with teeth, or hidden from view, by means of appropriate user specified commands on the user interface. For example, the screen display showing the target or current stage can have an icon indicating hide brackets, or display brackets, and activating the icon causes the brackets to be hid or displayed. The same is true for other virtual objects that exist independently of.other objects, such as tooth models and the archwire.

With the teeth now separated into individual tooth objects, the orthodontist can now view the current target stage, custom design a target situation for the patient, and design the appliance to treat the malocclusion. These aspects will now be described in further detail.

Viewing the observed (current) stage

Figure 69 is a screen shot showing a three-dimensional model 18 of a malocclusion, showing the teeth 312 in both the upper and lower arches 326 and 328, respectively. The screen 330 includes a row of icons 332 across the upper portion of the display, which are associated with various tools available to the user to view the dentition, virtual brackets, and current and target archforms. The lower portion 334 of the screen includes a set of tabs 336 that are accessed in various aspects of treatment planning. These tabs 336 include a patient tab 338, which accesses the screen of Figure 66. A limits tab 340 allows a user to breakdown the tooth movement between observed and target stages into stages, such as 30 percent, 50 percent and 75 percent, and display the tooth positions for both arches at these positions. A differences tab 342 quantifies the differences (in terms of translation and rotation) between the observed and target stages for each tooth. The space management tab 344 permits the user to simulate extraction of one or more teeth and adjust the spacing between teeth in either arch. A bonding correction tab 346 allows for adjustment of tooth position to be realized via bonding corrections. The technique tab 348 allows the user to select a bracket prescription and default settings for bracket height (distance from bracket slot to incisal edge of tooth). The tab also displays the parameters for the bracket prescription chosen by the user. The upper/lower (U/L) relations tab 327, selected in the screen shot of Figure 69, allows the user to modify the relation of the upper and lower jaws, by both translation in three axes (transversal, sagittal and vertical directions) and by rotation about these axes. The user manually enters values in the field 350 to change any parameter, and the change is immediately reflected in the view of the model of the dentition.

The tabs also include a bracket offset tab 352 that allows a user to reposition the bracket on a tooth and specifies numerical values for each bracket placement modification. A brackets tab 354 allows a user to enter information as to the type or manufacturer of brackets for each tooth in the both arches.

A further "morphing" tab could be provided which would animate the movement of the teeth from malocclusion to target situations based on treatment steps or limits defined by the user (explained in further detail below).

The screen shot of Figure 69 also includes a region 356 that allows the user to navigate between views of the observed stage and views of the target stage. Here, the user has highlighted or selected both arches in the observed stage, so the screen display shows the model of the dentition in the current or observed stage.

Referring to Figure 67, the treatment planning software preferably displays a plurality of icons 331, not all of which are shown in Figure 69, to enable the user to quickly and easily view the three dimensional model in various standard perspectives. For example, the icons 331 of Figure 67 include icons 333, 335, 337 and 339 for viewing the dentition in top, bottom, right hand side and left hand side views, respectively. An icon 341 is provided which zooms in or out. Icons 343 and 345 allow the user to select for viewing just the upper or lower arches, respectively, including virtual teeth, virtual brackets and virtual archwire. The icon 347 allows the user to show or hide the virtual dentition, excluding brackets and archwires. An icon 349 allows the user to select or deselect the virtual brackets. A marker icon 341 is used for measurement functions (described below) and an object navigation icon 353 is used for manipulating any of the virtual objects on the screen.

When positioning multiple objects in the three-dimensional view, such as shown in Figure 69, the camera navigation icons of Figure 67 move all the elements together. As shown in Figure 70, the initial placement of the virtual brackets 400 can be displayed along with the teeth. Further, the camera navigational tools allow the user to zoom in an zoom out in any desired degree. However, the virtual teeth 312 and virtual brackets 400 are individual three-dimensional objects which can be selected and moved independently. One way of moving objects is by entering new positional values (e.g., in terms of mm of displacement or angle of rotation, as described later). Another method provided by the software is using object navigational controls, activated by clicking the icon 353 or by accessing the function via a tools menu. The object navigation controls allow the user to move the object based on orthodontic judgment and visual feedback. The amount of movement is stored and can be displayed using numerical position information. As will be discussed in further detail below, the bracket position can be individually adjusted on a tooth by tooth basis. Furthermore, the camera navigation icons permit navigation of the archforms (i.e., the teeth placed on some-selected archform), navigation of the brackets, or navigation of the archwire.

The object navigation tools first require an object (e.g., tooth, bracket, archwire, etc.) to be selected. To select and deselect any object displayed on the screen, the user places the cursor over the object and double clicks the mouse. The selected object is highlighted in a separate colour. Additional objects are selected by pressing and holding down the <CTRL> button while double clicking additional objects. To magnify the object, the object is selected as described above and the icon 341 is clicked.

To move the selected objects, the software provides an object navigation icon 353. When the icon 353 is selected, object navigational tools appear on the screen 330. These navigational tools 355 are shown in Figure 68 and Figure 69. The object navigational tools 355 comprise a large circle 357, a small circle 359 and a small rectangle 361. First, the object is selected as described above. Then, the object navigation icon 353 is clicked, activating the tools 355 such that they are displayed. The user then positions the mouse pointer relative to the tools 355 and presses and drags as described below to position the object. When the mouse pointer is outside the large circle 357, when they start dragging the object is turned either clockwise or counterclockwise depending on the direction of dragging. When the mouse pointer is positioned within the large circle 357, when they start dragging they rotate the object in any direction. When they start dragging from inside the small circle 359, the object is moved in one plane. When they start dragging from inside the rectangle 361, by dragging down the object is moved closer, by dragging upward the object is moved farther away.

## Measuring objects

Referring again to Figure 67, the icon 351 allows the user to establish a measurement marker on any portion of the virtual model of the dentition. The user uses the icon 351 to place markers at any two points on the dentition and the distance between the markers is displayed on the screen.

To use the icon 351, the user clicks on the icon, and then clicks anywhere in the 3-D view of the dentition to place markers. A straight line is drawn between two markers. The distance between the markers appears on the screen, e.g., at the upper left hand corner of the windowpane of the 3-D view. A tool in the Tools menu in includes a DELETE ALL MARKERS function to delete the markers.

The measurement function allows the user to measure tooth size, inter-molar width, inter-canine width, the arch length, curve of spee, and other important orthodontic and diagnostic parameters. The measurement aspect is particularly significant in that it permits precise quantification of simulated tooth movement, both in terms of establishing initial treatment plan as well as monitoring treatment.

Viewing cross-sections of model

The viewing options also include a clipping plane feature by which cross-sections of the teeth through any desired plane are possible. As shown in Figure 73, the clipping plane is shown through the upper jaw, but the user at can move this plane in three-dimensional space at will. If the teeth are magnified, this clipping plane feature is very useful for inspecting contact points between the upper and lower jaw, viewing and adjusting the upper and lower jaws in the initial bite registration, and adjusting the location of the occlusal plane. For example, in Figure 74 the clipping plane is shown through the upper and lower incisors 312A and 312B.

The clipping plane is manipulated like an object with the object navigational tools shown in Figure 68. The plane is accessed using a tools menu and the user highlights or selects SHOW CLIPPING PLANE. A plane appears on the screen. The user then clicks on the object navigation icon 353 (Figure 67). The object navigational controls 355 of Figure 68 then are displayed. The user then positions the mouse pointer over the navigational controls 353 to adjust the position of the clipping plane. When they start dragging in the region outside the large circle 357 (Figure 68, Figure 73), the plane is turned clockwise or counterclockwise. Then they start dragging inside the large circle 357, the plane is rotated in the direction indicated by the dragging. When they start dragging from inside the small circle 359, the clipping plane is moved in the direction of the dragging. When they start from inside the rectangle 361, if they drag up they cut less into the model, by dragging down they cut further into the model.

Viewing and adjusting initial bite registration

The first step in typical treatment planning is deciding where to place the teeth in three-dimensional space. This will ordinarily involve a definition or fixation of the vertical level of the teeth relative to the bones, and defining an occlusal plane, and adjusting the occlusal plane sagittally and transversely. This, in turn, will ordinarily involves an assessment of structural relationship between the teeth and the maxilla and mandible. The orthodontist performs this by accessing and studying X-ray, CAT scan, photographs or other two dimensional data stored in the patient records portion of the treatment planning software, and of course the three-dimensional model of the malocclusion, with the teeth either represented as individual tooth objects or in context with the surrounding anatomical tissue. The mid-sagittal profile of the incisors and molars is set up by superimposing the mid-sagittal plane of the teeth over the X-ray.

Figure 66 is a screen shot from the workstation running the treatment planning software showing a patient information screen. The screen includes a region 500 for storing various photographs 502 of the patient's head and face, and various views of the patients dentition. The photographs are taken with a digital camera and loaded into the workstation, and accessed from the treatment planning software. The patient information section of the software also includes separate screens for entering and displaying other pertinent information for the patient, accessed through a menu 504. These additional screens (not shown) include the patient demographics, patient medical, dental and orthodontic history, examination notes, and X-rays.

To assist this process, the treatment planning software provides the ability to view and adjust the initial bite registration. The initial bite registration between the upper and lower arches can be modified using the U/L relation tab 327 of Figure 69. The user can move or rotate the lower jaw relative to the upper jaw by entering values in the field 366. The user can also simulate a chewing motion of the upper and lower jaws by moving the slide bar 368 down. During this simulation the lower jaw moves from side to side and up and down to simulate a chewing motion.

Figure 71 shows how the three-dimensional viewing area on the screen on the workstation can be broken up into separate screens using known windowpane techniques. Here, in windowpane 360, the area of the molar in the observed stage is displayed, with the orthodontist able to assess the upper and lower relation and change values for the upper and lower relation in three planes of space. Simultaneously, windowpane 362 shows the upper and lower jaws as seen from above. Windowpane 364 shows the dentition looking from the molars out towards the incisors, a view the orthodontist would otherwise not be able to access without the three-dimensional virtual model. These various views, plus the clipping plane tool, and the X-Ray and patient photograph data in the patient records portion of the software, provide a complete suite of tools for effective orthodontic diagnosis, treatment planning, and appliance design, including initial bite registration.

Figure 72 is another screen shot showing the three-dimensional model of the dentition in the observed stage.

No values have been entered in the malocclusion field 366 in the U/L Relations tab. By inspection of the upper and lower jaws (using magnification or clipping plane features if needed), the user can set numerical values in field 366 and immediately simulate the corresponding tooth movement to arrive at a desired upper and lower initial bite registration.

Design of Target Archform

Referring to Figure 75, after the user has set the initial level of the occlusal plane and inspected the initial observed situation, the next step is selection of a desired or target archform for the patient and the midline. The orthodontist will have previously selected or indicated a general type of archform for the patient, e.g., Roth. The treatment planning software allows the user to generate a target arch based on this archform for the individual patient. The user highlights the observed stage in region 356 and then right clicks the mouse. The pop-up menu 370 appears and the user selects INSERT TARGET STAGE. The target stage is arrived at by creating a flat virtual wire that runs through the virtual bracket slots to create a virtual arch line. The arch shape is based on the user's selected preference for the arch shape, based on the patient's facial anatomy, bone structure, malocclusion, and other parameters using the orthodontist's judgment. The wire target shape has a best fit through the malocclusion bracket slot positions. In one embodiment this wire is flat. It is also possible to design the wire to adapt to the malocclusion in the vertical direction to create a Curve of Spee if desired. The geometry in the transverse direction can also be changed, such as the transverse curve of Monson establishing an inclination of the teeth in the coronal plane.

Figure 76 is a screen shot from an orthodontic workstation showing the computer model of the patient's teeth objects 312 positioned in a target or desired condition. The illustration is the result of the user selecting an archform for the patient from a known type of archform and the computer placing the virtual brackets along the arch selected by the user. This is executed by placing or threading the virtual brackets along the archform or curve selected by the orthodontist. The brackets are omitted from Figure 76, but are shown in Figure 77. When the initial target archform is created, the slide line tab shown in Figure 67 is activated.

The initial target archform presented to the user in Figure 76 is only an initial archform. The treatment planning software allows the orthodontist to change many variables in the target situation, simply by entering new values in the slide line area 402 of the screen display. Figure 19 shows some of the parameters by which the orthodontist can adjust the shape of the arch, including the distance between the cuspids, the distance between the rearmost molars, the centre line offset, and the radius of curvature at the front of the teeth. Slide line area also permits the user to select a symmetrical archform or an asymmetrical archform, and apply corrections on the right and left quadrants as indicated. As values are entered in area 402, the shape of the archform is instantaneously modified on the screen display, allowing the user to simulate various potential archform configurations for the patient.

In generating the archforms shown in Figures 76 and 77, the user will ordinarily set up the lower arch first. The upper arch is automatically derived from the lower arch. The user can view the arch forms in several ways using three arch icons 390, 392 and 394. The arch icon 390 is for the malocclusion arch, which causes a blue line to appear on the screen which exhibits the curvature of the observed arch or malocclusion. The line passes through the slots on the virtual brackets, as placed on the teeth. The arch icon 392 is for the target arch, which represents a custom archwire passing through the bracket slots of the teeth in the target situation. The line 396 in Figure 76 represents this arch. The arch icon 394 if for an ideal spline or curve generated by the software to represent an optimal shape. The arch retains a parabolic shape as the user manipulates the target arch using the entries in the slide line. The numerical values in region 398 of the slide line tab represent checkpoints and boundary conditions on the ideal spline associated with the icon 394. These values can be edited as indicated.

Since the software allows for generation and display of a malocclusion archform and a planned archform, the difference between the two archforms indicates the space needed to control arch length inadequacy; i.e., to identify the need for interproximal reduction, extraction, or control of gap size. Interproximal reduction can be achieved by the clipping plane feature, and the simulation of shaping of individual tooth objects in three planes of space. The simulation of extractions and control of gap is provided for as explained next.

Space Management -- Management of Extractions and Gaps Between Teeth

The treatment planning software provides a space management tab 344 that is used for managing space between teeth, including tooth extraction. Figure 82 is a screen shot showing the target situation of both the upper and lower jaws. The user clicks on the space management tab 344, and the region 420 of the tab appears. The region 420 allows the user to simulate the extraction of a tooth in either the current or target stage. Here the user is simulating the extraction of tooth number 41 by clicking on the appropriate cell for that tooth in the rows for current and target stages. The result is shown in Figure 83. The extraction of tooth 41 is simulated. The region 422 also allows the user to enter values for the mesial gap size between teeth. A default value of 0.1 mm is provided. However, the user can change these values. For example, the user can enter any values in the row of cells for mesial gap size as shown in Figure 84 (in this example 2 mm). Note also in Figure 84 that the simulation of the extraction of tooth 41 is not being performed since the cell 423 is not checked. The tooth moves distally if a positive number is typed in the cell for that tooth in the mesial gap size row 424. If a negative number is typed into the cell, the tooth moves mesially.

Adjusting virtual bracket position

After the upper and lower archforms have been optimized, the user may wish to adjust the position of the virtual brackets 400 on the teeth. The step of adjusting the virtual bracket position can also be performed prior to the design of the archform.

The vertical position of the virtual brackets relative to the incisal edge of the virtual teeth is one adjustment that can be made. This distance can be measured using the measurement, tool described earlier. Bracket manufacturers have recommendations for this distance. If the initial landmarking has placed the brackets outside of the recommended distance, this distance can be adjusted by using the object navigational tools. Alternatively, the user can select preferred values for the archform and the bracket set (bracket manufacture, bracket technique and wire type, e.g., straight) and the landmarks and virtual brackets will be placed on the teeth at the recommended distance.

The bracket placement can also be performed interactively by the user. The user looks at every tooth 312 one by one (using a screen such as the screen shot of Figure 75) to see if they are basically satisfied with the bracket 400 position, e.g., angulation, side to side position, and its relation to teeth in the opposing jaw. The user may be performing this step while viewing the malocclusion, or the target stage. To improve the position of the virtual bracket 400, the user selects the bracket, zooms in if necessary, and adjusts the position of the bracket on the tooth surface using the navigational tools as described earlier.

Correction of Individual Tooth Position In Target Archform

After the archform has been designed and the bracket placement optimized, the user can adjust the individual tooth position on a tooth by tooth basis in the target arch. There are three ways this can be performed. First, the user can use the tables in the Target Correction tab. See, for example, Figure 78, in which the user has entered a value of -15 degrees for rotation of tooth number 41, and the tooth is rotated by that amount. The correction is realized by a bend in the archwire 396. The bracket position on the tooth does not change in this example. The target corrections tab 410 permits any values to be entered for mesial, buccal and coronal translation in three planes of space, and torque, angulation and rotation movements about three orthogonal axis. Thus, independent tooth position corrections are available in 6 degrees of freedom for every tooth, merely by entering values in the tables in the target corrections tab 410. Another example is shown in Figure 81, in which a new target position is shown for tooth number 41.

Secondly, the user can move teeth using the Bonding Corrections tab 346 of Figure 69. An example is shown in Figure 85. The bonding corrections tab 346 allow the user to enter new values for tooth position for any tooth in the arch by merely entering values in the appropriate cell. The selected tooth 312A (tooth number 41) is moved as indicated (here, a new rotation value of- 15 degrees is entered) . The virtual bracket remains in the same location in space and the gap 412 between the pad of the bracket and the surface of the tooth is taken up by a bonding adhesive when the bracket is bonded to the tooth.

Examples of common tooth movements that can be simulated using the bonding corrections tab or the target corrections tab are moving the lower incisors buccally or lingually, inclining the incisors axially, and levelling the incisors.

Thirdly, the user can simulate tooth position corrections interactively using the navigational tools. The user displays a target stage as needed. A tooth object is selected as explained above. The user clicks on the zoom icon 341 of Figure 67 to zoom in or out as needed. The user then clicks on the object navigation icon 353 to display the object navigation controls. The user then uses the navigation controls to move the tooth as desired. The movement of the tooth is recorded as new values in the bonding correction and target correction tables, in case the user wants to quantify the movement or use those tables for further modification of the tooth position. After the user has moved the tooth to the new position, they click one of two check mark icons 414, 416 (Figure 77, 78) that are highlighted on the screen. The blue check mark 414 realized the new tooth position via a bonding correction. The red check mark 416 realizes the new tooth position via a wire correction.

Another example of correction of individual tooth position is shown in Figures 79 and 80. In Figure 80, the target situation is shown, both with the virtual tooth objects 312 and the virtual brackets 400. Note that with tooth 16 (312A), there is a gap between the rearmost cusp of the tooth and the opposing tooth. The orthodontist can correct this gap by building in a bracket offset, basically repositioning the location of the bracket 400 on the tooth 312A by entering an amount in the table 450 in the angulation cell for tooth number 16 (here -15 degrees). The result is shown in Figure 80. The change in the angulation value causes tooth number 16 to rotate back into a more desirable occlusion with the opposing tooth.

Wire Tab

The wire tab 432 of Figure 86 allows the user to make wire configuration changes, while not changing bracket position. Note that in Figure 86, the virtual wire 396 is shown isolated from the teeth and brackets. The virtual wire can be scaled to actual size and printed out and provide a template for a manual bending of a wire if the orthodontist chooses not to obtain a wire from the precision appliance manufacturing centre. The tab includes a section 430 where the user can view the distance in mm in which the wire will slide relative to the brackets, on a tooth by tooth basis, when the teeth are moved from the current situation to the target situation. Note that a collision is detected for tooth 22 in movement of the tooth from the current situation to the target situation. This can be resolved in several possible ways in accordance with the teachings of U.S. Patent US6250918 to Sachdeva et al., serial no. 09/451,609 allowed December 7, 2000.

Additional Wire Bending Corrections

The wire offsets tab 426 (see Figure 73 and Figure 87) allows the user to simulate bending a wire 396 to the corrected tooth position while simultaneously retaining the original bracket position. Note that in Figure 87 the wire 396 is flat with no corrections indicated. The user highlights one of the teeth in the virtual model and enters new values for tooth position. The change is reflected in a new shape for the wire 396. The tab also allows the user to build in over/ under compensation into the archwire. These settings do not affect the bracket position. The characteristics of the wire bends, such as the radius of curvature, can be controlled by accessing a sidings tab (not shown). Another tab, forces 428, displays approximations of the forces the wire applies to each tooth to displace it from its initial position to the target position.

Treatment Stages

Since the difference between the current situation and the target situation is quantifiable in terms of millimetres of movement or degrees of rotation about three axes, the treatment for the patient can be broken up into segments or stages with each stage defined arbitrarily. For example, the orthodontist can specify a first treatment stage as the movement of the teeth from the initial position half-way to the final position. The software includes a screen that permits the user to view the position of the teeth when moved to the half-way position. Basically, the simulation simply multiplies the differences in tooth position between initial and target stages by 0.5 and the resulting tooth positions are displayed on the workstation. Additionally, the user can specify further intermediate positions, such as one fourth or three fourths. With this feature, the orthodontist can monitor treatment and compare the progress of the treatment with the limits or stages that have been set. When the patient comes in for a visit during treatment, the patient's dentition is scanned. The three-dimensional model of the now current situation is compared with the defined stages and perhaps with the target situation. Difference between actual progress and the planned treatment can be quantified. Changes to the archwire can be designed using the treatment planning software to move the teeth in the desired direction to account for unexpected biological influences that may be observed.

The above description of treatment planning has been predicated on the use of a three-dimensional model of the dentition from the scanner, obtained as described above. However, it is possible to perform digital treatment planning by importing into the software three-dimensional software from other sources. It is known today that three-dimensional models can be exchanged with different software programs in different file formats, similar to the translation programs that convert text documents from one type of file (e.g., Microsoft Word to WordPerfect). Most three-dimensional applications have several import filters for different 3D formats.

However, there are generally two different ways to describe three- dimensional objects: by surface representations and by solid representations. A 3D file that holds 3D data in a surface description consists typically of triangles that form the surface of the object. The STL format is one of the oldest and therefore most common formats that uses triangles. It is used to feed information to stereolithography machines. A more detailed description of STL can be found at http : //www.mmsonline.com/ articles. 019704.html.

Treatment Monitoring

Interactive, computer- based treatment monitoring is a significant advantage provided the treatment planning and appliance design aspects of the system described herein. Typically, when the patient comes into to the office during treatment, they will be scanned and a new digital model of the dentition is acquired. From this new model, differences can be monitored between the current situation and the original malocclusion, and differences between the current situation and the target situation or pre- defined limits or treatment stages as defined earlier. These differences can be quantified with precision. For example, a point on the tooth in the current model is selected, and the model of the tooth at the original malocclusion is overlaid on the screen. The superposition of the two teeth allows the user to view the change in position that has occurred. The measurement marker features described earlier allow the user to quantify precisely the amount of movement.

Any deviations between the therapeutic result that is observed and the expected result can be captured precisely and at an early stage in treatment using the scanning and treatment planning features described herein, and corrected for. For example, the orthodontist may need to place additional bends in the archwire. Such additional bends can be performed by simulating the wire shape on the screen, displaying the wire only on the screen, and printing out the screen and using it as a template for bending the wire. The current situation could also be forwarded to the precision appliance centre for manufacture of a new appliance. Of course, these monitoring and treatment corrections are applicable to any type of appliance selected for the patient.

Other inventive features disclosed herein

*Orthodontic Appliance Optimization*

Generally, the present system also provides for a method and apparatus for arch wire receptacle (e.g., brackets, bands, headgear tubes, etc.) optimization. Such a method and apparatus includes processing that begins by obtaining a digital model of an orthodontic structure of an orthodontic patient. This may be done in accordance with the teachings described above. The processing then continues by retrieving a digital model of an initial arch wire receptacle (e.g., a bracket, a band, a headgear tube, etc.) that was selected from a plurality of digital models of arch wire receptacles. Generation of a digital model of an arch wire receptacle may be further performed as described above. The processing then continues by digitally placing the digital model of the initial arch wire receptacle on a given tooth of the digital model of the orthodontic structure to provide a digital arch wire receptacle placement. The process then proceeds by retrieving a digital model of an arch wire. For the given tooth, the processing continues by digitally modelling a force system on the tooth based on the digital model of the initial arch wire receptacle, the digital arch wire receptacle placement, and the digital model of the arch wire. Having done this, when the force system is not optimal, the force system is modified to achieve an optimal force system. The force system includes the effects of bonding pad thickness, bonding agent properties, the arch wire, ligation forces, and biological characteristics (e.g., growth, tooth interference, tooth anatomy) . Each of these factors may be adjusted individually or in combination to optimize the force system. With such a method and apparatus, optimal force systems given orthodontic parameter constraints may be obtained without the limitations of prior art orthodontic practices.

*Determining and Monitoring Orthodontic Treatment*

Generally, the present system also provides for a method and apparatus for treating an orthodontic patient. Such a method and apparatus include processing that begins by generating digital information regarding the orthodontic patient by a site orthodontic system. The digital information may include a three- dimensional scan image of the patient's orthodontic structure (e.g., teeth, jaw bone, gums, and other facial features) , x- rays, clinical examination and measurements, dental history, medical history, photographs, etc. The site orthodontic system then transmits the digital information to an orthodontic server. The orthodontic server and/or the site orthodontic system creates an electronic patient record from the digital information. The electronic patient record includes, but is not limited to, clinical examination interpretations, radiology examination measurements, automatic and manual cephalometric analysis, a created electronic cephalometric tracings, an electronic composite including an integration of the three- dimensional images, radiographic data (e.g., x- rays, CAT scans, MRI scans) , photographs, a generated electronic articulation module, measurements and analysis of electronic models, data quality assurance checks, and/or supplemental data.

The orthodontic server then generates an initial treatment from the electronic patient record, wherein the initial treatment plan includes precise steps to obtain a desired orthodontic structure. In other words, the initial treatment plan includes a series of steps, wherein, for each step, a corresponding orthodontic apparatus will be designed to reshape the patient's orthodontic structure into a desired structure for the precise step. The orthodontic server then transmits a digital version of the initial treatment plan to the site orthodontic system. Upon confirmation from the site orthodontic system, the orthodontic server designs an orthodontic apparatus (e.g., brackets, bands, wires, head gear, rubber band placement, and/or a retainer) for one of the precise steps based on the treatment plan. The orthodontic apparatus is then fabricated/assembled and provided to the site orthodontic system. In addition, the orthodontic server may provide practitioner and/or patient instructions for use, maintenance, and/or installation of the orthodontic apparatus.

Upon receipt of the orthodontic apparatus, the orthodontist verifies receipt of the orthodontic apparatus and subsequently installs it. At a predetermined point in time after the installation in accordance with the treatment plan, the patient's mouth is electronically scanned to obtain updated digital information. The site orthodontic system provides the updated digital information to the orthodontic server, which uses the updated digital information to update the electronic patient record. From the updated electronic patient record, the orthodontic server determines whether the actual movement of the patient's teeth is as predicted. Such a determination may be supplemented with input from a practitioner (e.g., a technician, dental assistant, orthodontist, specialist, consultant, etc.) at the site or at a remote site with respect to the patient. If so, the next step of the initial treatment plan is executed (e.g., the wire for the next step is fabricated, provided to the orthodontist, and installed). If, however, the actual movement is not as predicted, the orthodontic server adjusts the treatment plan to obtain the desired results. After the treatment plan has been adjusted, the next step of the revised treatment plan is executed. This monitoring of a patient's progress and revising the treatment plan, when necessary, continues throughout the treatment. Thus, with such a method and apparatus, a scientific approach is provided to orthodontic treatment throughout the treatment, maintains the treatment costs at reasonable levels, and provides a more consistent and reduced treatment time. In addition, the present method and apparatus allow orthodontic practitioners to have just-in-time inventory, allows for treatment adjustments that would be required due to changes in tooth position and/or jaw development, and further allows for changes in the orthodontic structure if the force system becomes suboptimal.

*Treating an Orthodontic Patient*

Generally, the present system also provides for a method and apparatus for treating an orthodontic patient. Such a method and apparatus include processing that begins by generating digital information regarding the orthodontic patient by a site orthodontic system. The digital information may include a three- dimensional scan image of the patient's orthodontic structure (e.g., teeth, jaw bone, gums, and other facial features) , x- rays, clinical examination and measurements, dental history, medical history, photographs, etc. The site orthodontic system then transmits the digital information to an orthodontic server. The orthodontic server and/or the site orthodontic system creates an electronic patient record from the digital information. The electronic patient record includes, but is not limited to, clinical examination interpretations, radiology examination measurements, automatic and manual cephalometric analysis, a created electronic cephalometric tracings, an electronic composite including an integration of the three- dimensional images, radiographic data (e.g., x- rays, CAT scans, MRI scans) , photographs, a generated electronic articulation module, measurements and analysis of electronic models, data quality assurance checks, and/or supplemental data.

The orthodontic server then generates an initial treatment from the electronic patient record, wherein the initial treatment plan includes precise steps to obtain a desired orthodontic structure. In other words, the initial treatment plan includes a series of steps, wherein, for each step, a corresponding orthodontic apparatus will be designed to reshape the patient's orthodontic structure into a desired structure for the precise step. The orthodontic server then transmits a digital version of the initial treatment plan to the site orthodontic system. Upon confirmation from the site orthodontic system, the orthodontic server designs an orthodontic apparatus (e.g., brackets, bands, wires, head gear, rubber band placement, and/or a retainer) for one of the precise steps based on the treatment plan. The orthodontic apparatus is then fabricated/assembled and provided to the site orthodontic system. In addition, the orthodontic server may provide practitioner and/or patient instructions for use, maintenance, and/or installation of the orthodontic apparatus.

Upon receipt of the orthodontic apparatus, the orthodontist verifies receipt of the orthodontic apparatus and subsequently installs it. At a predetermined point in time after the installation in accordance with the treatment plan, the patient's mouth is electronically scanned to obtain updated digital information. The site orthodontic system provides the updated digital information to the orthodontic server, which uses the updated digital information to update the electronic patient record. From the updated electronic patient record, the orthodontic server determines whether the actual movement of the patient's teeth is as predicted. Such a determination may be supplemented with input from a practitioner (e.g., a technician, dental assistant, orthodontist, specialist, consultant, etc.) at the site or at a remote site with respect to the patient. If so, the next step of the initial treatment, plan is executed (e.g., the wire for the next step is fabricated, provided to the orthodontist, and installed). If, however, the actual movement is not as predicted, the orthodontic server adjusts the treatment plan to obtain the desired results. After the treatment plan has been adjusted, the next step of the revised treatment plan is executed. This monitoring of a patient's progress and revising the treatment plan, when necessary, continues throughout the treatment. Thus, with such a method and apparatus, a scientific approach is provided to orthodontic treatment throughout the treatment, maintains the treatment costs at reasonable levels, and provides a more consistent and reduced treatment time. In addition, the present method and apparatus allow orthodontic practitioners to have just-in-time inventory, allows for treatment adjustments that would be required due to changes in tooth position and/or jaw development, and further allows for changes in the orthodontic structure if the force system becomes suboptimal.

*Producing a Three-Dimensional Digital Model of an Orthodontic Patient*

Generally, the present system also provides for a method and apparatus for producing a three- dimensional digital model of an orthodontic patient. Such a method and apparatus include processing that begins by obtaining data of an orthodontic structure of the orthodontic patient. The processing then continues by obtaining at least one scaling reference point of the orthodontic structure. For example, the scaling reference point may be a marking placed on a tooth prior to obtaining the data. The processing continues by scaling the data of the orthodontic structure based on the at least one scaling reference point to produce scaled data. Note that the data may be video data, x- rays, CAT scans, ultrasound, and/or magnetic resonance images. The process then continues by obtaining at least two orientation reference points relating to the orthodontic structure (i.e., fixed physical attributes of the orthodontic structure that will not change, or will change with negligible effects, during the course of treatment) . The processing then continues by mapping the scaled data to a coordinate system based on at least two orientation reference points to produce an enhanced three-dimensional digital model of the orthodontic patient. With such a method and apparatus, the automated treatment of an orthodontic patient occurs in a scientific and controlled manner. By obtaining an accurate three- dimensional digital model that includes a dentition structure of the orthodontic patient, patient treatment can be simulated by a computer in three- dimensional space, thereby providing a scientific approach to orthodontic treatment.

*Producing a Desired Three-Dimensional Digital Model of an Orthodontic Structure*

Generally, the present system also provides for a method and apparatus for generating a three- dimensional digital model of a desired orthodontic structure. Such a method and apparatus include processing that begins by obtaining a three- dimensional model of an actual orthodontic structure, wherein the three- dimensional digital model is defined in x, y, z space. The processing then continues by generating an interim three- dimensional model of the desired orthodontic structure less teeth. The interim three- dimensional model is designed in x, y, z space and includes the desired placement of an occlusal plane, an upper- arch form, a lower- arch form, an upper- arch midline, and a lower-arch midline. The processing then continues by positioning the upper and lower teeth with respect to the interim digital model and the defined x, y, z space to obtain a first pass three dimensional digital model of the desired orthodontic structure. The processing continues by determining whether achieving the first pass three- dimensional model is feasible. In essence, the determination is based on treatment constraints and whether the desired orthodontic structure can be achieved from the actual orthodontic structure given such constraints. When achieving the first pass three- dimensional image is feasible, the processing continues by utilizing the first pass three- dimensional model as the desired three-dimensional digital model. With such a method and apparatus, automation of orthodontic treatment is realized thereby providing a scientific approach to practice of orthodontics.

*Designing an Orthodontic Apparatus to Provide Tooth Movement*

Generally, the present system also provides for a method and apparatus for designing an orthodontic structure to provide tooth movement. Such a method and apparatus includes processing that begins by obtaining a three- dimensional digital model of a desired orthodontic structure. The orthodontic structure includes a plurality of teeth that have been positioned into a desired location. The processing continues by generating a zero force arch wire based on the three- dimensional digital model of the desired orthodontic structure. The processing continues by placing brackets on at least some of the plurality of teeth in a zero force position with respect to the zero force arch wire. Having placed the brackets on the desired orthodontic structure, the placement is transferred to a three- dimensional digital model of an actual orthodontic structure. Having transferred the bracket placement, a jig may be formed from the digital model of the actual orthodontic structure with the brackets installed. The jig holds the physical embodiments of the brackets such that the brackets may be placed in the patient’s mouth in the position determined by the automated process. With such a method and apparatus, the practice of orthodontics is transformed from an art to a science throughout the treatment process, reduces costs, and provides for more consistent treatment times.

*Automated Generation of a Patient Treatment Plan*

Generally, the present system also provides for a method and apparatus for generating a patient treatment plan. Such a method and apparatus includes processing that begins by providing a list of health care services to a patient and/or care provider. The processing continues by prompting for input of digital information regarding the patient when a health care service has been selected. The processing continues by determining whether a sufficient amount of digital information has been received. If so, the processing continues by simulating treatment of a patient based on the digital information, a treatment objective, and normalized patient data. The processing then continues by generating the patient treatment plan in accordance with the simulating of the treatment when the simulated treatment results have been acknowledged. With such a method and apparatus, the generation of a patient treatment plan may be automated for particular types of health care services, including orthodontic care, dental care, and medical care.

While presently preferred embodiments have been described for purposes of illustration of the best mode contemplated by the inventors for practicing the invention, wide variation from the details described herein is foreseen without departure from the scope of the invention. This scope is to be determined by reference to the appended claims.

## Claims

1. A system for creating an individual, three-dimensional virtual tooth model representing a tooth found in a dentition assigned to a patient, comprising:

   a memory storing at least one virtual three-dimensional model of a template object corresponding to a tooth, including a template tooth corresponding to said tooth;
   a data processing system including a memory storing a virtual three-dimensional model of at least a part of the dentition;
   wherein said data processing system further comprises software processing said virtual three-dimensional model of at least a part of the dentition and said virtual model of the template object and responsively deriving said individual, three-dimensional, virtual tooth model;
   wherein said software comprises (1) a routine superimposing said virtual template object corresponding to a tooth on said virtual model of at least a part of the dentition; (2) a routine drawing vectors from said virtual template object to a surface of said virtual model of the dentition and storing a set of points where said vectors intersect said surface; (3) a routine forming a new surface comprised of said set of points; and (4) a routine filling missing parts of said surface, if any, from said template tooth.

2. The system of claim 1, wherein said template object comprises one or more specific characteristics of a tooth selected from the group consisting of cusps, tooth width, occlusion characteristics, two-dimensional cross-section in an occlusal plane, roots and flatness.

3. The system of claim 1, wherein said template object comprises a surface formed of a mesh of polygon surfaces.

4. The system of claim 1, wherein said template object comprises a cloud of three-dimensional points.

5. The system of claim 1, wherein said system further comprises a user interface, and wherein said software displays said individual virtual tooth model on said user interface.

6. The system of claim 5, wherein said system includes software that permits a user to place a landmark on a virtual representation of said tooth of the patient via said user interface, said landmark used to align said virtual template object corresponding to a tooth with said virtual three-dimensional model of at least a part of the dentition.

7. The system of claim 6, wherein said user interface displays a superposition of said individual virtual tooth model and said virtual three-dimensional model of at least a part of the patient's dentition.

8. The system of claim 6, wherein said data processing system generates individual virtual tooth models for all of the teeth of a patient and displays said virtual tooth models on a user interface.

9. The system of claim 8, wherein said virtual tooth models are displayed on said user interface in a position relative to each other in three dimensions that substantially matches the current position of the teeth in the dentition of the patient.

10. The system of claim 1, wherein the software module executes routines (1), (2), (3) and (4) in an iterative fashion, with the surface after execution of step (3) or step (4) replacing the virtual model of the dentition at step (1) in successive iterations.

11. The system of claim 10, wherein said iterative execution of steps (1), (2), (3) and (4) terminates, the surface generated after the final iteration is displayed for the user as a superposition of original data obtained from scanning and the surface created by said software at the end of step (3) or step (4) after the last iteration.

## Patentansprüche

1. System zum Erstellen eines individuellen, dreidimensionalen virtuellen Zahnmodells, das einen Zahn darstellt, der sich in einem Gebiss befindet, das einem Patienten zugeordnet ist, umfassend:

   - einen Speicher, der mindestens ein virtuelles dreidimensionales Modell eines Musterobjekts speichert, das einem Zahn entspricht, wozu ein Musterzahn gehört, der dem Zahn entspricht;
- ein Datenverarbeitungssystem, das einen Speicher umfasst, der ein virtuelles dreidimensionales Modell mindestens eines Teils des Gebisses speichert;

wobei das Datenverarbeitungssystem ferner Software umfasst, die das virtuelle dreidimensionale Modell mindestens eines Teils des Gebisses und das virtuelle Modell des Musterobjekts verarbeitet und daraufhin das individuelle, dreidimensionale virtuelle Zahnmodell ableitet;
wobei die Software Folgendes umfasst: (1) eine Routine, die das virtuelle Musterobjekt, das einem Zahn entspricht, dem virtuellen Modell mindestens eines Teils des Gebisses überlagert; (2) eine Routine, die Vektoren von dem virtuellen Musterobjekt zu einer Oberfläche des virtuellen Modells des Gebisses zieht und einen Satz Punkte speichert, an denen die Vektoren die Oberfläche schneiden; (3) eine Routine, die eine neue Oberfläche bildet, die aus dem Satz Punkte besteht; und (4) eine Routine, die gegebenenfalls fehlende Teile der Oberfläche aus dem Musterzahn ausfüllt.

**2.** System nach Anspruch 1, wobei das Musterobjekt eine oder mehrere spezifische Charakteristiken eines Zahns umfasst, die aus der Gruppe ausgewählt werden, die aus Höckern, Zahnbreite, Okklusionscharakteristiken, zweidimensionalem Querschnitt in einer Okklusionsebene, Wurzeln und Planheit besteht.

**3.** System nach Anspruch 1, wobei das Musterobjekt eine Oberfläche umfasst, die aus einem Polygonflächennetz gebildet wird.

**4.** System nach Anspruch 1, wobei das Musterobjekt eine Wolke von dreidimensionalen Punkten umfasst.

**5.** System nach Anspruch 1, wobei das System ferner eine Benutzerschnittstelle umfasst, und wobei die Software das individuelle virtuelle Zahnmodell an der Benutzerschnittstelle anzeigt.

**6.** System nach Anspruch 5, wobei das System Software umfasst, die es einem Benutzer erlaubt, anhand der Benutzerschnittstelle eine Markierung auf einer virtuellen Darstellung des Zahns des Patienten zu setzen, wobei die Markierung verwendet wird, um das virtuelle Musterobjekt, das einem Zahn entspricht, auf das virtuelle dreidimensionale Modell mindestens eines Teils des Gebisses auszurichten.

**7.** System nach Anspruch 6, wobei die Benutzerschnittstelle eine Überlagerung des individuellen virtuellen Zahnmodells und des virtuellen dreidimensionalen Modells mindestens eines Teils des Gebisses des Patienten anzeigt.

**8.** System nach Anspruch 6, wobei das Datenverarbeitungssystem individuelle virtuelle Zahnmodelle für alle Zähne eines Patienten generiert und die virtuellen Zahnmodelle an einer Benutzerschnittstelle anzeigt.

**9.** System nach Anspruch 8, wobei die virtuellen Zahnmodelle an der Benutzerschnittstelle in einer Position im Verhältnis zueinander in drei Dimensionen angezeigt werden, die im Wesentlichen mit der aktuellen Position der Zähne im Gebiss des Patienten übereinstimmen.

**10.** System nach Anspruch 1, wobei das Software-Modul die Routinen (1), (2), (3) und (4) iterativ ausführt, wobei die Oberfläche nach dem Ausführen von Schritt (3) oder Schritt (4) das virtuelle Modell des Gebisses in Schritt (1) in aufeinanderfolgenden Iterationen ersetzt.

**11.** System nach Anspruch 10, wobei [wenn] die iterative Ausführung der Schritte (1), (2), (3) und (4) beendet ist, die Oberfläche, die nach der endgültigen Iteration generiert ist, für den Benutzer als Überlagerung von Originaldaten, die durch Scannen erzielt werden, und der Oberfläche, die von der Software am Ende von Schritt (3) oder Schritt (4) nach der letzten Iteration erstellt wird, angezeigt wird.

## Revendications

**1.** Système de création d'un modèle de dent individuel, tridimensionnel, virtuel représentant une dent figurant dans une dentition attribuée à un patient, comprenant :

une mémoire qui mémorise au moins un modèle virtuel tridimensionnel d'une instance de patron correspondant à une dent, comportant un patron de dent correspondant à ladite dent ;

un système de traitement de données comportant une mémoire qui mémorise un modèle virtuel tridimensionnel d'au moins une partie de la dentition ;
dans lequel ledit système de traitement de données comprend en outre un logiciel qui traite ledit modèle virtuel tridimensionnel d'au moins une partie de la dentition et ledit modèle virtuel de l'instance de patron et dérive en réponse ledit modèle de dent individuel, tridimensionnel, virtuel ;
dans lequel ledit logiciel comprend (1) un sous-programme qui superpose ladite instance de patron virtuelle correspondant à une dent sur ledit modèle virtuel d'au moins une partie de la dentition ; (2) un sous-programme qui dessine des vecteurs depuis ladite instance de patron virtuelle jusqu'à une surface dudit modèle virtuel de la dentition et mémorise un ensemble de points où lesdits vecteurs intersectent ladite surface ; (3) un sous-programme qui forme une nouvelle surface composée dudit ensemble de points ; et (4) un sous-programme qui remplit des parties manquantes, éventuelles, de ladite surface, dudit patron de dent.

**2.** Système selon la revendication 1, dans lequel ladite instance de patron comprend une ou plusieurs caractéristiques spécifiques d'une dent sélectionnées dans le groupe consistant en cuspides, largeur de dent, caractéristiques d'occlusion, coupe transversale bidimensionnelle dans un plan d'occlusion, racines et planéité.

**3.** Système selon la revendication 1, dans lequel ladite instance de patron comprend une surface formée d'une maille de surfaces polygonales.

**4.** Système selon la revendication 1, dans lequel ladite instance de patron comprend un nuage de points tridimensionnels.

**5.** Système selon la revendication 1, dans lequel ledit système comprend en outre une interface utilisateur, et dans lequel ledit logiciel affiche ledit modèle de dent individuel virtuel sur ladite interface utilisateur.

**6.** Système selon la revendication 5, dans lequel ledit système comporte un logiciel qui permet à un utilisateur de placer un repère sur une représentation virtuelle de ladite dent du patient par l'intermédiaire de ladite interface utilisateur, ledit repère servant à aligner ladite instance de patron virtuelle correspondant à une dent avec ledit modèle tridimensionnel virtuel d'au moins une partie de la dentition.

**7.** Système selon la revendication 6, dans lequel ladite interface utilisateur affiche une superposition dudit modèle de dent individuel virtuel et dudit modèle virtuel tridimensionnel d'au moins une partie de la dentition du patient.

**8.** Système selon la revendication 6, dans lequel ledit système de traitement de données génère des modèles de dents individuels virtuels de toutes les dents d'un patient et affiche lesdits modèles de dents virtuels sur une interface utilisateur.

**9.** Système selon la revendication 8, dans lequel lesdits modèles de dents virtuels sont affichés en trois dimensions sur ladite interface utilisateur à une position relative aux autres qui correspond sensiblement à la position courante des dents dans la dentition du patient.

**10.** Système selon la revendication 1, dans lequel le module logiciel exécute des sous-programmes (1), (2), (3) et (4) de façon itérative, la surface après l'exécution de l'étape (3) ou de l'étape (4) remplaçant le modèle virtuel de la dentition à l'étape (1) par itérations successives.

**11.** Système selon la revendication 10, dans lequel ladite exécution itérative des étapes (1), (2), (3) et (4) se termine, la surface générée après la dernière itération est affichée à l'utilisateur en tant que superposition de données initiales obtenues par balayage et de la surface créée par ledit logiciel à la fin de l'étape (3) ou de l'étape (4) après la dernière itération.

FIG. 1

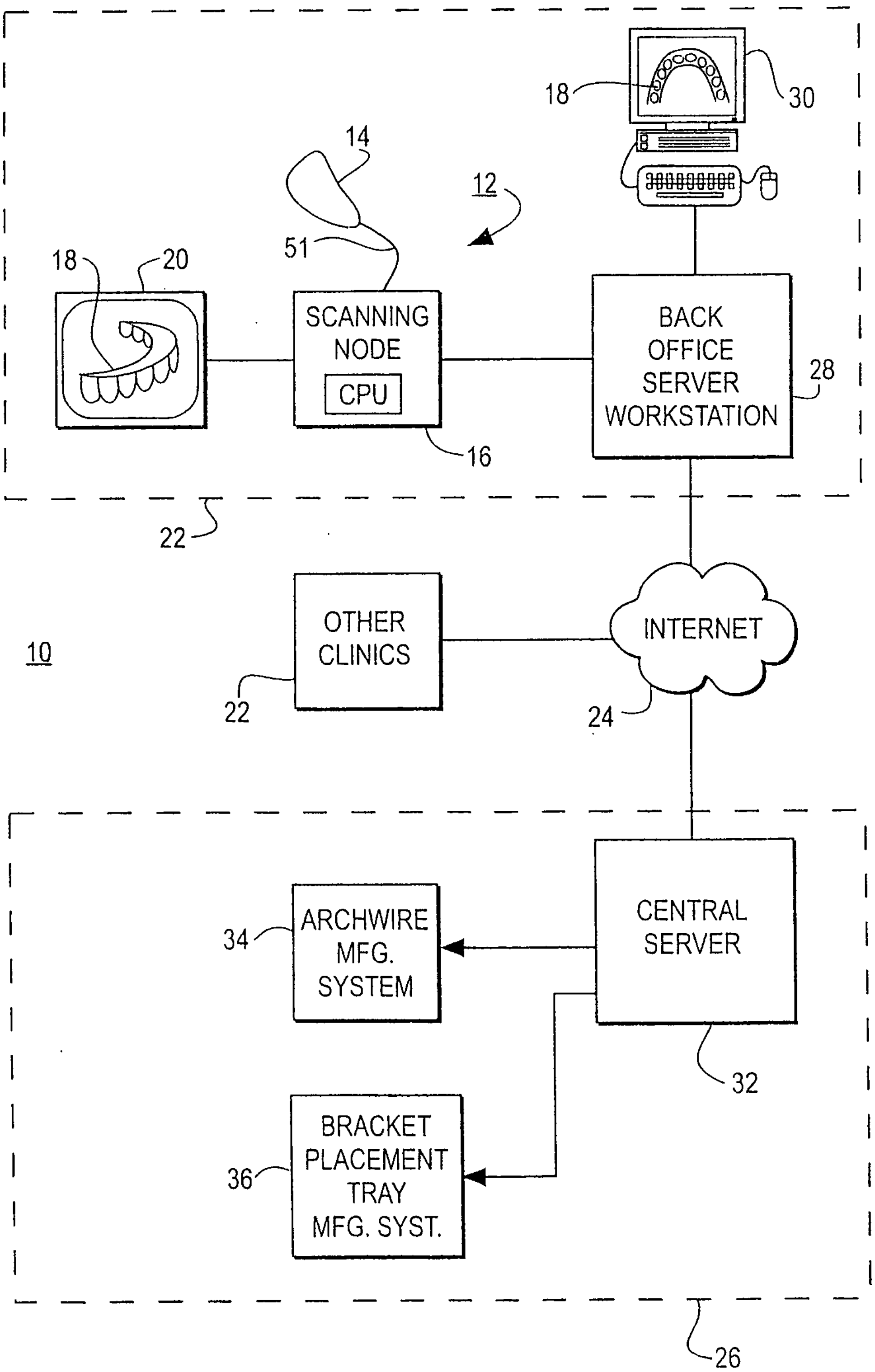
14
12
18
30
51
18
20
SCANNING NODE
CPU
BACK OFFICE SERVER WORKSTATION
28
16
22
OTHER CLINICS
INTERNET
10
22
24
CENTRAL SERVER
ARCHWIRE MFG. SYSTEM
34
32
BRACKET PLACEMENT TRAY MFG. SYST.
36
26

FIG. 2

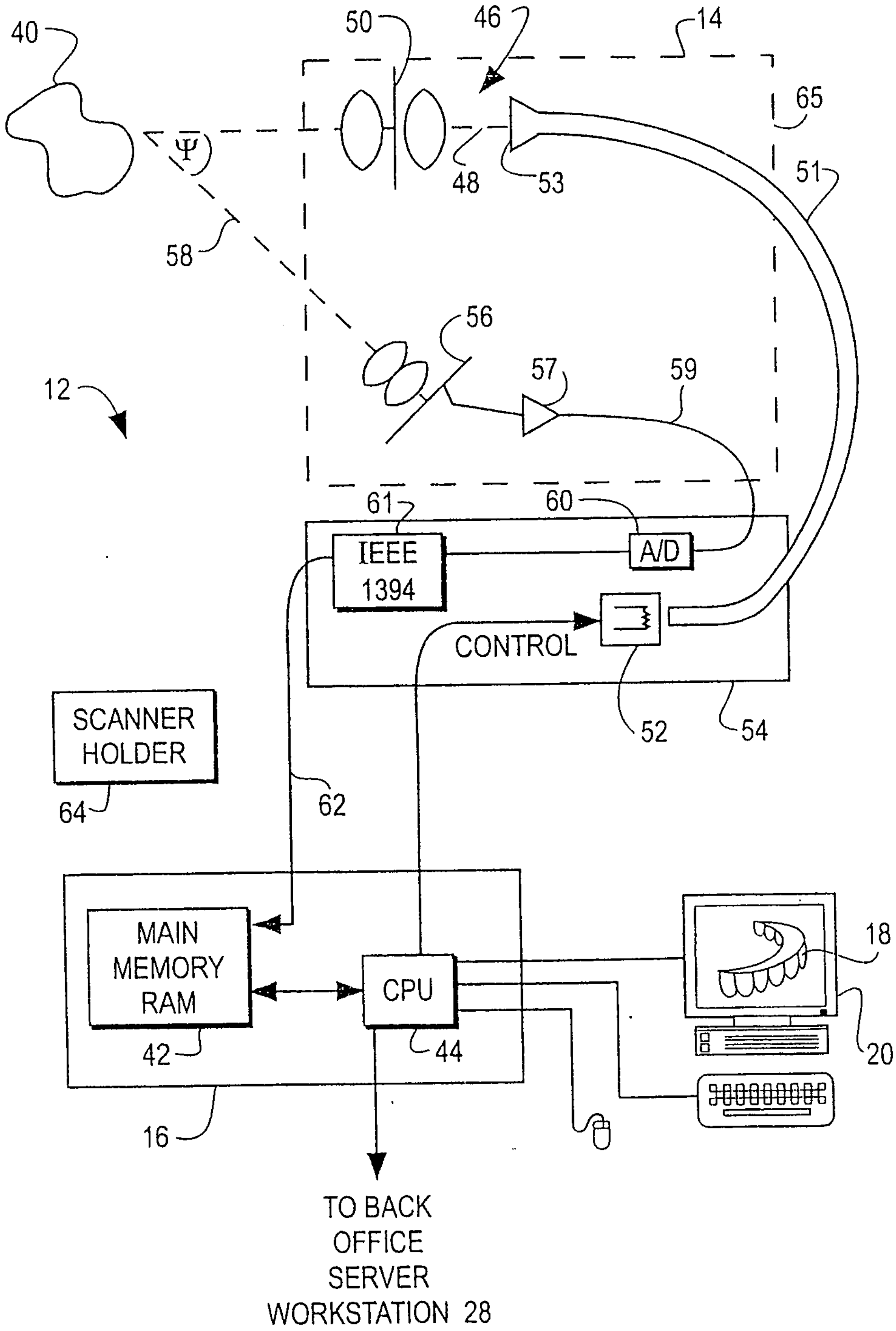
40
50
46
14
65
Ψ
48
53
51
58
56
57
59
12
61
60
IEEE
1394
A/D
CONTROL
52
54
SCANNER
HOLDER
64
62
MAIN
MEMORY
RAM
CPU
42
44
18
20
16
TO BACK
OFFICE
SERVER
WORKSTATION 28

FIG. 3

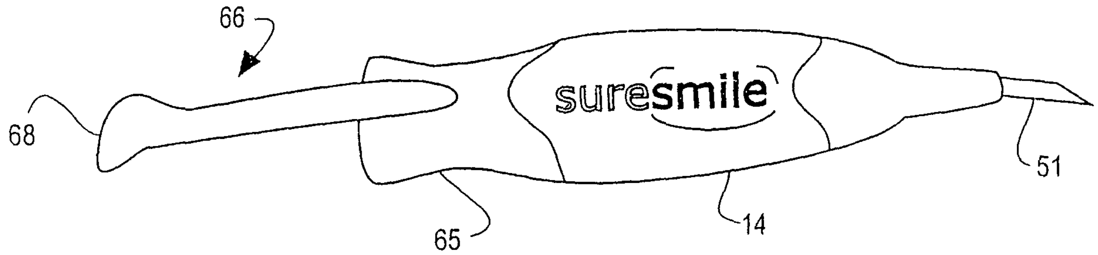


FIG. 4

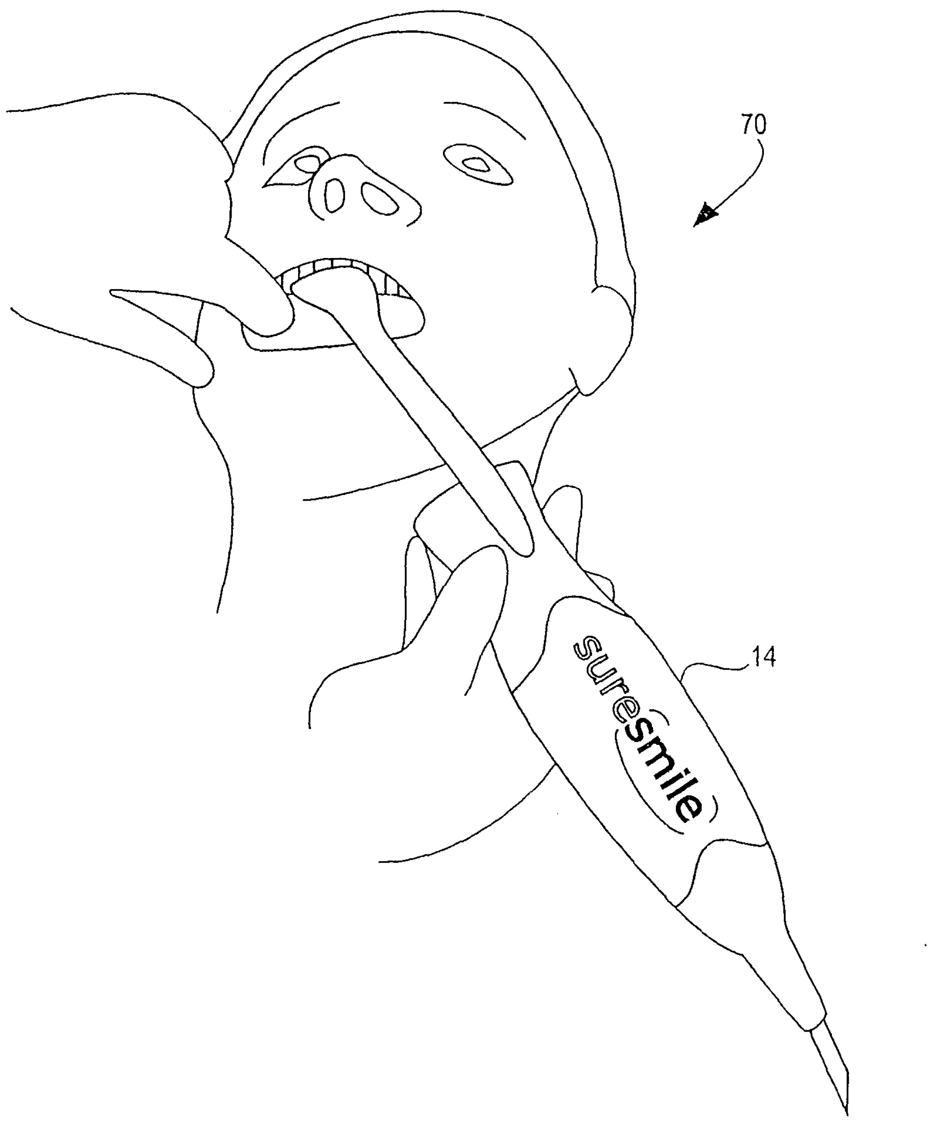

FIG. 5

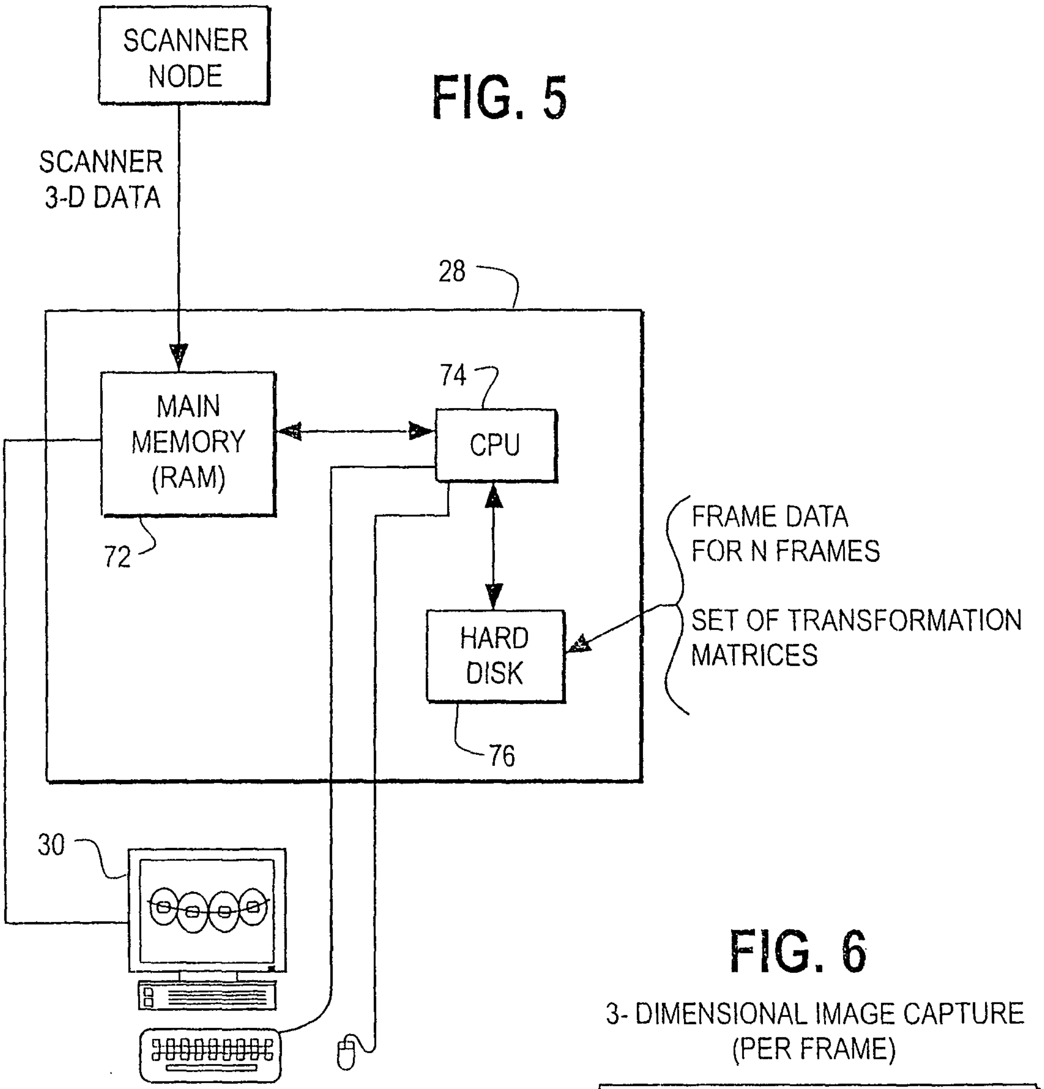
SCANNER NODE
SCANNER 3-D DATA
28
MAIN MEMORY (RAM)
72
74
CPU
HARD DISK
76
FRAME DATA FOR N FRAMES
SET OF TRANSFORMATION MATRICES
30

FIG. 6

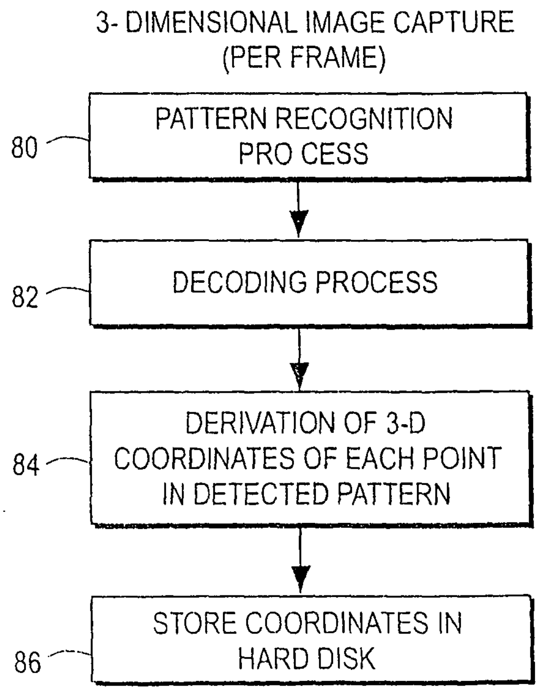
3- DIMENSIONAL IMAGE CAPTURE (PER FRAME)
80
PATTERN RECOGNITION PRO CESS
82
DECODING PROCESS
84
DERIVATION OF 3-D COORDINATES OF EACH POINT IN DETECTED PATTERN
86
STORE COORDINATES IN HARD DISK

FIG. 7

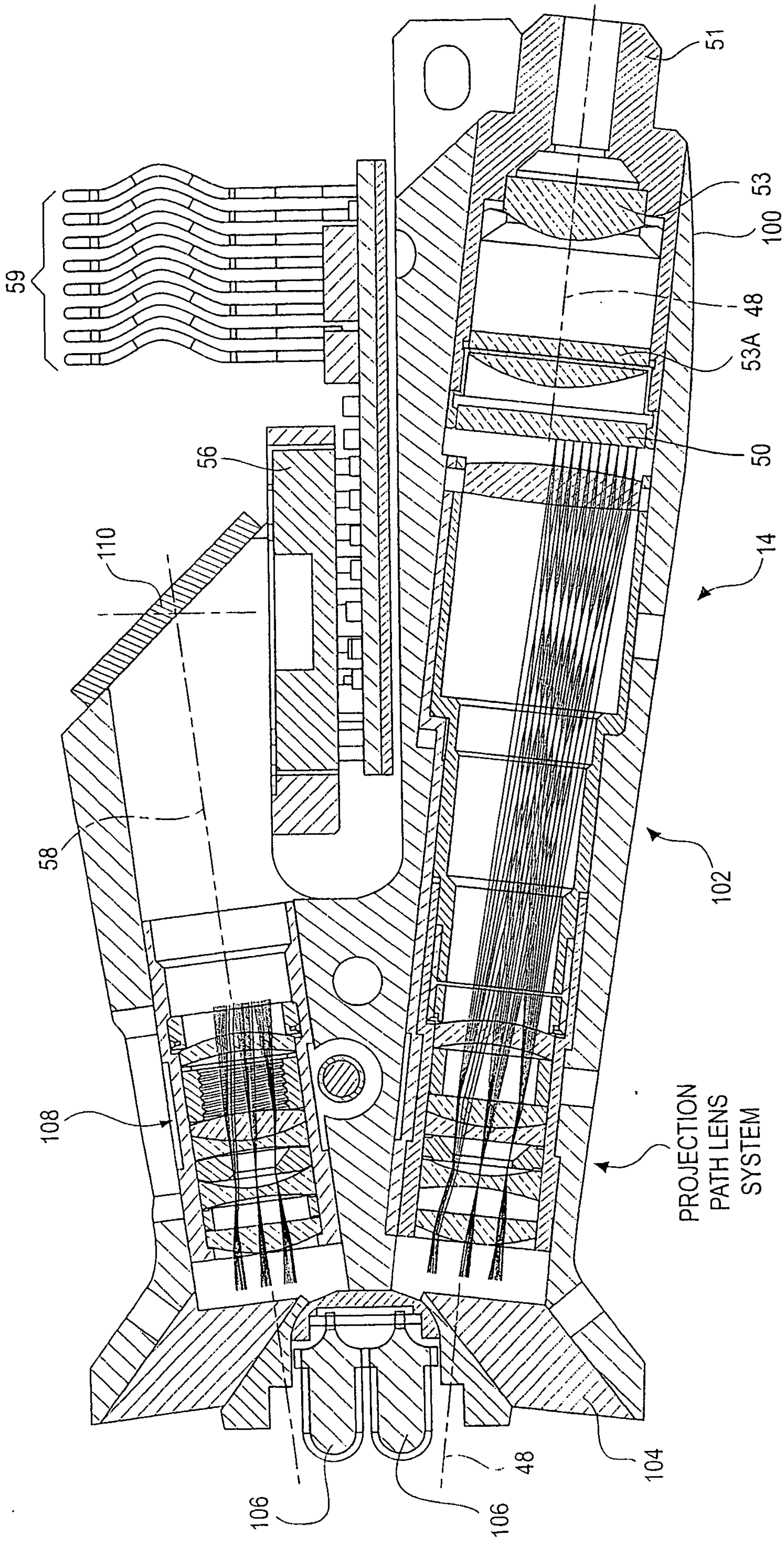
59
56
110
58
108
51
53
100
48
53A
50
14
102
PROJECTION
PATH LENS
SYSTEM
104
48
106
106

FIG. 8

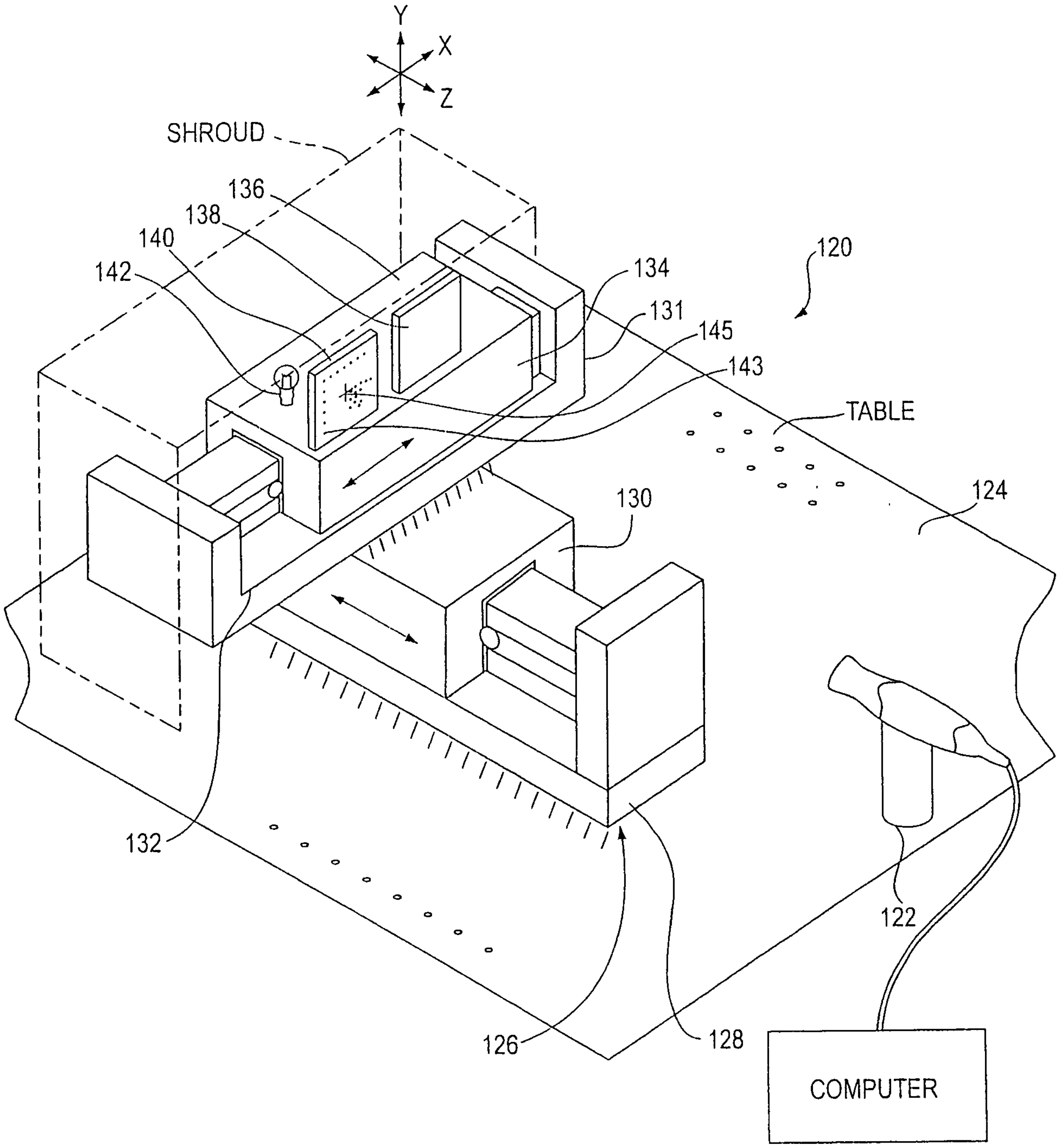
Y
X
Z
SHROUD
136
138
140
142
120
134
131
145
143
TABLE
124
130
132
122
126
128
COMPUTER

FIG. 8A

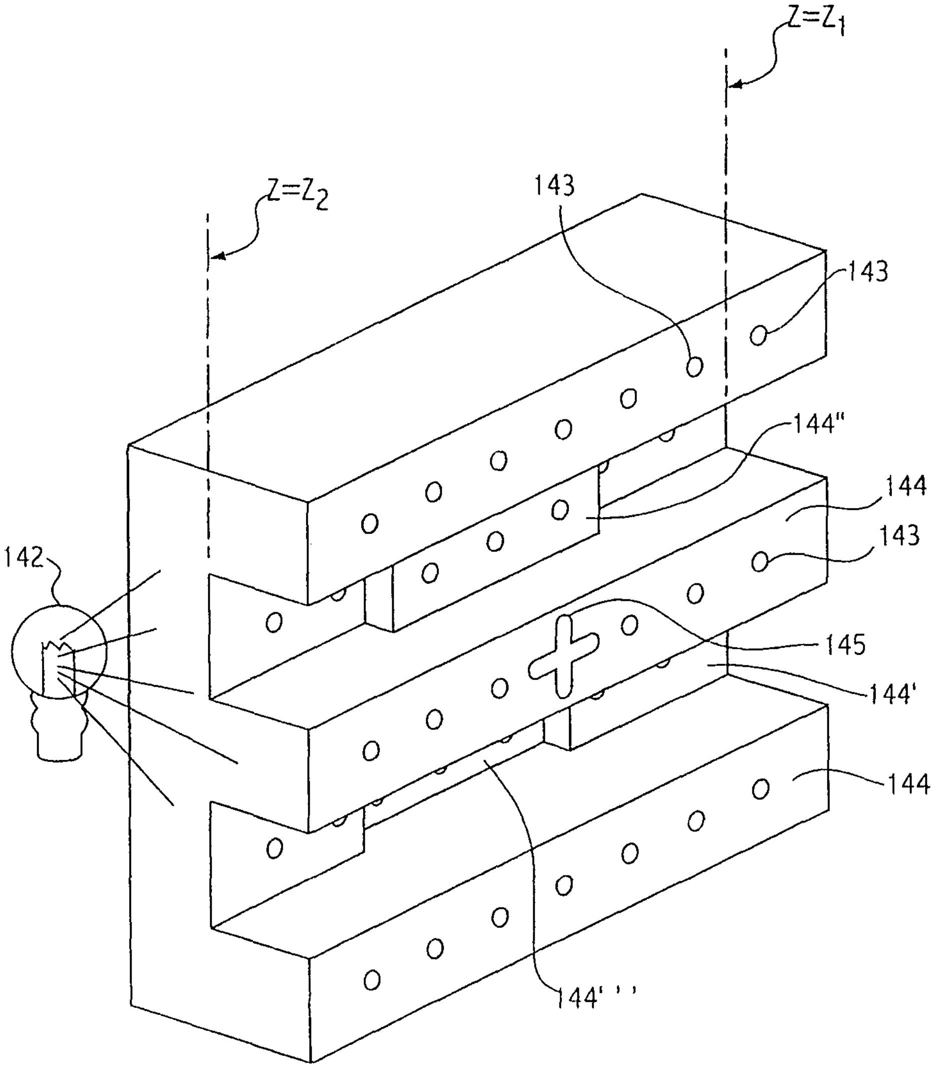

FIG. 9

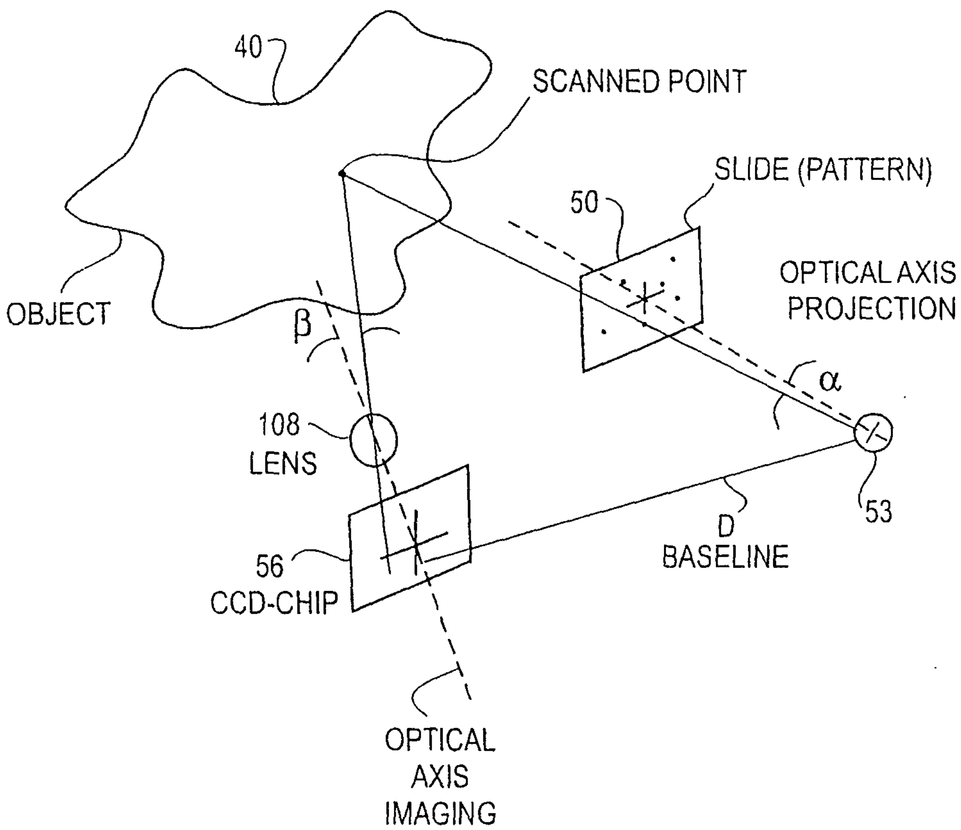
40
SCANNED POINT
SLIDE (PATTERN)
50
OPTICAL AXIS
PROJECTION
OBJECT
β
α
108
LENS
53
D
BASELINE
56
CCD-CHIP
OPTICAL
AXIS
IMAGING

FIG. 9A

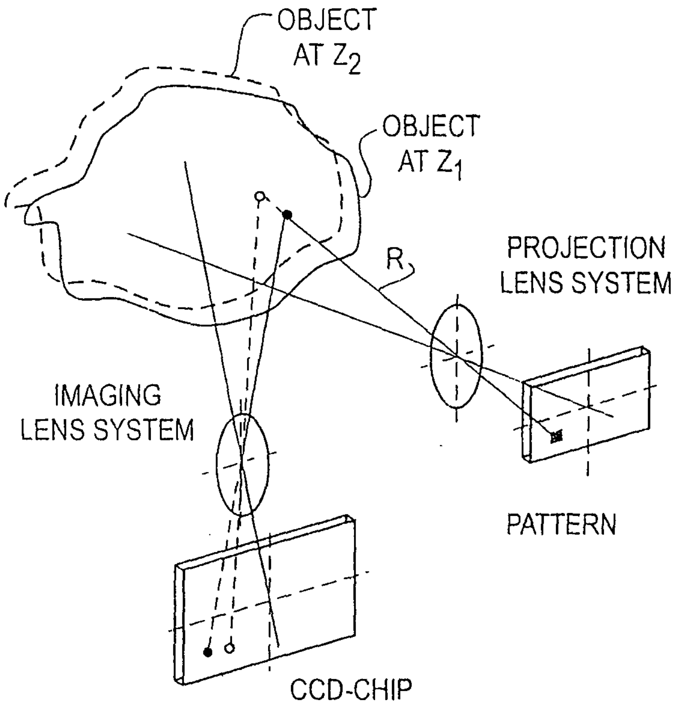


FIG. 9B

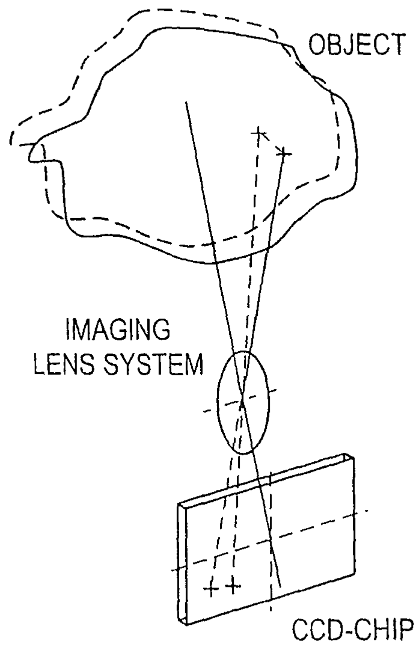


FIG. 9C

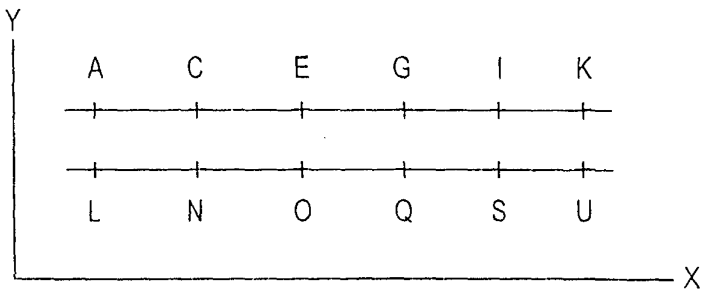


PIXEL COORDINATES FOR PORTIONS OF THE PATTERN ASSIGNED TO A CERTAIN Z-LEVEL

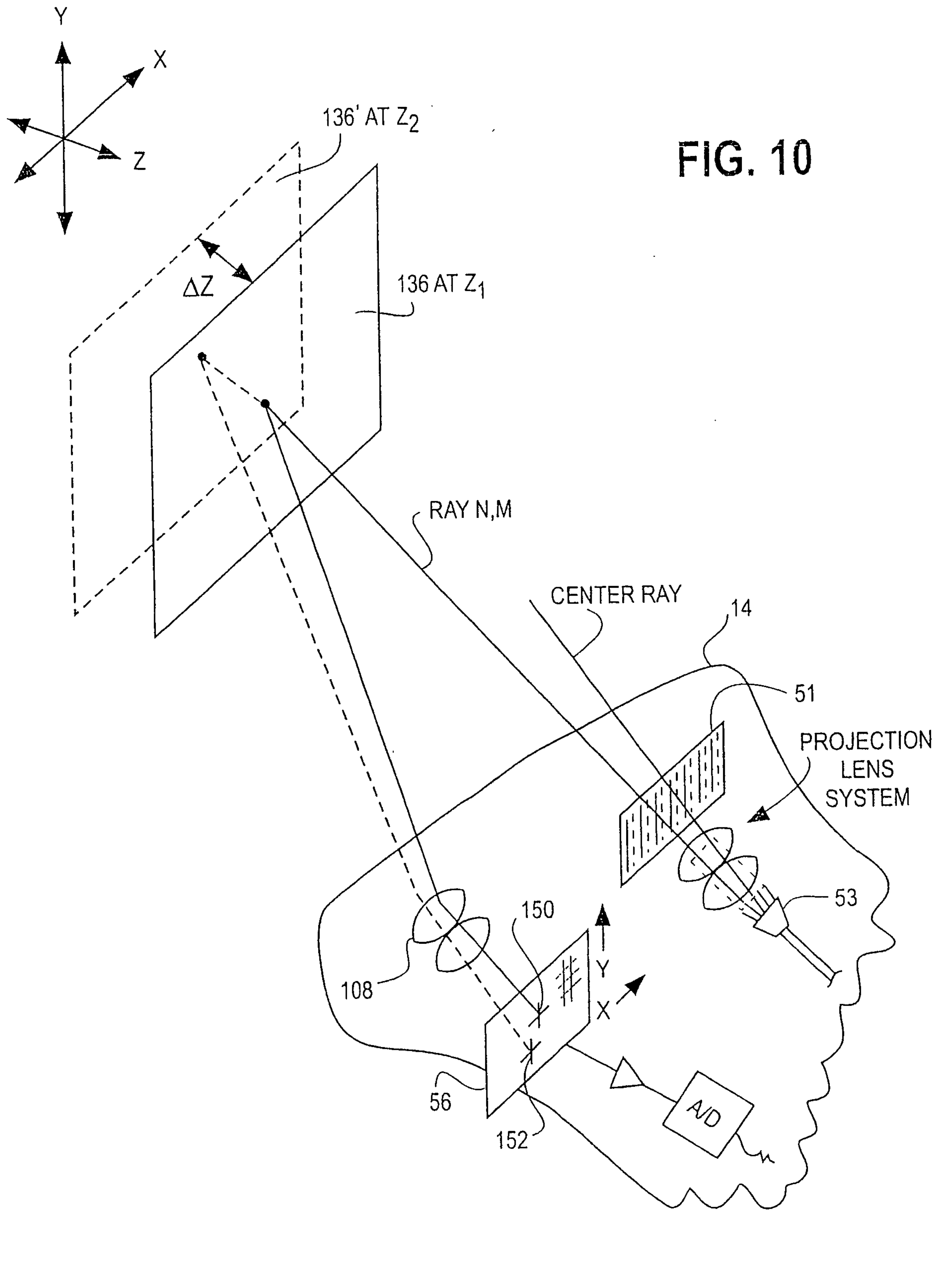
Y
X
Z
136' AT $Z_2$
ΔZ
136 AT $Z_1$
RAY N,M
CENTER RAY
14
51
PROJECTION
LENS
SYSTEM
53
150
108
Y
X
56
152
A/D

FIG. 10

FIG. 11

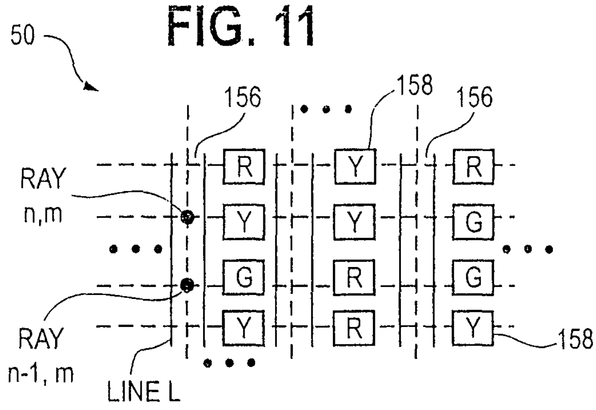


FIG. 12

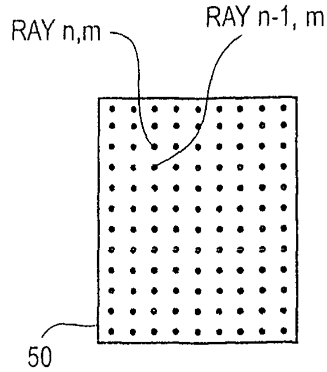


FIG. 13

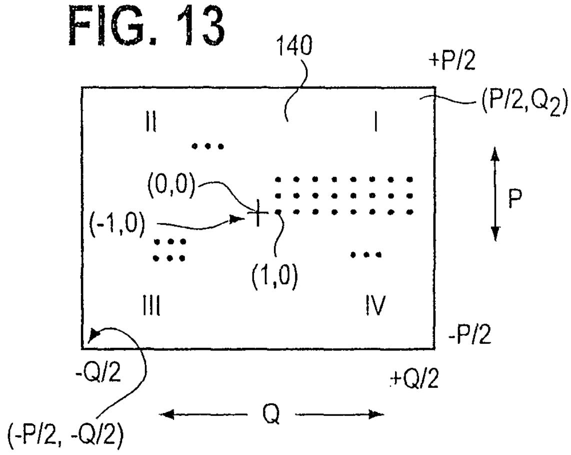


FIG. 14

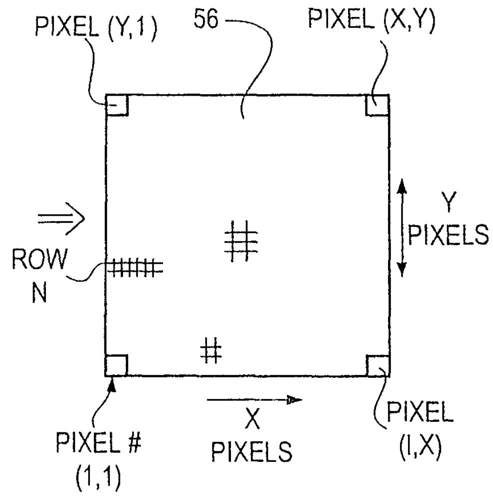


FIG. 15

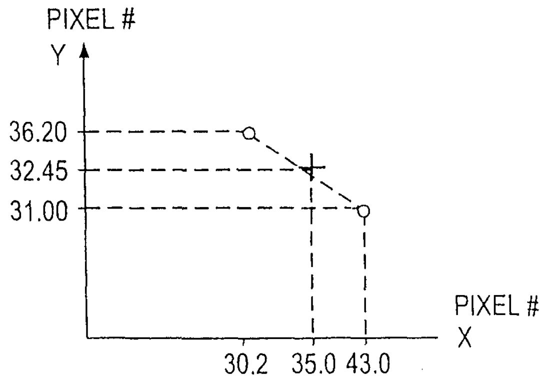


FIG. 16

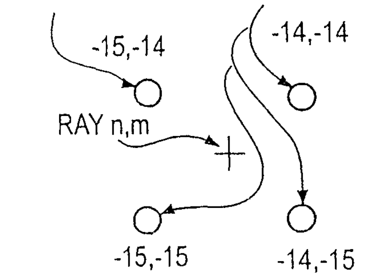

FIG. 17

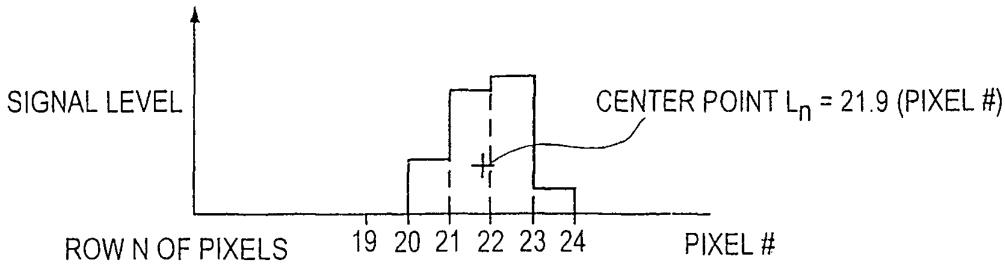


FIG. 18

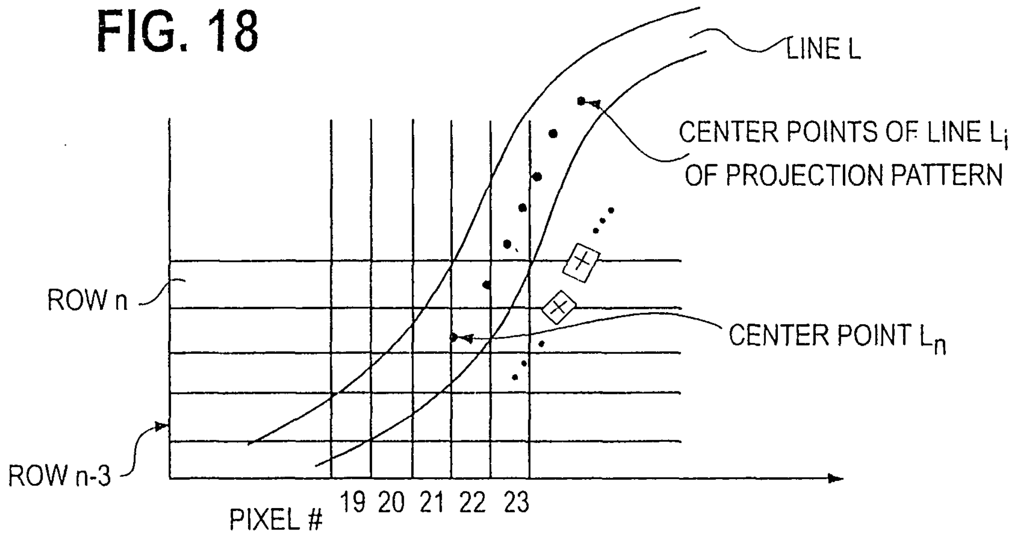


FIG. 19

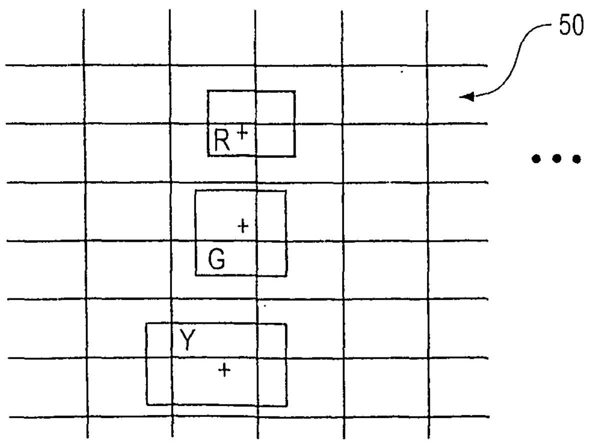

FIG. 20

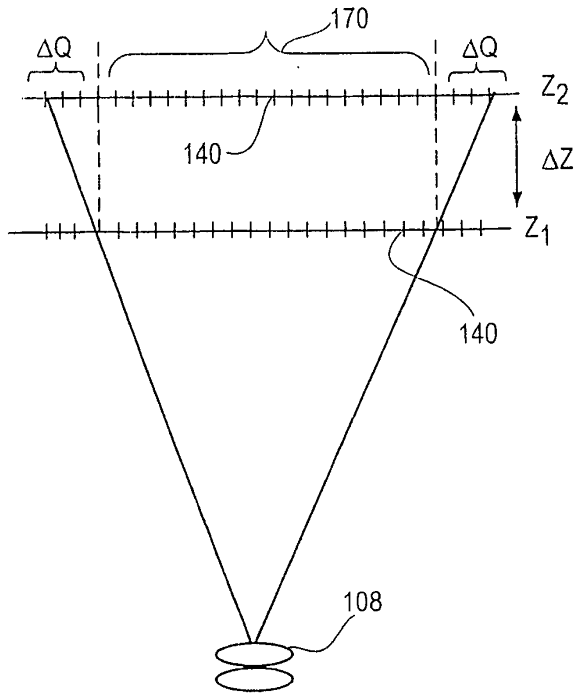
170
ΔQ
ΔQ
Z2
140
ΔZ
Z1
140
108

FIG. 21

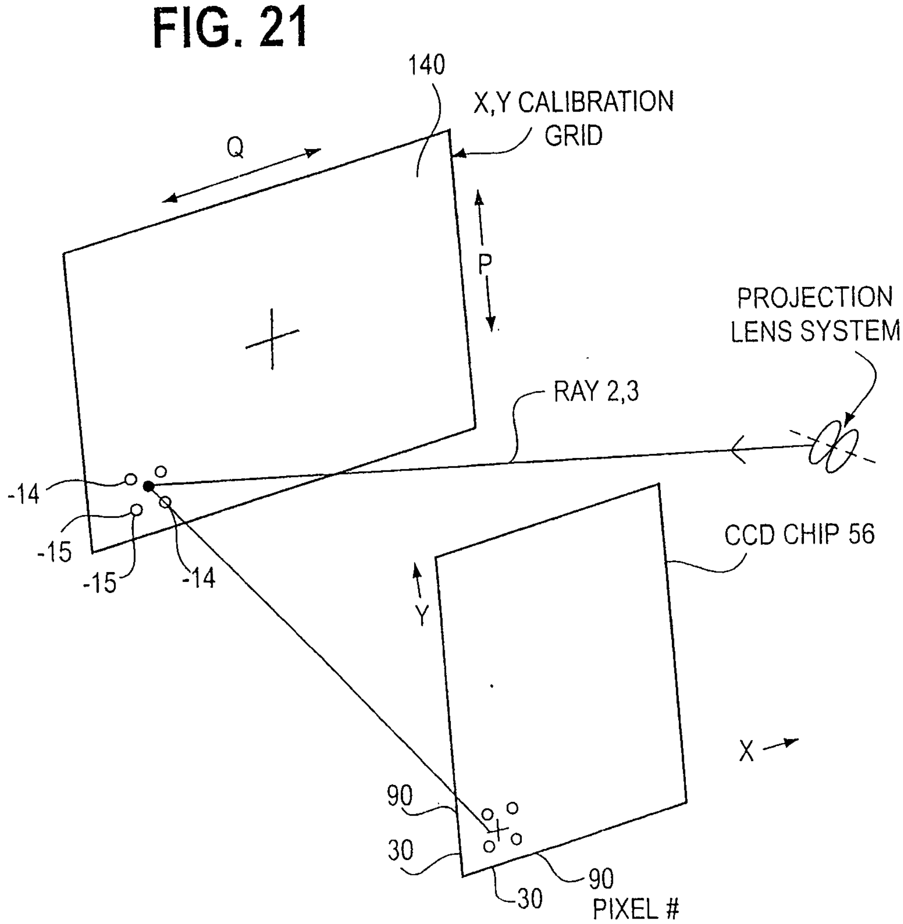
140
X,Y CALIBRATION GRID
Q
P
PROJECTION LENS SYSTEM
RAY 2,3
-14
-15
-15
-14
CCD CHIP 56
Y
X
90
30
30
90
PIXEL #

FIG. 22

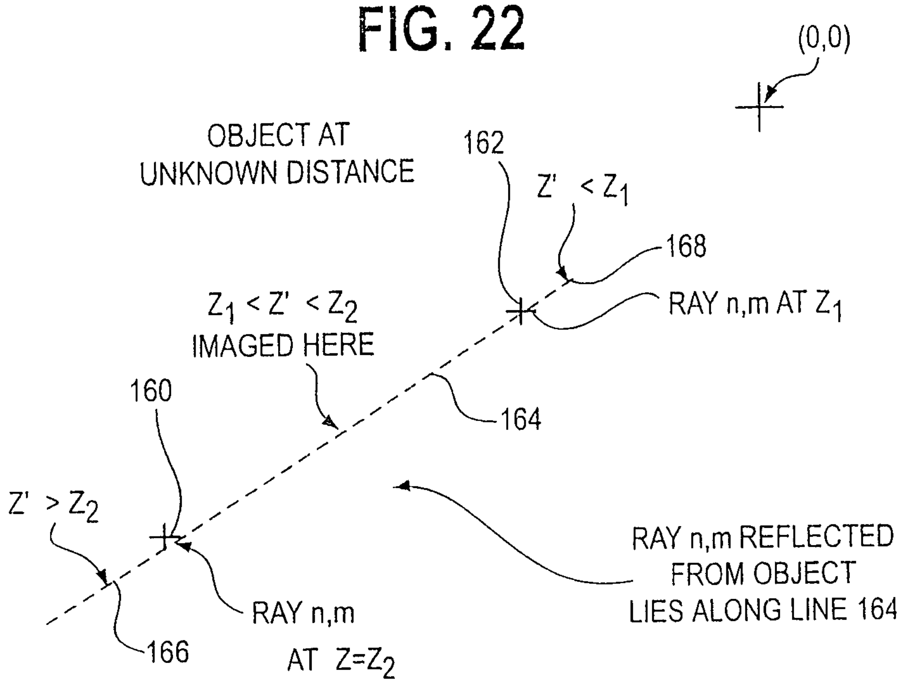
(0,0)
OBJECT AT
UNKNOWN DISTANCE
162
Z' < Z1
168
RAY n,m AT Z1
Z1 < Z' < Z2
IMAGED HERE
160
164
Z' > Z2
RAY n,m REFLECTED
FROM OBJECT
LIES ALONG LINE 164
166
RAY n,m
AT Z=Z2

FIG. 23

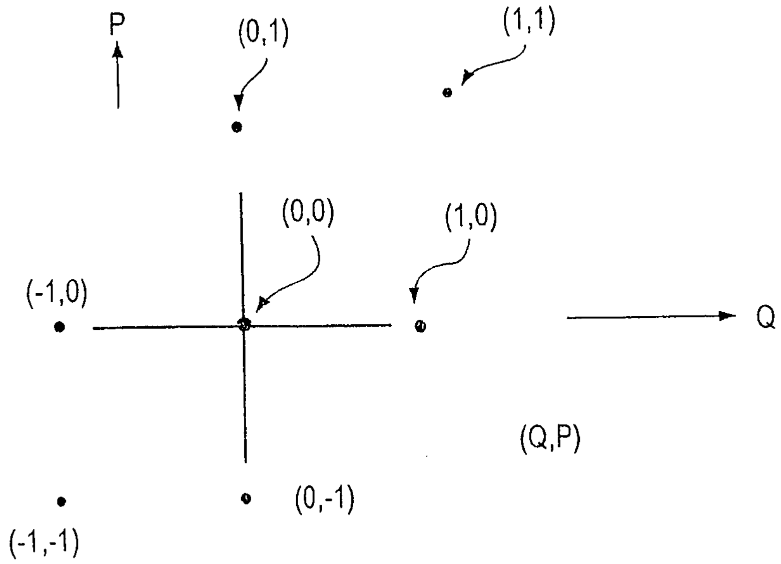
P
(0,1)
(1,1)
(0,0)
(1,0)
(-1,0)
Q
(Q,P)
(0,-1)
(-1,-1)

# FIG. 24

$CCD_X$, $CCD_Y$ = PIXEL #, IN SUBPIXEL RESOLUTION

CALIBRATION TABLE #1

(BEFORE)

| | | LINE 1 | | | | | | LINE 2 | | | | | • • • | LINE N | | | |
|---|---|---|---|---|---|---|---|---|---|---|---|---|---|---|---|---|---|
| | | ROW 1 | ROW 2 | ROW 3 | ROW 4 | • • • | ROW M | ROW 1 | ROW 2 | ROW 3 | ROW 4 | • • • | ROW M | ROW 1 | ROW 2 | • • • | ROW M |
| $Z_1$ | $CCD_X$ | 1.0 | 1.1 | 1.5 | 2.1 | | • • • | 27.1 | 29.5 | 30.2 | 37.1 | | | | | | |
| | MM DIST. | | | | | | | | | | | | | | | | |
| | $CCD_Y$ | 10.2 | 20.4 | 32.8 | 44.5 | | | 11.5 | 21.6 | 36.2 | 44 | | | | | | |
| | MM DIST. | | | | | | | | | | | | | | | | |
| $Z_2$ | $CCD_X$ | 3.9 | 4.5 | 6.8 | 12.2 | | | 34.0 | 41.1 | 43.0 | 46 | | | | | | |
| | MM DIST. | | | | | | | | | | | | | | | | |
| | $CCD_Y$ | 12.1 | 21.5 | 30.4 | 46.3 | | | 13.2 | 21.8 | 31.0 | 48.2 | | | | | | |
| | MM DIST. | | | | | | | | | | | | | | | | |

# FIG. 25

CALIBRATION TABLE #2 (Q,P)

QUADRANT I — ROW 0 — ROW 1 — ROW + P/2

| | | (0,0) | (1,0) | (2,0) | (3,0) | • • • | (Q/2 - ΔQ,0) | • • • | (Q/210) | (0,1) | (1,1) | (2,1) | • • • | • • • | (0, P/2) | (1, P/2) | • • • | (Q/2, P/2) |
|---|---|---|---|---|---|---|---|---|---|---|---|---|---|---|---|---|---|---|
| $Z_1$ | $CCD_X$ | 640.1 | 700.2 | 760.6 | 820.5 | • • • | • • • | | — | 640.1 | 700.2 | • • • | | | • • • | • • • | | 1,279.5 |
| | $CCD_Y$ | 640.1 | 640.1 | 640.3 | 640.4 | | • • • | | — | 701.2 | 701.5 | • • • | | | • • • | • • • | | 1,279.4 |
| $Z_2$ | $CCD_X$ | 640.2 | 680.3 | 741.2 | 801.6 | | • • • | | 1,279.5 | • • • | 681.2 | • • • | | | • • • | • • • | | 1,256.4 |
| | $CCD_Y$ | 640.2 | 640.3 | 640.1 | 640.1 | | • • • | | 640.2 | • • • | 680.9 | • • • | | | • • • | • • • | | 1,251.5 |

QUADRANT II — ROW — ROW 1 — ROW +P/2

| | | (-1,0) | (-2,0) | (-3,0) | (-4, 0) | • • • | (-Q/2 - ΔQ,0) | • • • | (-Q/210) | (-1,1) | (-2,1) | (-3,1) | • • • | • • • | (-1, P/2) | (-2, P/2) | • • • | (-Q/2, P/2) |
|---|---|---|---|---|---|---|---|---|---|---|---|---|---|---|---|---|---|---|
| $Z_1$ | $CCD_X$ | | | | | | | | | | | | | | | | | |
| | $CCD_Y$ | | | | | | | | | | | | | | | | | |
| $Z_2$ | $CCD_X$ | | | | | | | | | | | | | | | | | |
| | $CCD_Y$ | | | | | | | | | | | | | | | | | |

QUADRANT III

| | | (-1,-1) | (-2,-1) | • • • | • • • | • • • |
|---|---|---|---|---|---|---|
| $Z_1$ | $CCD_X$ | — | — | | | |
| | $CCD_Y$ | — | — | | | |
| $Z_2$ | $CCD_X$ | — | — | | | |
| | $CCD_Y$ | — | — | | | |

QUADRANT IV

| | | (0,-1) | (1,-1) | • • • | • • • | • • • |
|---|---|---|---|---|---|---|
| $Z_1$ | $CCD_X$ | — | — | | | |
| | $CCD_Y$ | — | — | | | |
| $Z_2$ | $CCD_X$ | — | — | | | |
| | $CCD_Y$ | — | — | | | |

# FIG. 26

$CCD_X$, $CCD_Y$ = PIXEL #, IN SUBPIXEL RESOLUTION

CALIBRATION TABLE #1 (AFTER)

| | | PATTERN LINE 1 | | | | | | PATTERN LINE 2 | | | | | | LINE N | | | |
|---|---|---|---|---|---|---|---|---|---|---|---|---|---|---|---|---|---|
| | | ROW 1 | ROW 2 | ROW 3 | ROW 4 | • • • | ROW M | ROW 1 | ROW 2 | ROW 3 | ROW 4 | • • • | ROW M | ROW 1 | ROW 2 | • • • | ROW M |
| $Z_1$ | $CCD_X$ | 1.0 | 1.1 | 1.5 | 2.1 | | • • • | 27.1 | 29.5 | 30.2 | 37.1 | | | | | | |
| | MM DIST. | | | | | | | | | -14.6 | | | | | | | |
| | $CCD_Y$ | 10.2 | 20.4 | 32.8 | 44.5 | | | 11.5 | 21.6 | 36.2 | 44 | | | | | | |
| | MM DIST. | | | | | | | | | -14.4 | | | | | | | |
| $Z_2$ | $CCD_X$ | 3.9 | 4.5 | 6.8 | 12.2 | | | 34.0 | 41.1 | 43.0 | 46 | | | | | | |
| | MM DIST. | | | | | | | | | -14.8 | | | | | | | |
| | $CCD_Y$ | 12.1 | 21.5 | 30.4 | 46.3 | | | 13.2 | 21.8 | 31.0 | 48.2 | | | | | | |
| | MM DIST. | | | | | | | | | -15.8 | | | | | | | |

Fig. 27

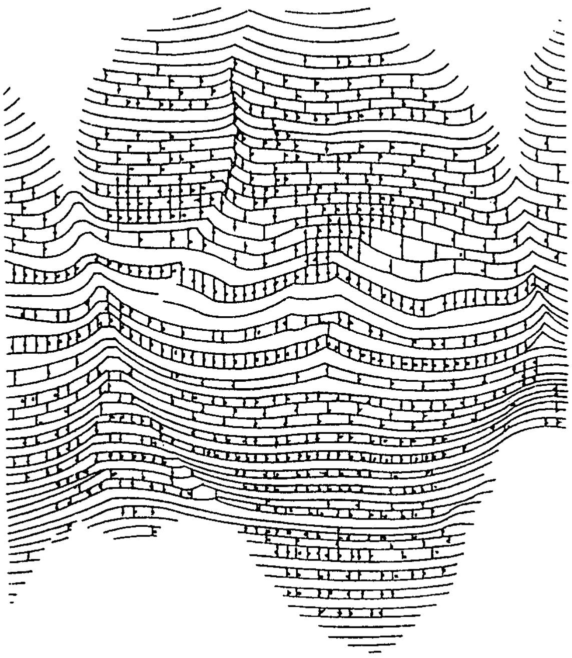

Fig. 28

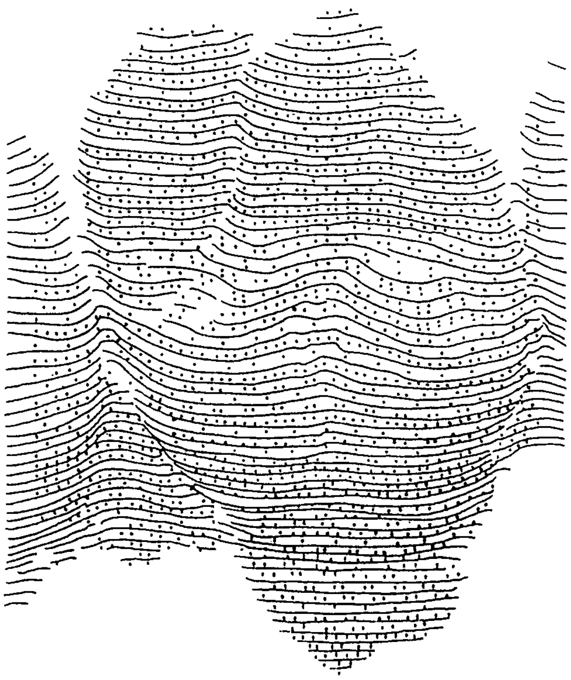

Fig. 29

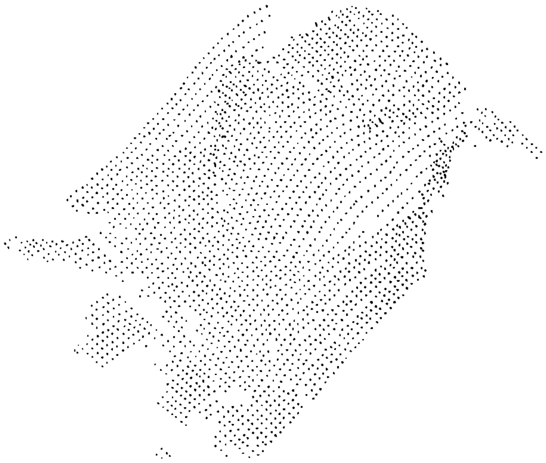

Fig. 30

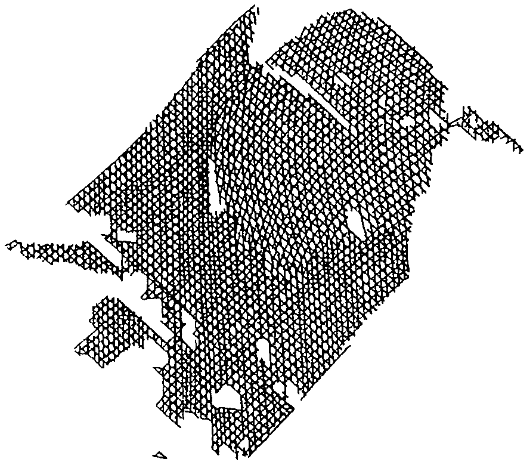

Fig. 31

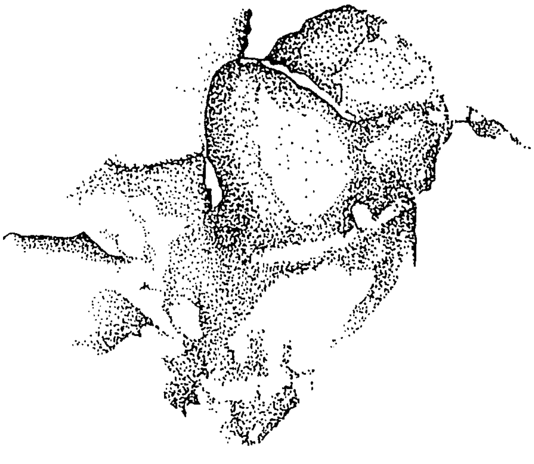

Fig. 32

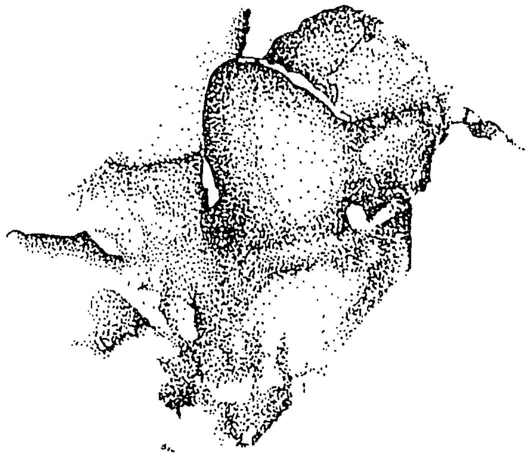

Fig. 33

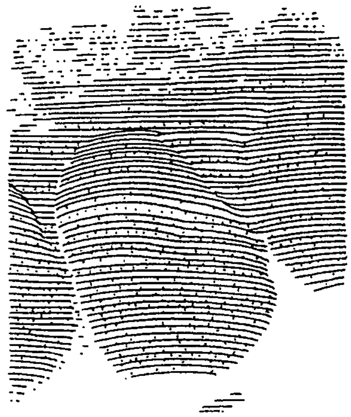

Fig. 34

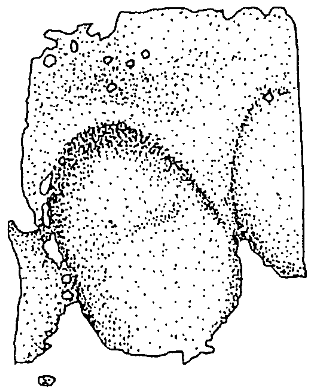

Fig. 35

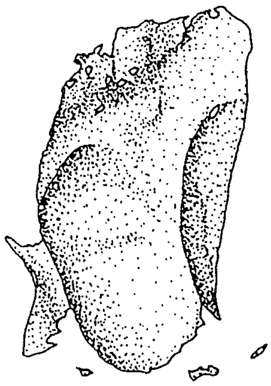

FIG. 36

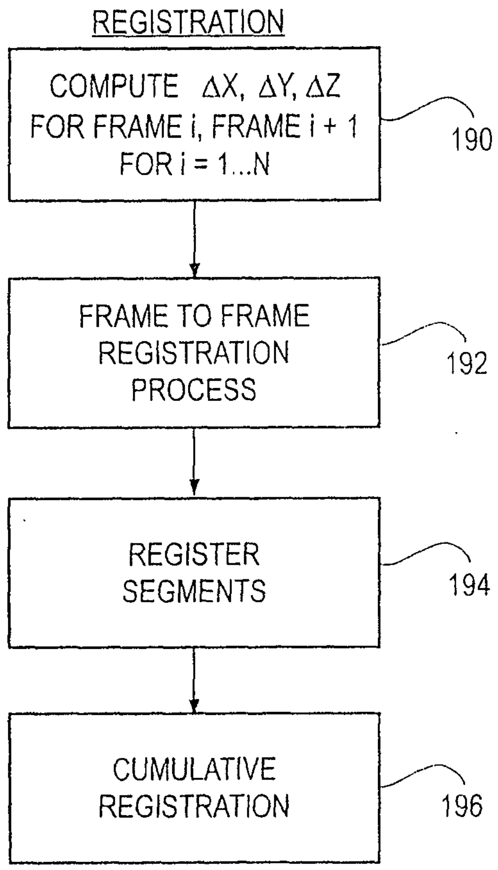
REGISTRATION
COMPUTE ΔX, ΔY, ΔZ FOR FRAME i, FRAME i + 1 FOR i = 1...N
190
FRAME TO FRAME REGISTRATION PROCESS
192
REGISTER SEGMENTS
194
CUMULATIVE REGISTRATION
196

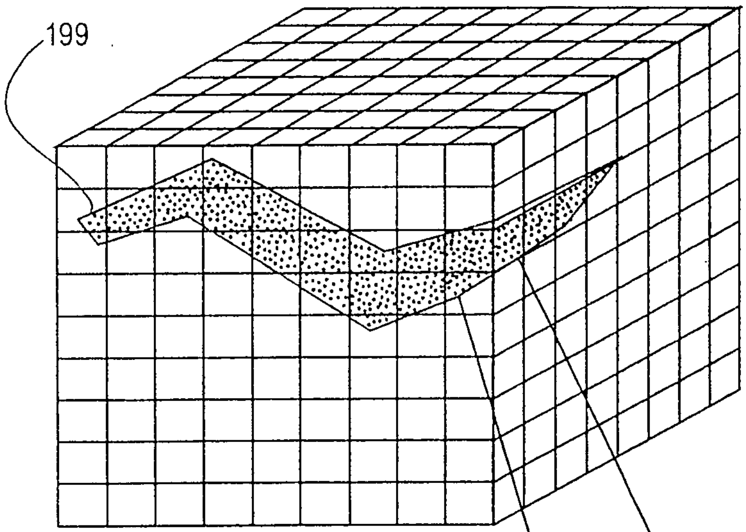


FIG. 37A

FIG. 37B

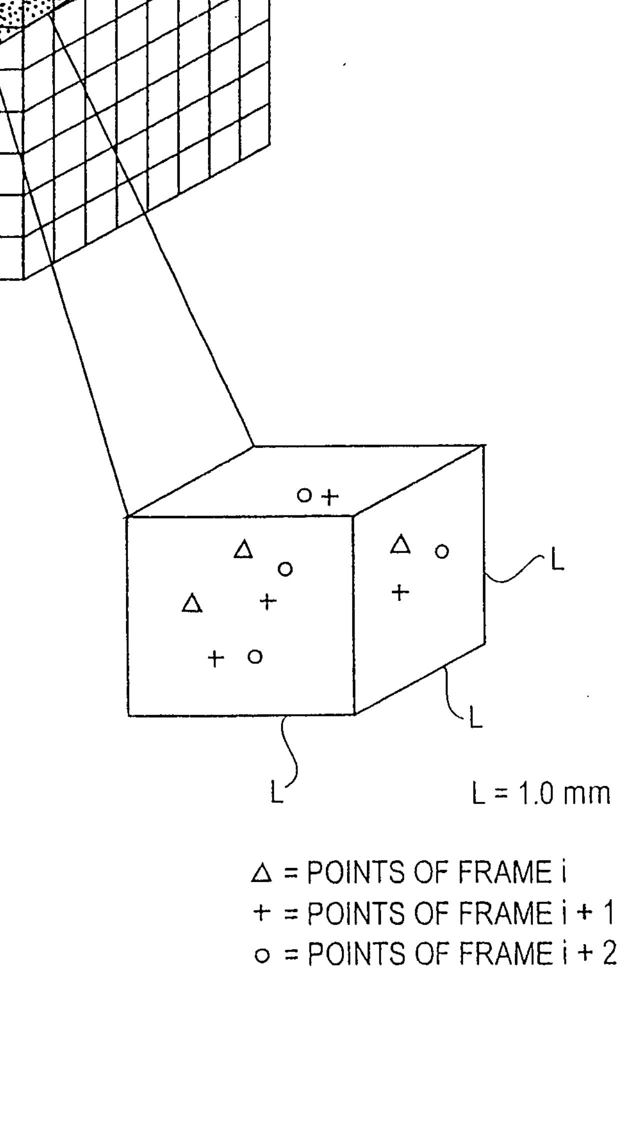

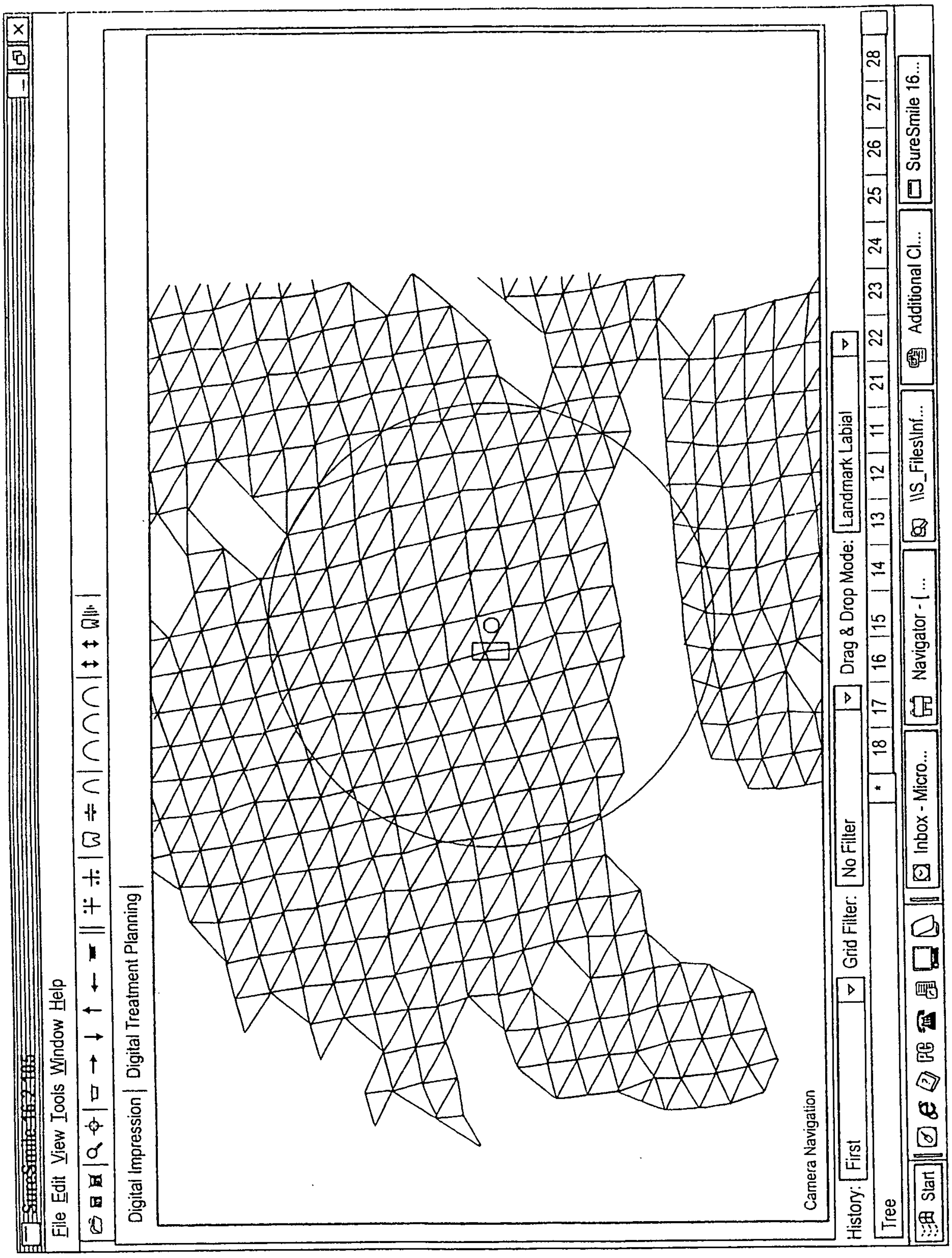
SureSmile 16.2.105
File Edit View Tools Window Help
Digital Impression
Digital Treatment Planning
Camera Navigation
History: First
Grid Filter: No Filter
Drag & Drop Mode: Landmark Labial
18 17 16 15 14 13 12 11 21 22 23 24 25 26 27 28
Tree
Start
Inbox - Micro...
Navigator - ( ...
\\S_FilesInf...
Additional Cl...
SureSmile 16...

FIG. 37C

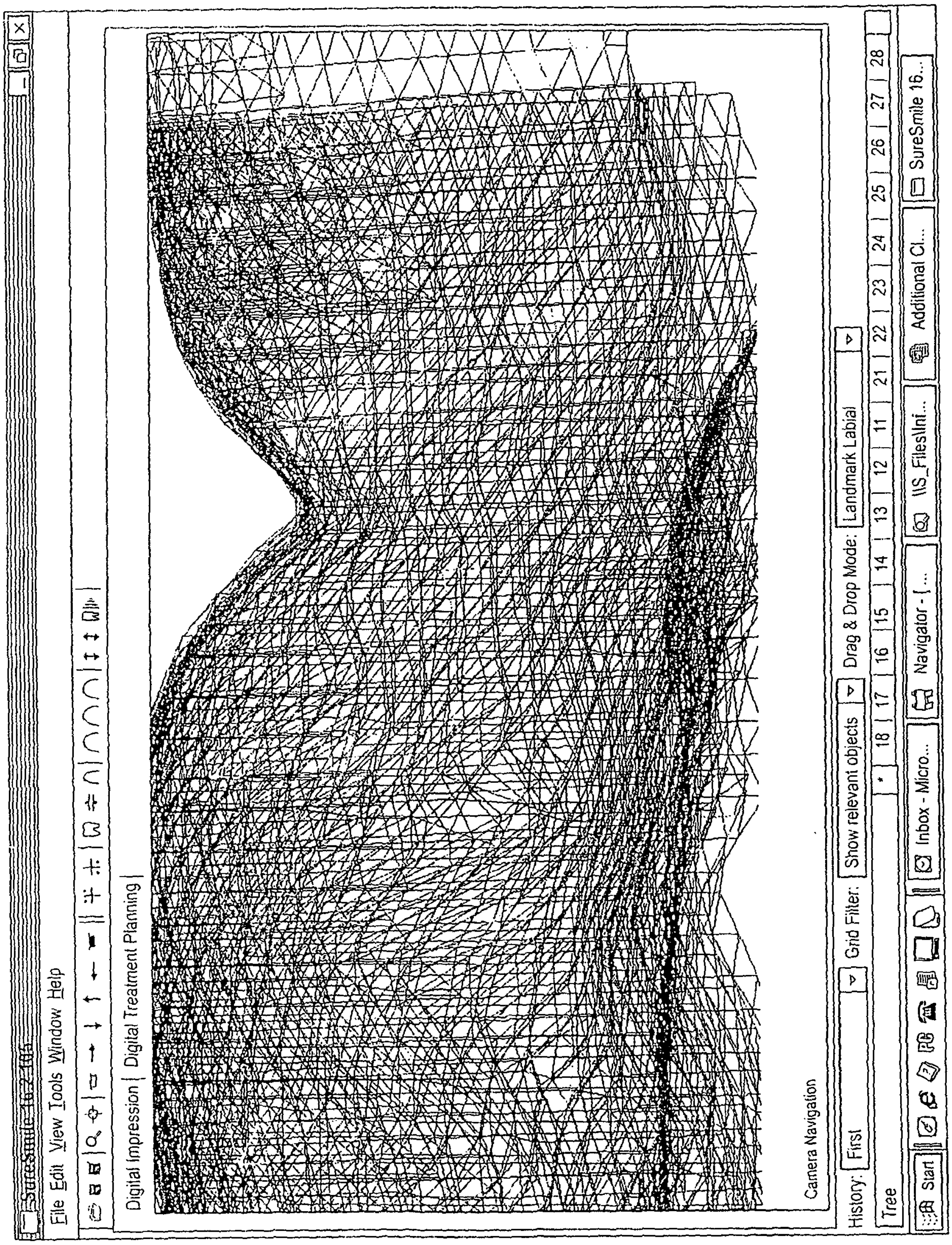
File Edit View Tools Window Help
Digital Impression
Digital Treatment Planning
Camera Navigation
History: First
Grid Filter: Show relevant objects
Drag & Drop Mode: Landmark Labial
Tree
18 17 16 15 14 13 12 11 21 22 23 24 25 26 27 28
Start
Inbox - Micro...
Navigator - [...
\\S_Files\Ini...
Additional Cl...
SureSmile 16...

FIG. 37D

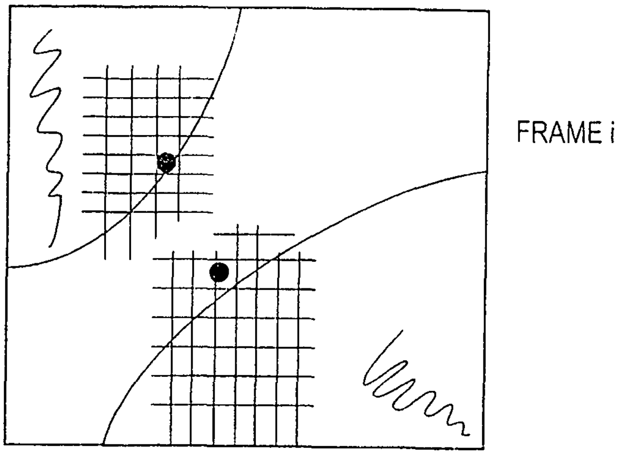


FIG. 38A

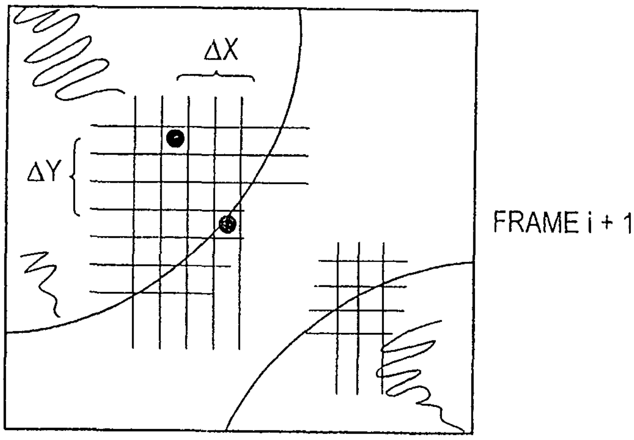


FIG. 38B

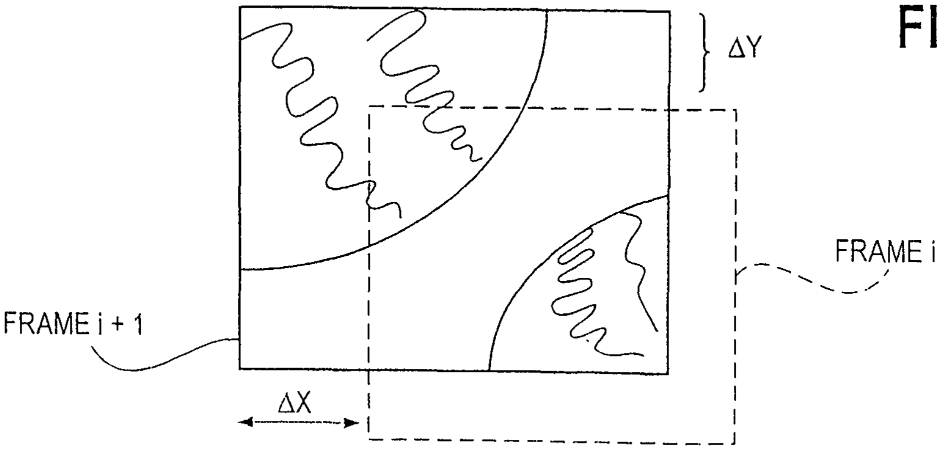


FIG. 38C

FIG. 39A

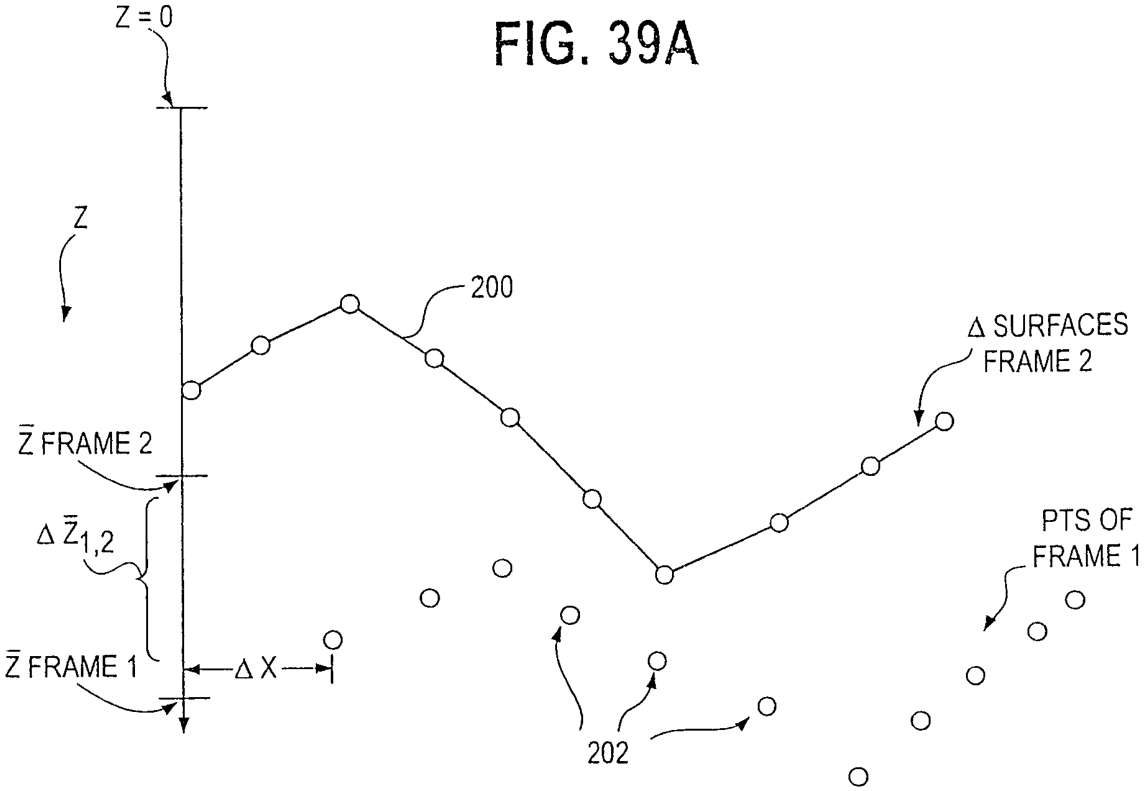


FIG. 39B

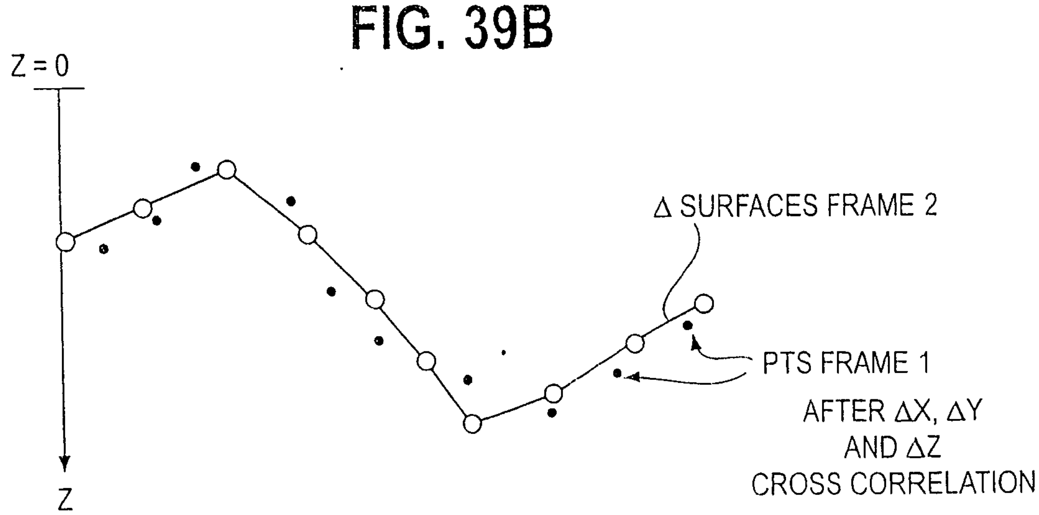

FIG. 40A

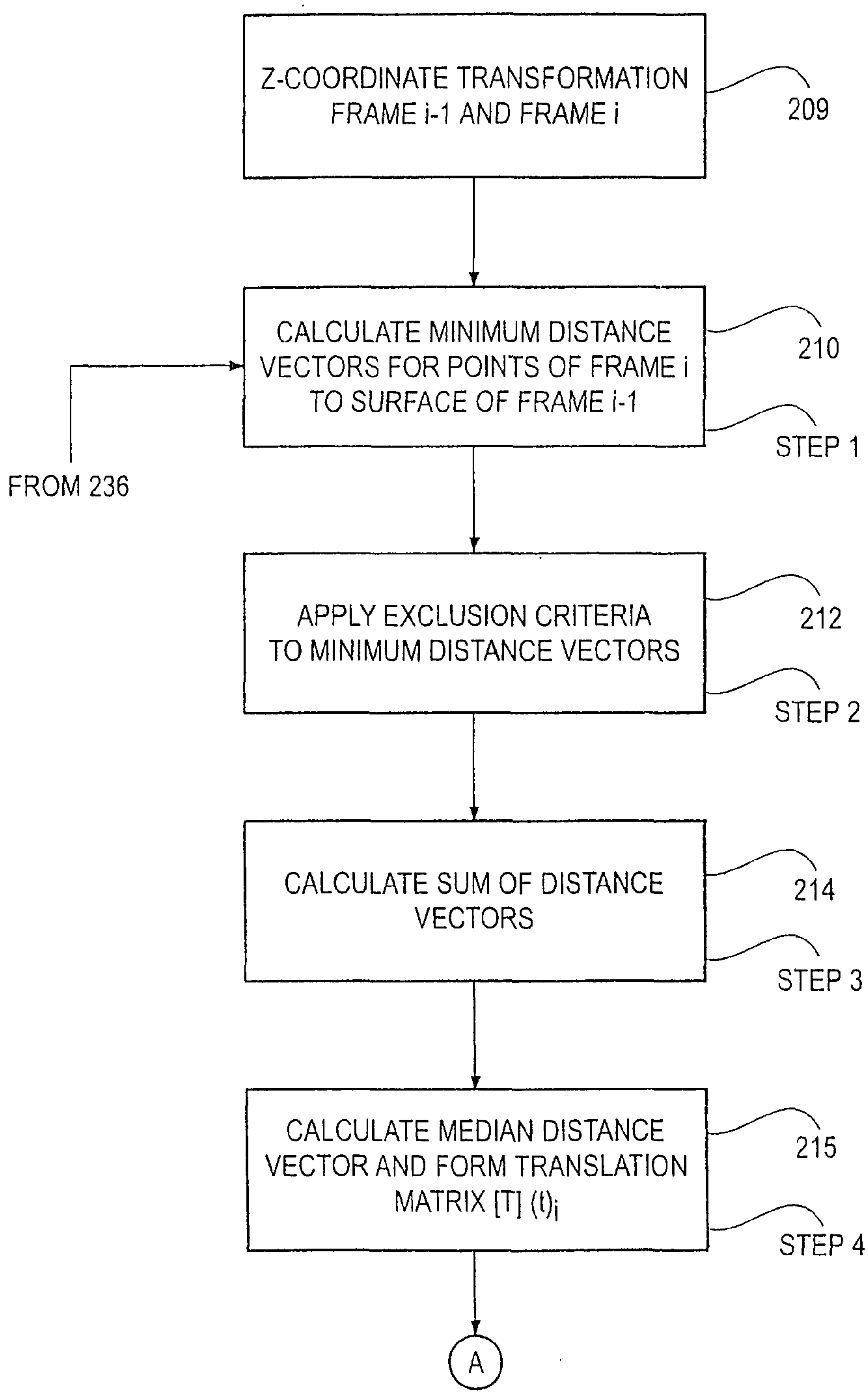
Z-COORDINATE TRANSFORMATION FRAME i-1 AND FRAME i
209
CALCULATE MINIMUM DISTANCE VECTORS FOR POINTS OF FRAME i TO SURFACE OF FRAME i-1
210
STEP 1
FROM 236
APPLY EXCLUSION CRITERIA TO MINIMUM DISTANCE VECTORS
212
STEP 2
CALCULATE SUM OF DISTANCE VECTORS
214
STEP 3
CALCULATE MEDIAN DISTANCE VECTOR AND FORM TRANSLATION MATRIX [T] (t)i
215
STEP 4
A

FIG. 40B

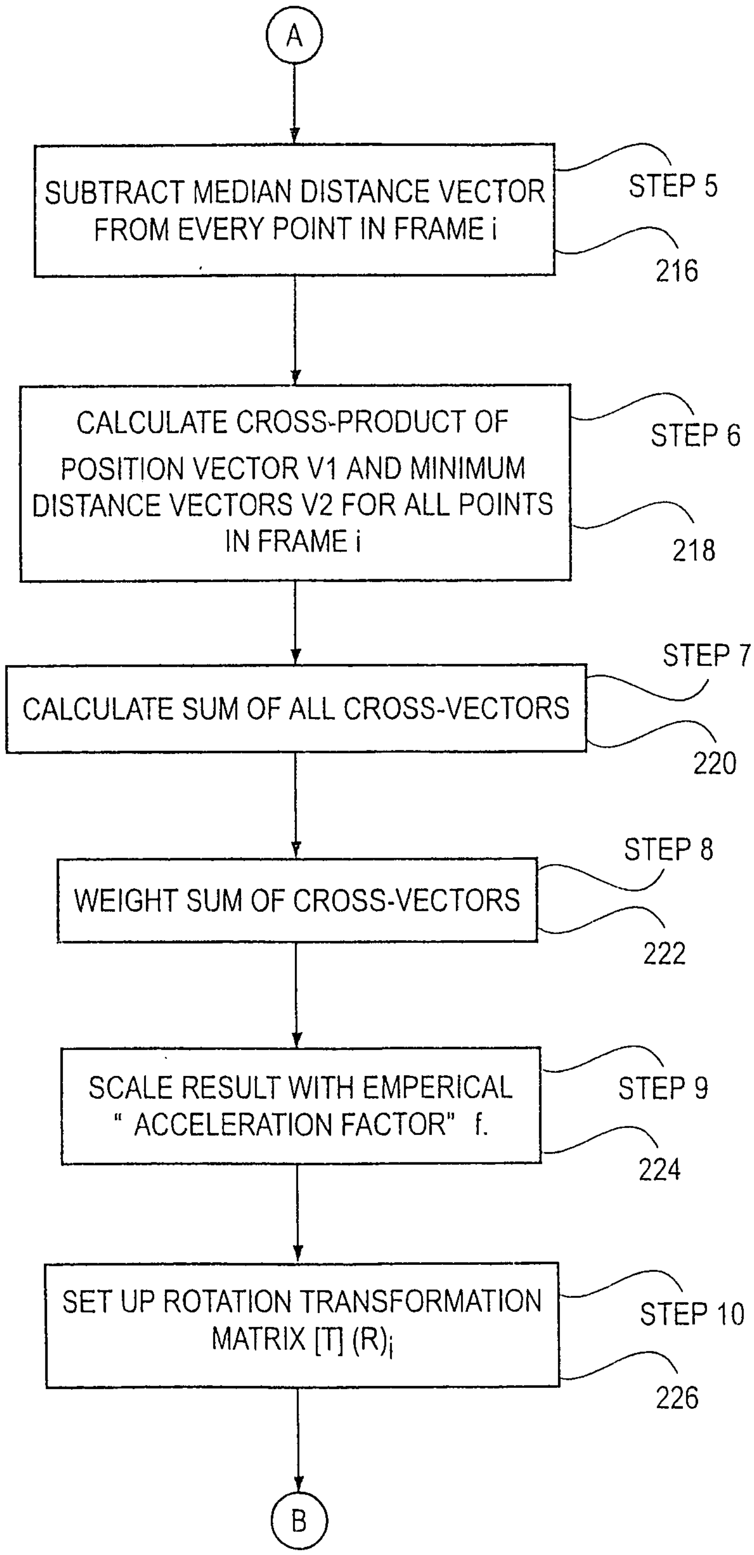
A
SUBTRACT MEDIAN DISTANCE VECTOR FROM EVERY POINT IN FRAME i
STEP 5
216
CALCULATE CROSS-PRODUCT OF POSITION VECTOR V1 AND MINIMUM DISTANCE VECTORS V2 FOR ALL POINTS IN FRAME i
STEP 6
218
CALCULATE SUM OF ALL CROSS-VECTORS
STEP 7
220
WEIGHT SUM OF CROSS-VECTORS
STEP 8
222
SCALE RESULT WITH EMPERICAL " ACCELERATION FACTOR" f.
STEP 9
224
SET UP ROTATION TRANSFORMATION MATRIX [T] (R)i
STEP 10
226
B

FIG. 40C

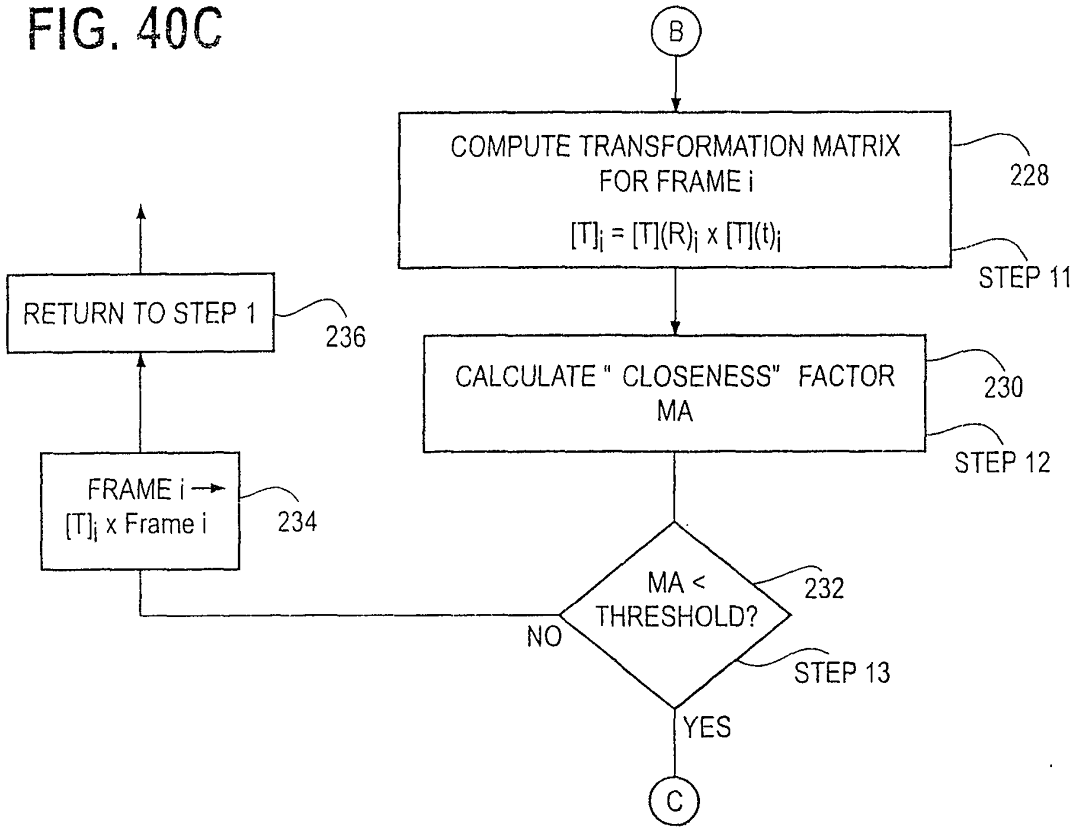


FIG. 40D

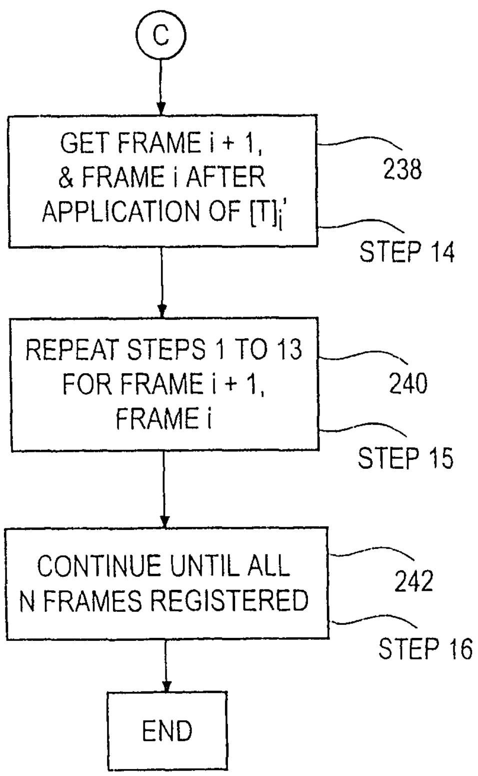

FIG. 41

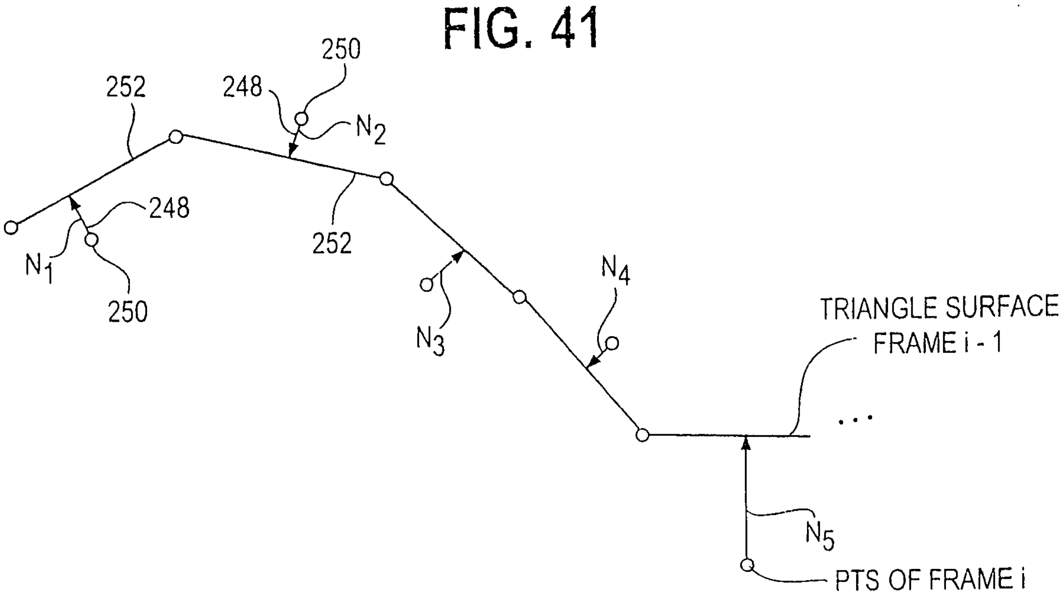


FIG. 42

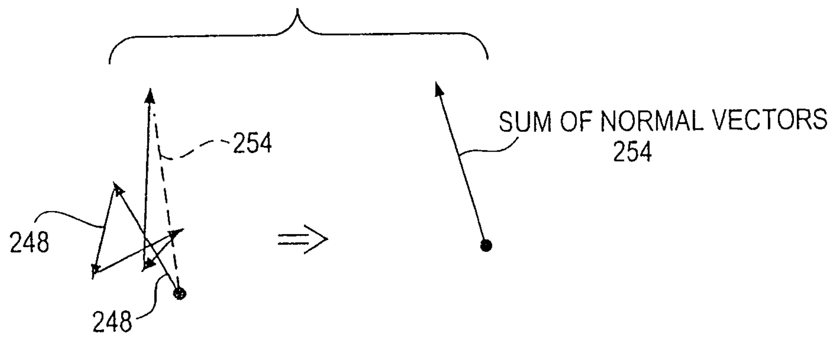


FIG. 43

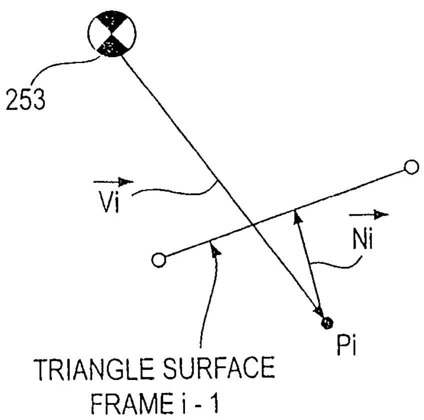


FIG. 44

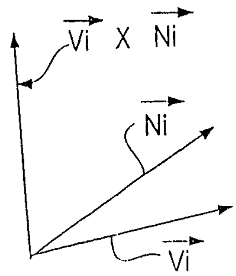

FIG. 45

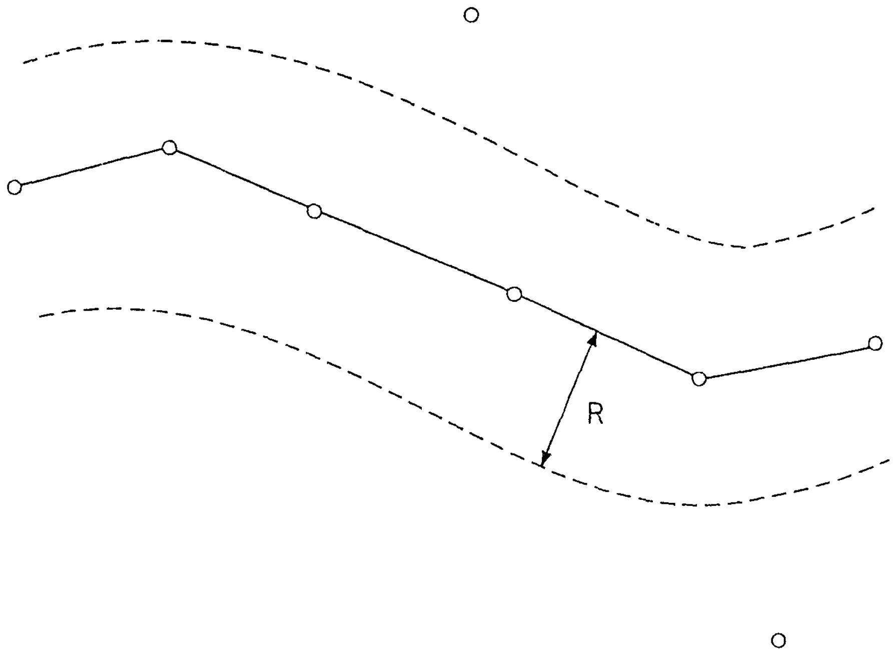
R

FIG. 46

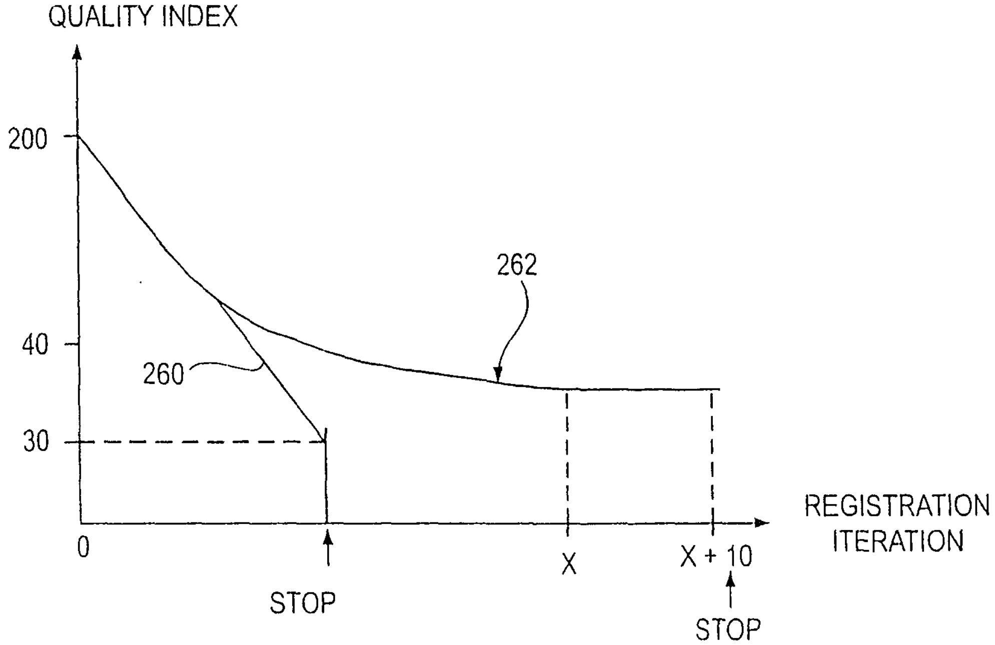
QUALITY INDEX
200
40
30
262
260
0
STOP
X
X + 10
STOP
REGISTRATION
ITERATION

FIG. 47A

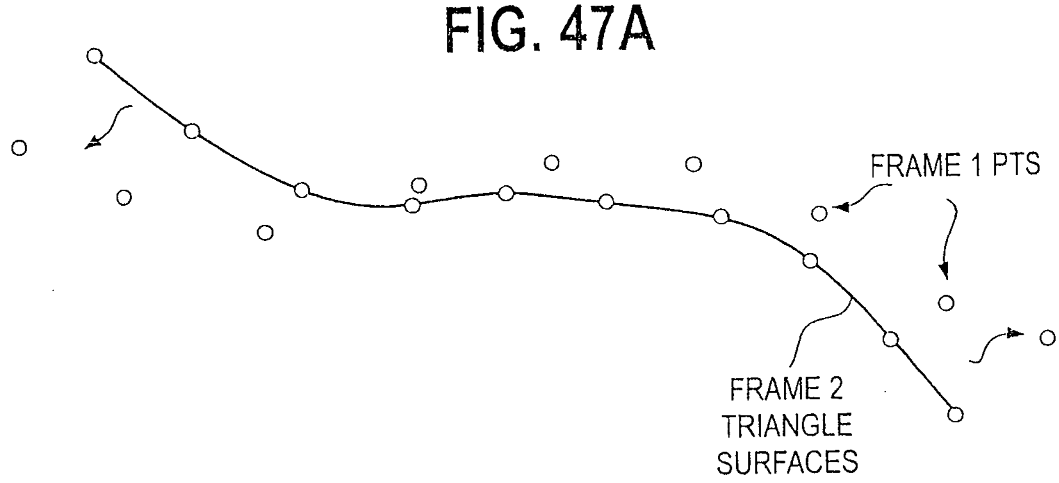


FIG. 47B

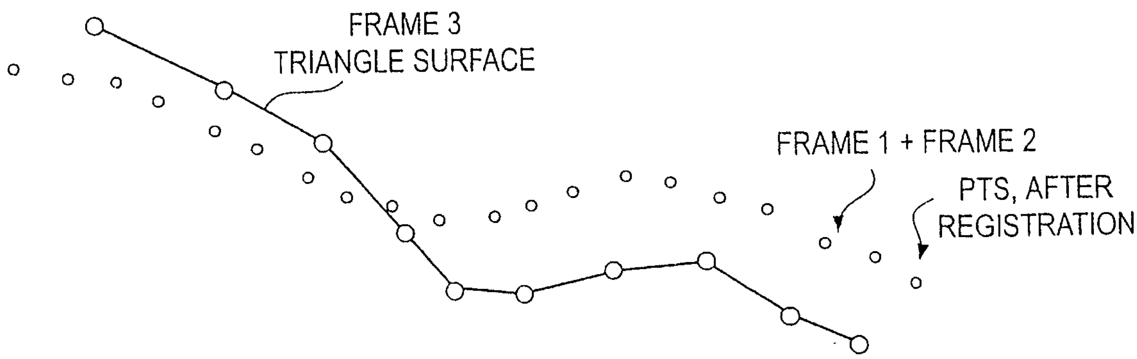

FIG. 48A

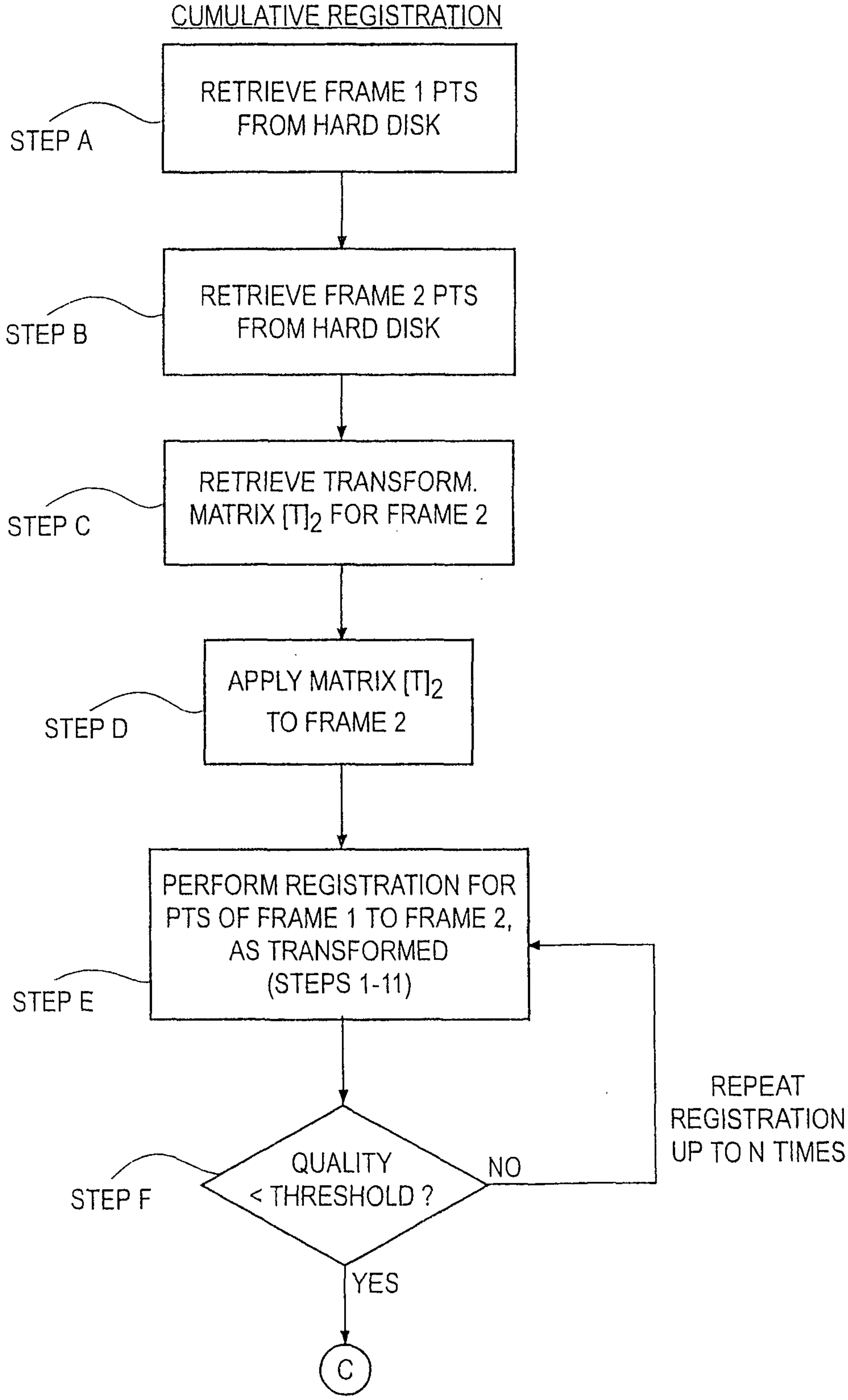
CUMULATIVE REGISTRATION
STEP A
RETRIEVE FRAME 1 PTS FROM HARD DISK
STEP B
RETRIEVE FRAME 2 PTS FROM HARD DISK
STEP C
RETRIEVE TRANSFORM. MATRIX $[T]_2$ FOR FRAME 2
STEP D
APPLY MATRIX $[T]_2$ TO FRAME 2
STEP E
PERFORM REGISTRATION FOR PTS OF FRAME 1 TO FRAME 2, AS TRANSFORMED (STEPS 1-11)
STEP F
QUALITY < THRESHOLD ?
NO
REPEAT REGISTRATION UP TO N TIMES
YES
C

FIG. 48B

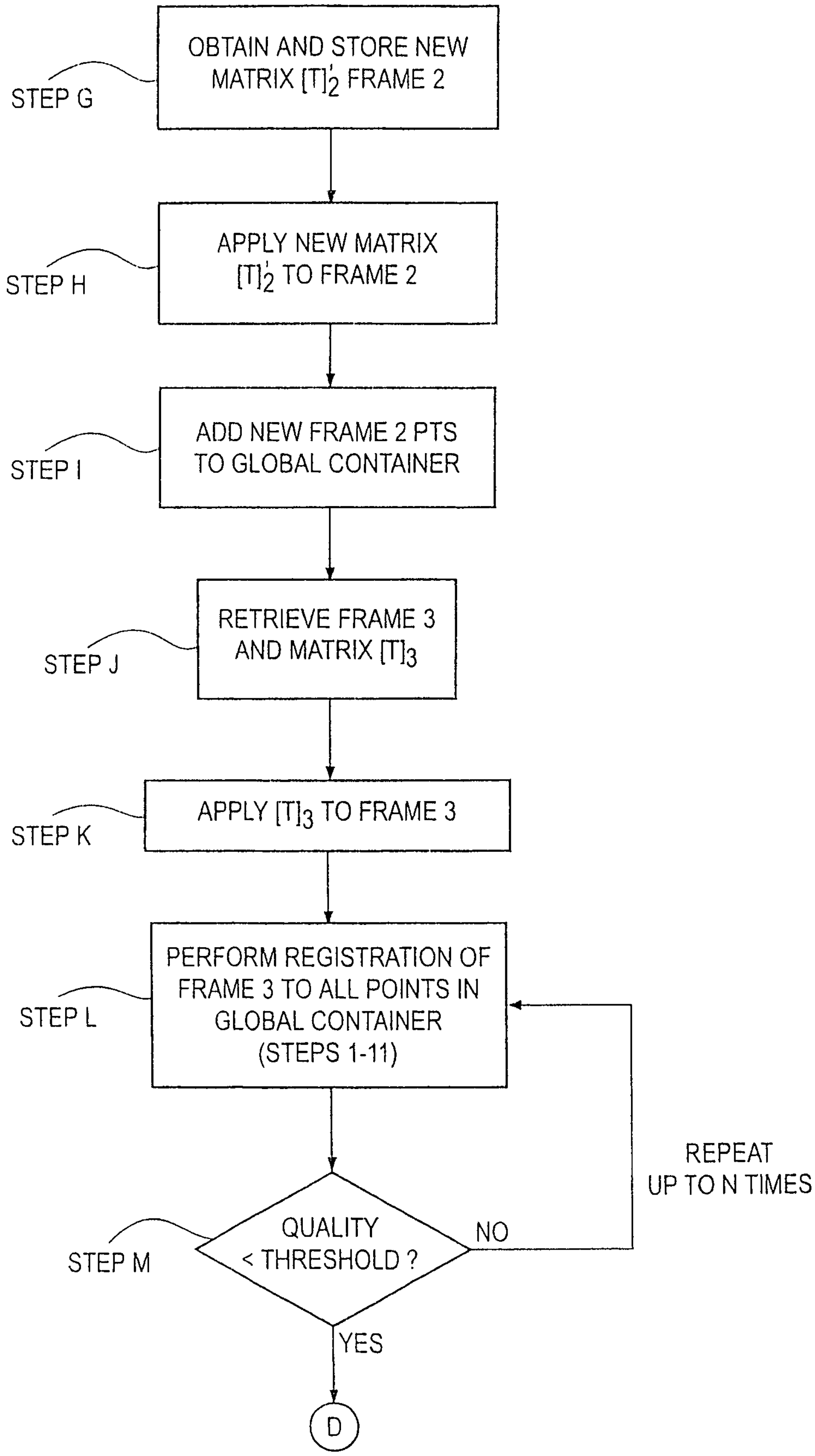
OBTAIN AND STORE NEW MATRIX [T]'2 FRAME 2
STEP G
APPLY NEW MATRIX [T]'2 TO FRAME 2
STEP H
ADD NEW FRAME 2 PTS TO GLOBAL CONTAINER
STEP I
RETRIEVE FRAME 3 AND MATRIX [T]3
STEP J
APPLY [T]3 TO FRAME 3
STEP K
PERFORM REGISTRATION OF FRAME 3 TO ALL POINTS IN GLOBAL CONTAINER (STEPS 1-11)
STEP L
REPEAT UP TO N TIMES
QUALITY < THRESHOLD ?
STEP M
NO
YES
D

FIG. 48C

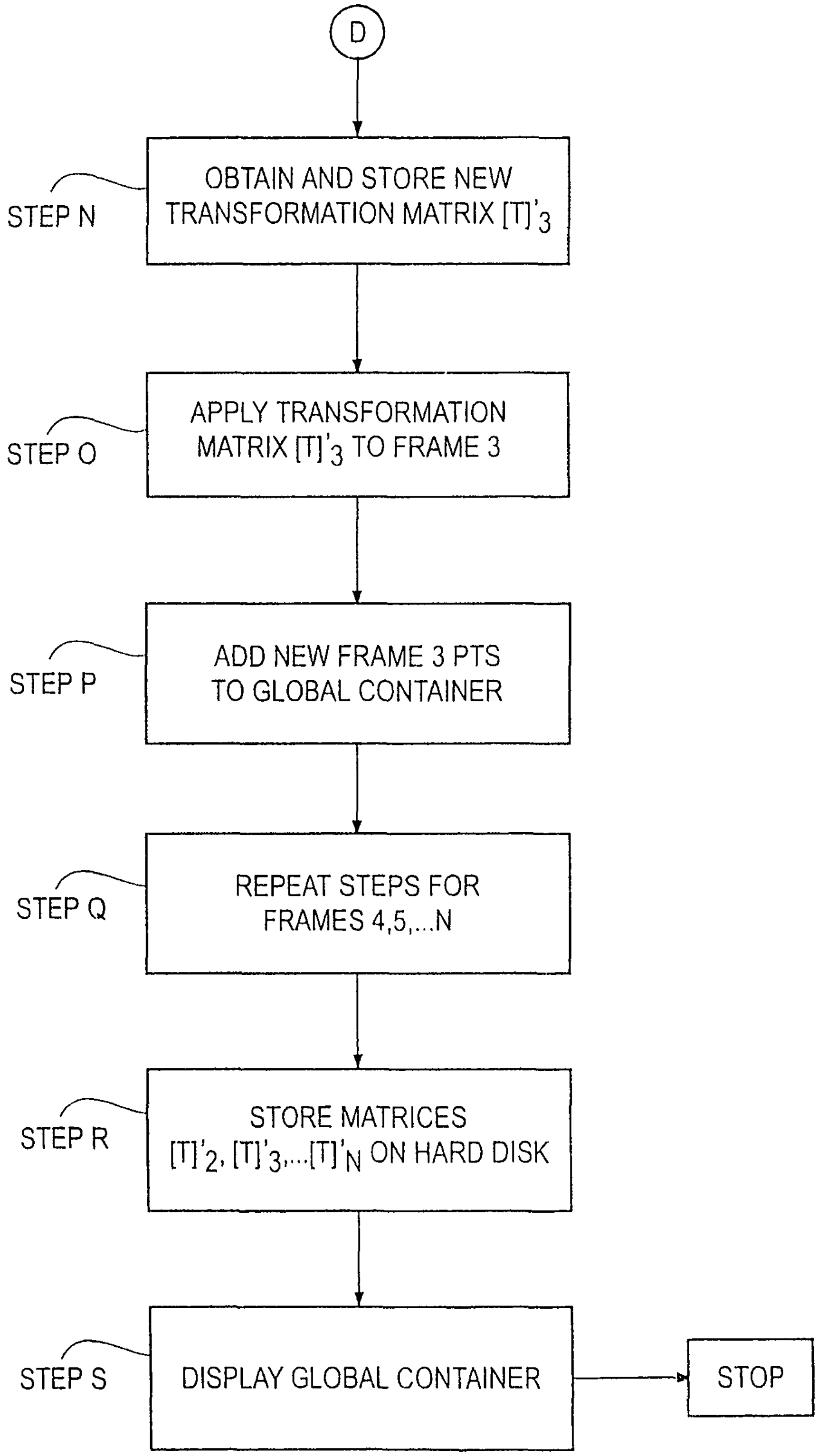
D
STEP N
OBTAIN AND STORE NEW TRANSFORMATION MATRIX [T]'3
STEP O
APPLY TRANSFORMATION MATRIX [T]'3 TO FRAME 3
STEP P
ADD NEW FRAME 3 PTS TO GLOBAL CONTAINER
STEP Q
REPEAT STEPS FOR FRAMES 4,5,...N
STEP R
STORE MATRICES [T]'2, [T]'3,...[T]'N ON HARD DISK
STEP S
DISPLAY GLOBAL CONTAINER
STOP

FIG. 49

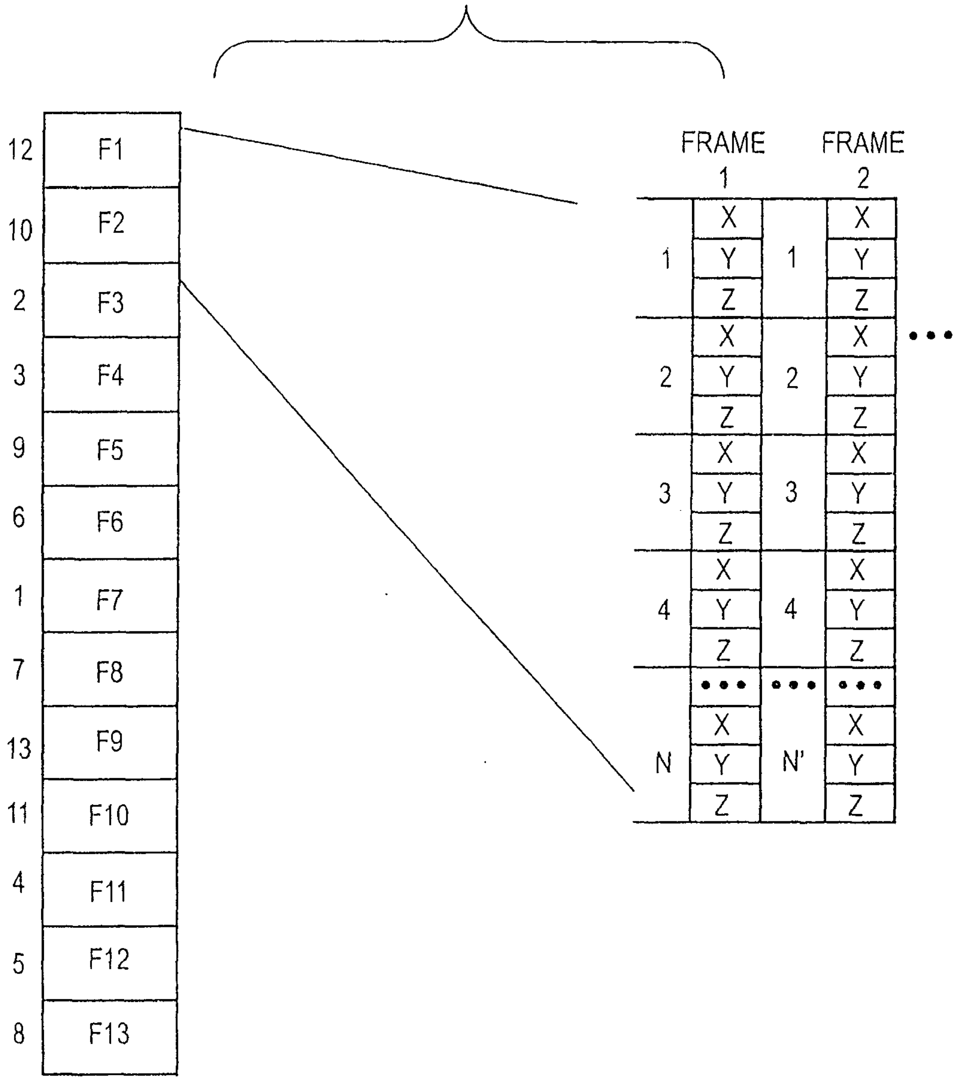
12 F1
10 F2
2 F3
3 F4
9 F5
6 F6
1 F7
7 F8
13 F9
11 F10
4 F11
5 F12
8 F13
FRAME 1
FRAME 2
X
Y
Z
1
2
3
4
N
N'

FIG. 50

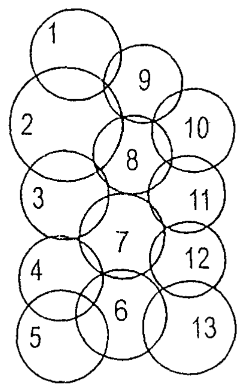
1
2
3
4
5
6
7
8
9
10
11
12
13

FIG. 51

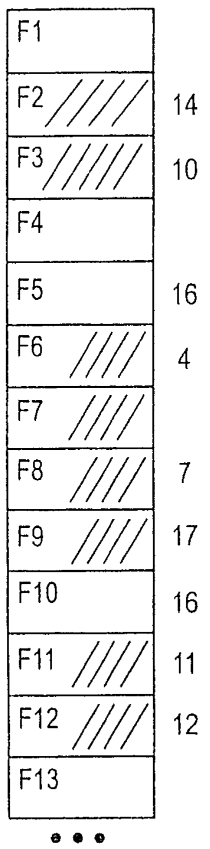


FIG. 52

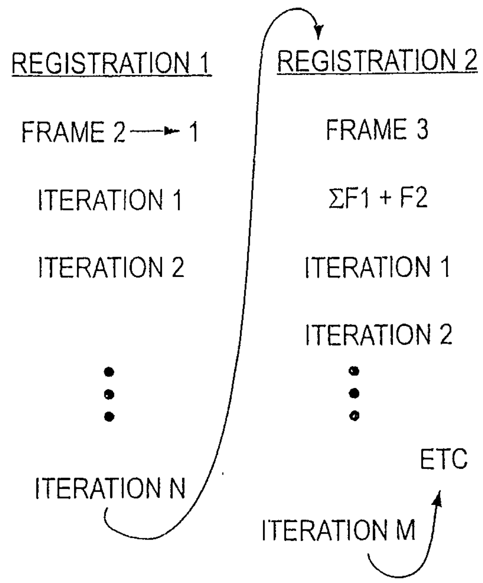


FIG. 53

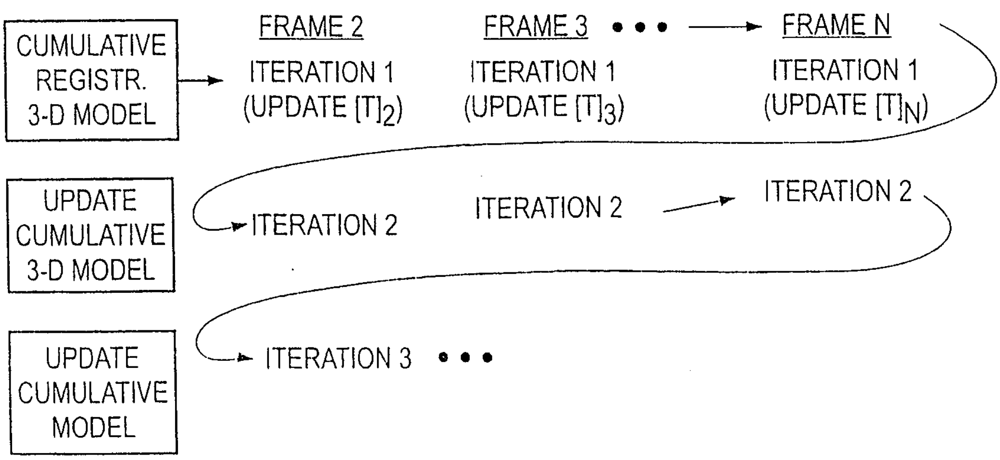

FIG. 54

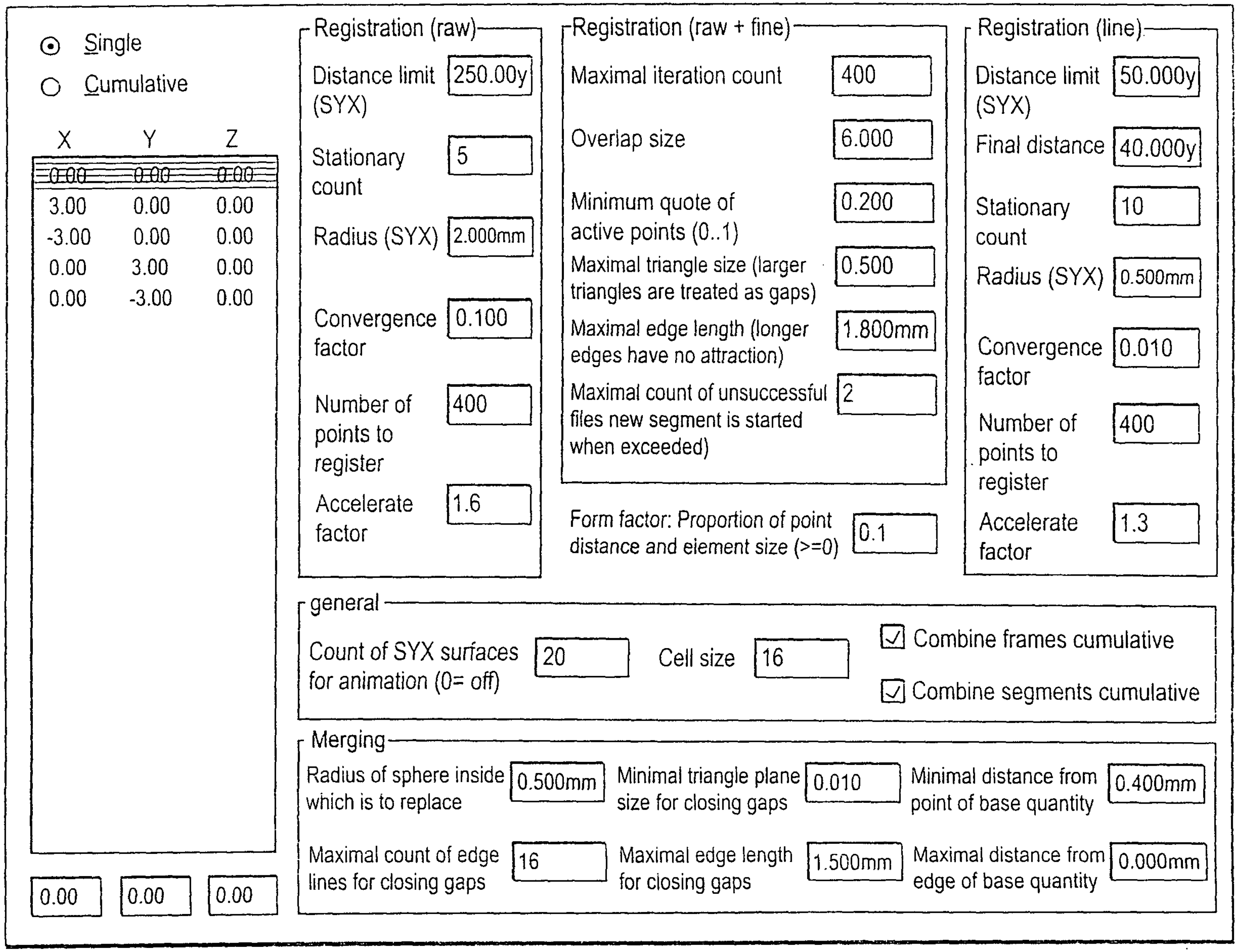
Single
Cumulative
X
Y
Z
0.00
0.00
0.00
3.00
0.00
0.00
-3.00
0.00
0.00
0.00
3.00
0.00
0.00
-3.00
0.00
0.00
0.00
0.00
Registration (raw)
Distance limit (SYX)
250.00y
Stationary count
5
Radius (SYX)
2.000mm
Convergence factor
0.100
Number of points to register
400
Accelerate factor
1.6
Registration (raw + fine)
Maximal iteration count
400
Overlap size
6.000
Minimum quote of active points (0..1)
0.200
Maximal triangle size (larger triangles are treated as gaps)
0.500
Maximal edge length (longer edges have no attraction)
1.800mm
Maximal count of unsuccessful files new segment is started when exceeded)
2
Form factor: Proportion of point distance and element size (>=0)
0.1
Registration (line)
Distance limit (SYX)
50.000y
Final distance
40.000y
Stationary count
10
Radius (SYX)
0.500mm
Convergence factor
0.010
Number of points to register
400
Accelerate factor
1.3
general
Count of SYX surfaces for animation (0= off)
20
Cell size
16
Combine frames cumulative
Combine segments cumulative
Merging
Radius of sphere inside which is to replace
0.500mm
Minimal triangle plane size for closing gaps
0.010
Minimal distance from point of base quantity
0.400mm
Maximal count of edge lines for closing gaps
16
Maximal edge length for closing gaps
1.500mm
Maximal distance from edge of base quantity
0.000mm

FIG. 55

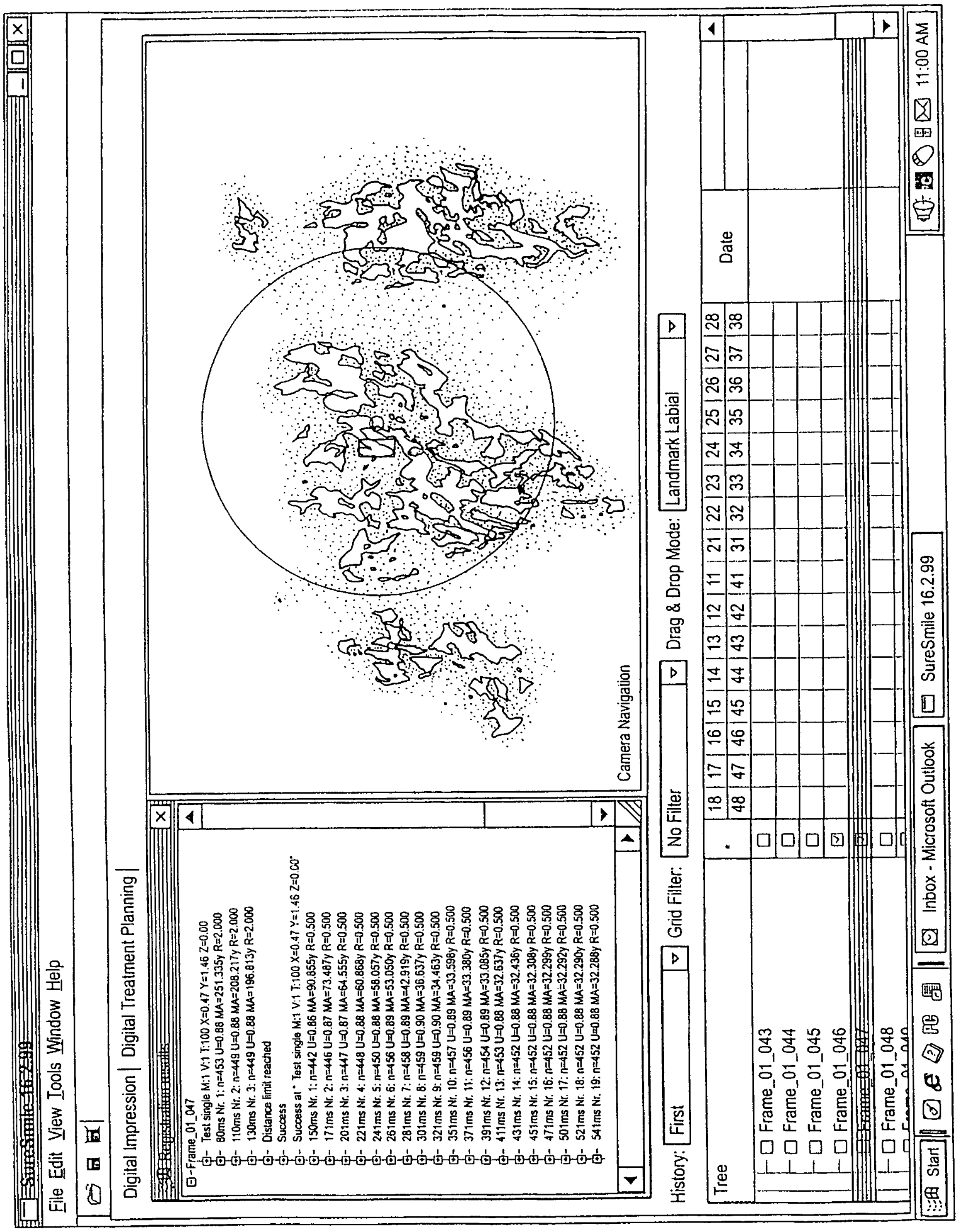
SureSmile 16.2.99
File Edit View Tools Window Help
Digital Impression
Digital Treatment Planning
Registration results
Camera Navigation
History: First
Grid Filter: No Filter
Drag & Drop Mode: Landmark Labial
Tree
Date
Frame_01_043
Frame_01_044
Frame_01_045
Frame_01_046
Frame_01_047
Frame_01_048
Start
Inbox - Microsoft Outlook
SureSmile 16.2.99
11:00 AM

FIG. 56

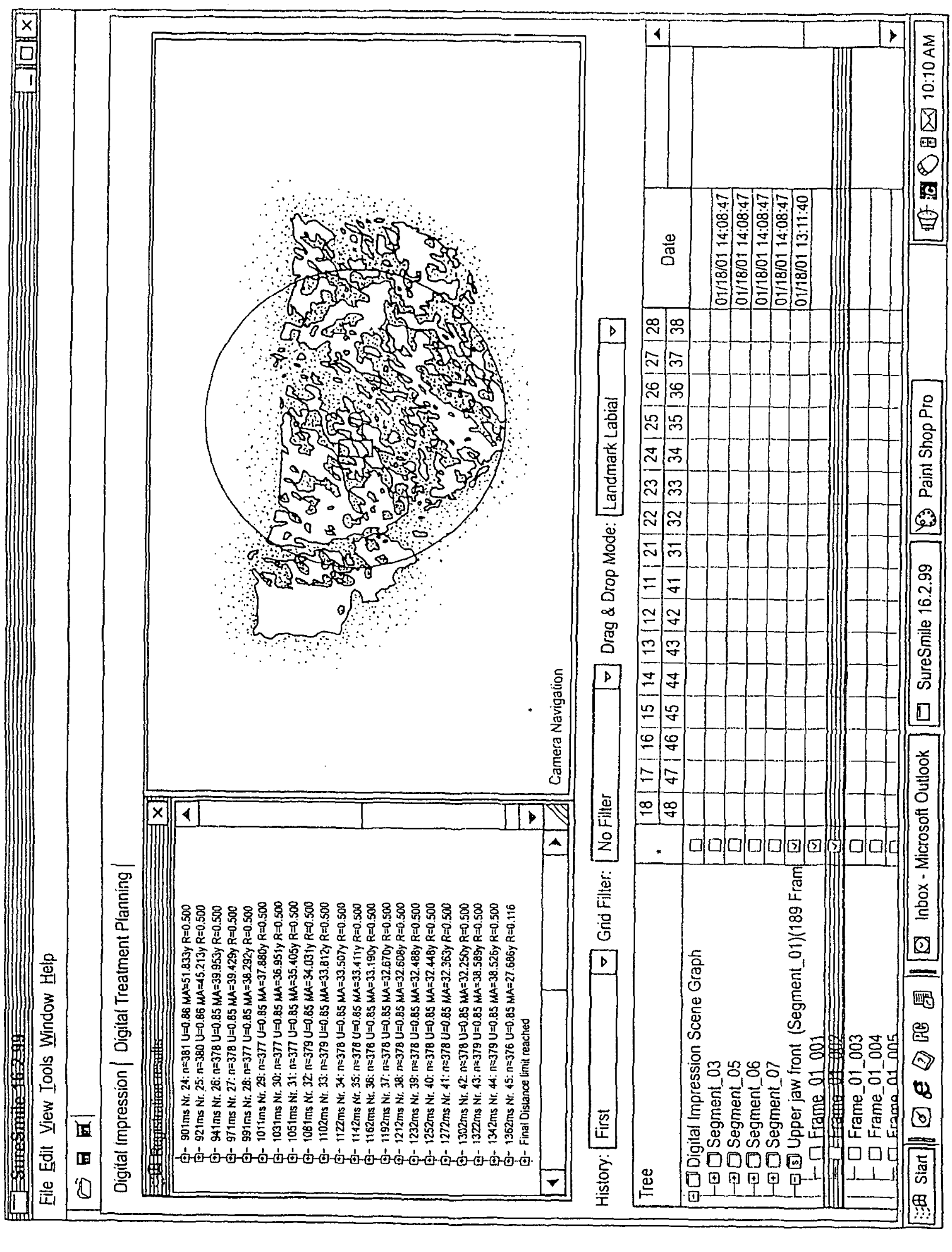
SureSmile 16.2.99
File Edit View Tools Window Help
Digital Impression
Digital Treatment Planning
Registration results
Final Distance limit reached
Camera Navigation
History: First
Grid Filter: No Filter
Drag & Drop Mode: Landmark Labial
Tree
Date
Digital Impression Scene Graph
Segment_03
Segment_05
Segment_06
Segment_07
Upper jaw front (Segment_01)(189 Fram
Frame_01_001
Frame_01_003
Frame_01_004
18 17 16 15 14 13 12 11 21 22 23 24 25 26 27 28
48 47 46 45 44 43 42 41 31 32 33 34 35 36 37 38
01/18/01 14:08:47
01/18/01 13:11:40
Start
Inbox - Microsoft Outlook
SureSmile 16.2.99
Paint Shop Pro
10:10 AM

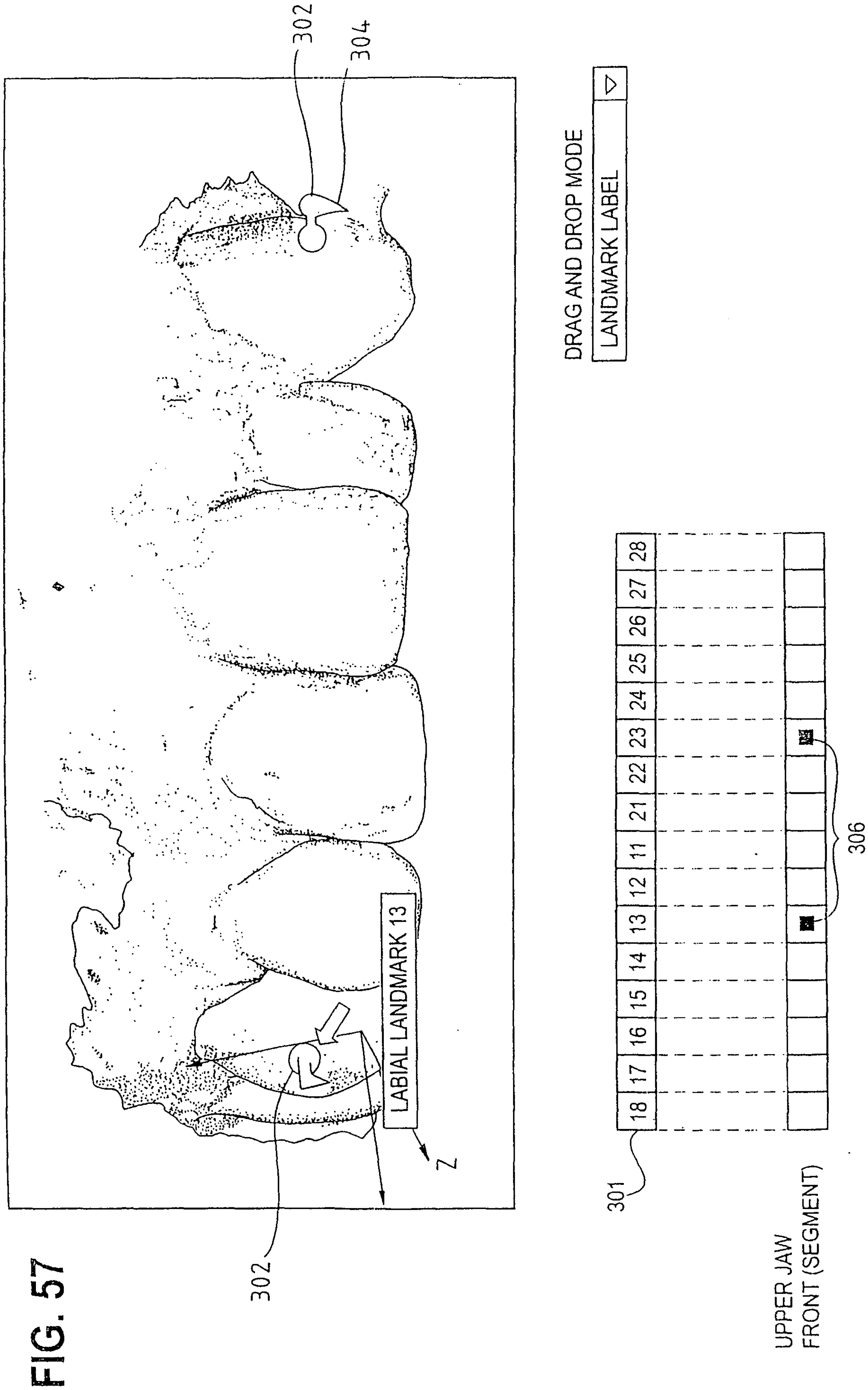
302
304
DRAG AND DROP MODE
LANDMARK LABEL
LABIAL LANDMARK 13
Z
302
301
18
17
16
15
14
13
12
11
21
22
23
24
25
26
27
28
306
UPPER JAW
FRONT (SEGMENT)

FIG. 57

Fig. 58A

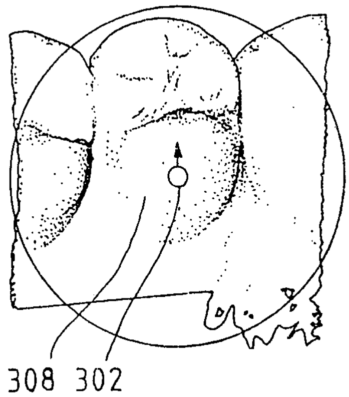


Fig. 58B

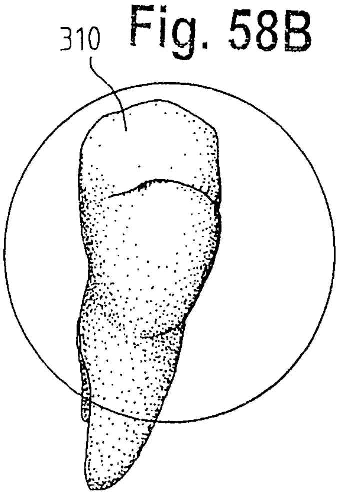


Fig. 58C

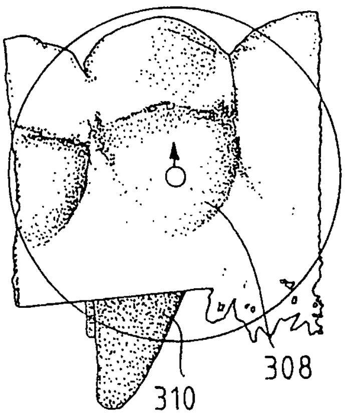


Fig. 58D

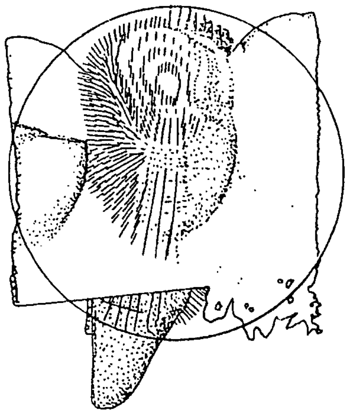

Fig. 58E

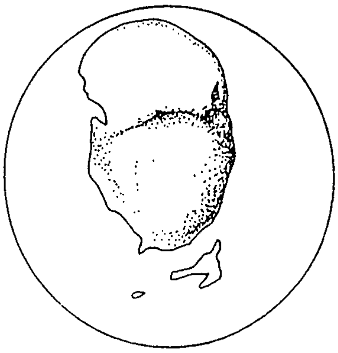

Fig. 58F

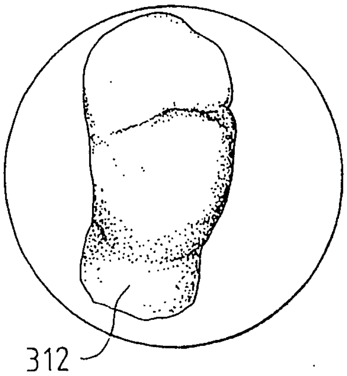

Fig. 59

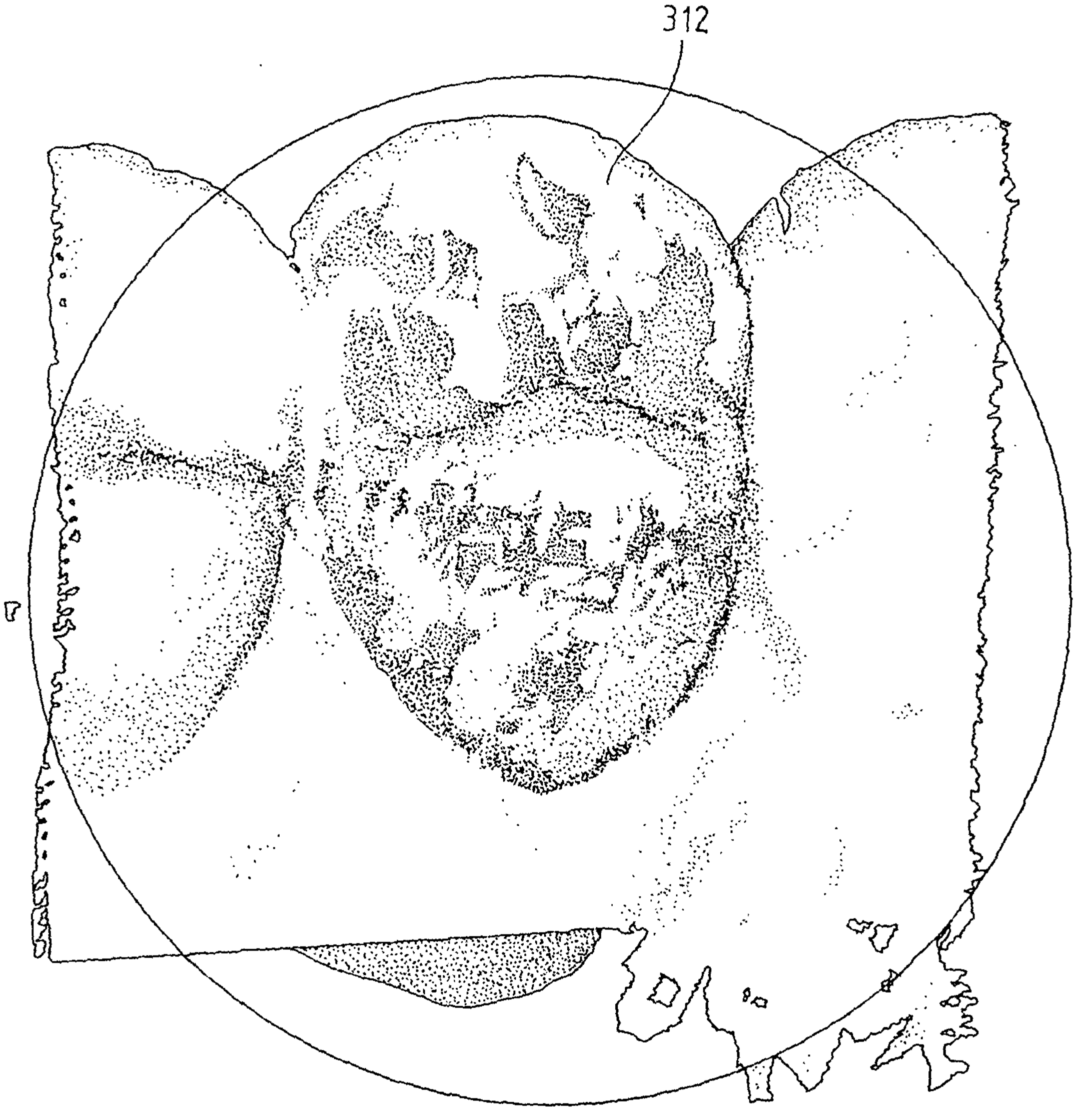

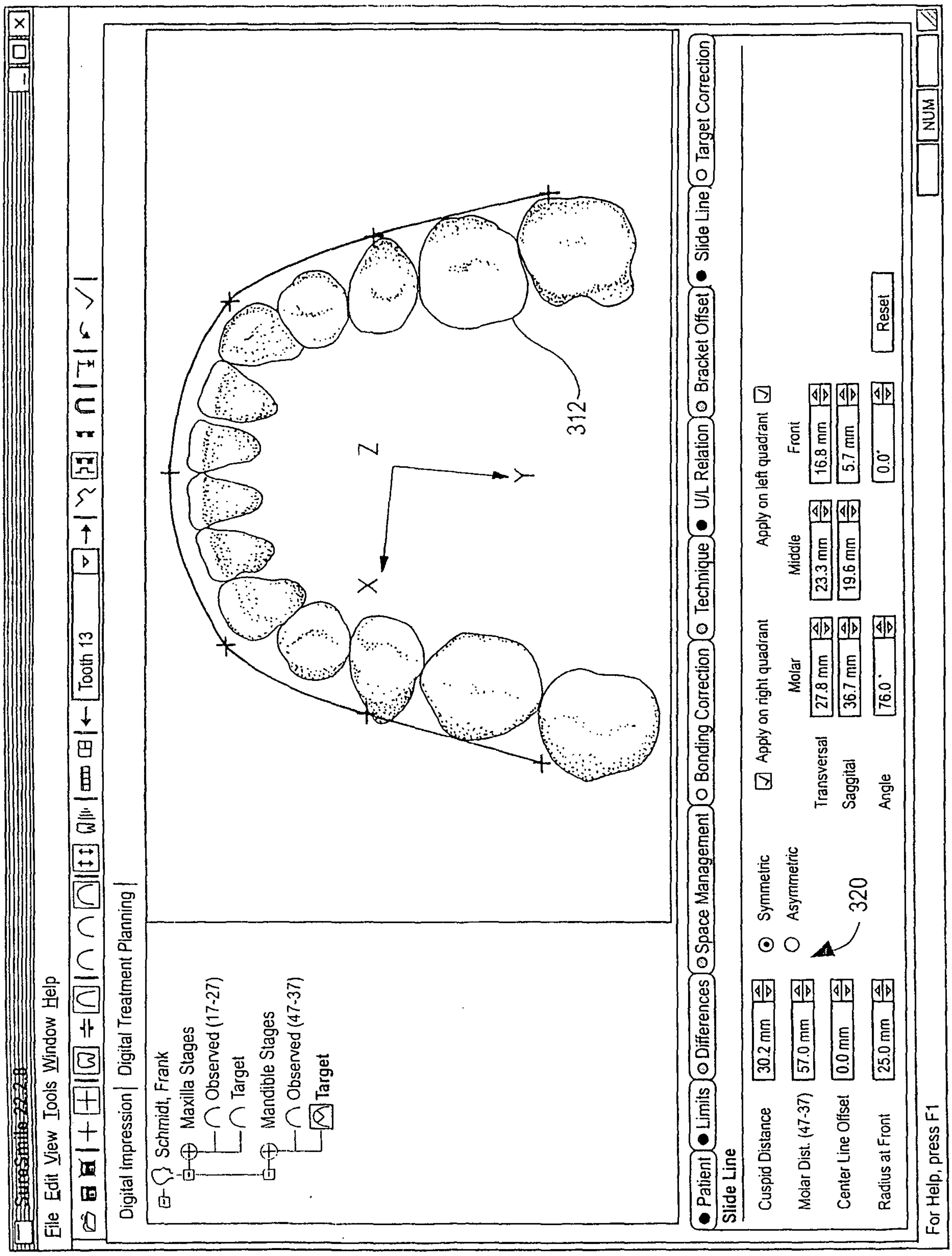
File Edit View Tools Window Help
Tooth 13
Digital Impression
Digital Treatment Planning
Schmidt, Frank
Maxilla Stages
Observed (17-27)
Target
Mandible Stages
Observed (47-37)
Target
Z
Y
X
312
320
Patient
Limits
Differences
Space Management
Bonding Correction
Technique
U/L Relation
Bracket Offset
Slide Line
Target Correction
Slide Line
Cuspid Distance
30.2 mm
Molar Dist. (47-37)
57.0 mm
Center Line Offset
0.0 mm
Radius at Front
25.0 mm
Symmetric
Asymmetric
Apply on right quadrant
Apply on left quadrant
Molar
Middle
Front
Transversal
27.8 mm
23.3 mm
16.8 mm
Saggital
36.7 mm
19.6 mm
5.7 mm
Angle
76.0°
0.0°
Reset
For Help, press F1
NUM

FIG. 60

FIG. 61

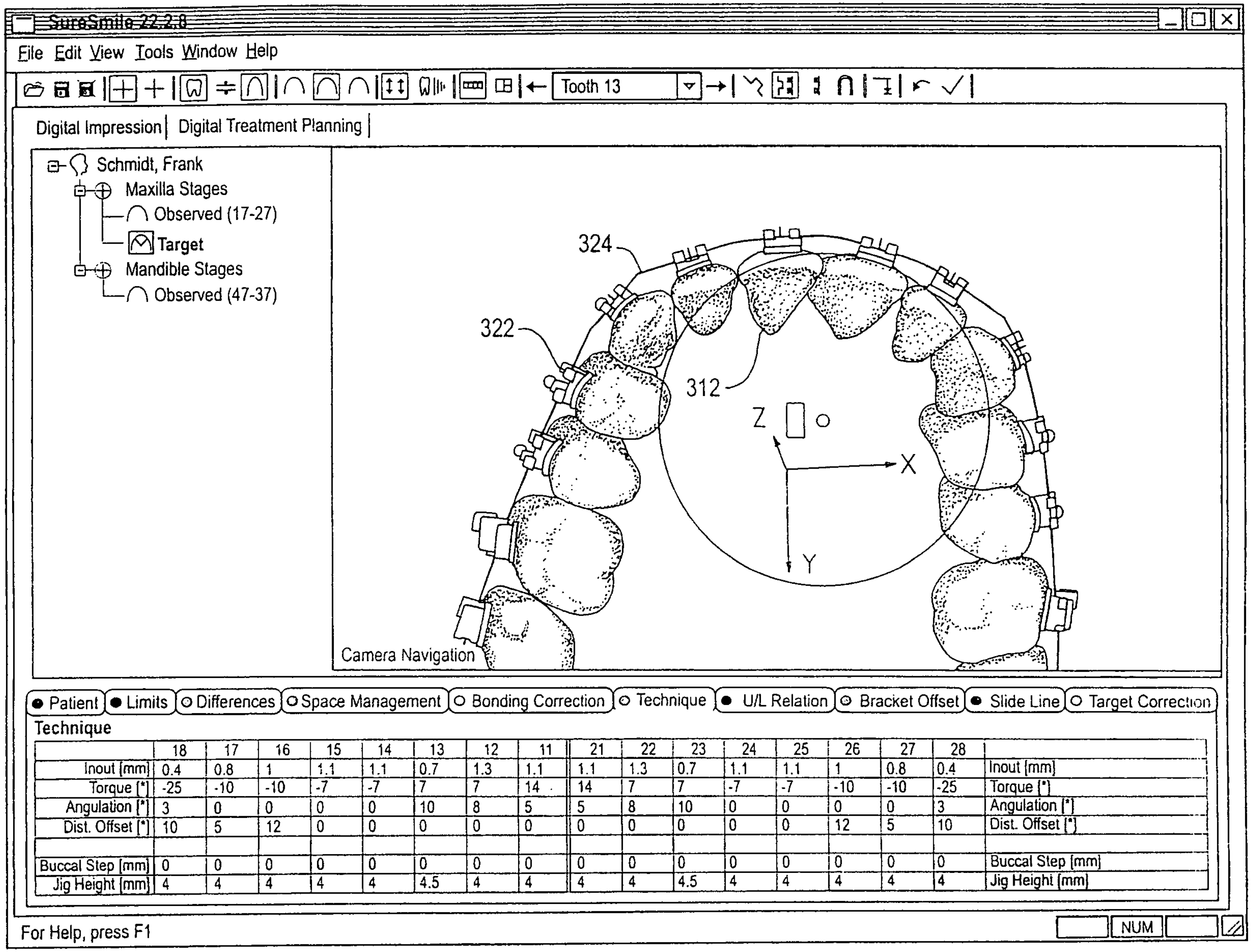
SureSmile 22.2.8
File Edit View Tools Window Help
Tooth 13
Digital Impression
Digital Treatment Planning
Schmidt, Frank
Maxilla Stages
Observed (17-27)
Target
Mandible Stages
Observed (47-37)
324
322
312
Z
X
Y
Camera Navigation
Patient
Limits
Differences
Space Management
Bonding Correction
Technique
U/L Relation
Bracket Offset
Slide Line
Target Correction
Technique
18 17 16 15 14 13 12 11 21 22 23 24 25 26 27 28
Inout [mm] 0.4 0.8 1 1.1 1.1 0.7 1.3 1.1 1.1 1.3 0.7 1.1 1.1 1 0.8 0.4 Inout [mm]
Torque [°] -25 -10 -10 -7 -7 7 7 14 14 7 7 -7 -7 -10 -10 -25 Torque [°]
Angulation [°] 3 0 0 0 0 10 8 5 5 8 10 0 0 0 0 3 Angulation [°]
Dist. Offset [°] 10 5 12 0 0 0 0 0 0 0 0 0 0 12 5 10 Dist. Offset [°]
Buccal Step [mm] 0 0 0 0 0 0 0 0 0 0 0 0 0 0 0 0 Buccal Step [mm]
Jig Height [mm] 4 4 4 4 4 4.5 4 4 4 4 4.5 4 4 4 4 4 Jig Height [mm]
For Help, press F1
NUM

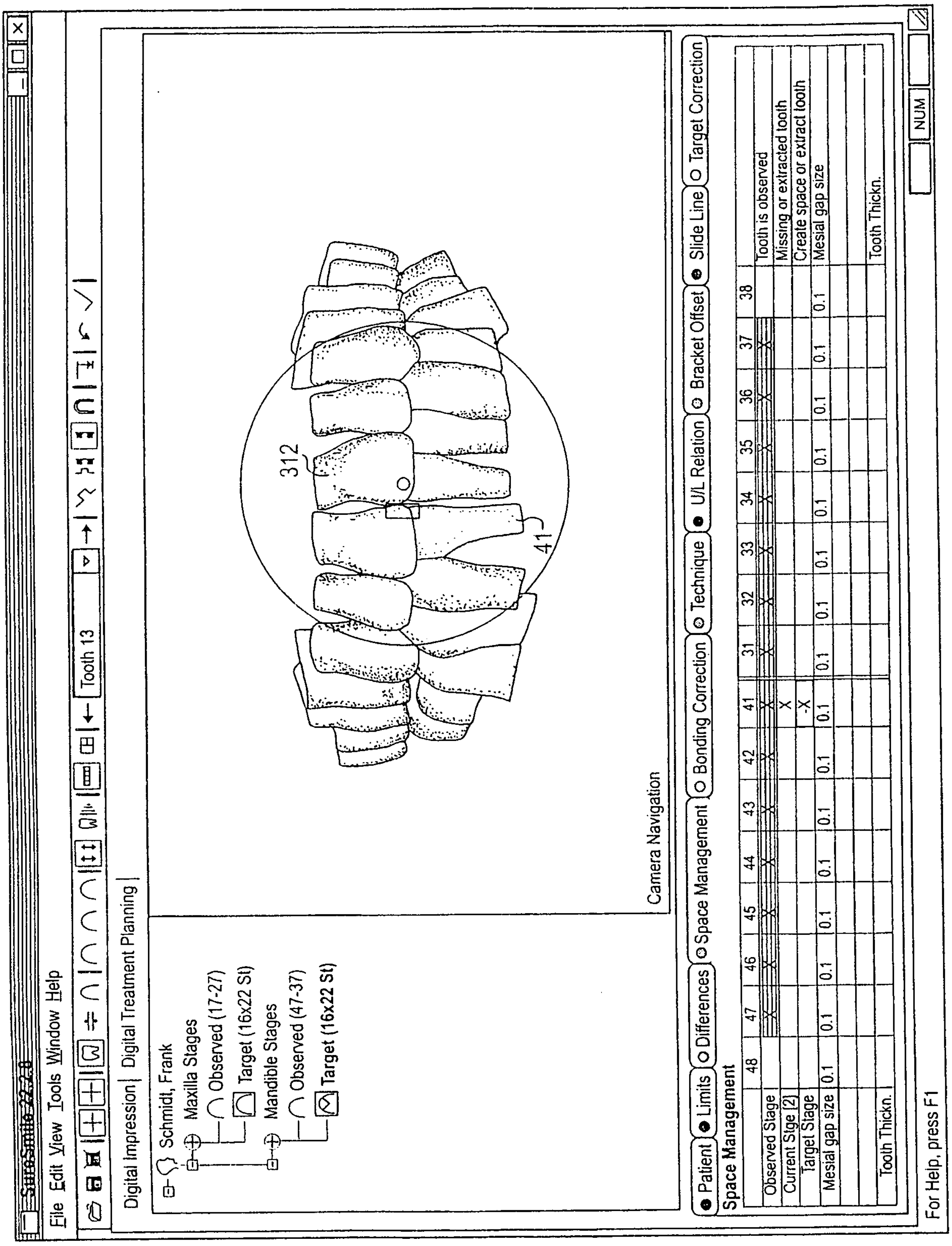
SureSmile 22.2.0
File Edit View Tools Window Help
Tooth 13
Digital Impression
Digital Treatment Planning
Schmidt, Frank
Maxilla Stages
Observed (17-27)
Target (16x22 St)
Mandible Stages
Observed (47-37)
Target (16x22 St)
Camera Navigation
312
41
Patient
Limits
Differences
Space Management
Bonding Correction
Technique
U/L Relation
Bracket Offset
Slide Line
Target Correction
Space Management
Observed Stage
Current Stge [2]
Target Stage
Mesial gap size
Tooth Thickn.
48
47
46
45
44
43
42
41
31
32
33
34
35
36
37
38
Tooth is observed
Missing or extracted tooth
Create space or extract tooth
Mesial gap size
For Help, press F1
NUM

FIG. 62

FIG. 63

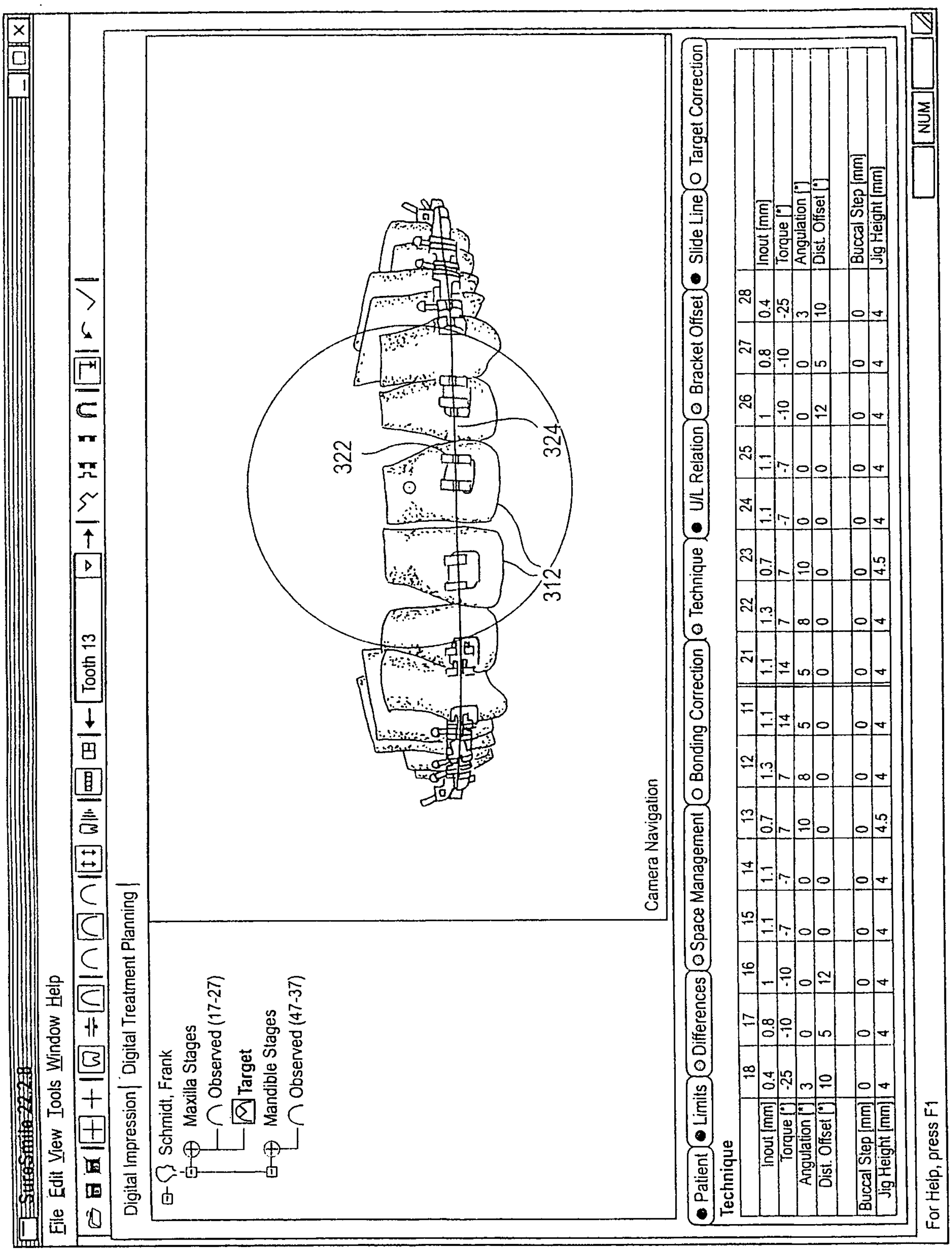


| | 18 | 17 | 16 | 15 | 14 | 13 | 12 | 11 | 21 | 22 | 23 | 24 | 25 | 26 | 27 | 28 | |
|---|---|---|---|---|---|---|---|---|---|---|---|---|---|---|---|---|---|
| Inout [mm] | 0.4 | 0.8 | 1 | 1.1 | 1.1 | 0.7 | 1.3 | 1.1 | 1.1 | 1.3 | 0.7 | 1.1 | 1.1 | 1 | 0.8 | 0.4 | Inout [mm] |
| Torque [°] | -25 | -10 | -10 | -7 | -7 | 7 | 7 | 14 | 14 | 7 | 7 | -7 | -7 | -10 | -10 | -25 | Torque [°] |
| Angulation [°] | 3 | 0 | 0 | 0 | 0 | 10 | 8 | 5 | 5 | 8 | 10 | 0 | 0 | 0 | 0 | 3 | Angulation [°] |
| Dist. Offset [°] | 10 | 5 | 12 | 0 | 0 | 0 | 0 | 0 | 0 | 0 | 0 | 0 | 0 | 12 | 5 | 10 | Dist. Offset [°] |
| | | | | | | | | | | | | | | | | | |
| Buccal Step [mm] | 0 | 0 | 0 | 0 | 0 | 0 | 0 | 0 | 0 | 0 | 0 | 0 | 0 | 0 | 0 | 0 | Buccal Step [mm] |
| Jig Height [mm] | 4 | 4 | 4 | 4 | 4 | 4.5 | 4 | 4 | 4 | 4 | 4.5 | 4 | 4 | 4 | 4 | 4 | Jig Height [mm] |

Fig. 64A

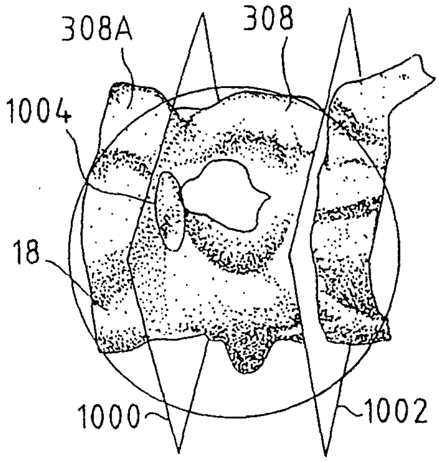


Fig. 64B

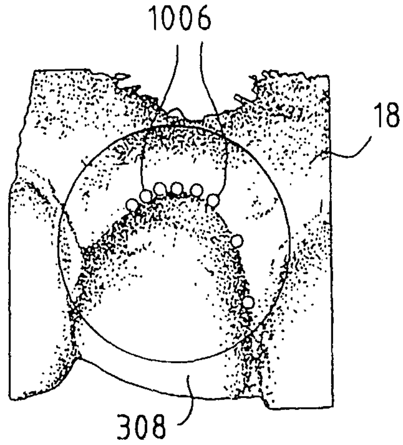


Fig. 64C

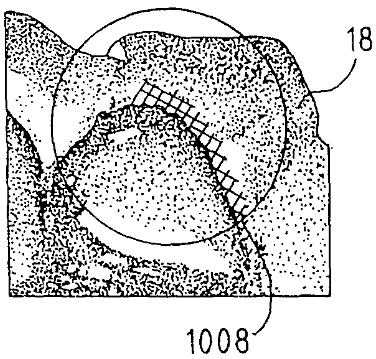


Fig. 64D

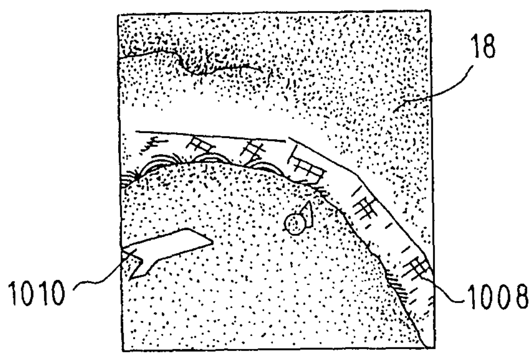

FIG. 65

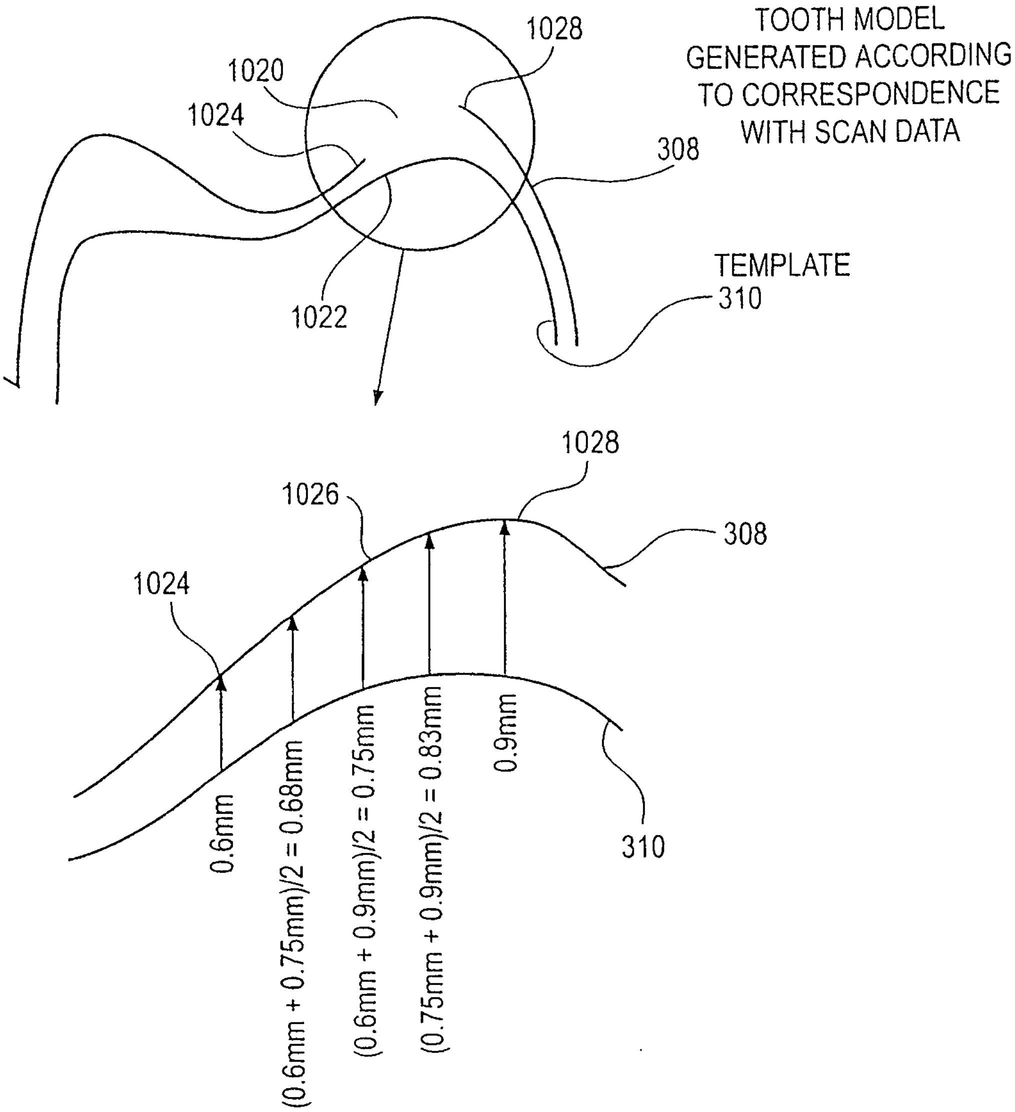
1028
TOOTH MODEL
GENERATED ACCORDING
TO CORRESPONDENCE
WITH SCAN DATA
1020
1024
308
1022
TEMPLATE
310
1028
1026
308
1024
0.6mm
(0.6mm + 0.75mm)/2 = 0.68mm
(0.6mm + 0.9mm)/2 = 0.75mm
(0.75mm + 0.9mm)/2 = 0.83mm
0.9mm
310

FIG. 66

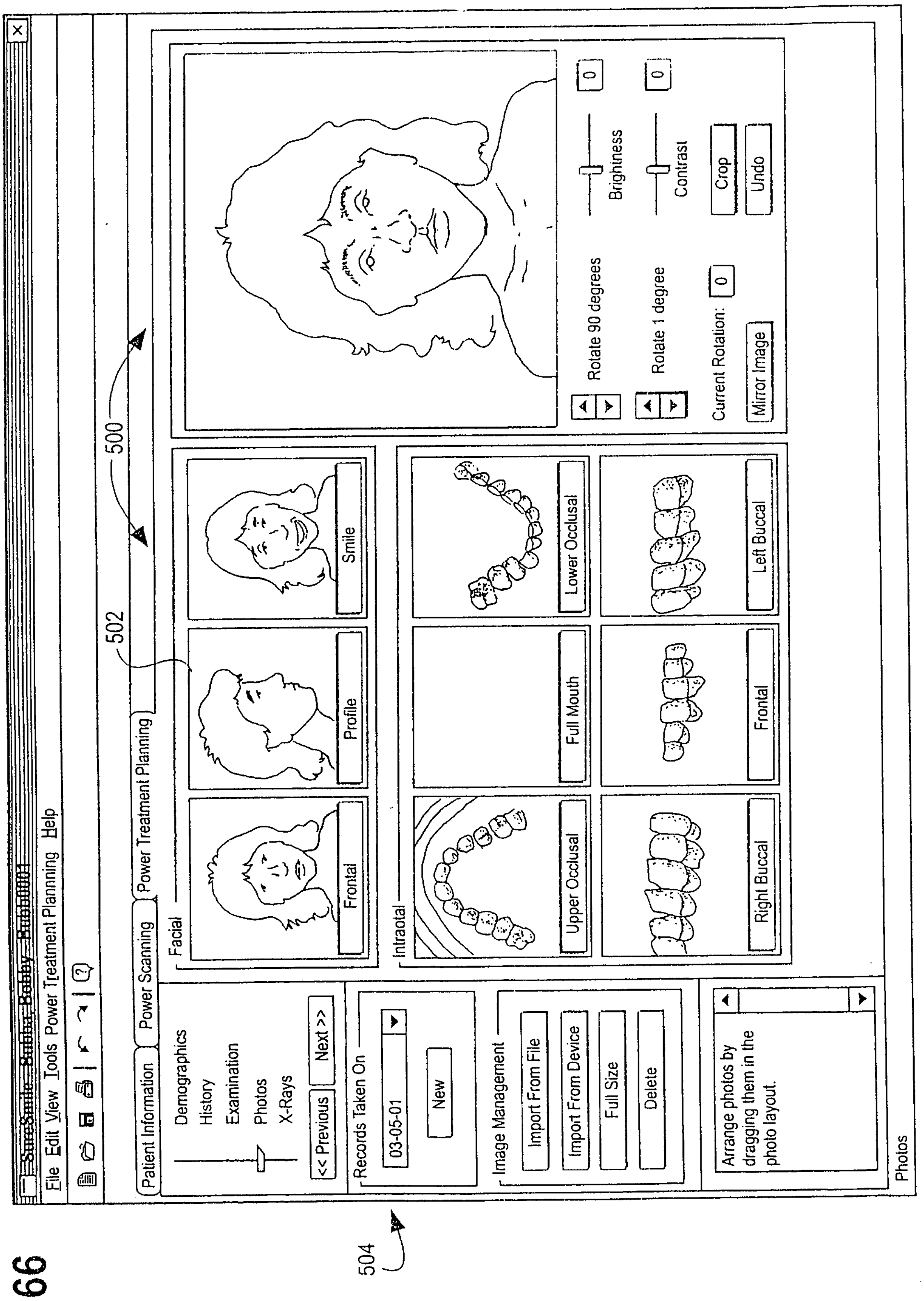

SureSmile - Bubba, Bobby - Bub0001
File Edit View Tools Power Treatment Planning Help
Patient Information
Power Scanning
Power Treatment Planning
Demographics
History
Examination
Photos
X-Rays
<< Previous
Next >>
Records Taken On
03-05-01
New
Image Management
Import From File
Import From Device
Full Size
Delete
Arrange photos by dragging them in the photo layout.
Photos
Facial
Frontal
Profile
Smile
Intraotal
Upper Occlusal
Full Mouth
Lower Occlusal
Right Buccal
Frontal
Left Buccal
Rotate 90 degrees
Rotate 1 degree
Current Rotation:
0
Mirror Image
Brightness
0
Contrast
0
Crop
Undo
500
502
504

FIG. 67

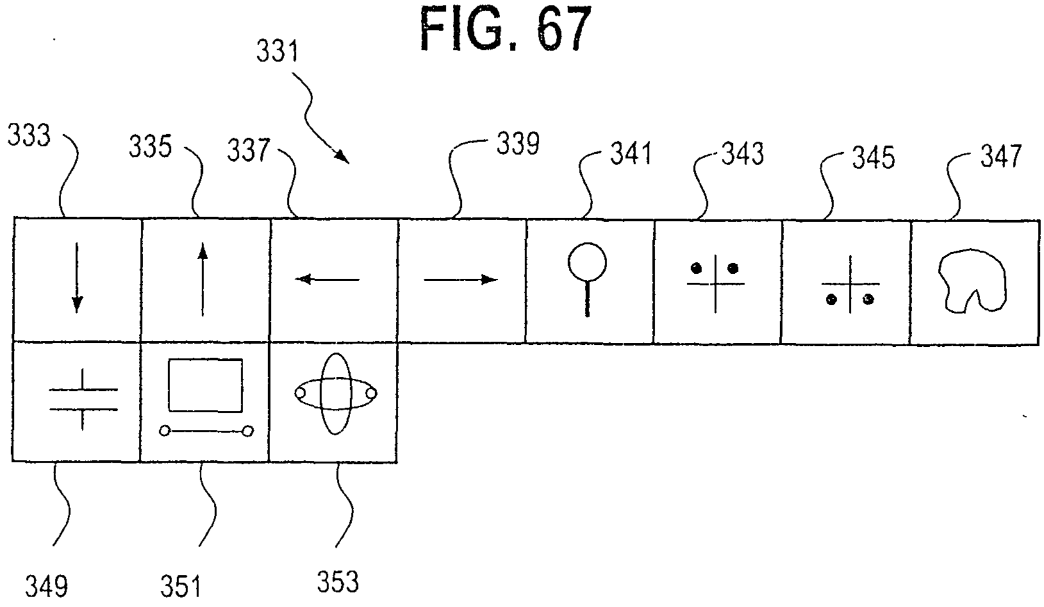
331
333
335
337
339
341
343
345
347
349
351
353

FIG. 68

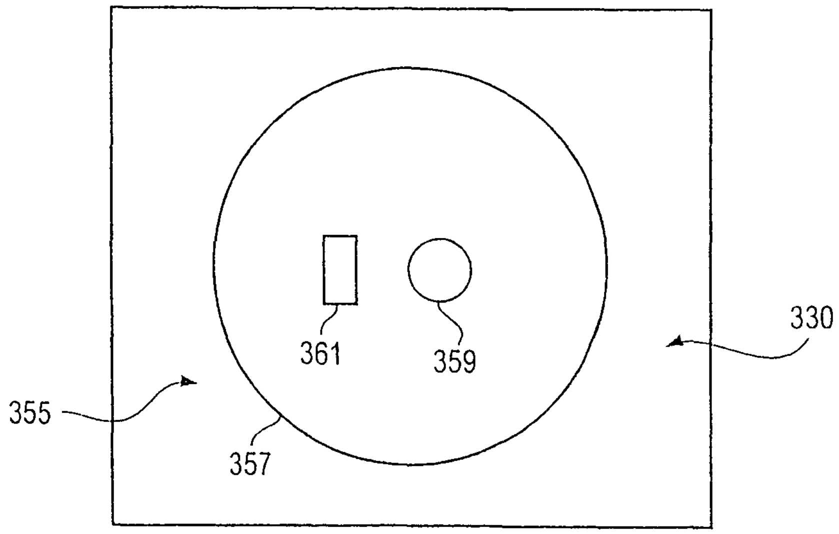
361
359
330
355
357

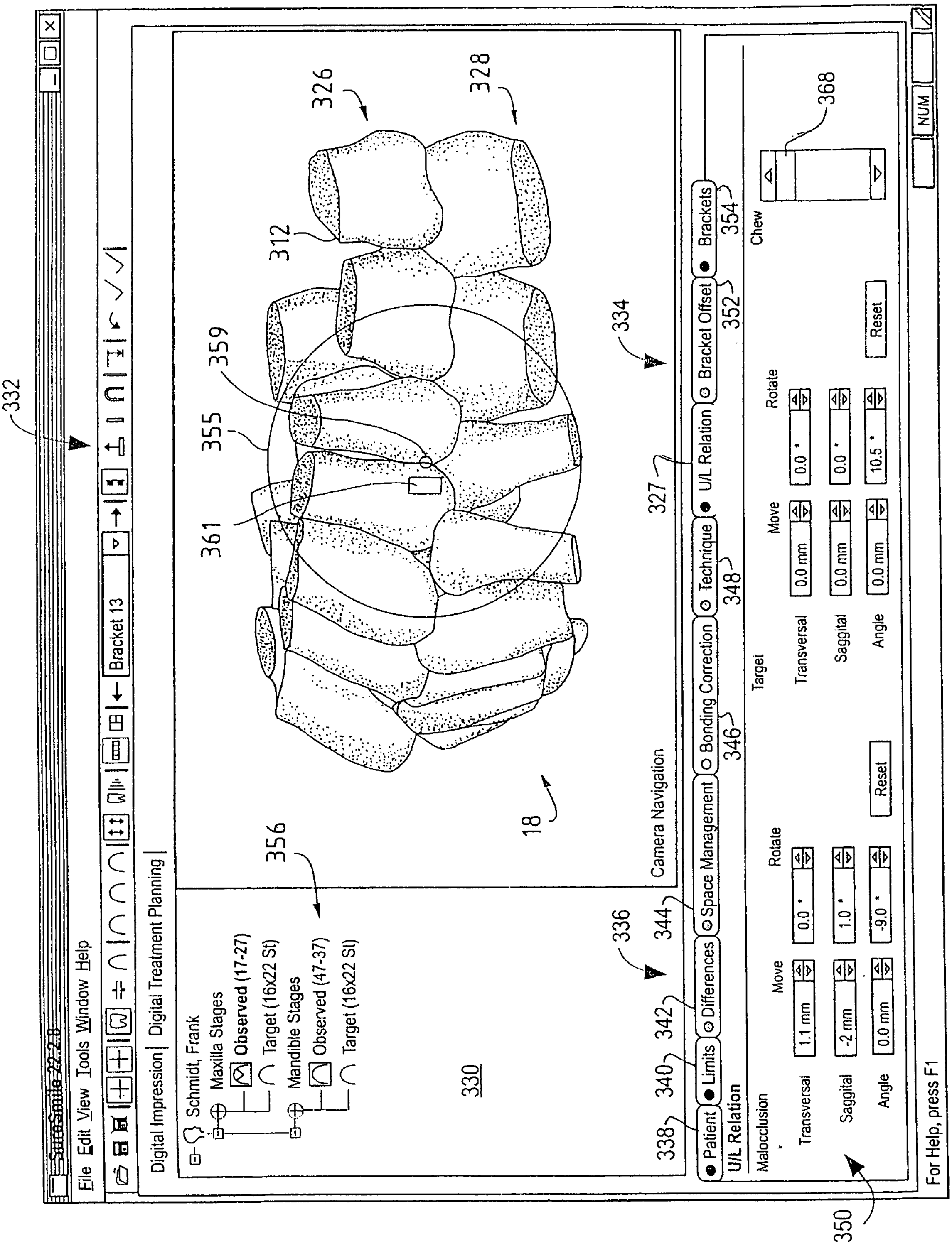
File Edit View Tools Window Help
Bracket 13
Digital Impression
Digital Treatment Planning
Schmidt, Frank
Maxilla Stages
Observed (17-27)
Target (16x22 St)
Mandible Stages
Observed (47-37)
Target (16x22 St)
330
Camera Navigation
Patient
Limits
Differences
Space Management
Bonding Correction
Technique
U/L Relation
Bracket Offset
Brackets
U/L Relation
Malocclusion
Move
Rotate
Transversal
1.1 mm
0.0 °
Saggital
-2 mm
1.0 °
Angle
0.0 mm
-9.0 °
Reset
Target
Move
Rotate
Transversal
0.0 mm
0.0 °
Saggital
0.0 mm
0.0 °
Angle
0.0 mm
10.5 °
Reset
Chew
NUM
For Help, press F1
332
326
328
312
359
355
361
356
18
334
336
338
340
342
344
346
348
327
352
354
368
350

FIG. 69

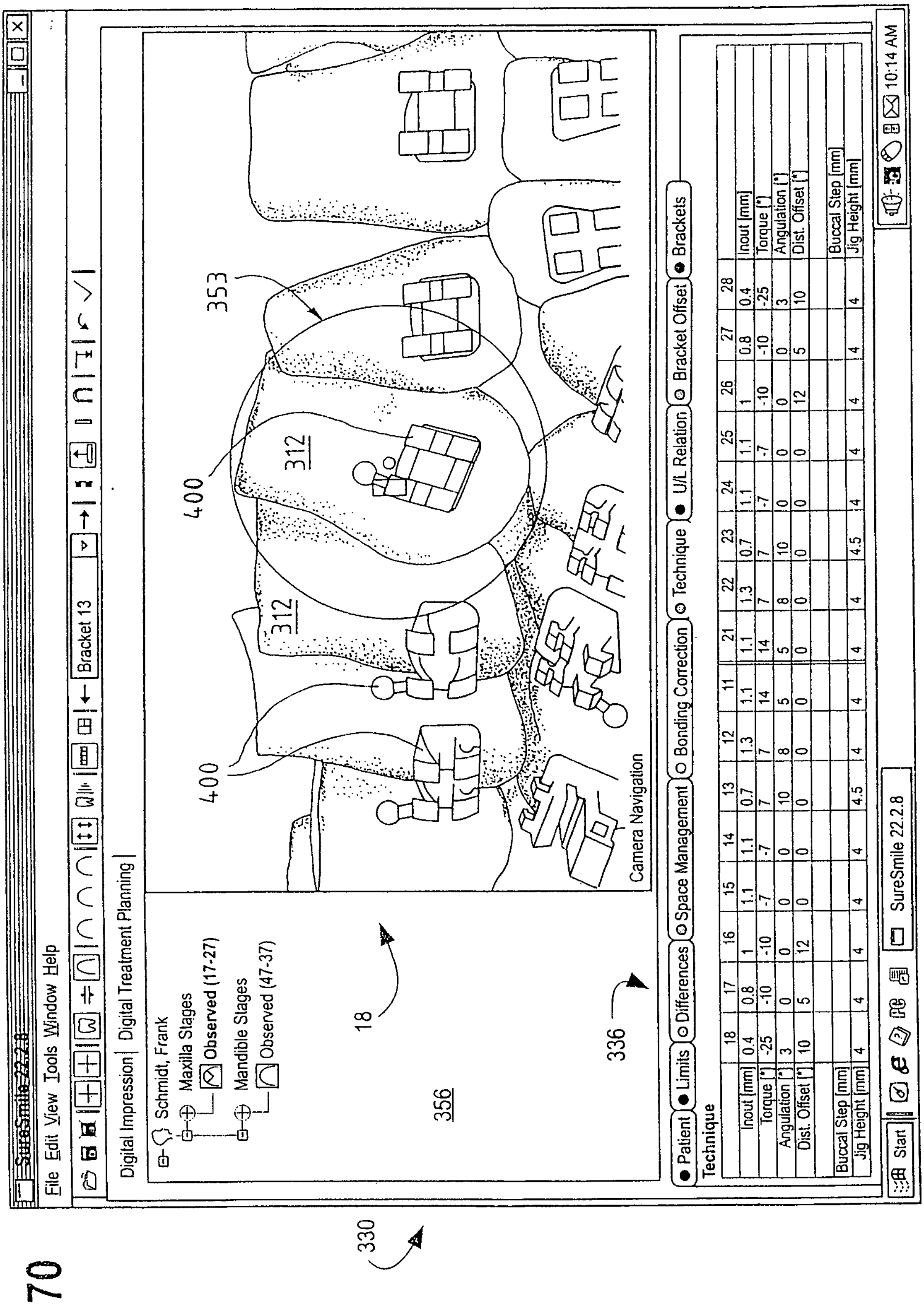
SureSmile 22.2.8
File Edit View Tools Window Help
Bracket 13
Digital Impression
Digital Treatment Planning
Schmidt, Frank
Maxilla Stages
Observed (17-27)
Mandible Stages
Observed (47-37)
Camera Navigation
Patient
Limits
Differences
Space Management
Bonding Correction
Technique
U/L Relation
Bracket Offset
Brackets
Technique
18 17 16 15 14 13 12 11 21 22 23 24 25 26 27 28
Inout [mm] 0.4 0.8 1 1.1 1.1 0.7 1.3 1.1 1.1 1.3 0.7 1.1 1.1 1 0.8 0.4
Torque [°] -25 -10 -10 -7 -7 7 7 14 14 7 7 -7 -7 -10 -10 -25
Angulation [°] 3 0 0 0 0 10 8 5 5 8 10 0 0 0 0 3
Dist. Offset [°] 10 5 12 0 0 0 0 0 0 0 0 0 0 12 5 10
Buccal Step [mm]
Jig Height [mm] 4 4 4 4 4 4.5 4 4 4 4 4.5 4 4 4 4 4
Start
SureSmile 22.2.8
10:14 AM
330
18
336
356
400
400
312
312
353

FIG. 70

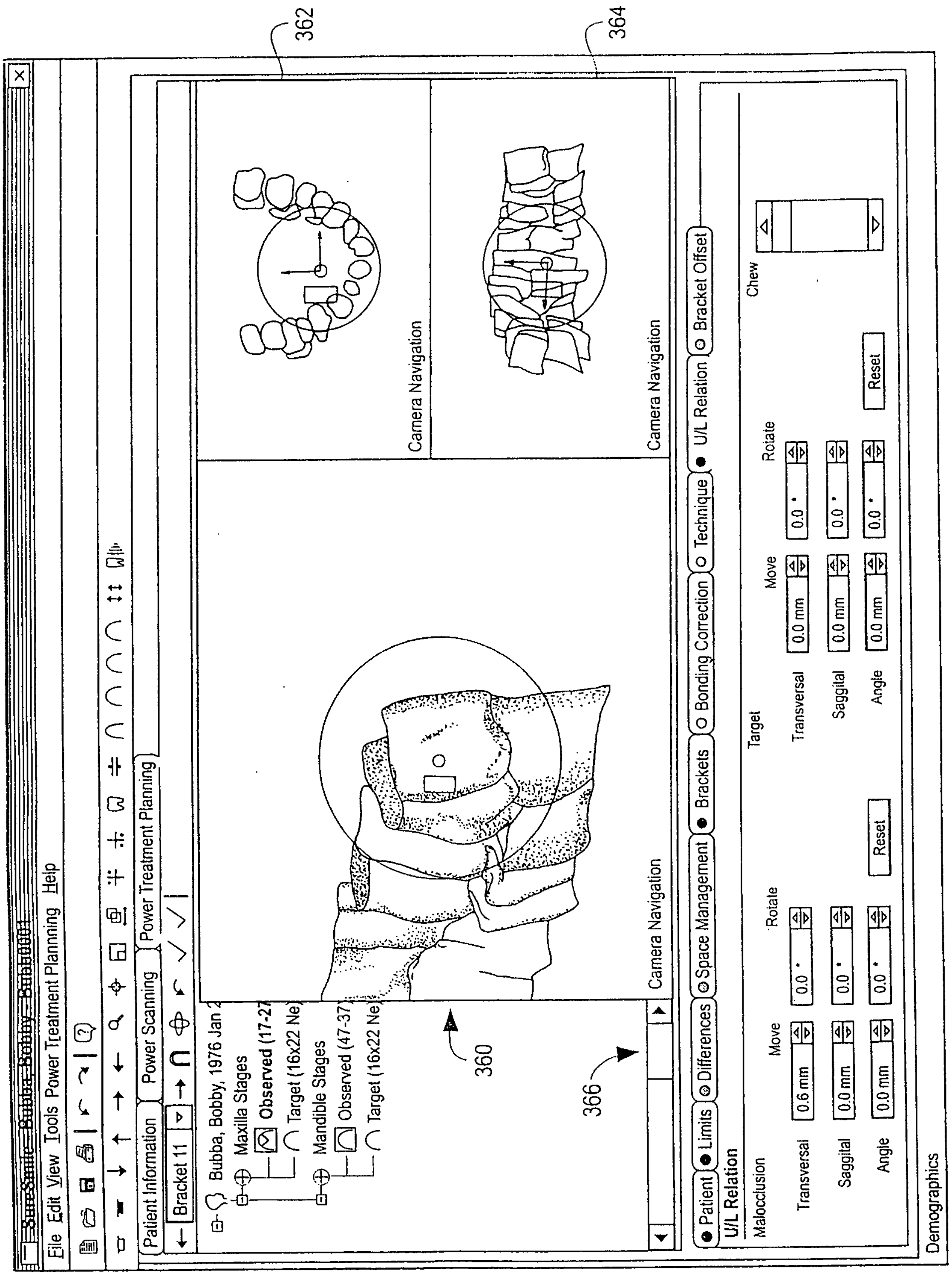
362
364
SureSmile - Bubba, Bobby - Bubb0001
File Edit View Tools Power Treatment Plannning Help
Patient Information
Power Scanning
Power Treatment Planning
Bracket 11
Bubba, Bobby, 1976 Jan 2
Maxilla Stages
Observed (17-27
Target (16x22 Ne
Mandible Stages
Observed (47-37
Target (16x22 Ne
Camera Navigation
Camera Navigation
Camera Navigation
360
366
Patient
Limits
Differences
Space Management
Brackets
Bonding Correction
Technique
U/L Relation
Bracket Offset
U/L Relation
Malocclusion
Move
Rotate
Transversal
0.6 mm
0.0 °
Saggital
0.0 mm
0.0 °
Angle
0.0 mm
0.0 °
Reset
Target
Move
Rotate
Transversal
0.0 mm
0.0 °
Saggital
0.0 mm
0.0 °
Angle
0.0 mm
0.0 °
Reset
Chew
Demographics

FIG. 71

FIG. 72

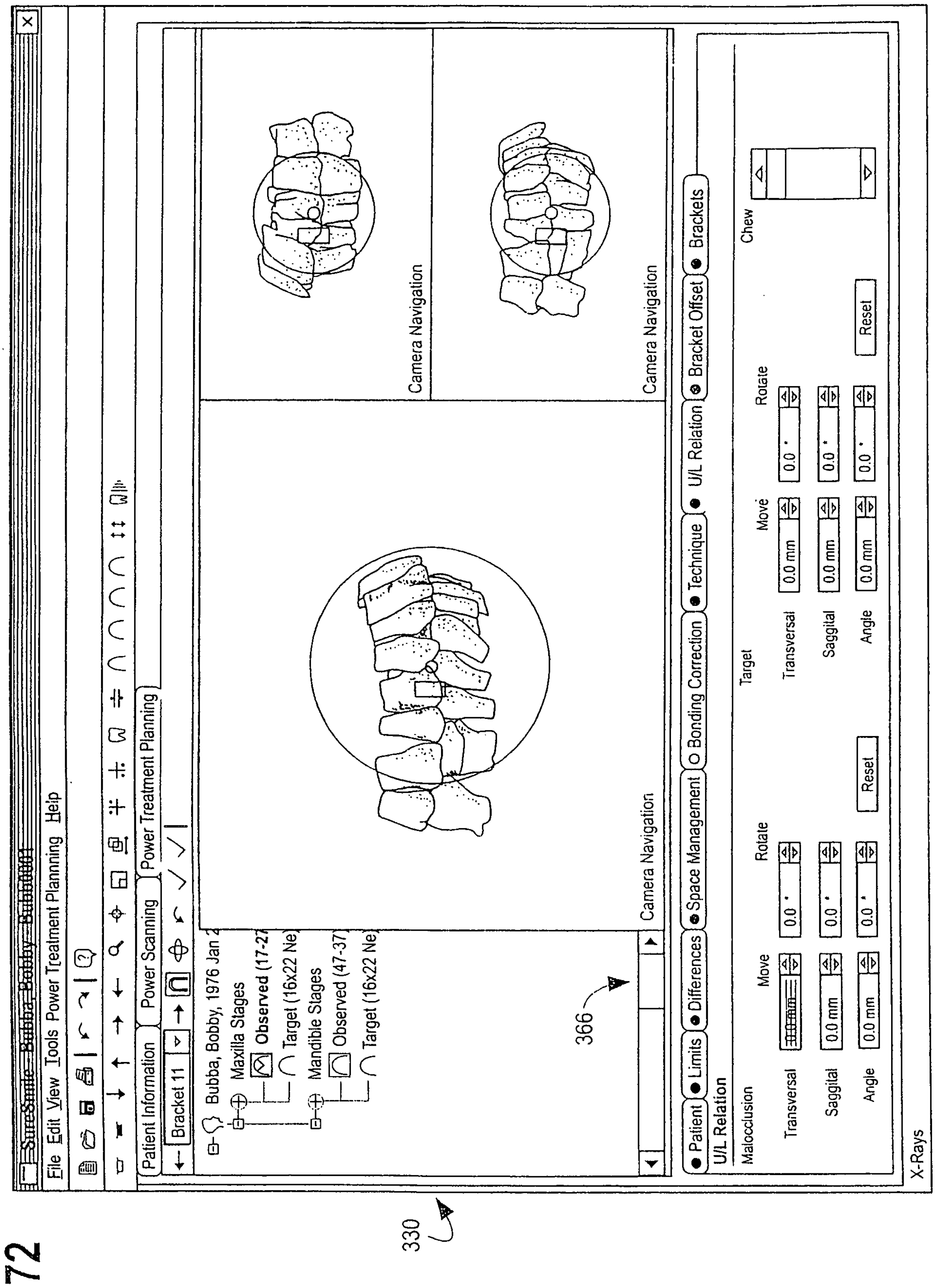
SureSmile - Bubba, Bobby - Bubb0001
File Edit View Tools Power Treatment Planning Help
Patient Information
Power Scanning
Power Treatment Planning
Bracket 11
Bubba, Bobby, 1976 Jan 2
Maxilla Stages
Observed (17-27
Target (16x22 Ne
Mandible Stages
Observed (47-37
Target (16x22 Ne
Camera Navigation
Camera Navigation
Camera Navigation
366
Patient
Limits
Differences
Space Management
Bonding Correction
Technique
U/L Relation
Bracket Offset
Brackets
U/L Relation
Malocclusion
Move
Rotate
Transversal
Saggital
Angle
0.0 mm
0.0 °
Reset
Target
Chew
X-Rays
330

FIG. 73

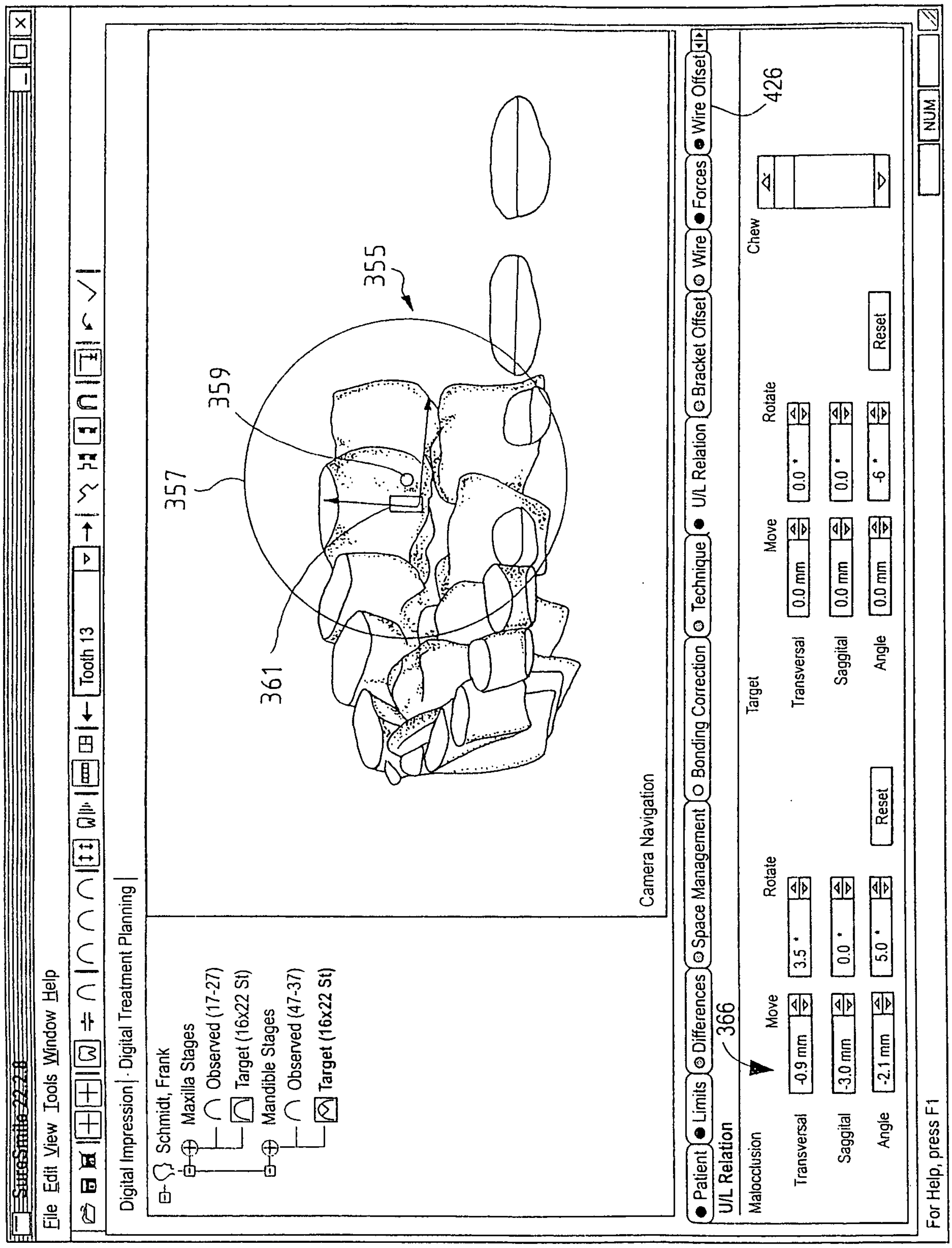
SureSmile 22.2.0
File Edit View Tools Window Help
Tooth 13
Digital Impression | Digital Treatment Planning
Schmidt, Frank
Maxilla Stages
Observed (17-27)
Target (16x22 St)
Mandible Stages
Observed (47-37)
Target (16x22 St)
Camera Navigation
355
357
359
361
Patient
Limits
Differences
Space Management
Bonding Correction
Technique
U/L Relation
Bracket Offset
Wire
Forces
Wire Offset
U/L Relation
366
426
Malocclusion
Move
Rotate
Transversal
Saggital
Angle
-0.9 mm
3.5 °
-3.0 mm
0.0 °
-2.1 mm
5.0 °
Reset
Target
0.0 mm
0.0 °
0.0 mm
0.0 °
0.0 mm
-6 °
Reset
Chew
For Help, press F1
NUM

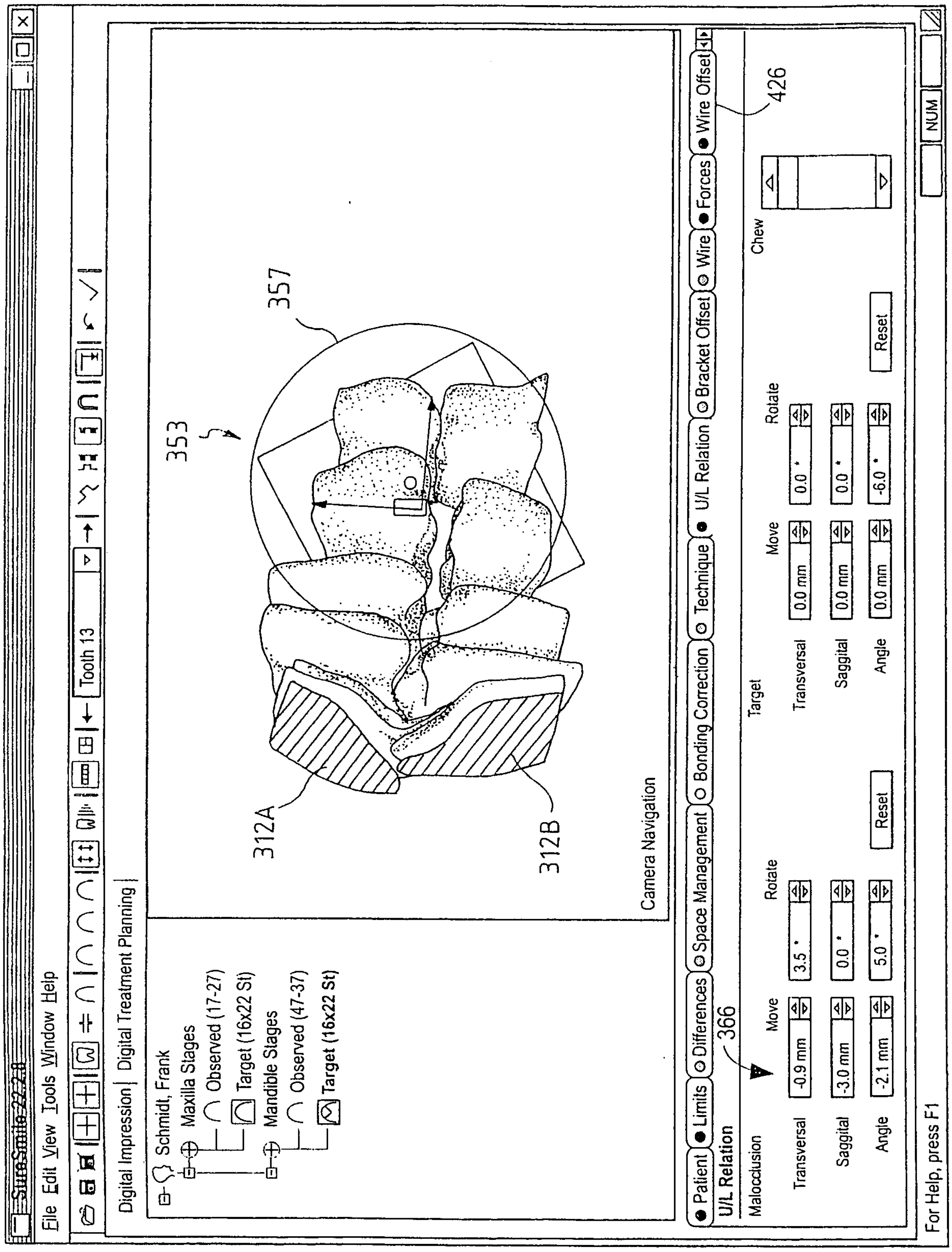
SureSmile 2.2.8
File Edit View Tools Window Help
Tooth 13
Digital Impression | Digital Treatment Planning
Schmidt, Frank
Maxilla Stages
Observed (17-27)
Target (16x22 St)
Mandible Stages
Observed (47-37)
Target (16x22 St)
353
357
312A
312B
Camera Navigation
Patient
Limits
Differences
Space Management
Bonding Correction
Technique
U/L Relation
Bracket Offset
Wire
Forces
Wire Offset
366
426
U/L Relation
Malocclusion
Target
Move
Rotate
Transversal
Saggital
Angle
-0.9 mm
3.5 °
-3.0 mm
0.0 °
-2.1 mm
5.0 °
0.0 mm
0.0 °
0.0 mm
0.0 °
0.0 mm
-6.0 °
Reset
Reset
Chew
NUM
For Help, press F1

FIG. 74

FIG. 75

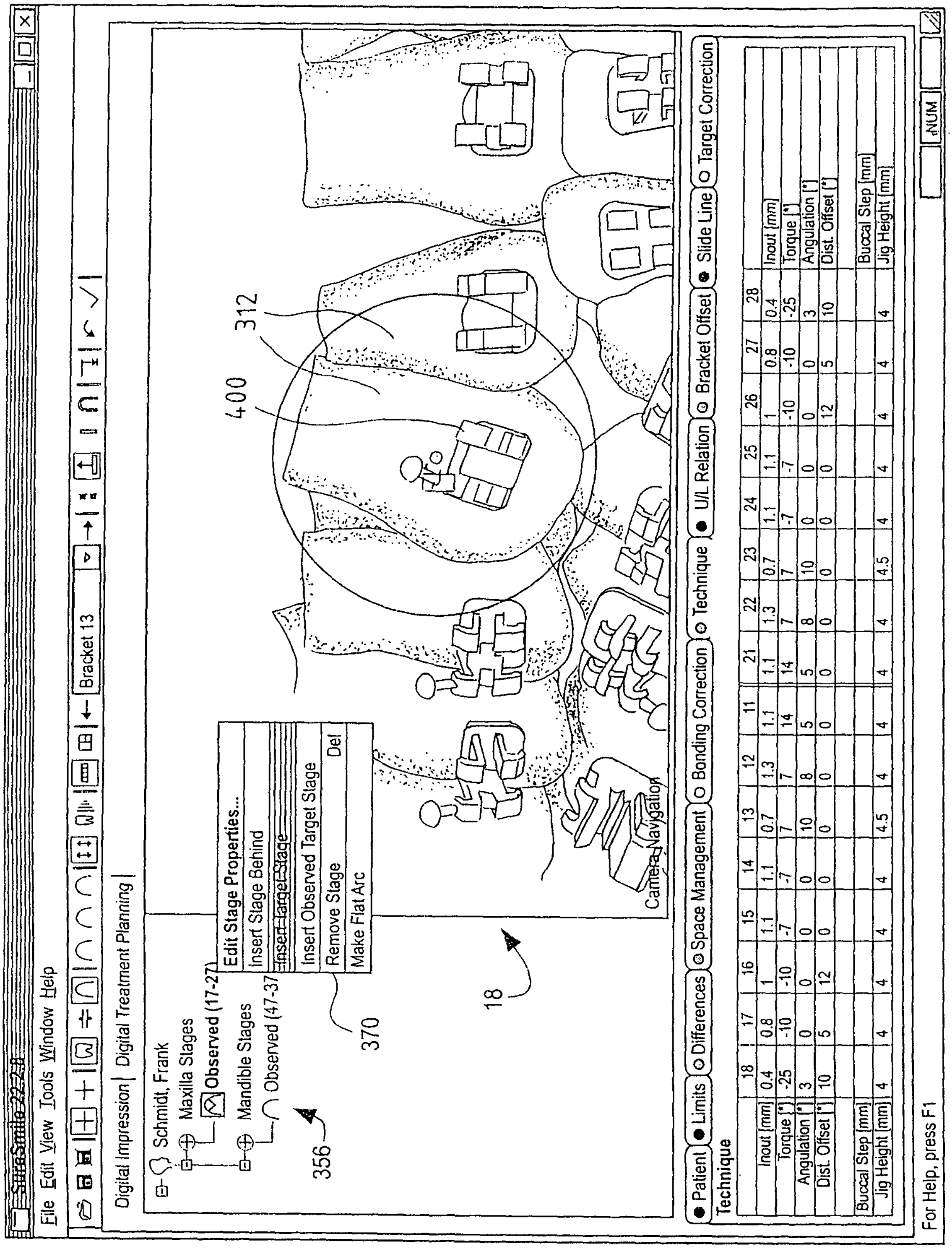
SureSmile 22.2.8
File Edit View Tools Window Help
Bracket 13
Digital Impression
Digital Treatment Planning
Schmidt, Frank
Maxilla Stages
Observed (17-27)
Mandible Stages
Observed (47-37
Edit Stage Properties...
Insert Stage Behind
Insert Target Stage
Insert Observed Target Stage
Remove Stage Del
Make Flat Arc
Camera Navigation
356
370
18
400
312
Patient
Limits
Differences
Space Management
Bonding Correction
Technique
U/L Relation
Bracket Offset
Slide Line
Target Correction
Technique
18 17 16 15 14 13 12 11 21 22 23 24 25 26 27 28
Inout [mm] 0.4 0.8 1 1.1 1.1 0.7 1.3 1.1 1.1 1.3 0.7 1.1 1.1 1 0.8 0.4
Torque [°] -25 -10 -10 -7 -7 17 7 14 14 7 7 -7 -7 -10 -10 -25
Angulation [°] 3 0 0 0 0 10 8 5 5 8 10 0 0 0 0 3
Dist. Offset [°] 10 5 12 0 0 0 0 0 0 0 0 0 0 12 5 10
Buccal Step [mm]
Jig Height [mm] 4 4 4 4 4 4.5 4 4 4 4 4.5 4 4 4 4 4
For Help, press F1
NUM

FIG. 76

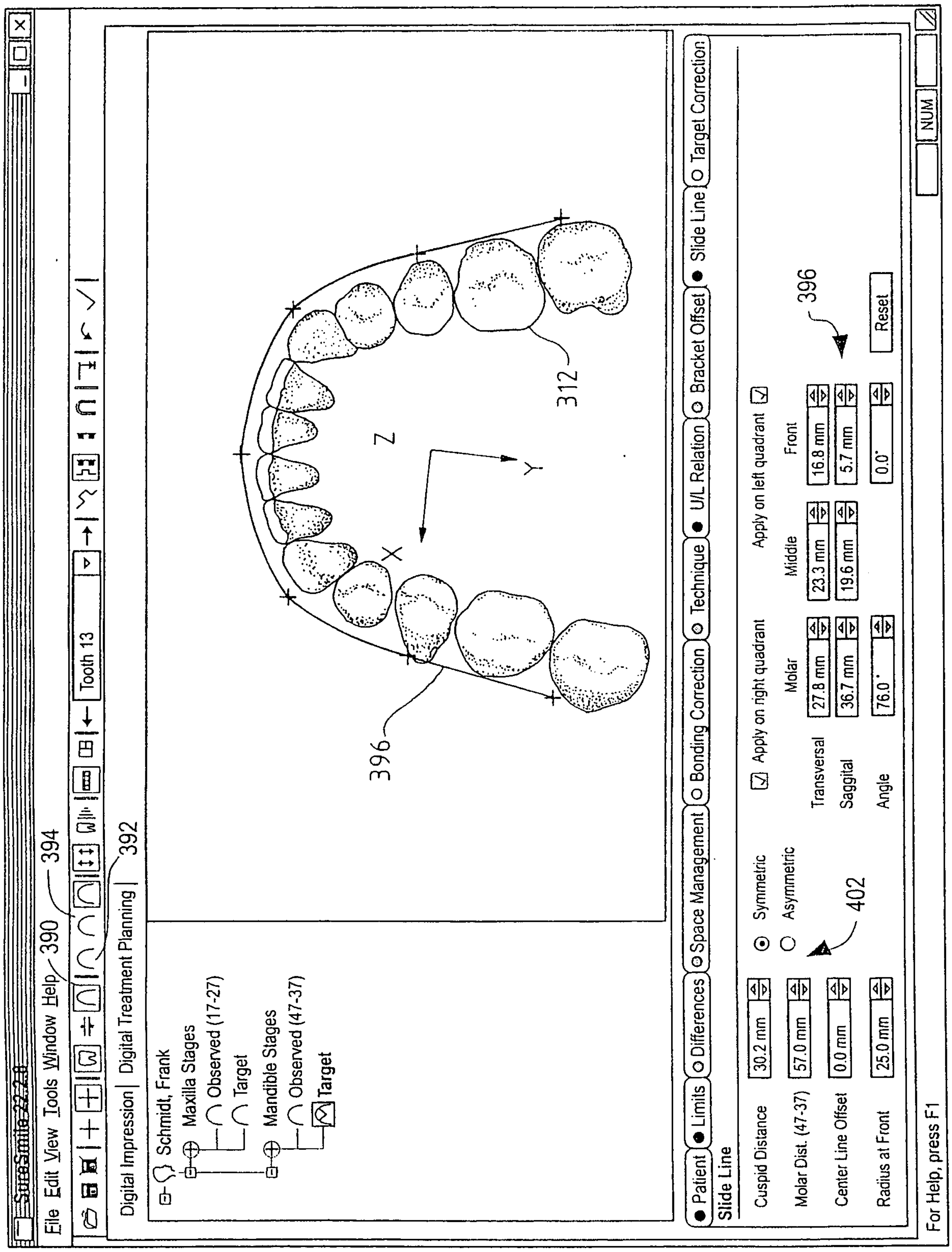
SureSmile 2.2.7.0
File Edit View Tools Window Help
390
394
392
Tooth 13
Digital Impression
Digital Treatment Planning
Schmidt, Frank
Maxilla Stages
Observed (17-27)
Target
Mandible Stages
Observed (47-37)
Target
396
312
Z
X
Y
Patient
Limits
Differences
Space Management
Bonding Correction
Technique
U/L Relation
Bracket Offset
Slide Line
Target Correction
Slide Line
Cuspid Distance
30.2 mm
Molar Dist. (47-37)
57.0 mm
Center Line Offset
0.0 mm
Radius at Front
25.0 mm
Symmetric
Asymmetric
402
Apply on right quadrant
Apply on left quadrant
Molar
Middle
Front
Transversal
27.8 mm
23.3 mm
16.8 mm
Saggital
36.7 mm
19.6 mm
5.7 mm
Angle
76.0°
0.0°
396
Reset
For Help, press F1
NUM

FIG. 77

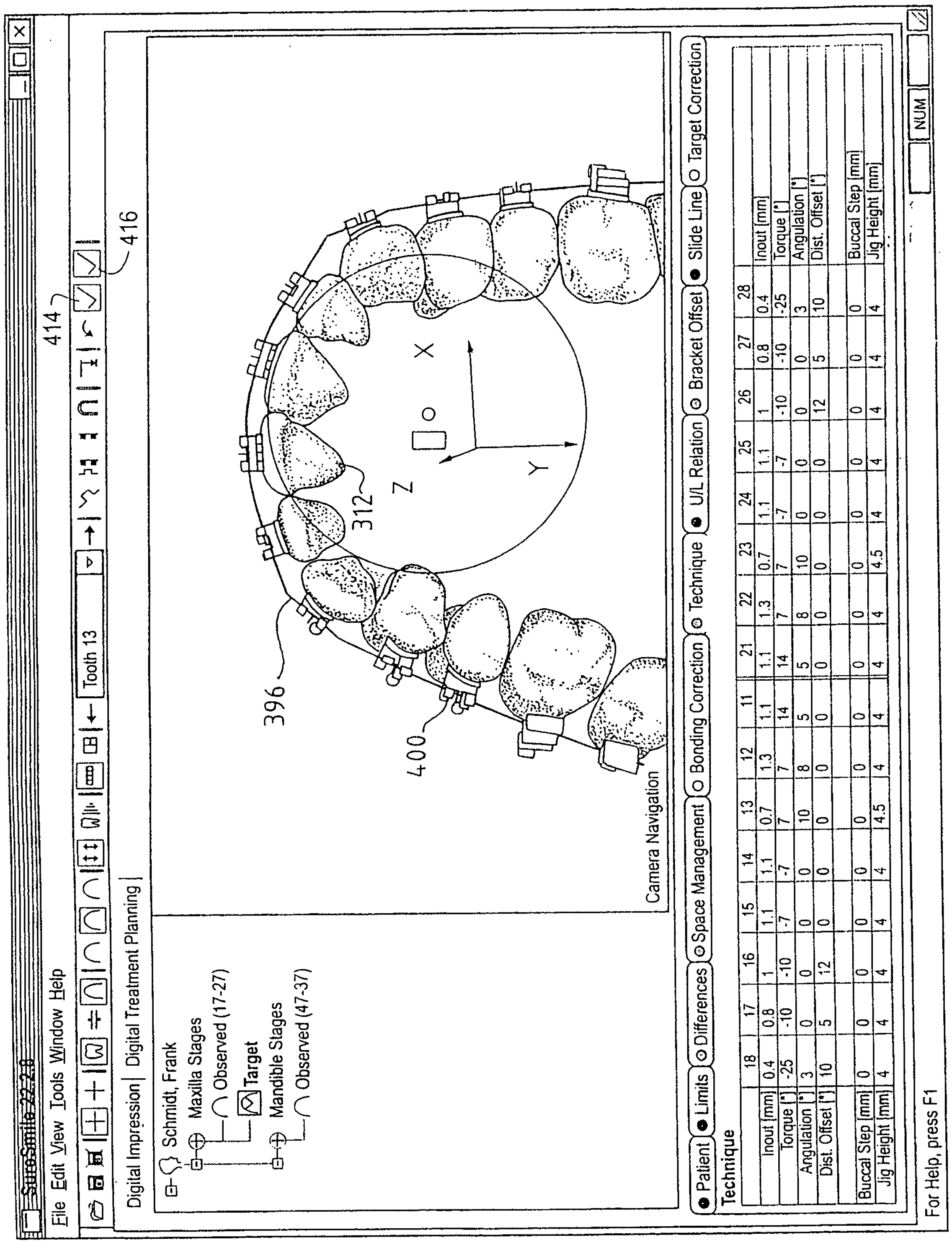
SureSmile 22.2.8
File Edit View Tools Window Help
Tooth 13
414
416
Digital Impression
Digital Treatment Planning
Schmidt, Frank
Maxilla Stages
Observed (17-27)
Target
Mandible Stages
Observed (47-37)
396
400
312
Z
X
Y
Camera Navigation
Patient
Limits
Differences
Space Management
Bonding Correction
Technique
U/L Relation
Bracket Offset
Slide Line
Target Correction
Technique
18 17 16 15 14 13 12 11 21 22 23 24 25 26 27 28
Inout [mm] 0.4 0.8 1 1.1 1.1 0.7 1.3 1.1 1.1 1.3 0.7 1.1 1.1 1 0.8 0.4
Torque [°] -25 -10 -10 -7 -7 7 7 14 14 7 7 -7 -7 -10 -10 -25
Angulation [°] 3 0 0 0 0 10 8 5 5 8 10 0 0 0 0 3
Dist. Offset [°] 10 5 12 0 0 0 0 0 0 0 0 0 0 12 5 10
Buccal Step [mm] 0 0 0 0 0 0 0 0 0 0 0 0 0 0 0 0
Jig Height [mm] 4 4 4 4 4 4.5 4 4 4 4 4.5 4 4 4 4 4
For Help, press F1
NUM

FIG. 78

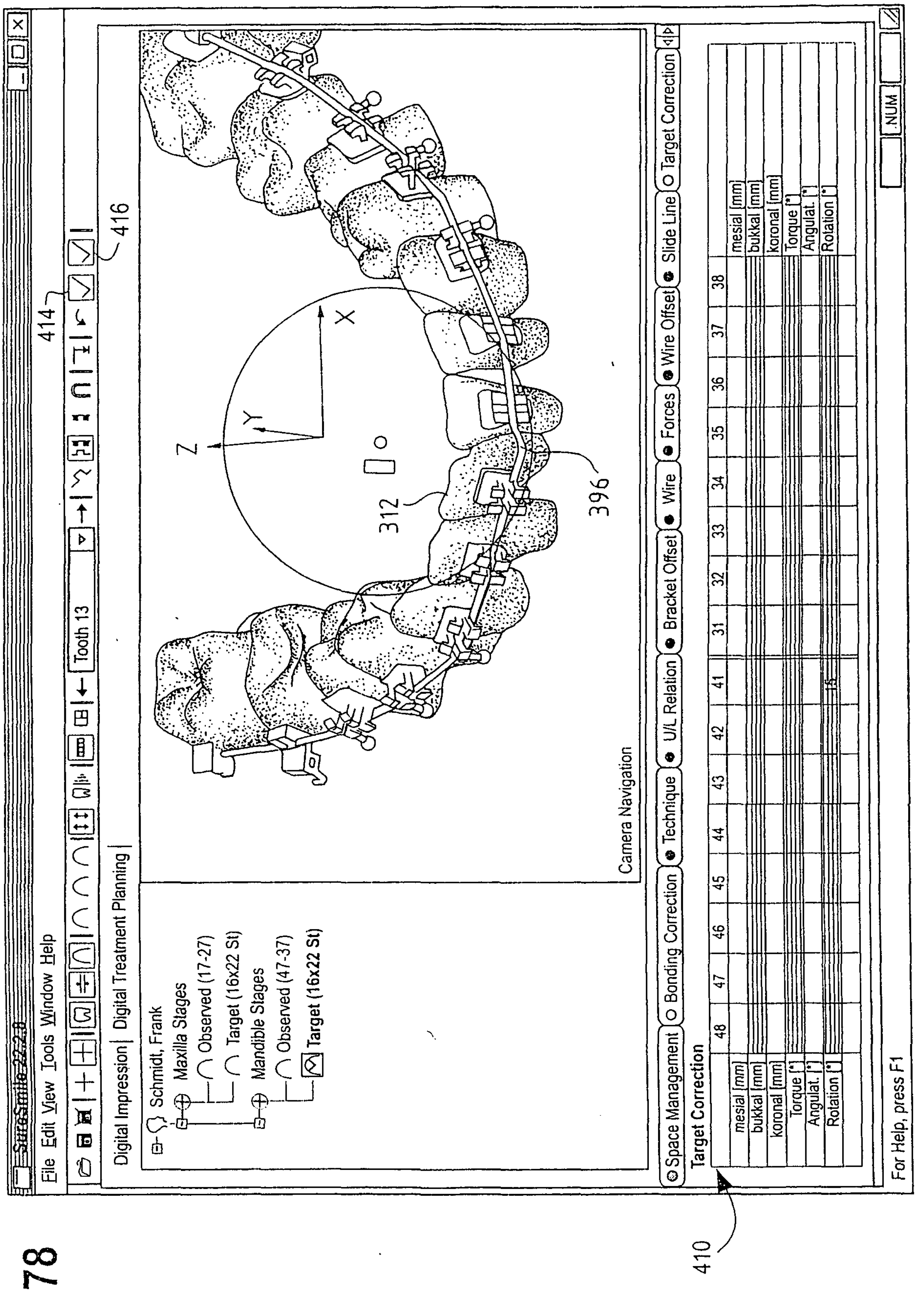

SureSmile 2.2.0
File Edit View Tools Window Help
Tooth 13
414
416
Digital Impression | Digital Treatment Planning |
Schmidt, Frank
Maxilla Stages
Observed (17-27)
Target (16x22 St)
Mandible Stages
Observed (47-37)
Target (16x22 St)
Z
Y
X
312
396
Camera Navigation
Space Management
Bonding Correction
Technique
U/L Relation
Bracket Offset
Wire
Forces
Wire Offset
Slide Line
Target Correction
Target Correction
48
47
46
45
44
43
42
41
31
32
33
34
35
36
37
38
mesial [mm]
bukkal [mm]
koronal [mm]
Torque [°]
Angulat. [°]
Rotation [°]
For Help, press F1
NUM
410

FIG. 79

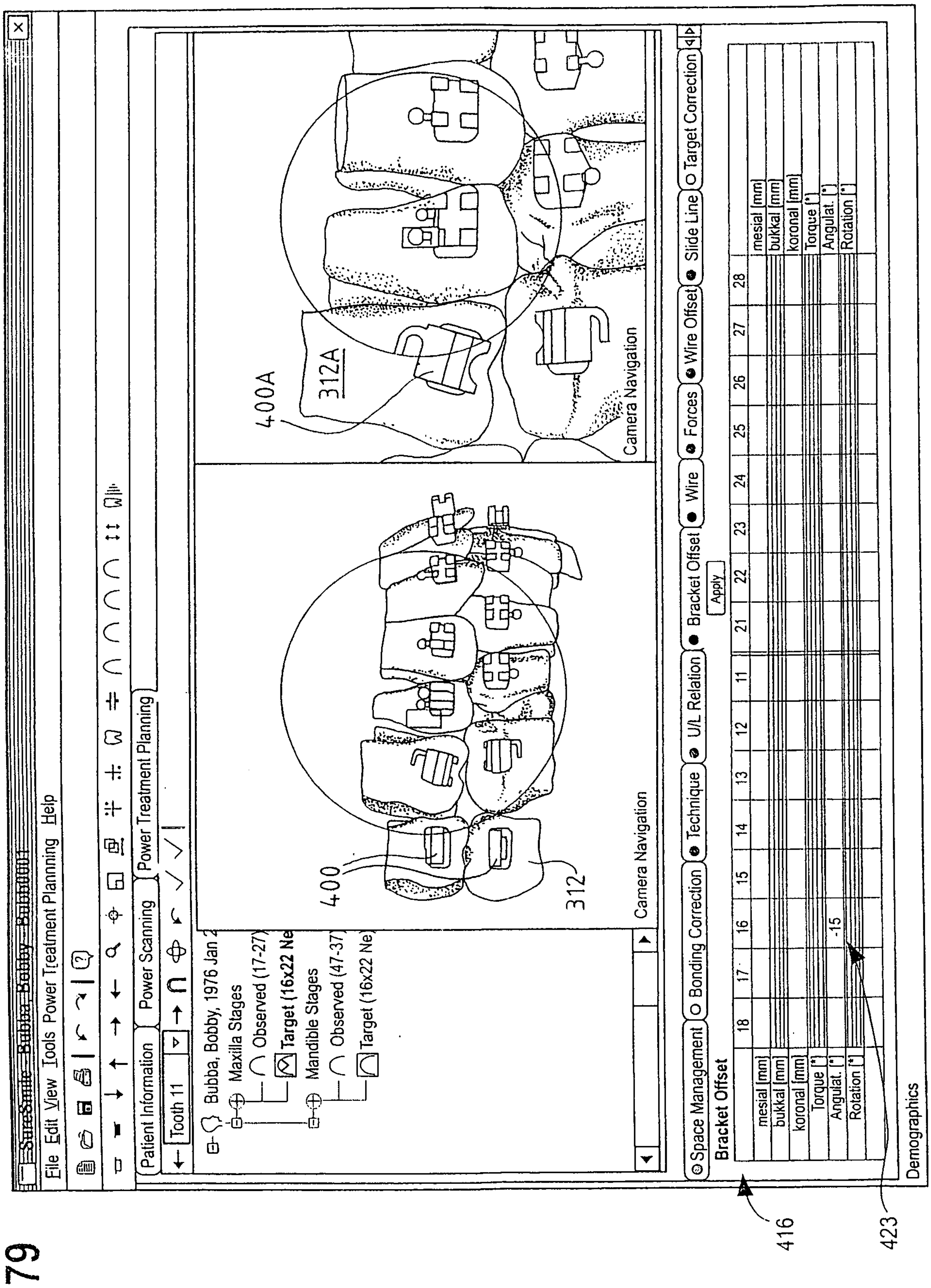
SureSmile - Bubba, Bobby - Bubb0001
File Edit View Tools Power Treatment Plannning Help
Patient Information
Power Scanning
Power Treatment Planning
Tooth 11
Bubba, Bobby, 1976 Jan 2
Maxilla Stages
Observed (17-27)
Target (16x22 Ne
Mandible Stages
Observed (47-37)
Target (16x22 Ne
Camera Navigation
400
312
400A
312A
Camera Navigation
Space Management
Bonding Correction
Technique
U/L Relation
Bracket Offset
Wire
Forces
Wire Offset
Slide Line
Target Correction
Apply
Bracket Offset
18
17
16
15
14
13
12
11
21
22
23
24
25
26
27
28
mesial [mm]
bukkal [mm]
koronal [mm]
Torque [°]
Angulat. [°]
Rotation [°]
-15
Demographics
416
423

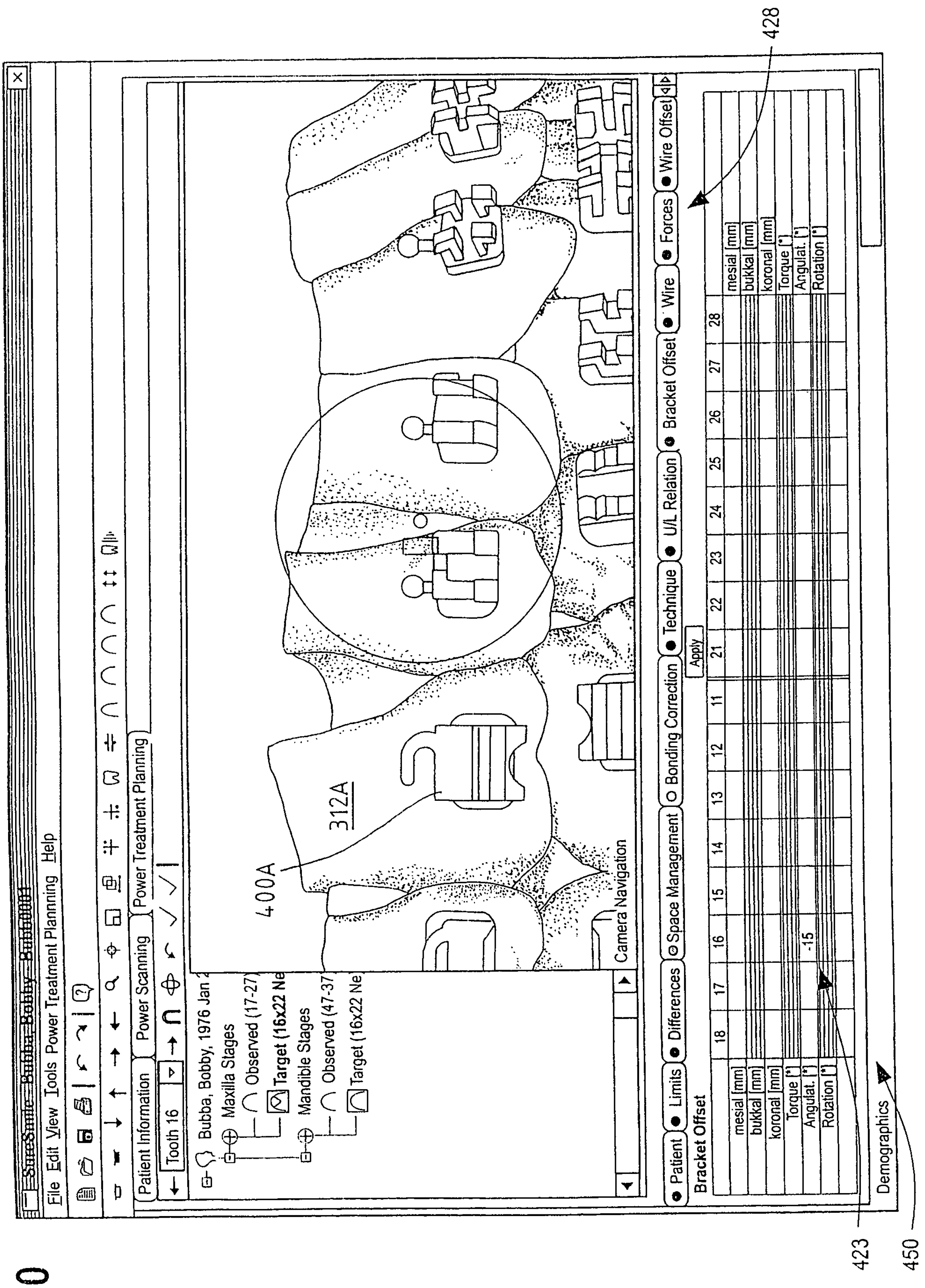
428
400A
312A
423
450
File Edit View Tools Power Treatment Planning Help
Patient Information
Power Scanning
Power Treatment Planning
Tooth 16
Maxilla Stages
Mandible Stages
Camera Navigation
Patient
Limits
Differences
Space Management
Bonding Correction
Technique
U/L Relation
Bracket Offset
Wire
Forces
Wire Offset
Apply
Bracket Offset
mesial [mm]
bukkal [mm]
koronal [mm]
Torque [°]
Angulat. [°]
Rotation [°]
-15
Demographics

FIG. 80

FIG. 81

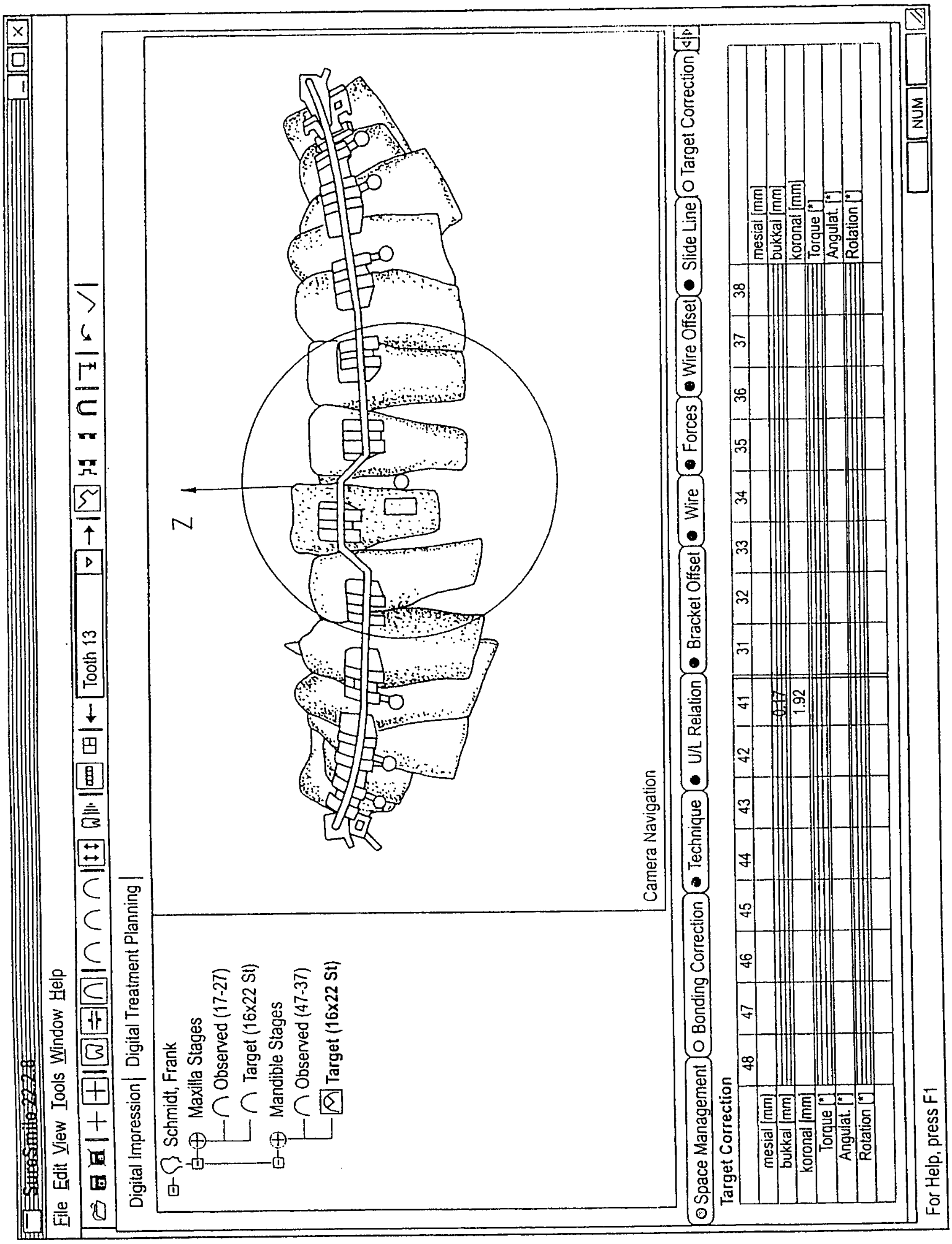
SureSmile 22.2.8
File Edit View Tools Window Help
Tooth 13
Digital Impression
Digital Treatment Planning
Schmidt, Frank
Maxilla Stages
Observed (17-27)
Target (16x22 St)
Mandible Stages
Observed (47-37)
Target (16x22 St)
Z
Camera Navigation
Space Management
Bonding Correction
Technique
U/L Relation
Bracket Offset
Wire
Forces
Wire Offset
Slide Line
Target Correction
48
47
46
45
44
43
42
41
31
32
33
34
35
36
37
38
mesial [mm]
bukkal [mm]
koronal [mm]
Torque [°]
Angulat. [°]
Rotation [°]
0.17
1.92
For Help, press F1
NUM

FIG. 82

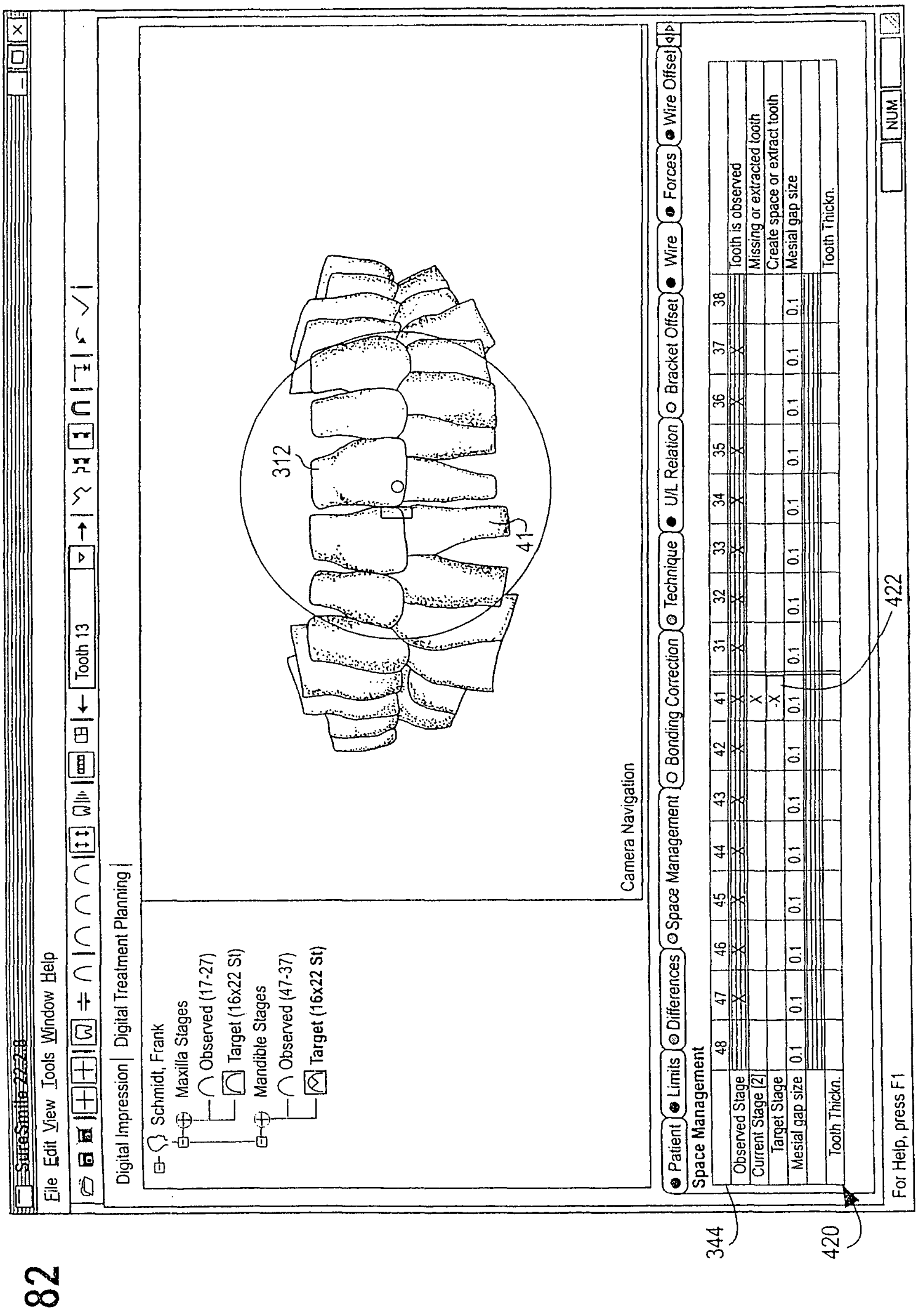
SureSmile 22-2-8
File Edit View Tools Window Help
Tooth 13
Digital Impression
Digital Treatment Planning
Schmidt, Frank
Maxilla Stages
Observed (17-27)
Target (16x22 St)
Mandible Stages
Observed (47-37)
Target (16x22 St)
Camera Navigation
312
41
Patient
Limits
Differences
Space Management
Bonding Correction
Technique
U/L Relation
Bracket Offset
Wire
Forces
Wire Offset
Observed Stage
Current Stage [2]
Target Stage
Mesial gap size
Tooth Thickn.
Tooth is observed
Missing or extracted tooth
Create space or extract tooth
Mesial gap size
For Help, press F1
NUM
344
420
422

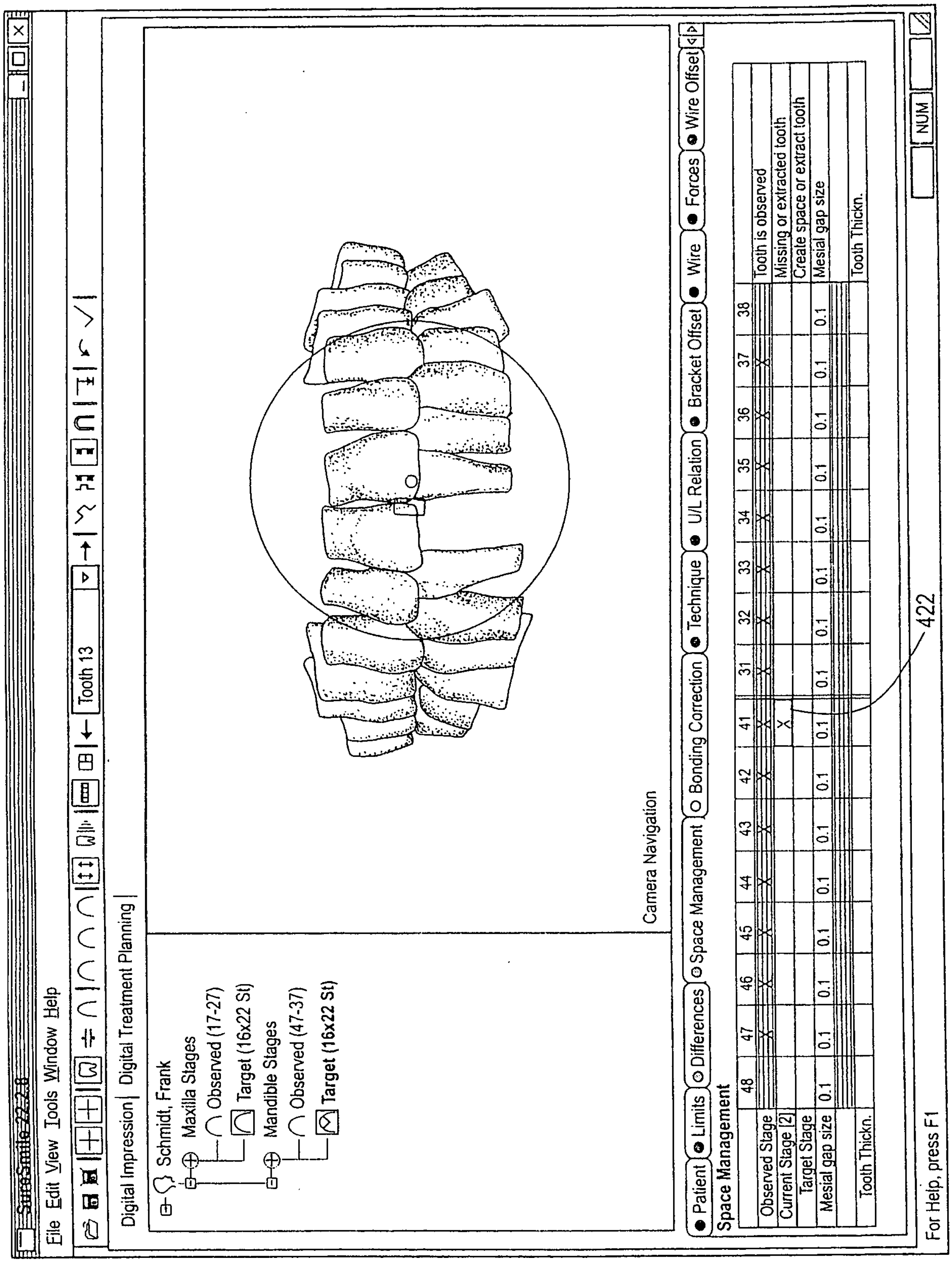
SureSmile 2.2.2.8
File Edit View Tools Window Help
Tooth 13
Digital Impression
Digital Treatment Planning
Schmidt, Frank
Maxilla Stages
Observed (17-27)
Target (16x22 St)
Mandible Stages
Observed (47-37)
Target (16x22 St)
Camera Navigation
Patient
Limits
Differences
Space Management
Bonding Correction
Technique
U/L Relation
Bracket Offset
Wire
Forces
Wire Offset
Space Management
Observed Stage
Current Stage [2]
Target Stage
Mesial gap size
Tooth Thickn.
48 47 46 45 44 43 42 41 31 32 33 34 35 36 37 38
Tooth is observed
Missing or extracted tooth
Create space or extract tooth
Mesial gap size
Tooth Thickn.
422
For Help, press F1
NUM

FIG. 83

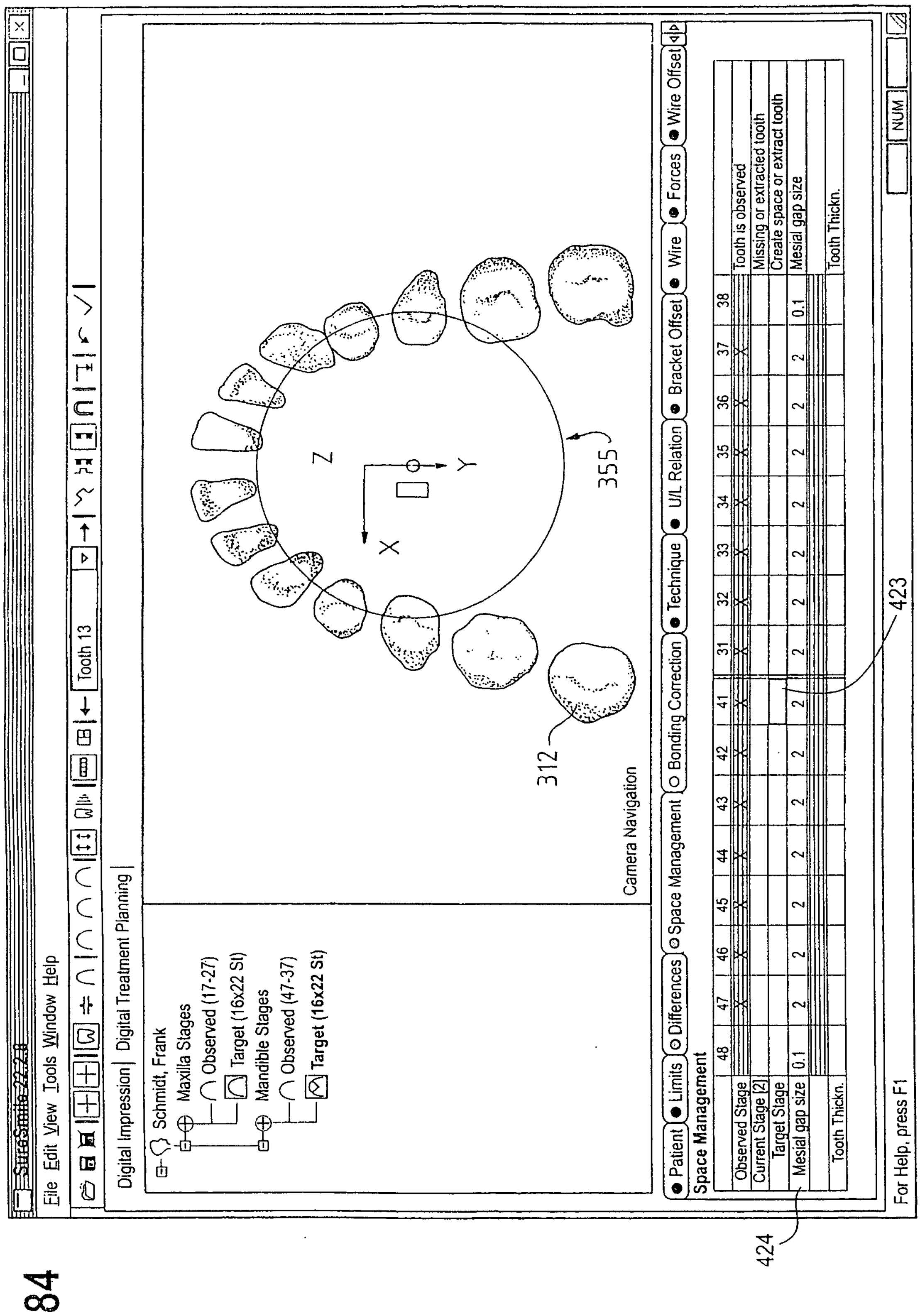
SureSmile 2.2.0
File Edit View Tools Window Help
Tooth 13
Digital Impression
Digital Treatment Planning
Schmidt, Frank
Maxilla Stages
Observed (17-27)
Target (16x22 St)
Mandible Stages
Observed (47-37)
Target (16x22 St)
Camera Navigation
Z
Y
X
355
312
Patient
Limits
Differences
Space Management
Bonding Correction
Technique
U/L Relation
Bracket Offset
Wire
Forces
Wire Offset
Space Management
48 47 46 45 44 43 42 41 31 32 33 34 35 36 37 38
Observed Stage
Current Stage [2]
Target Stage
Mesial gap size
Tooth Thickn.
Tooth is observed
Missing or extracted tooth
Create space or extract tooth
Mesial gap size
423
424
For Help, press F1
NUM

FIG. 84

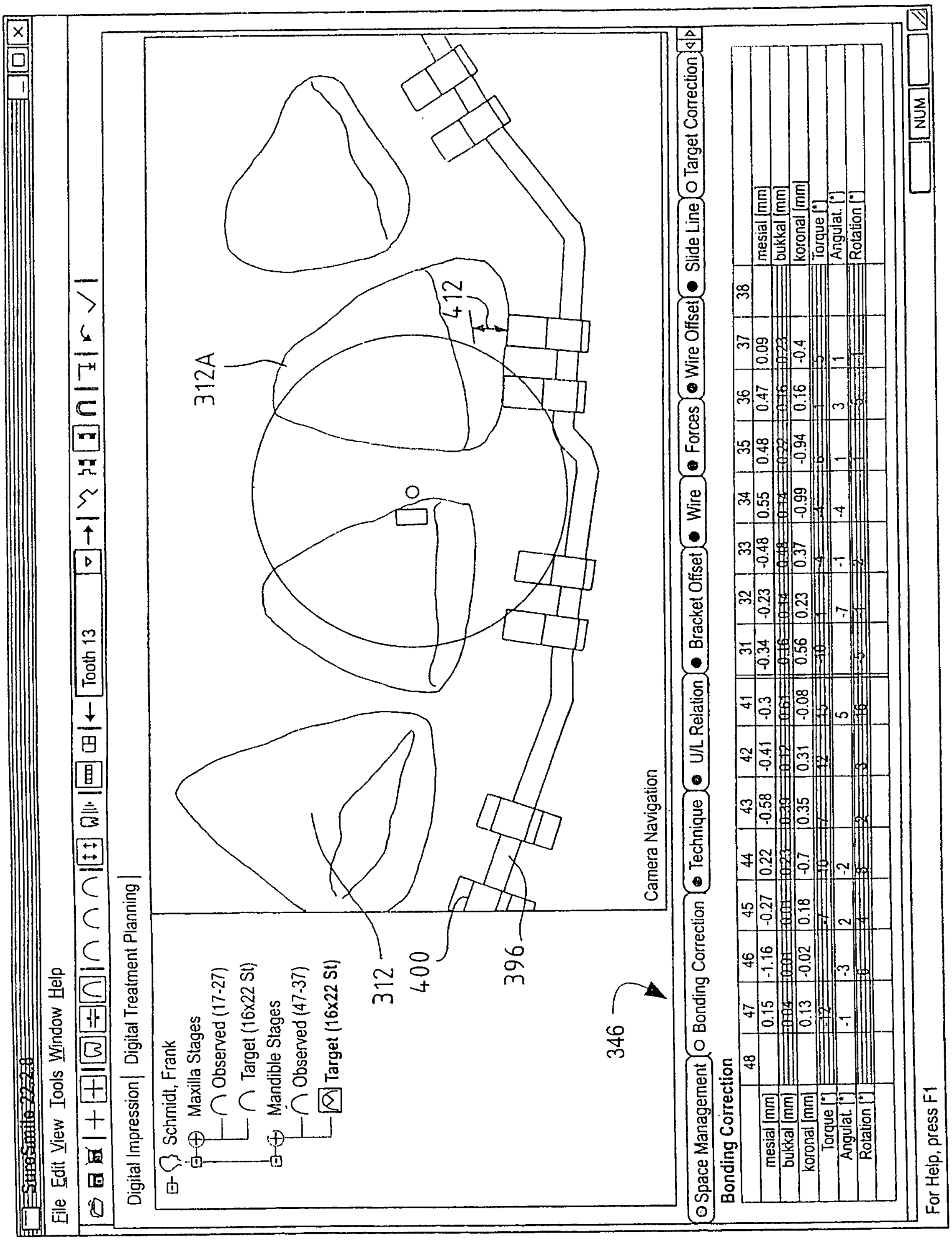
SureSmile 22.2.8
File Edit View Tools Window Help
Tooth 13
Digital Impression
Digital Treatment Planning
Schmidt, Frank
Maxilla Stages
Observed (17-27)
Target (16x22 St)
Mandible Stages
Observed (47-37)
Target (16x22 St)
Camera Navigation
312A
412
312
400
396
346
Space Management
Bonding Correction
Technique
U/L Relation
Bracket Offset
Wire
Forces
Wire Offset
Slide Line
Target Correction
Bonding Correction
mesial [mm]
bukkal [mm]
koronal [mm]
Torque [°]
Angulat. [°]
Rotation [°]
For Help, press F1
NUM

FIG. 85

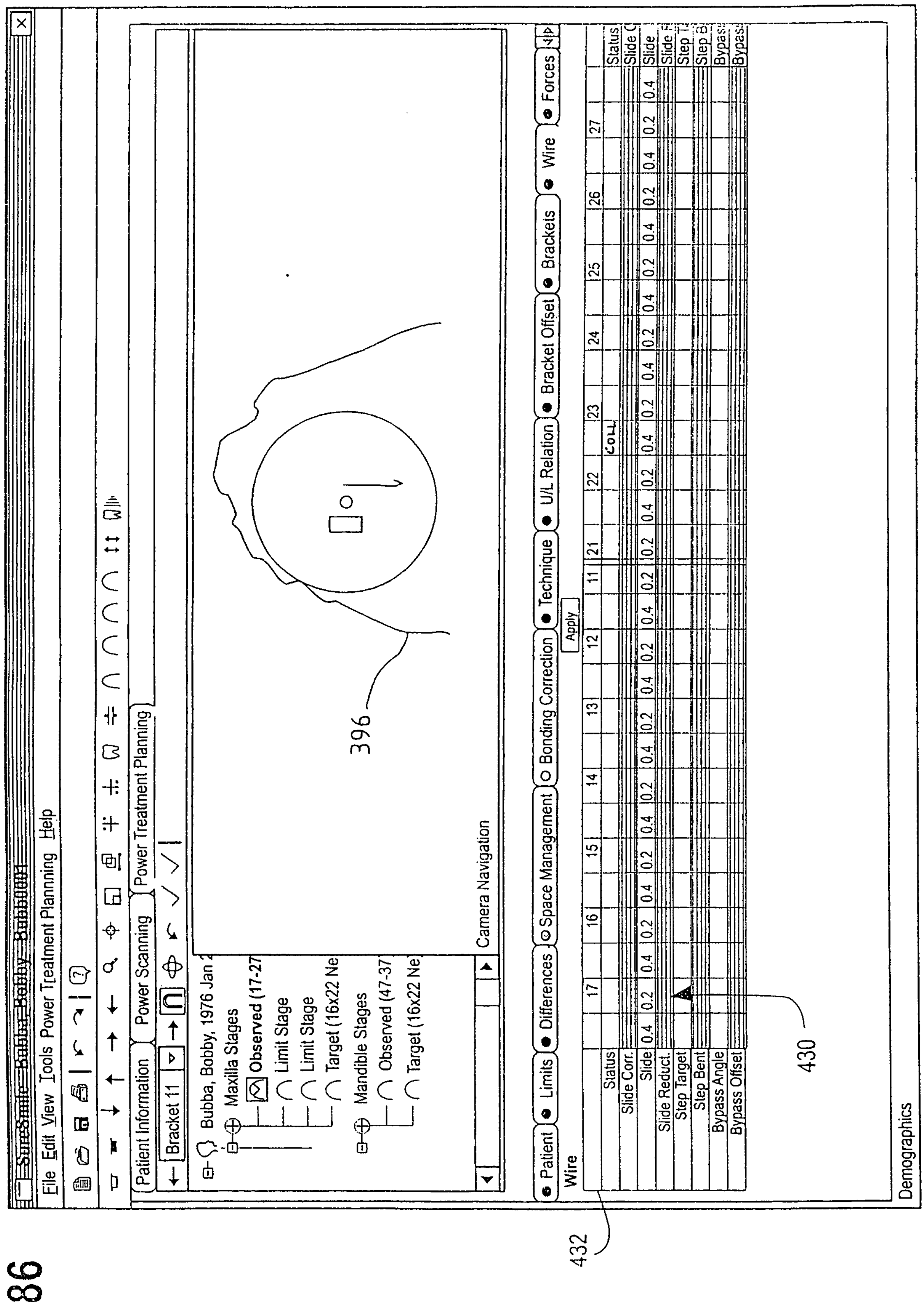
SureSmile - Bubba, Bobby - Bubb0001
File Edit View Tools Power Treatment Planning Help
Patient Information
Power Scanning
Power Treatment Planning
Bracket 11
Bubba, Bobby, 1976 Jan 2
Maxilla Stages
Observed (17-27
Limit Stage
Limit Stage
Target (16x22 Ne
Mandible Stages
Observed (47-37
Target (16x22 Ne
Camera Navigation
396
Patient
Limits
Differences
Space Management
Bonding Correction
Technique
U/L Relation
Bracket Offset
Brackets
Wire
Forces
Apply
Wire
Status
Slide Corr.
Slide
Slide Reduct.
Step Target
Step Bent
Bypass Angle
Bypass Offset
17 16 15 14 13 12 11 21 22 23 24 25 26 27
COLL
430
432
Demographics

FIG. 86

FIG. 87

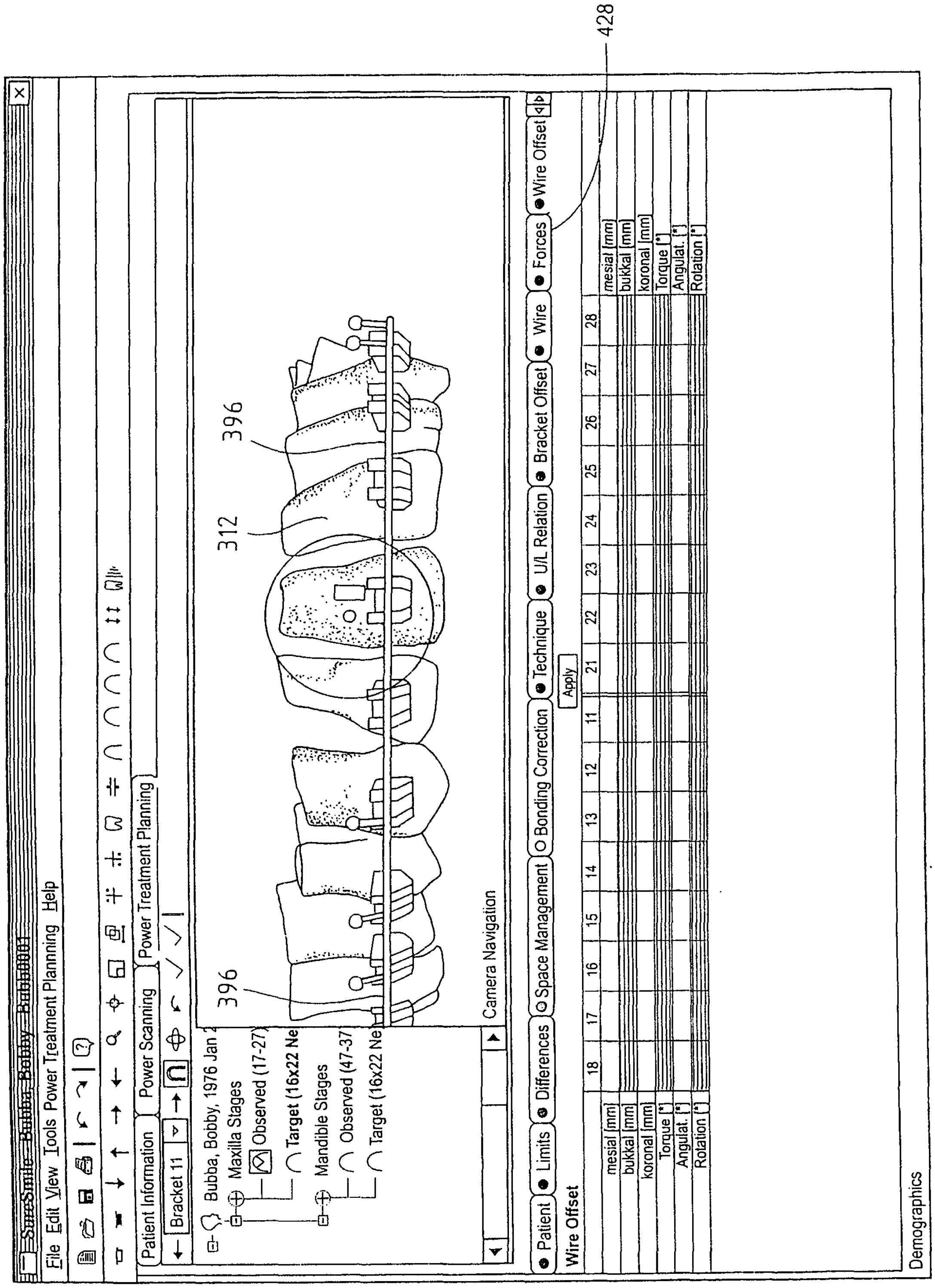

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5431562 A, Andreiko
- US 4837732 A
- US 4575805 A, Brandestini and Moermann
- US 5372502 A, Massen
- US 5027281 A, Rekow
- WO 0019935 A
- WO 0019929 A
- WO 09254755 A, Rüdger Rubbert
- WO 091560131 A
- US 09673863 B
- US 5155597 A, Lareau
- WO 09560131 A, Rubbert
- WO 09254843 A, Rüdger Rubbert
- DE 9701797 W, Rubbert
- US 6250918 B, Sachdeva
- US 09451609 B